(19) Europäisches Patentamt / European Patent Office / Office européen des brevets

(11) **EP 4 678 643 A1**

(12) **EUROPEAN PATENT APPLICATION**
published in accordance with Art. 153(4) EPC


(43) Date of publication:
**14.01.2026 Bulletin 2026/03**

(21) Application number: **24788218.6**

(22) Date of filing: **12.04.2024**

(51) International Patent Classification (IPC):
***C07D 471/04*** $^{(2006.01)}$ ***C07D 519/00*** $^{(2006.01)}$
***A61P 35/00*** $^{(2006.01)}$ ***A61P 35/02*** $^{(2006.01)}$
***A61K 31/519*** $^{(2006.01)}$

(52) Cooperative Patent Classification (CPC):
**A61K 31/519; A61K 31/55; A61K 31/553; A61P 35/00; A61P 35/02; C07D 471/04; C07D 519/00**

(86) International application number:
**PCT/CN2024/087520**

(87) International publication number:
**WO 2024/213122 (17.10.2024 Gazette 2024/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.04.2023 CN 202310400536**



(71) Applicant: **Sunshine Lake Pharma Co., Ltd.**
**Dongguan, Guangdong 523000 (CN)**

(72) Inventors:
- **XIE, Hongming**
**Dongguan, Guangdong 523871 (CN)**
- **LIU, Haiwang**
**Dongguan, Guangdong 523871 (CN)**
- **LUO, Guolin**
**Dongguan, Guangdong 523871 (CN)**
- **FENG, Xihui**
**Dongguan, Guangdong 523871 (CN)**
- **YAN, Zihui**
**Dongguan, Guangdong 523871 (CN)**
- **ZHANG, Yingjun**
**Dongguan, Guangdong 523871 (CN)**

(74) Representative: **Becker Kurig & Partner Patentanwälte mbB**
**Bavariastraße 7**
**80336 München (DE)**


(54) **KRAS INHIBITOR COMPOUND, PHARMACEUTICAL COMPOSITION THEREOF, AND USE THEREOF**


(57) The present invention belongs to the technical field of medicines, and specifically relates to a KRAS inhibitor compound, a pharmaceutical composition thereof, and a use thereof. Specifically, the present invention relates to the compound shown in formula (I), or a stereoisomer, tautomer, nitrogen oxide, solvate, metabolite, pharmaceutically acceptable salt, or prodrug of the compound shown in formula (I). The compound and pharmaceutical composition thereof to which the present invention relates can be used for preparing a drug for preventing or treating diseases related to wild-type KRAS, or diseases related to the mutation of KRAS G12A, KRAS G12C, KRAS G12D, KRAS G12R, KRAS G12S, KRAS G12V, KRAS G13D, or KRAS Q61H and are especially useful in preparing drugs for preventing or treating cancers.

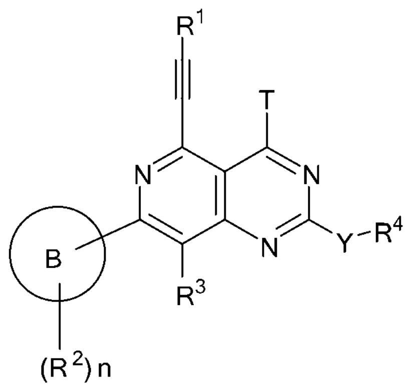

(I),


EP 4 678 643 A1

## Description

### FIELD OF THE INVENTION

The present invention belongs to the field of pharmaceutical technology. Specifically, the present invention relates to a compound capable of inhibiting one or more KRAS mutants, a pharmaceutical composition thereof, and the use of the compound and the pharmaceutical composition thereof in preparing a drug for preventing or treating KRAS-mediated related diseases.

### BACKGROUND ART

The RAS gene is one of the most commonly mutated genes in cancer (20%-25%). Currently known members of the RAS gene family include KRAS, NRAS and HRAS, among which KRAS mutation is the most common, accounting for approximately 85%. The mutation rate of KRAS is highest in pancreatic ductal adenocarcinoma (PDAC), reaching 97%, followed by colorectal cancer, multiple myeloma and lung cancer, at 52%, 42% and 32%, respectively. The most common mutation sites of the KRAS gene are codons 12, 13 and 61, and the mutation forms include KRAS G12C, KRAS G12D, KRAS G12R, KRAS G12S, KRAS G12A, KRAS G12V, KRAS Gl3D and KRAS Q61H. RAS gene mutations are often associated with poor prognosis in cancer. KRAS can be transiently activated by upstream growth factors or tyrosine kinases (such as EGFR). The activated KRAS can activate downstream pathways, including the PI3K-AKT-mTOR signaling pathway that controls cell generation and the RAS-RAF-MEK-ERK signaling pathway that controls cell proliferation. This also lays a biological foundation for the combination of many targets.

In recent years, some progress has been made in drug development using the allosteric sites of the KRAS G12C mutant. For example, in 2013, a research group reported the discovery of a small molecule inhibitor of KRAS G12C (Nature, 2013, 503, 548-551). In addition, Mirati disclosed KRAS G12D inhibitor compounds in patent WO2021041671, and a class of broad-spectrum KRAS (including KRAS G12A, KRAS G12C, KRAS G12D, KRAS G12R, KRAS G12S, KRAS G12V, KRAS G13D and KRAS Q61H) inhibitors in patent WO2022132200 for the treatment of KRAS-mediated cancers.

The discovery that KRAS is frequently mutated in various tumor types makes KRAS as an extremely attractive target for cancer treatment by the pharmaceutical field, and compounds that inhibit KRAS activity remain very worthy of investigation.

### SUMMARY

The present invention provides a compound that can inhibit one or more KRAS mutants, including KRAS G12A, KRAS G12C, KRAS G12D, KRAS G12R, KRAS G12S, KRAS G12V, KRAS G13D and KRAS Q61H. In addition, it relates to a pharmaceutical composition containing such compound, and the use of such compound and its pharmaceutical composition in the preparation of a drug for preventing or treating a related disease mediated by KRAS. The drug treats diseases and/or conditions, especially cancer, by inhibiting KRAS activity.

In one aspect, the present invention provides a compound having Formula (I) or a stereoisomer, a tautomer, an N-oxide, a solvate, a metabolite, a pharmaceutically acceptable salt or a prodrug thereof,

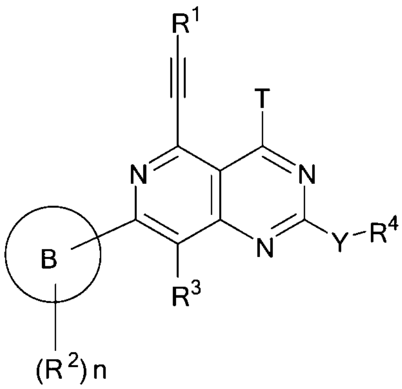

(I),

wherein,

$R^1$ is -H, -D, -CN, $-NH_2$, -C(=O)H, -C(=O)OH, $-C(=O)OR^{6a}$, $-C(=O)NR^6R^7$, $-NR^6C(=O)R^7$, $-NR^{6a}C(=O)NR^6R^7$, -C(=O)

$R^{6a}$, $-C(=O)OR^{6a}$, $-NR^6S(=O)_2R^7$, $-S(=O)_2NR^6R^7$, $-NR^{6a}S(=O)_2NR^6R^7$, $-NR^6R^7$, $-C_{1-6}$ alkyl $NR^6C(=O)R^7$, $-C_{1-6}$ alkyl $NR^6R^7$, $-C_{1-6}$ alkyl-$C(=O)OR^6$, $-C_{1-6}$ alkyl-$C(=O)NR^6R^7$, $C_{1-6}$ alkyl, $C_{1-6}$ cyanoalkyl, $C_{1-6}$ hydroxyalkyl, $C_{1-6}$ haloalkyl, $C_{1-6}$ alkoxy $C_{1-6}$ alkyl, ($C_{3-12}$ cycloalkyl)-$C_{1-6}$ alkyl, (3-12 membered heterocyclyl)-$C_{1-6}$ alkyl, ($C_{6-10}$ aryl)-$C_{1-6}$ alkyl, (5-12 membered heteroaryl)-$C_{1-6}$ alkyl, ($C_{3-12}$ cycloalkyl)-O-$C_{1-6}$ alkyl, (3-12 membered heterocyclyl)-O-$C_{1-6}$ alkyl, $C_{1-6}$ mercaptoalkyl, $C_{6-12}$ aryl, 5-12 membered heteroaryl, $C_{3-12}$ cycloalkyl or 3-12 membered heterocyclyl; wherein the $C_{1-6}$ alkoxy $C_{1-6}$ alkyl, ($C_{3-12}$ cycloalkyl)-$C_{1-6}$ alkyl, (3-12 membered heterocyclyl)-$C_{1-6}$ alkyl, ($C_{6-10}$ aryl)-$C_{1-6}$ alkyl, (5-12 membered heteroaryl)-$C_{1-6}$ alkyl, ($C_{3-12}$ cycloalkyl)-O-$C_{1-6}$ alkyl, (3-12 membered heterocyclyl)-O-$C_{1-6}$ alkyl, $C_{6-12}$ aryl, 5-12 membered heteroaryl, $C_{3-12}$ cycloalkyl and 3-12 membered heterocyclyl are each independently optionally substituted with 1, 2, 3 or 4 substituents selected from D, -OH, -F, -Cl, -Br, -I, CN, $-C(=O)OR^6$, $-NR^6R^7$, $-C(=O)NR^6R^7$, $-NR^6C(=O)R^7$ or $C_{1-6}$ alkyl;

T is

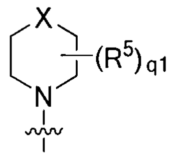

;

X is a bond, -O-, -S-, -NH-, $-CH_2-$, $-CH_2-NH-$, $-CH_2-NH-CH_2-$, $-(CH_2)_3-$, $-(CH_2)_2-$, $-NH-CH_2-$, $-O-CH_2-$, $-CH_2-O-$, $-CH_2-S-$ or $-S-CH_2-$;

each $R^5$ is independently H, -D, -OH, -F, -Cl, -Br, -I, -CN, -SH, -COOH, oxo, -NHC(=O)H, $-C(=O)R^{6c}$, $-C(=O)OR^{6c}$, $-C(=O)NR^{6b}R^{7b}$, $-NR^{6b}C(=O)R^{7b}$, $-NR^{6b}S(=O)_2R^{7b}$, $-S(=O)_2N(R^{7b})_2$, $-NR^{6b}C(=O)N(R^{7b})2$, $-NR^{6b}S(=O)_2N(R^{76})_2$, $-OS(=O)_2N(R^{7b})_2$, $-S(=O)_2R^{6c}$, $-C_{1-4}$ alkylene $-S(=O)_2N(R^{76})_2$, $C_{1-6}$ alkyl, $C_{1-6}$ alkylthio, $C_{1-6}$ alkoxy, $C_{2-6}$ alkenyl, $C_{2-6}$ alkynyl, $C_{1-6}$ cyanoalkyl, $C_{1-6}$ hydroxyalkyl, $C_{1-6}$ haloalkyl, -NH($C_{1-6}$ alkyl), -N($C_{1-6}$ alkyl)$_2$, $C_{2-6}$ haloalkenyl, $C_{2-6}$ haloalkynyl, $C_{1-6}$ haloalkoxy, $C_{1-6}$ haloalkylthio, $C_{6-10}$ aryl, 5-10 membered heteroaryl, $C_{3-6}$ cycloalkyl or 3-6 membered heterocyclyl, wherein the $C_{1-6}$ alkyl, $C_{1-6}$ alkylthio, $C_{1-6}$ alkoxy, $C_{2-6}$ alkenyl, $C_{2-6}$ alkynyl, -NH($C_{1-6}$ alkyl), -N($C_{1-6}$ alkyl)$_2$, $C_{6-10}$ aryl, 5-10 membered heteroaryl, $C_{3-6}$ cycloalkyl and 3-6 membered heterocyclyl are each independently optionally substituted with 1, 2, 3 or 4 $R^8$;

or, two $R^5$ attached to the same carbon atom together form

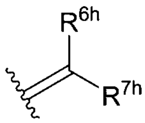 or 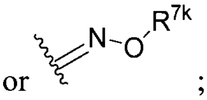

;

or, two $R^5$ attached to the same carbon atom together with the carbon atom to which they are attached form a $C_{3-6}$ cycloalkyl or 3-6 membered heterocyclyl, wherein the $C_{3-6}$ cycloalkyl and 3-6 membered heterocyclyl are each independently optionally substituted with 1, 2, 3 or 4 $R^8$;

or, two $R^5$ attached to two adjacent atoms together with the two adjacent atoms to which they are attached form a $C_{3-6}$ cycloalkyl or 3-6 membered heterocyclyl, wherein the $C_{3-6}$ cycloalkyl and 3-6 membered heterocyclyl are each independently optionally substituted with 1, 2, 3 or 4 $R^8$

each $R^8$ is independently -D, -OH, -F, -Cl, -Br, -I, -CN, $-NH_2$, -C(=O)OH, $-C(=O)NH_2$, oxo, $C_{1-6}$ alkyl, $C_{1-6}$ alkoxy, $C_{1-6}$ hydroxyalkyl, $C_{6-10}$ aryl, 6-10 membered heteroaryl, -C(=O)O$C_{1-6}$ alkyl, -C(=O)NH$C_{1-6}$ alkyl or -C(=O)N($C_{1-6}$ alkyl)$_2$;

with the proviso that T is not

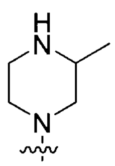 , 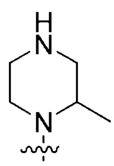 , 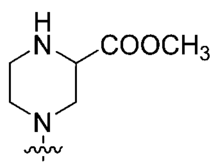 , 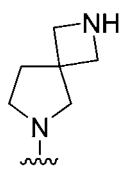 ,

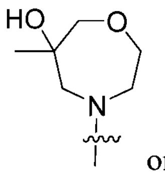 or 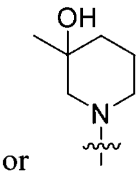 ;

Ring B is $C_{6-12}$ aryl or 5-12 membered heteroaryl;

each $R^2$ is independently -D, -OH, -F, -Cl, -Br, -I, -CN, -SH, $-CH_2C(=O)NR^{6b}R^{7b}$, $-C(=O)R^{6c}$, $-C(=O)OR^{6c}$, $-C(=O)NR^{6b}R^{7b}$, $-NR^{6b}C(=O)R^{7b}$, $-NR^{6b}R^{7b}$, $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, $C_{6-12}$ aryl, 5-12 membered heteroaryl, $C_{3-6}$ cycloalkyl or 3-6 membered heterocyclyl, wherein the $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, $C_{6-12}$ aryl, 5-12 membered heteroaryl, $C_{3-6}$ cycloalkyl and 3-6 membered heterocyclyl are each independently optionally substituted with 1, 2, 3 or 4 substituents selected from -D, -OH, -F, -Cl, -Br, -I, -CN, $-NH_2$, -C(=O)H, -C(=O)OH, $C_{1-6}$ alkyl, $C_{1-6}$ alkoxy, $C_{3-6}$ cycloalkyl and 3-6 membered heterocyclyl;

$R^3$ is -H, -D, -OH, -SH, -F, -Cl, -Br, -I, -CN, methyl, ethyl, n-propyl, i-propyl, n-butyl or $C_{1-4}$ haloalkyl;

Y is bond, O or S;

$R^4$ is 3-10 membered heterocyclyl, -L-(3-12 membered heterocyclyl), -L-($C_{3-12}$ cycloalkyl), -L-(5-12 membered heteroaryl) or -L-($C_{6-10}$ aryl); wherein the 3-10 membered heterocyclyl, -L-($C_{3-12}$ cycloalkyl), -L-(5-12 membered heteroaryl) and -L-($C_{6-10}$ aryl) are each independently optionally substituted with 1, 2, 3 or 4 $R^{9a}$; and the -L-(3-12 membered heterocyclyl) is optionally substituted with 1, 2, 3 or 4 $R^{9b}$.

$R^{9a}$ and $R^{9b}$ are each independently -D, -OH, -F, -Cl, -Br, -I, -CN, $-NR^{6d}R^{7d}$, $-C(=O)NR^{6d}R^{7d}$, $-CH_2NR^{6d}R^{7d}$, $-CH_2OC(=O)NR^{6d}R^{7d}$, $C_{1-6}$ alkyl, $C_{1-6}$ alkoxy, $C_{1-6}$ haloalkyl, $C_{1-6}$ haloalkoxy, ($C_{6-10}$ aryl)-$C_{1-6}$ alkyl, (5-12 membered heteroaryl)-$C_{1-6}$ alkyl, (3-6 membered heterocyclyl)-$C_{1-6}$ alkyl, ($C_{3-6}$ cycloalkyl)-$C_{1-6}$ alkyl, $C_{3-6}$ cycloalkyl or 3-6 membered heterocyclyl;

wherein the $C_{1-6}$ alkyl, $C_{1-6}$ alkoxy, $C_{1-6}$ haloalkyl, $C_{1-6}$ haloalkoxy, ($C_{6-10}$ aryl)-$C_{1-6}$ alkyl, (5-12 membered heteroaryl)-$C_{1-6}$ alkyl, (3-6 membered heterocyclyl)-$C_{1-6}$ alkyl, ($C_{3-6}$ cycloalkyl)-$C_{1-6}$ alkyl, $C_{3-6}$ cycloalkyl and 3-6 membered heterocyclyl are each independently optionally substituted with 1, 2, 3 or 4 substituents selected from -D, -F, -Cl, -Br, -I, -OH, -CN, $-NR^{6e}R^{7e}$, $-C(=O)C_{1-6}$ alkyl and $C_{1-6}$ alkyl;

or, two $R^{9b}$ attached to the same carbon atom together form

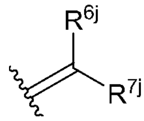 ;

L is $C_{1-6}$ alkylene;

$R^6$, $R^7$, $R^{6b}$, $R^{7b}$, $R^{6d}$, $R^{7d}$, $R^{6e}$ and $R^{7e}$ are each independently -H, -D or $C_{1-6}$ alkyl, wherein the $C_{1-6}$ alkyl is independently optionally substituted with 1, 2, 3 or 4 substituents selected from -D, -OH, -F, -Cl, -Br, -I, -CN, -C(=O)H, -C(=O)OH, $-NR^{6g}R^{7g}$, $C_{1-6}$ alkoxy, $C_{6-12}$ aryl, $C_{3-6}$ cycloalkyl and 3-6 membered heterocyclyl;

or $R^6$ and $R^7$, or $R^{6b}$ and $R^{7b}$, or $R^{6d}$ and $R^{7d}$, or $R^{6e}$ and $R^{7e}$, independently together with the N atom to which they are attached to form a 4-6 membered heterocyclic ring; wherein the 4-6 membered heterocyclic ring is optionally substituted with 1, 2, 3 or 4 substituents selected from -D, -OH, -F, -Cl, -Br, -I, -CN, $-NH_2$, $C_{1-6}$ alkyl, $C_{1-6}$ alkylamino, $C_{3-6}$ cycloalkyl, 3-6 membered heterocyclyl, $C_{1-6}$ alkoxy, $C_{1-6}$ cyanoalkyl, $C_{1-6}$ hydroxyalkyl, $C_{1-6}$ haloalkoxy and $C_{1-6}$ haloalkyl;

$R^{6a}$, $R^{6c}$, $R^{6g}$, $R^{7g}$, $R^{6h}$, $R^{7h}$, $R^{7k}$, $R^{6j}$ and $R^{7j}$ are each independently -H, -D or $C_{1-6}$ alkyl;

n is 0, 1, 2, 3, 4, 5, 6 or 7;

q1 is 0, 1, 2, 3, 4, 5, 6, 7 or 8.

In some embodiments, $R^1$ is -H, -D, -CN, $-NH_2$, -C(=O)H, -C(=O)OH, $-C(=O)OR^{6a}$, $-C(=O)NR^6R^7$, $-NR^6C(=O)R^7$, $-NR^{6a}C(=O)NR^6R^7$, $-C(=O)R^{6a}$, $-C(=O)OR^{6a}$, $-NR^6S(=O)_2R^7$, $-S(=O)_2NR^6R^7$, $-NR^{6a}S(=O)_2NR^6R^7$, $-NR^6R^7$, $-C_{1-4}$ alkyl $NR^6C(=O)R^7$, $-C_{1-4}$ alkyl $NR^6R^7$, $-C_{1-4}$ alkyl-$C(=O)OR^6$, $-C_{1-4}$ alkyl-$C(=O)NR^6R^7$, $C_{1-6}$ alkyl, $C_{1-4}$ cyanoalkyl, $C_{1-4}$ hydroxyalkyl, $C_{1-4}$ haloalkyl, $C_{1-4}$ alkoxy $C_{1-4}$ alkyl, ($C_{3-6}$ cycloalkyl)-$C_{1-4}$ alkyl, ($C_{7-12}$ cycloalkyl)-$C_{1-4}$ alkyl, (3-6 membered heterocyclyl)-$C_{1-4}$ alkyl, (7-12 membered heterocyclyl)-$C_{1-4}$ alkyl, phenyl-$C_{1-4}$ alkyl, (5-6 membered heteroaryl)-$C_{1-4}$ alkyl, ($C_{3-6}$ cycloalkyl)-O-$C_{1-4}$ alkyl, ($C_{7-12}$ cycloalkyl)-O-$C_{1-4}$ alkyl, (3-6 membered heterocyclyl)-O-$C_{1-6}$ alkyl, (3-7 membered heterocyclyl)-O-$C_{1-6}$ alkyl, (7-12 membered heterocyclyl)-O-$C_{1-4}$ alkyl, $C_{1-4}$ mercaptoalkyl, $C_{6-10}$ aryl, 5-12

membered heteroaryl, $C_{3-6}$ cycloalkyl or 3-6 membered heterocyclyl; wherein the $C_{1-4}$ alkoxy $C_{1-4}$ alkyl, ($C_{3-6}$ cycloalkyl)-$C_{1-4}$ alkyl, ($C_{7-12}$ cycloalkyl)-$C_{1-4}$ alkyl, (3-6 membered heterocyclyl)-$C_{1-4}$ alkyl, (7-12 membered heterocyclyl)-$C_{1-4}$ alkyl, phenyl-$C_{1-4}$ alkyl, (5-6 membered heteroaryl)-$C_{1-4}$ alkyl, ($C_{3-6}$ cycloalkyl)-O-$C_{1-4}$ alkyl, ($C_{7-12}$ cycloalkyl)-O-$C_{1-4}$ alkyl, (3-6 membered heterocyclyl)-O-$C_{1-6}$ alkyl, (3-7 membered heterocyclyl)-O-$C_{1-6}$ alkyl, (7-12 membered heterocyclyl)-O-$C_{1-4}$ alkyl, $C_{6-10}$ aryl, 5-12 membered heteroaryl, $C_{3-6}$ cycloalkyl and 3-6 membered heterocyclyl are each independently optionally substituted with 1, 2, 3 or 4 substituents selected from D, -OH, -F, -Cl, -Br, -I, CN, -C(=O)$OR^6$, -$NR^6R^7$, -C(=O)$NR^6R^7$, -$NR^6$C(=O)$R^7$ and $C_{1-4}$ alkyl; wherein, $R^6$, $R^7$ and $R^{6a}$ are each as defined herein.

In some embodiments, $R^1$ is -H, -D, -CN, -$NH_2$, -C(=O)H, -C(=O)OH, -C(=O)$OR^{6a}$, -C(=O)$NR^6R^7$, -$NR^6$C(=O)$R^7$, -$NR^{6a}$C(=O)$NR^6R^7$, -C(=O)$R^{6a}$, -C(=O)$OR^{6a}$, -$NR^6S(=O)_2R^7$, -$S(=O)_2NR^6R^7$, -$NR^{6a}S(=O)_2NR^6R^7$, -$NR^6R^7$, -$CH_2NR^6$C(=O)$R^7$, -$CH_2NR^6R^7$, -$(CH_2)_2NR^6R^7$, -$CH_2$C(=O)$OR^6$, -$(CH_2)_2$C(=O)$OR^6$, -$(CH_2)_3$C(=O)$OR^6$, -$CH_2$C(=O)$NR^6R^7$, -$(CH_2)_2$C(=O)$NR^6R^7$, -$(CH_2)_3$C(=O)$NR^6R^7$, -$CH_3$, -$CH_2CH_3$, -$(CH_2)_2CH_3$, -$(CH_2)_2CH(CH_3)_2$, -$(CH_2)_3CH_3$, -$C(CH_3)_3$, -$CH(CH_3)_2$, -$CH_2CH(CH_3)_2$, -$CH_2CN$, -$(CH_2)_2CN$, -$(CH_2)_3CN$, -$CH(CH_3)CN$, -$C(CH_3)_2CN$, -$CH_2OH$, -$(CH_2)_2OH$, -$(CH_2)_3OH$, -$CH(OH)CH_3$, -$(CH_2)_2CHF_2$, -$(CH_2)_2CF(CF_3)_2$, -$CF_3$, -$CHF_2$, -$CH_2F$, -$(CH_2)_2F$, -$(CH_2)_2Cl$, -$CH_2CF_3$, -$CH_2OCH_3$, -$(CH_2)_2OCH_3$, -$(CH_2)_2OCH_2CH_3$, -$CH_2OCH_2CH_3$, -$CH_2OC(CH_3)_3$, -$CH_2$-cyclopropyl, -$CH_2$-cyclobutyl, -$CH_2$-cyclopentyl, -$CH_2$-cyclohexyl, -$(CH_2)_2$-cyclopentyl, -$(CH_2)_3$-cyclopentyl, -$(CH_2)_2$-cyclohexyl, -$CH_2$-phenyl, -$CH_2$-imidazolyl, -$CH_2$-pyrazolyl, -$CH_2$O-cyclopropyl, -$CH_2$O-cyclobutyl, -$(CH_2)_2$O-cyclobutyl, -$CH_2$O-cyclopentyl, -$(CH_2)_2$O-cyclopentyl, -$CH_2$O-azetidinyl, -$CH_2$O-oxetanyl, -$CH_2$O-tetrahydrofuranyl, -$CH_2$O-pyrrolidinyl, -$CH_2$O-spiro[2.3]hexanyl, -$CH_2$O-spiro[3.3]heptanyl, -$CH_2SH$, -$(CH_2)_2SH$, phenyl, naphthyl, pyridinyl, pyrimidinyl, cyclopropyl, cyclobutyl, cyclopentyl, cyclohexyl, oxiranyl, oxetanyl, tetrahydropyranyl, azetidinyl or pyrrolidinyl; wherein the -$CH_2OCH_3$, -$(CH_2)_2OCH_3$, -$(CH_2)_2OCH_2CH_3$, -$CH_2OCH_2CH_3$, -$CH_2OC(CH_3)_3$, -$CH_2$-cyclopropyl, -$CH_2$-cyclobutyl, -$CH_2$-cyclopentyl, -$CH_2$-cyclohexyl, -$(CH_2)_2$-cyclopentyl, -$(CH_2)_3$-cyclopentyl, -$(CH_2)_2$-cyclohexyl, -$CH_2$-phenyl, -$CH_2$-imidazolyl, -$CH_2$-pyrazolyl, -$CH_2$O-cyclopropyl, -$CH_2$O-cyclobutyl, -$(CH_2)_2$O-cyclobutyl, -$CH_2$O-cyclopentyl, -$(CH_2)_2$O-cyclopentyl, -$CH_2$O-azetidinyl, -$CH_2$O-oxetanyl, -$CH_2$O-tetrahydrofuranyl, -$CH_2$O-pyrrolidinyl, -$CH_2$O-spiro[2.3]hexanyl, -$CH_2$O-spiro[3.3]heptanyl, phenyl, naphthyl, pyridinyl, pyrimidinyl, cyclopropyl, cyclobutyl, cyclopentyl, cyclohexyl, oxiranyl, oxetanyl, tetrahydropyranyl, azetidinyl and pyrrolidinyl are each independently optionally substituted with 1, 2, 3 or 4 substituents selected from D, -OH, -F, -Cl, -Br, -I, CN, -C(=O)$OR^6$, -$NR^6R^7$, -C(=O)$NR^6R^7$, -$NR^6$C(=O)$R^7$, methyl, ethyl, n-propyl, isopropyl, n-butyl, isobutyl and tert-butyl; wherein, $R^6$, $R^7$ and $R^{6a}$ are each as defined herein.

In some embodiments, T is

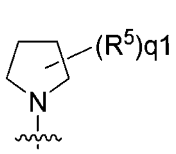 , 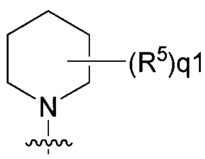 , 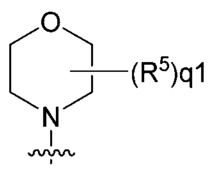 , 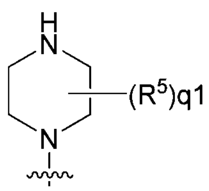 ,

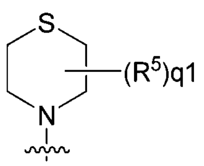 , 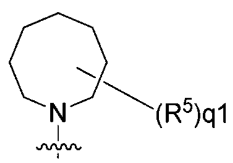 , 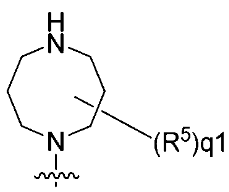 , 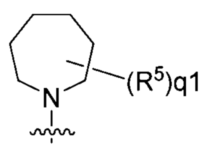 , 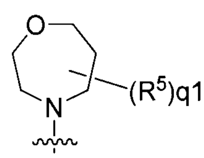 ,

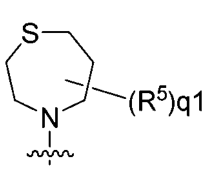 or 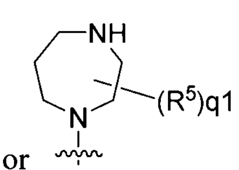 ,

each $R^5$ is independently -H, -D, -OH, -F, -Cl, -Br, -I, -CN, -SH, -COOH, oxo, -NHC(=O)H, -C(=O)$R^{6c}$, -C(=O)$OR^{6c}$, -C(=O)$NR^{6b}R^{7b}$, -$NR^{6b}$C(=O)$R^{7b}$, -$NR^{6b}S(=O)_2R^{7b}$, -$S(=O)_2N(R^{7b})_2$, -$NR^{6b}$C(=O)$N(R^{7b})$2, -$NR^{6b}S(=O)_2N(R^{7b})_2$, -$OS(=O)_2N(R^{7b})_2$, -$S(=O)_2R^{6c}$, -$C_{1-4}$ alkylene-$S(=O)_2N(R^{7b})_2$, $C_{1-4}$ alkyl, $C_{1-4}$ alkylthio, $C_{1-4}$ alkoxy, $C_{2-4}$ alkenyl, $C_{2-4}$ alkynyl, $C_{1-4}$ cyanoalkyl, $C_{1-4}$ hydroxyalkyl, $C_{1-4}$ haloalkyl, -NH($C_{1-4}$ alkyl), -N($C_{1-4}$ alkyl)$_2$, $C_{2-4}$ haloalkenyl, $C_{2-4}$ haloalkynyl, $C_{1-4}$ haloalkoxy, $C_{1-4}$ haloalkylthio, 6-10 membered aryl, 5-10 membered heteroaryl, $C_{3-6}$ cycloalkyl or 3-6 membered heterocyclyl; wherein the $C_{1-4}$ alkyl, $C_{1-4}$ alkylthio, $C_{1-4}$ alkoxy, $C_{2-4}$ alkenyl, $C_{2-4}$ alkynyl, -NH($C_{1-4}$ alkyl), -N($C_{1-4}$ alkyl)$_2$, 6-10 membered aryl, 5-10 membered heteroaryl, $C_{3-6}$ cycloalkyl and 3-6 membered heterocyclyl are each independently optionally substituted with 1, 2, 3 or 4 $R^8$;
or, two $R^5$ attached to the same carbon atom together form

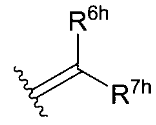 or 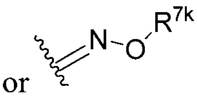 ;

or, two $R^5$ attached to the same carbon atom together with the carbon atom to which they are attached form a $C_{3-6}$ cycloalkyl or 3-6 membered heterocyclyl, wherein the $C_{3-6}$ cycloalkyl and 3-6 membered heterocyclyl are each independently optionally substituted with 1, 2, 3 or 4 $R^8$;
or, two $R^5$ attached to two adjacent atoms together with the two adjacent atoms to which they are attached form a $C_{3-6}$ cycloalkyl or 3-6 membered heterocyclyl, wherein the $C_{3-6}$ cycloalkyl and 3-6 membered heterocyclyl are each independently optionally substituted with 1, 2, 3 or 4 $R^8$;
each $R^8$ is independently -D, -OH, -F, -Cl, -Br, -I, -CN, $-NH_2$, -C(=O)OH, $-C(=O)NH_2$, oxo, $C_{1-4}$ alkyl, $C_{1-4}$ alkoxy, $C_{1-4}$ hydroxyalkyl, $C_{6-10}$ aryl, 6-10 membered heteroaryl, $-C(=O)OC_{1-4}$ alkyl, $-C(=O)NHC_{1-4}$ alkyl, $-C(=O)N(C_{1-4}\ alkyl)_2$;
$R^{6b}$, $R^{7h}$ and $R^{7k}$ are each independently -H, -D or $C_{1-4}$ alkyl;
wherein, $R^{6b}$, $R^{6c}$ and $R^{7b}$ are each as defined herein.

In some embodiments, T is

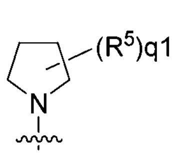 , 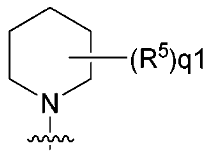 , 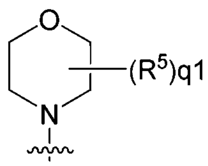 , 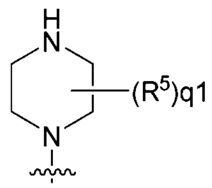 , 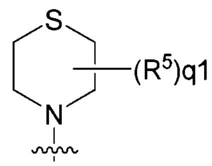 ,

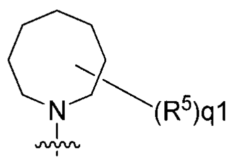 , 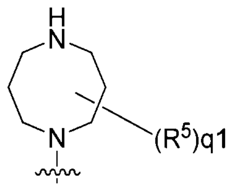 , 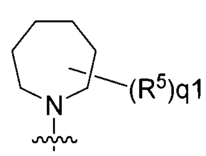 , 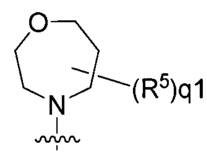 ,

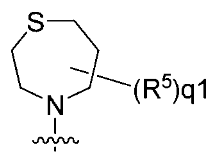 or 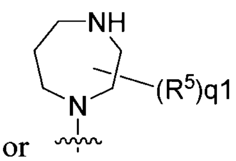 ,

each $R^5$ is independently -H, -D, -OH, -F, -Cl, -Br, -I, -CN, -SH, -COOH, oxo, -NH(C=O)H, $-C(=O)R^{6c}$, $-C(=O)OR^{6c}$, $-C(=O)NR^{6b}R^{7b}$, $-NR^{6b}C(=O)R^{7b}$, $-NR^{6b}S(=O)_2R^{7b}$, $-S(=O)_2N(R^{7b})_2$, $-NR^{6b}C(=O)N(R^{7b})2$, $-NR^{6b}S(=O)_2N(R^{7b})_2$, $-OS(=O)_2N(R^{7b})_2$, $-S(=O)_2R^{6c}$, $-CH_2S(=O)_2N(R^{7b})_2$, $-(CH_2)_2S(=O)_2N(R^{7b})_2$, $-CH_3$, $-CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-S(CH_2)_2CH_3$, $-SCH_2CH(CH_3)_2$, $-SCH(CH_3)_2$, $-OCH_3$, $-OCH_2CH_3$, $-O(CH_2)_2CH_3$, $-OCH_2CH(CH_3)_2$, $-OCH(CH_3)_2$, $-CH=CH_2$, $-CH=CHCH_3$, $-CH_2CH=CH_2$, -C≡CH, $-C≡CCH_3$, $-CH_2C≡CH$, $-CH_2CN$, $-(CH_2)_2CN$, $-(CH_2)_3CN$, $-CH_2OH$, $-(CH_2)_2OH$, $-(CH_2)_3OH$, $-CH(OH)CH_3$, $-CF_3$, $-CHF_2$, $-CH_2F$, $-(CH_2)_2F$, $-(CH_2)_2Cl$, $-CH_2CF_3$, $-NHCH_3$, $-NH(CH_2CH_3)$, $-NH((CH_2)_2CH_3)$, $-NH((CH_2)_3CH_3)$, $-NH(CH(CH_3)_2)$, $-N(CH_3)_2$, $-N(CH_2CH_3)_2$, $-N((CH_2)_2CH_3)_2$, $-CHFCH=CH_2$, -CH=CHF, -CH=CHCl, $-CH=CHCH_2F$, $-C≡CCH_2F$, $-OCF_3$, $-OCH_2F$, $-OCHF_2$, $-OCH_2CF_3$, $-OCH_2CHF_2$, $-SCF_3$, $-SCH_2F$, $-SCHF_2$, $-SCH_2CF_3$, $-SCH_2CHF_2$, phenyl, furyl, imidazolyl, isoxazolyl, oxazolyl, pyrrolyl, pyrazolyl, pyridinyl, pyrimidinyl, pyridazinyl, pyrazinyl, thiophenyl, thiazolyl, triazolyl, tetrazolyl, benzopyridinyl, benzimidazolyl, benzopyrrolyl, benzopyrazolyl, cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein the $-CH_3$, $-CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-S(CH_2)_2CH_3$, $-SCH_2CH(CH_3)_2$, $-SCH(CH_3)_2$, $-OCH_3$, $-OCH_2CH_3$, $-O(CH_2)_2CH_3$, $-OCH_2CH(CH_3)_2$, $-OCH(CH_3)_2$, $-CH=CH_2$,

$-CH=CHCH_3$, $-CH_2CH=CH_2$, $-C\equiv CH$, $-C\equiv CCH_3$, $-CH_2C\equiv CH$, $-CH_2CN$, $-(CH_2)_2CN$, $-(CH_2)_3CN$, $-CH_2OH$, $-(CH_2)_2OH$, $-(CH_2)_3OH$, $-CH(OH)CH_3$, $-CHF_2$, $-CH_2F$, $-(CH_2)_2F$, $-(CH_2)_2Cl$, $-CH_2CF_3$, $-NHCH_3$, $-NH(CH_2CH_3)$, $-NH((CH_2)_2CH_3)$, $-NH((CH_2)_3CH_3)$, $-NH(CH(CH_3)_2)$, $-N(CH_3)_2$, $-N(CH_2CH_3)_2$, $-N((CH_2)_2CH_3)_2$, $-CHFCH=CH_2$, $-CH=CHF$, $-CH=CHCl$, $-CH=CHCH_2F$, $-C\equiv CCH_2F$, $-OCH_2F$, $-OCHF_2$, $-OCH_2CF_3$, $-OCH_2CHF_2$, $-SCH_2F$, $-SCHF_2$, $-SCH_2CF_3$, $-SCH_2CHF_2$, phenyl, furyl, imidazolyl, isoxazolyl, oxazolyl, pyrrolyl, pyrazolyl, pyridinyl, pyrimidinyl, pyridazinyl, pyrazinyl, thiophenyl, thiazolyl, triazolyl, tetrazolyl, benzopyridinyl, benzimidazolyl, benzopyrrolyl, benzopyrazolyl, cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl are each independently optionally substituted with 1, 2, 3 or 4 $R^8$;

or, two $R^5$ attached to the same carbon atom together form

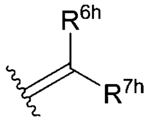 or 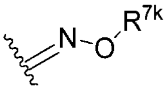 ;

or, two $R^5$ attached to the same carbon atom together with the carbon atom to which they are attached form a cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein the cyclopropyl, cyclobutyl, cyclopentyl, cyclohexyl, oxiranyl, aziridinyl, azetidinyl, oxetanyl, thietanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl are each independently optionally substituted with 1, 2, 3 or 4 $R^8$;

or, two $R^5$ attached to two adjacent atoms together with the two adjacent atoms to which they are attached form a cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl are each independently optionally substituted with 1, 2, 3 or 4 $R^8$;

each $R^8$ is independently -D, -OH, -F, -Cl, -Br, -I, -CN, $-NH_2$, -C(=O)OH, $-C(=O)NH_2$, oxo, $-CH_3$, $-CH_2CH_3$, $-(CH_2)_2CH_3$, $-(CH_2)_3CH_3$, $-CH(CH_3)_2$, $-CH_2CH(CH_3)_2$, $-C(CH_3)_3$, $-OCH_3$, $-OCH_2CH_3$, $-O(CH_2)_2CH_3$, $-CH_2OH$, $-(CH_2)_2OH$, $-(CH_2)_3OH$, $-CH(OH)CH_3$, phenyl, furyl, imidazolyl, isoxazolyl, oxazolyl, pyrrolyl, pyrazolyl, pyridinyl, pyrimidinyl, pyridazinyl, pyrazinyl, thiophenyl, thiazolyl, triazolyl, tetrazolyl, benzopyridinyl, benzimidazolyl, benzopyrrolyl, benzopyrazolyl, $-C(=O)OCH_3$, $-C(=O)OCH_2CH_3$, $-C(=O)O(CH_2)_2CH_3$, $-C(=O)OCH(CH_3)_2$, $-C(=O)NHCH_3$, $-C(=O)NHCH_2CH_3$, $-C(=O)N(CH_3)_2$ or $-C(=O)N(CH_3)CH_2CH_3$;

$R^{6h}$, $R^{7h}$ and $R^{7k}$ are each independently -H, -D, methyl, ethyl, n-propyl, isopropyl, n-butyl, isobutyl or *tert*-butyl;

wherein, $R^{6b}$, $R^{6c}$ and $R^{7b}$ are each as defined herein.

In some embodiments, Ring B is one of the following sub-structures,

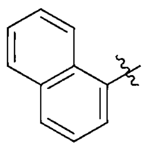 , 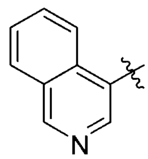 , 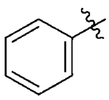 , 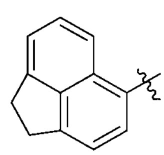 , 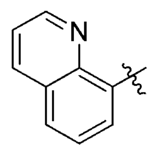 , 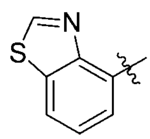 , 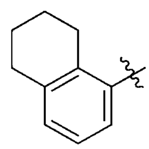 ,

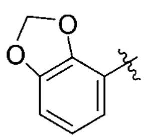 , 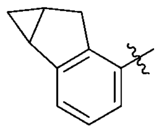 , 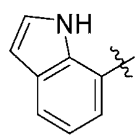 , 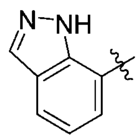 , 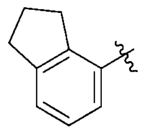 , 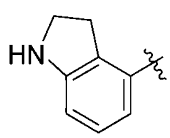 , 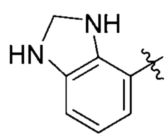 ,

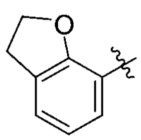 , 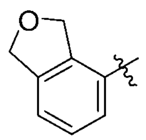 , 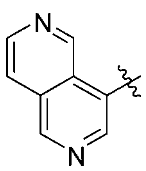 , 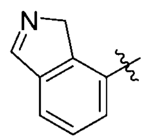 , 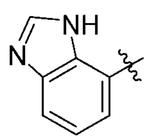 , 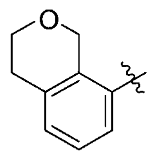 , 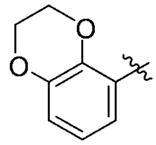

or

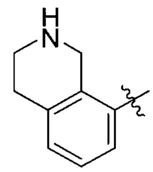

;

each $R^2$ is independently D, -OH, -F, -Cl, -Br, -I, -CN, -SH, $-CH_2C(=O)NR^{6b}R^{7b}$, $-C(=O)R^{6c}$, $-C(=O)OR^{6c}$, $-C(=O)NR^{6b}R^{7b}$, $-NR^{6b}C(=O)R^{7b}$, $-NR^{6b}R^{7b}$, $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, $C_{6-12}$ aryl, 5-12 membered heteroaryl, $C_{3-6}$ cycloalkyl or 3-6 membered heterocyclyl; wherein the $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, $C_{6-10}$ aryl, 5-12 membered heteroaryl, $C_{3-6}$ cycloalkyl and 3-6 membered heterocyclyl are each independently optionally substituted with 1, 2, 3 or 4 substituents selected from -D, -OH, -F, -Cl, -Br, -I, -CN, $-NH_2$, -C(=O)H, -C(=O)OH, $C_{1-4}$ alkyl, $C_{1-4}$ alkoxy, $C_{3-6}$ cycloalkyl and 3-6 membered heterocyclyl; wherein, $R^{6b}$, $R^{6c}$ and $R^{7b}$ are each as defined herein.

In some embodiments, each $R^2$ is independently -D, -OH, -F, -Cl, -Br, -I, -CN, -SH, $-CH_2C(=O)NR^{6b}R^{7b}$, $-C(=O)R^{6c}$, $-C(=O)OR^{6c}$, $-C(=O)NR^{6b}R^{7b}$, $-NR^{6b}C(=O)R^{7b}$, $-NR^{6b}R^{7b}$, $-CH_3$, $-CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-CH=CH_2$, $-CH=CHCH_3$, $-CH_2CH=CH_2$, -C=CH, $-C\equiv CCH_3$, $-CH_2C\equiv CH$, $-C\equiv CCH_2OH$, $-C\equiv C(CH_2)_2OH$, $-OCH_3$, $-OCH_2CH_3$, $-O(CH_2)_2CH_3$, $-CH_2CN$, $-(CH_2)_2CN$, $-(CH_2)_3CN$, $-CH_2OH$, $-(CH_2)_2OH$, $-(CH_2)_3OH$, $-CH(OH)CH_3$, $-(CH_2)_2F$, $-CH_2CHF_2$, $-CF_3$, $-CH_2CF_3$, $-CHF_2$, $-CH_2F$, $-(CH_2)_2Cl$, -CH=CHF, -CH=CHCl, $-CH=CHCH_2F$, $-C\equiv CCH_2F$, $-C\equiv C(CH_2)_2F$, -C=CF, $-OCF_3$, $-OCHF_2$, $-OCH_2CHF_2$, $-OCH_2CF_3$, $-OCHClCHCl_2$, $-OCH_2CH_2F$, $-SCF_3$, $-SCH_2CF_3$, $-SCH_2CHF_2$, phenyl, naphthyl, pyridinyl, pyrimidinyl, cyclopropyl, cyclobutyl, cyclopentyl, cyclohexyl, pyrrolidinyl, oxazolidinyl, tetrahydrofuranyl, piperidyl or piperazinyl, wherein the $-CH_3$, $-CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-CH=CH_2$, $-CH=CHCH_3$, $-CH_2CH=CH_2$, $-C\equiv CH$, $-C\equiv CCH_3$, $-CH_2C\equiv CH$, $-C\equiv CCH_2OH$, $-C\equiv C(CH_2)_2OH$, $-OCH_3$, $-OCH_2CH_3$, $-O(CH_2)_2CH_3$, $-CH_2CN$, $-(CH_2)_2CN$, $-(CH_2)_3CN$, $-CH_2OH$, $-(CH_2)_2OH$, $-(CH_2)_3OH$, $-CH(OH)CH_3$, $-(CH_2)_2F$, $-CH_2CHF_2$, $-CH_2CF_3$, $-CHF_2$, $-CH_2F$, $-(CH_2)_2Cl$, -CH=CHF, -CH=CHCl, $-CH=CHCH_2F$, $-C\equiv CCH_2F$, $-C\equiv C(CH_2)_2F$, $-OCHF_2$, $-OCH_2CHF_2$, $-OCH_2CF_3$, $-OCHClCHCl_2$, $-OCH_2CH_2F$, $-SCH_2CF_3$, $-SCH_2CHF_2$, phenyl, naphthyl, pyridinyl, pyrimidinyl, cyclopropyl, cyclobutyl, cyclopentyl, cyclohexyl, pyrrolidinyl, oxazolidinyl, tetrahydrofuranyl, piperidyl and piperazinyl are each independently optionally substituted with 1, 2, 3 or 4 substituents selected from -D, -OH, -F, -Cl, -Br, -I, -CN, $-NH_2$, -C(=O)H, -C(=O)OH, methyl, ethyl, *n*-propyl, isopropyl, methoxy, ethoxy, *n*-propoxy, *i*-propoxy, cyclopropyl, cyclobutyl, cyclopentyl, cyclohexyl, pyrrolidinyl, oxazolidinyl, tetrahydrofuranyl, piperidyl and piperazinyl; wherein $R^{6b}$, $R^{6c}$ and $R^{7b}$ are each as defined herein.

In some embodiments, $R^4$ is 3-6 membered heterocyclyl,

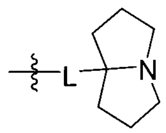, 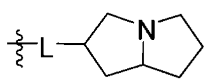,

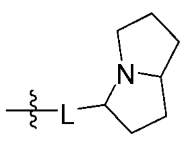, 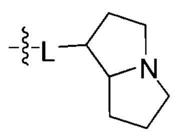, 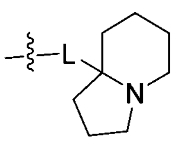, 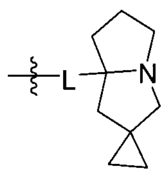,

-L-pyrrolidinyl, -L-piperidyl, -L-morpholinyl, -L-oxetanyl, -L-oxiranyl, -L-tetrahydrofuranyl, -L-octahydroindolizinyl, -L-cyclopropyl, -L-cyclopentyl, -L-octahydropentalenyl, -L-octahydro-1*H*-indenyl, -L-decalinyl, -L-pyridinyl, -L-pyrazolyl or -L-phenyl; wherein the 3-6 membered heterocyclyl, -L-cyclopropyl, -L-cyclopentyl, -L-octahydropentalenyl, -L-octahydro-1*H*-indenyl, -L-decalinyl, -L-pyridinyl, -L-pyrazolyl and -L-phenyl are each independently optionally substituted with 1, 2, 3 or 4 $R^{9a}$; and

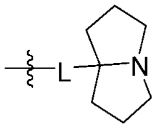, 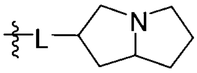, 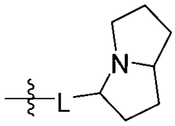, 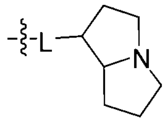, 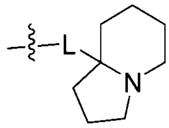, 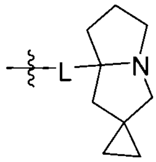,

-L-pyrrolidinyl, -L-piperidinyl, -L-morpholinyl, -L-oxetanyl, -L-oxiranyl, -L-tetrahydrofuranyl and -L-octahydroindolizinyl are each optionally substituted with 1, 2, 3 or 4 $R^{9b}$;

$R^{9a}$ and $R^{9b}$ are each independently -D, -OH, -F, -Cl, -Br, -I, -CN, $-NR^{6d}R^{7d}$, $-C(=O)NR^{6d}R^{7d}$, $-CH_2NR^{6d}R^{7d}$, $-CH_2OC(=O)NR^{6d}R^{7d}$, $C_{1-4}$ alkyl, $C_{1-4}$ alkoxy, $C_{1-4}$ haloalkyl, $C_{1-4}$ haloalkoxy, phenyl-$C_{1-4}$ alkyl, (5-6 membered heteroaryl)-$C_{1-4}$ alkyl, (3-6 membered heterocyclyl)-$C_{1-4}$ alkyl, ($C_{3-6}$ cycloalkyl)-$C_{1-4}$ alkyl, $C_{3-6}$ cycloalkyl or 3-6 membered heterocyclyl; wherein the $C_{1-4}$ alkyl, $C_{1-4}$ alkoxy, $C_{1-4}$ haloalkyl, $C_{1-4}$ haloalkoxy, phenyl-$C_{1-4}$ alkyl, (5-6 membered heteroaryl)-$C_{1-4}$ alkyl, (3-6 membered heterocyclyl)-$C_{1-4}$ alkyl, ($C_{3-6}$ cycloalkyl)-$C_{1-4}$ alkyl, $C_{3-6}$ cycloalkyl and 3-6 membered heterocyclyl are each independently optionally substituted with 1, 2, 3 or 4 substituents selected from -D, -F, -Cl, -Br, -I, -OH, -CN, $-NR^{6e}R^{7e}$, $-C(=O)C_{1-4}$ alkyl and $C_{1-4}$ alkyl; or,

two $R^{9b}$ attached to the same carbon atom together form

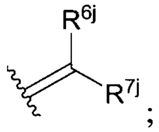;

L is $C_{1-4}$ alkylene;

$R^{6j}$ and $R^{7j}$ each are independently -H, -D or $C_{1-4}$ alkyl;

wherein, $R^{6d}$, $R^{7d}$, $R^{6d}$, $R^{7e}$ and $R^{7e}$ are each as defined herein.

In some embodiments,

$R^4$ is piperidyl, piperazinyl, pyrrolidinyl, imidazolidinyl,

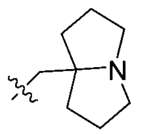,

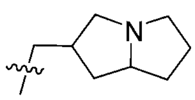, 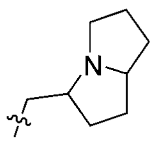, 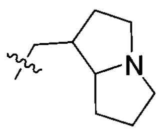, 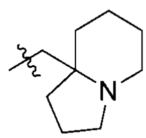, 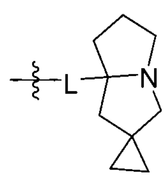,.

$-CH_2$-pyrrolidinyl, $-CH_2$-morpholinyl, $-(CH_2)_2$-morpholinyl, $-CH_2$-oxetanyl, $-CH_2$-oxiranyl, $-CH_2$-tetrahydrofuranyl, $-CH_2$-octahydroindolizinyl, $-CH_2$-cyclopropyl, $-CH_2$-cyclopentyl, $-CH_2$-octahydropentalenyl, $-CH_2$-octahydro-1*H*-indenyl, $-CH_2$-decahydronaphthalenyl, $-CH_2$-pyridinyl, $-(CH_2)_2$-pyridinyl, $-CH_2$-pyrazolyl, $-(CH_2)_2$-pyrazolyl or $-CH_2$-phenyl, wherein the piperidyl, piperazinyl, pyrrolidinyl, imidazolidinyl, $-CH_2$-cyclopropyl, $-CH_2$-cyclopentyl, $-CH_2$-octahydropentalenyl, $-CH_2$-octahydro-1*H*-indenyl, $-CH_2$-decahydronaphthalenyl, $-CH_2$-pyridinyl, $-(CH_2)_2$-pyridinyl, $-CH_2$-pyrazolyl, $-(CH_2)_2$-pyrazolyl and $-CH_2$-phenyl are each independently optionally substituted with 1, 2, 3 or 4 $R^{9a}$; and the

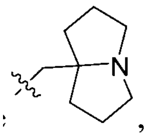, 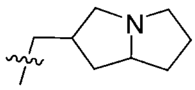, 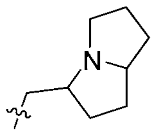, 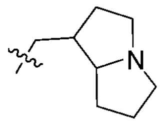, 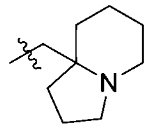,

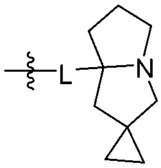,

$-CH_2$-pyrrolidinyl, $-CH_2$-morpholinyl, $-(CH_2)_2$-morpholinyl, $-CH_2$-oxetanyl, $-CH_2$-oxiranyl, $-CH_2$-tetrahydrofuranyl and $-CH_2$-octahydroindolizinyl are each optionally substituted with 1, 2, 3 or 4 $R^{9b}$;

$R^{9a}$ and $R^{9b}$ are each independently -D, -OH, -F, -Cl, -Br, -I, -CN, $-NR^{6d}R^{7d}$, $-C(=O)NR^{6d}R^{7d}$, $-CH_2NR^{6d}R^{7d}$, $-CH_2OC(=O)NR^{6d}R^{7d}$, methyl, ethyl, *n*-propyl, isopropyl, *n*-butyl, isobutyl, *tert*-butyl, methoxy, ethoxy, isopropoxy, $-CHF_2$, $-CF_3$, $-OCF_3$, benzyl, pyridinylmethyl, pyrazolylmethyl, morpholinylmethyl, pyrrolidinylmethyl, piperazinylmethyl, azetidinylmethyl, piperidinylmethyl, tetrahydropyranylmethyl, cyclopropylmethyl, cyclopentylmethyl, cyclohexylmethyl, cyclopentyl, cyclohexyl, morpholinyl, piperidinyl, pyrrolidinyl, piperazinyl or azetidinyl, wherein the methyl, ethyl, *n*-propyl, isopropyl, *n*-butyl, isobutyl, *tert*-butyl, methoxy, ethoxy, isopropoxy, $-CHF_2$, benzyl, pyridinylmethyl, pyrazolylmethyl, morpholinylmethyl, pyrrolidinylmethyl, piperazinylmethyl, azetidinylmethyl, piperidinylmethyl, tetrahydropyranylmethyl, cyclopropylmethyl, cyclopentylmethyl, cyclohexylmethyl, cyclopentyl, cyclohexyl, morpholinyl, piperidinyl, pyrrolidinyl, piperazinyl and azetidinyl are each independently optionally substituted with 1, 2, 3 or 4 substituents selected from -D, -F, -Cl, -Br, -I, -OH, -CN, $-NH_2$, $-NHCH_3$, $-N(CH_3)_2$, $-NHCH_2CH_3$, $-C(=O)CH_3$, $-C(=O)CH_2CH_3$, methyl, ethyl, *n*-propyl and isopropyl;

or,

two $R^{9b}$ attached to the same carbon atom together form

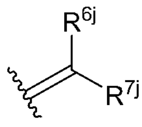;

$R^{6j}$ and $R^{7j}$ each are independently -H, -D, methyl, ethyl, n-propyl, isopropyl, n-butyl, isobutyl or *tert*-butyl;

wherein, $R^{6d}$, $R^{7d}$ , $R^{6d}$, $R^{7e}$ and $R^{7e}$ are as defined herein.

In some embodiments, $R^6$, $R^7$, $R^{6b}$, $R^{7b}$, $R^{6d}$, $R^{7d}$, $R^{6c}$ and $R^{7c}$ are each independently -H, -D or $C_{1-4}$ alkyl, wherein the $C_{1-4}$ alkyl is independently optionally substituted with 1, 2, 3 or 4 substituents selected from -D, -OH, -F, -Cl, -Br, -I, -CN, -C(=O)H, -C(=O)OH, $-NR^{6g}R^{7g}$, $C_{1-4}$ alkyl, $C_{6-10}$ aryl, $C_{3-6}$ cycloalkyl and 3-6 membered heterocyclyl;

or $R^6$ and $R^7$, or $R^{6b}$ and $R^{7b}$, or $R^{6d}$ and $R^{7d}$, or $R^{6e}$ and $R^{7e}$, independently together with the N atom to which they are attached form a 4-6 membered heterocyclic ring, wherein the 4-6 membered heterocyclic ring is optionally substituted with 1, 2, 3 or 4 substituents selected from -D, -OH, -F, -Cl, -Br, -I, -CN, $-NH_2$, $C_{1-4}$ alkyl, $C_{1-4}$ alkylamino, $C_{3-6}$ cycloalkyl, 3-6 membered heterocyclyl, $C_{1-4}$ alkoxy, $C_{1-4}$ cyanoalkyl, $C_{1-4}$ hydroxyalkyl, $C_{1-4}$ haloalkoxy and $C_{1-4}$ haloalkyl;

$R^{6a}$, $R^{6c}$, $R^{6g}$ and $R^{7g}$ are each independently -H, -D or $C_{1-4}$ alkyl.

In some embodiments, $R^6$, $R^7$, $R^{6b}$, $R^{7b}$, $R^{6d}$, $R^{7d}$, $R^{6e}$ and $R^{7e}$ are each independently -H, -D, methyl, ethyl, *n*-propyl, isopropyl, *n*-butyl, isobutyl or *tert*-butyl, wherein the methyl, ethyl, *n*-propyl, isopropyl, *n*-butyl, isobutyl and *tert*-butyl are each independently optionally substituted with 1, 2, 3 or 4 substituents selected from -D, -OH, -F, -Cl, -Br, -I, -CN, C(=O)H, -C(=O)OH, $-NR^{6g}R^{7g}$, methoxy, ethoxy, *n*-propoxy, isopropoxy, isobutoxy, cyclopropyl, cyclobutyl, cyclopentyl, cyclohexyl, phenyl, oxiranyl, oxetanyl, azetidinyl and pyrrolidinyl;

or $R^6$ and $R^7$, or $R^{6b}$ and $R^{7b}$, or $R^{6d}$ and $R^{7d}$, or $R^{6e}$ and $R^{7e}$, together with the nitrogen atom to which they are attached form pyrrolidine, piperazine, piperidine, morpholinyl, oxazolidine or imidazolidine, wherein the pyrrolidine, piperazine, piperidine, morpholinyl, oxazolidine and imidazolidine are each independently optionally substituted with 1, 2, 3 or 4 substituents selected from -D, -OH, -F, -Cl, -Br, -I, -CN, $-NH_2$, methyl, ethyl, *n*-propyl, isopropyl, *n*-butyl, isobutyl, *tert*-butyl, methylamino, dimethylamino, ethylamino, cyclopropyl, cyclopentyl, pyrrolidinyl, methoxy, ethoxy, isopropoxy,

cyanomethyl, hydroxymethyl, hydroxyethyl, trifluoromethoxy, monofluoromethyl, difluoromethyl, trifluoromethyl or 1,2-dichloroethyl;

$R^{6a}$, $R^{6c}$, $R^{6g}$ and $R^{7g}$ are each independently -H, -D, methyl, ethyl, n-propyl, isopropyl, n-butyl, isobutyl or *tert*-butyl.

In some embodiments, the compound of the present invention is a compound of Formula (I-1), or a stereoisomer, tautomer, nitrogen oxide, solvate, metabolite, pharmaceutically acceptable salt, or prodrug thereof,

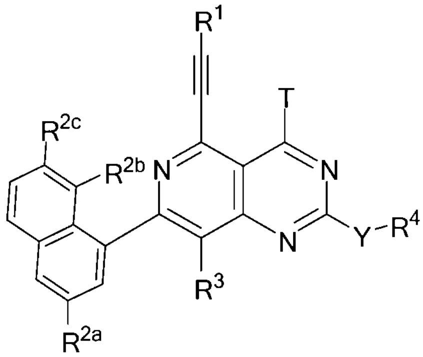

(I-1),

wherein, the $R^1$, $R^3$, $R^4$, Y and T are each as defined herein;

$R^{2a}$, $R^{2b}$ and $R^{2c}$ are each as defined herein. In other aspect, provided herein is a pharmaceutical composition comprising the compound disclosed herein.

In some embodiments, the pharmaceutical compositions of the present invention further comprise pharmaceutically acceptable adjuvant.

In some embodiments, the adjuvants of the present invention include, but are not limited to, carriers, excipients, diluents, vehicles or combinations thereof.

In some embodiments, the pharmaceutical composition may be in a liquid, solid, semi-solid, gel or spray dosage form.

In other aspect, the present invention provides a use of the pharmaceutical composition of the present invention in the preparation of a medicament for preventing, treating, or alleviating a disease associated with KRAS wild-type, or KRAS G12A, KRAS G12C, KRAS G12D, KRAS G12R, KRAS G12S, KRAS G12V, KRAS G13D or KRAS Q61H mutation.

In some embodiments, the disease associated with KRAS wild-type, or KRAS G12A, KRAS G12C, KRAS G12D, KRAS G12R, KRAS G12S, KRAS G12V, KRAS G13D or KRAS Q61H mutation according to the present invention is cancer.

In some embodiments, the cancer of the present invention is cardiac cancer: sarcoma, myxoma, rhabdomyoma, fibroma, lipoma or teratoma; lung cancer: bronchial carcinoma, non-small cell lung cancer, small cell lung cancer, alveolar carcinoma, bronchial adenoma, sarcoma, lymphoma, chondromatous hamartoma, mesothelioma; gastrointestinal cancer: esophageal cancer, gastric cancer, pancreatic cancer, small intestinal cancer, large intestinal cancer; urogenital tract cancer: kidney cancer, bladder and urethral cancer, prostate cancer, testicular cancer; liver cancer: hepatocellular carcinoma, hepatoblastoma, angiosarcoma, hepatocellular adenoma, hemangioma; biliary tract cancer: gallbladder cancer, ampullary cancer, cholangiocarcinoma; bone cancer: osteosarcoma, fibrosarcoma, malignant fibrous histiocytoma, chondrosarcoma, Ewing sarcoma, malignant lymphoma, multiple myeloma, malignant giant cell tumor, chordoma, enchondroma, chondroblastoma, chondromyxoid fibroma, osteoid osteoma, giant cell tumor; nervous system cancer: skull tumor, brain cancer; gynecological cancer: uterine cancer, vulvar cancer, vaginal cancer, fallopian tube carcinoma, ovarian cancer, breast cancer; hematologic cancer: acute or chronic myeloid leukemia, acute lymphoblastic leukemia, chronic lymphocytic leukemia, Hodgkin's disease, non-Hodgkin lymphoma; skin cancer: melanoma, basal cell carcinoma, squamous cell carcinoma, Kaposi sarcoma, dysplastic nevus, lipoma, hemangioma, dermatofibroma, keloid, psoriasis; adrenal cancer: neuroblastoma.

In other aspect, the present invention further provides a method for preventing or treating a disease associated with KRAS wild-type, or KRAS G12A, KRAS G12C, KRAS G12D, KRAS G12R, KRAS G12S, KRAS G12V, KRAS G13D or KRAS Q61H mutation, comprising administering to a patient a therapeutically effective amount of the compound according to the present invention or a pharmaceutical composition thereof.

In other aspect, the present invention further provides the compound or pharmaceutical composition thereof of the present invention for use in treating a disease associated with KRAS wild-type, or KRAS G12A, KRAS G12C, KRAS G12D, KRAS G12R, KRAS G12S, KRAS G12V, KRAS G13D or KRAS Q61H mutation.

In other aspect, the present invention relates to a method for the preparation, isolation, and purification of the compound of Formula (I) or (I-1).

Unless otherwise stated, all stereoisomers, tautomers, N-oxides, hydrates, solvates, metabolites, salts and pharmaceutically acceptable prodrugs of the compounds disclosed herein are within the scope of the invention.

In certain embodiments, the salt is a pharmaceutically acceptable salt. The phrase "pharmaceutically acceptable" refers to that the substance or composition must be compatible chemically and/or toxicologically, with the other ingredients comprising a formulation, and/or the mammal being treated therewith.

The compounds disclosed herein also include salts of the compounds which are not necessarily pharmaceutically acceptable salts, and which may be useful as intermediates for preparing and/or purifying compounds of Formula (1) or (I-1), and/or for separating enantiomers of compounds of Formula (I) or (I-1).

The foregoing merely summarizes certain aspects disclosed herein and is not intended to be limiting in nature. These aspects and other aspects and embodiments are described more fully below.

## DETAILED DESCRIPTION OF THE INVENTION

### DEFINITIONS AND GENERAL TERMINOLOGY

Reference will now be made in detail to certain embodiments of the invention, examples of which are illustrated in the accompanying structures and formulas. The invention is intended to cover all alternatives, modifications, and equivalents which may be included within the scope of the present invention as defined by the claims. One skilled in the art will recognize many methods and materials similar or equivalent to those described herein, which could be used in the practice of the present invention. The present invention is in no way limited to the methods and materials described herein. In the event that one or more of the incorporated literature, patents, and similar materials differs from or contradicts this application, including but not limited to defined terms, term usage, described techniques, or the like, this application controls.

It is further appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable subcombination.

Unless defined otherwise, all technical and scientific terms used herein have the same meaning as is commonly understood by one skilled in the art to which this invention belongs. All patents and publications referred to herein are incorporated by reference in their entirety.

As used herein, the term "subject" refers to an animal. Typically the animal is a mammal. A subject also refers to for example, primates (e.g., humans, male or female), cows, sheep, goats, horses, dogs, cats, rabbits, rats, mice, fish, birds and the like. In certain embodiments, the subject is a primate. In yet other embodiments, the subject is a human.

As used herein, "patient" refers to a human (including adults and children) or other animal. In one embodiment, "patient" refers to a human.

The term "comprise" is an open expression, it means comprising the contents disclosed herein, but don't exclude other contents.

"Stereoisomers" refers to compounds which have identical chemical constitution, but differ with regard to the arrangement of the atoms or groups in space. Stereoisomers include enantiomer, diastereomers, conformer (rotamer), geometric (*cis*/*trans*) isomer, atropisomer, etc. Unless otherwise indicated, all stereoisomers or mixtures of stereoisomers of the structural formulas described herein are encompassed within the scope of the present invention. Additionally, unless otherwise stated, structures depicted herein are also meant to include compounds that differ only in the presence of one or more isotopically enriched atoms.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994.

Any resulting mixtures of stereoisomers can be separated on the basis of the physicochemical differences of the constituents, into the pure or substantially pure geometric isomers, enantiomers, diastereomers, for example, by chromatography and/or fractional crystallization. Cis and trans isomers are diastereomer.

The term "tautomer" or "tautomeric form" refers to structural isomers of different energies which are interconvertible via a low energy barrier. Where tautomerization is possible (e.g. in solution), a chemical equilibrium of tautomers can be reached. For example, proton tautomers (also known as prototropic tautomers) include interconversions via migration of a proton, such as keto-enol and imine-enamine isomerizations. Valence tautomers include interconversions by reorganization of some of the bonding electrons. A specific example of keto-enol tautomerization is the interconversion of pentane-2,4-dione and 4-hydroxypent-3-en-2-one tautomers. Another example of tautomerization is phenol-keto tautomerization. The specific example of phenol-keto tautomerisms is pyridin-4-ol and pyridin-4(1*H*)-one tautomerism. Unless otherwise stated, all tautomeric forms of the compounds disclosed herein are within the scope of the invention.

As described herein, compounds disclosed herein may independently optionally be substituted with one or more substituents, such as are illustrated generally below, or as exemplified by particular classes, subclasses, and species of the invention. It should be understood that the terms "independently optionally substituted with..." and "optionally substituted with..." are interchangeably used with the term "substituted or unsubstituted." In general, the term "substituted" refers to the replacement of one or more hydrogen radicals in a given structure with the radical of a specified substituent. Unless otherwise indicated, an optionally substituted group may have a substituent at each substitutable position of the group. When more than one position in a given structure can be substituted with more than one substituent selected from a specified group, the substituent may be either the same or different at each position.

Furthermore, what need to be explained is that the phrase "each...is independently", "each of...and...is independently" and "...... are each independently", unless otherwise stated, should be broadly understood. The specific options expressed by the same symbol are independent of each other in different groups; or the specific options expressed by the same symbol are independent of each other in same groups.

At various places in the present specification, substituents of compounds disclosed herein are disclosed in groups or in ranges. It is specifically intended that the invention include each and every individual subcombination of the members of such groups and ranges. For example, the term "$C_{1-6}$ alkyl" is specifically intended to individually disclose methyl, ethyl, $C_3$ alkyl, $C_4$ alkyl, $C_5$ alkyl and $C_6$ alkyl.

At various places in the present specification, linking substituents are described. Where the structure clearly requires a linking group, the Markush variables listed for that group are understood to be linking groups. For example, if the structure requires a linking group and the Markush group definition for that variable lists "alkyl" or "aryl" then it is understood that the "alkyl" or "aryl" represents a linking alkylene group or arylene group, respectively.

The term "alkyl" refers to a saturated linear or branched-chain monovalent hydrocarbon group of 1-20 carbon atoms, wherein the alkyl group is optionally substituted with one or more substituents described herein. In some embodiments, the alkyl group contains 1-6 carbon atoms, referring to $C_{1-6}$ alkyl; in still other embodiments, the alkyl group contains 1-4 carbon atoms, referring to $C_{1-4}$ alkyl; in yet other embodiments, the alkyl group contains 1-3 carbon atoms, referring to $C_{1-3}$ alkyl. Examples of alkyl groups include, but are not limited to, methyl (Me, $-CH_3$), ethyl (Et, $-CH_2CH_3$), *n*-propyl (*n*-Pr, $-CH_2CH_2CH_3$), isopropyl (*i*-Pr, $-CH(CH_3)_2$), *n*-butyl (*n*-Bu, $-CH_2CH_2CH_2CH_3$), isobutyl (*i*-Bu, $-CH_2CH(CH_3)_2$), *sec*-butyl (*s*-Bu, $-CH(CH_3)CH_2CH_3$), *tert*-butyl (*t*-Bu, $-C(CH_3)_3$), *n*-pentyl ($-CH_2CH_2CH_2CH_2CH_3$), 2-pentyl ($-CH(CH_3)CH_2CH_2CH_3$), 3-pentyl ($-CH(CH_2CH_3)_2$), 2-methyl-2-butyl ($-C(CH_3)_2CH_2CH_3$), 3-methyl-2-butyl ($-CH(CH_3)CH(CH_3)_2$), 3-methyl-1-butyl ($-CH_2CH_2CH(CH_3)_2$), 2-methyl-1-butyl ($-CH_2CH(CH_3)CH_2CH_3$), *n*-hexyl ($-CH_2CH_2CH_2CH_2CH_2CH_3$), 2-hexyl ($-CH(CH_3)CH_2CH_2CH_2CH_3$), 3-hexyl ($-CH(CH_2CH_3)(CH_2CH_2CH_3)$), 2-methyl-2-pentyl ($-C(CH_3)_2CH_2CH_2CH_3$), 3-methyl-2-pentyl ($-CH(CH_3)CH(CH_3)CH_2CH_3$), 4-methyl-2-pentyl ($-CH(CH_3)CH_2CH(CH_3)_2$), 3-methyl-3-pentyl ($-C(CH_3)(CH_2CH_3)_2$), 2-methyl-3-pentyl ($-CH(CH_2CH_3)CH(CH_3)_2$), 2,3-dimethyl-2-butyl ($-C(CH_3)_2CH(CH_3)_2$), 3,3-dimethyl-2-butyl ($-CH(CH_3)C(CH_3)_3$), *n*-heptyl, *n*-octyl, and the like.

The term "alkylene" refers to a saturated divalent hydrocarbon group derived from a straight or branched chain saturated hydrocarbon by the removal of two hydrogen atoms. In some embodiments, the alkylene group contains 1-6 carbon atoms, referring to $C_{1-6}$ alkylene; in still other embodiments, the alkylene group contains 1-4 carbon atoms, referring to $C_{1-4}$ alkylene; in yet other embodiments, the alkylene group contains 1-3 carbon atoms, referring to $C_{1-3}$ alkylene; in yet other embodiments, the alkylene group contains 1-2 carbon atoms, referring to $C_{1-2}$ alkylene. Some non-limiting examples of the alkylene group include : $-CH_2-$, $-CH_2CH_2-$, $-CH(CH_3)CH_2-$, and the like.

The term "alkenyl" refers to linear or branched-chain monovalent hydrocarbon radical of 2 to 12 carbon atoms with at least one site of unsaturation, i.e., a carbon-carbon, $sp^2$ double bond, wherein the alkenyl radical may be optionally substituted independently with one or more substituents described herein, and includes radicals having "*cis*" and "*trans*" orientations, or alternatively, "*E*" and "*Z*" orientations. In some embodiments, the alkenyl contains 2 to 6 carbon atoms, denoted as $C_{2-6}$ alkenyl. In other embodiments, the alkenyl contains 2 to 4 carbon atoms, denoted as $C_{2-4}$ alkenyl. Some non-limiting examples of the alkenyl group include ethenyl or vinyl ($-CH=CH_2$), allyl ($-CH_2CH=CH_2$), 1-propenyl (i.e., propenyl, $-CH=CH-CH_3$), and the like.

The term "alkynyl" refers to a linear or branched monovalent hydrocarbon radical of 2 to 12 carbon atoms with at least one site of unsaturation, i.e., a carbon-carbon, sp triple bond, wherein the alkynyl radical may be optionally substituted independently with one or more substituents described herein. In some embodiments, the alkynyl contains 2 to 6 carbon atoms, denoted as $C_{2-6}$ alkynyl. In other embodiments, the alkynyl contains 2 to 4 carbon atoms, denoted as $C_{2-4}$ alkynyl. Examples of alkynyl include, but are not limited to, ethynyl ($-C=CH$), propargyl ($-CH_2C\equiv CH$), 1-propynyl ($-C\equiv C-CH_3$), and the like.

The term "cyanoalkyl" refers to an alkyl group substitued with one or more cyano groups, wherein the cyano group and the alkyl group are as defined herein. In some embodiments, "cyanoalkyl" refers to an alkyl group substitued with one cyano group. In some embodiments, "cyanoalkyl" is $C_{1-6}$ cyanoalkyl, that is, $C_{1-6}$ alkyl group substituted with one or more cyano groups. In some preferred embodiments, the $C_{1-6}$ cyanoalkyl is a $C_{1-6}$ alkyl group substituted with a single cyano group. In other embodiments, "cyanoalkyl" is $C_{1-4}$ cyanoalkyl, that is, $C_{1-4}$ alkyl group substituted with one or more cyano groups. Examples of cyanoalkyl include, but are not limited to, $-CH_2CN$, $-CH_2CH_2CH_2CH_2CN$, $-CH_2CH_2CN$,

$-CH_2CH(CN)CH_2CH_2CN$, $-CH_2CH(CN)CH_2CH(CH_3)CN$, and the like.

The term "hydroxyalkyl" refers to an alkyl group substitued with one or more hydroxy groups, wherein the alkyl group and the hydroxy group are as defined herein. In some embodiments, hydroxyalkyl refers to an alkyl group substitued with 1, 2, 3 or 4 hydroxy groups. In some embodiments, hydroxyalkyl refers to an alkyl group substitued with one or two hydroxyl groups. In some embodiments, hydroxyalkyl refers to $C_{1-6}$ hydroxyalkyl, that is, $C_{1-6}$ alkyl group substituted with one or more hydroxy groups. In some embodiments, hydroxyalkyl refers to $C_{1-4}$ hydroxyalkyl. In some embodiments, hydroxyalkyl refers to $C_{1-3}$ hydroxyalkyl. Examples of hydroxyalkyl include, but are not limited to, $-CH_2OH$, $-CH_2CH_2CH_2CH_2OH$, $-CH_2CH_2OH$, $-CH_2CH(OH)CH_2CH_2OH$, $-CH_2CH(OH)CH_2CH(CH_3)OH$, and the like.

The term "haloalkyl" refers to an alkyl group substituted with one or more halogen atoms, wherein the alkyl group and halogen atoms are as defined herein. In some embodiments, the haloalkyl is $C_{1-6}$ haloalkyl, referring to a $C_{1-6}$ alkyl group substituted with one or more halogen atoms; in other embodiments, the haloalkyl is $C_{1-4}$ haloalkyl, referring to a $C_{1-4}$ alkyl group substituted with one or more halogen atoms; in yet other embodiments, the haloalkyl is $C_{1-3}$ haloalkyl, referring to a $C_{1-3}$ alkyl group substituted with one or more halogen atoms. Some non-limiting examples of such group include monofluoromethyl, difluoromethyl, trifluoromethyl, 1-fluoroethyl, 2-fluoroethyl, 1,2-difluoroethyl, 1,1-difluoroethyl, 2,2-difluoroethyl, monochloromethyl, dichloromethyl, trichloromethyl, 2-chloroethyl, 1-chloroethyl, 1,2-dichloroethyl, 1,1-dichloroethyl, 2,2-dichloroethyl, 1,1-dibromoethyl, and the like.

The term "haloalkenyl" refers to an alkenyl group substituted with one or more halogen atoms, wherein the alkenyl group is as defined herein. In some embodiments, the haloalkenyl is $C_{2-6}$ haloalkenyl, referring to a $C_{2-6}$ alkenyl group substituted with one or more halogen atoms; in other embodiments, the haloalkenyl is $C_{2-4}$ haloalkenyl, referring to a $C_{2-4}$ alkenyl group substituted with one or more halogen atoms. Some non-limiting examples of such group include 1-chloroethenyl ($-CCl=CH_2$), 2-fluoroethenyl ($-CH=CHF$), 1-fluoroallyl ($-CHFCH=CH_2$), 3-fluoropropenyl (i.e., $-CH=CH-CH_2F$), 3,3-difluoropropenyl (i.e., $-CH=CH-CHF_2$).

The term "haloalkynyl" refers to an alkynyl group substituted with one or more halogen atoms, wherein the alkynyl group is as defined herein. In some embodiments, the haloalkynyl is $C_{2-6}$ haloalkynyl, referring to a $C_{2-6}$ alkynyl group substituted with one or more halogen atoms; in other embodiments, the haloalkynyl is $C_{2-4}$ haloalkynyl, referring to a $C_{2-4}$ alkynyl group substituted with one or more halogen atoms. Examples of such groups include, but are not limited to, 2-chloroethynyl ($-C\equiv CCl$), 1-chloropropargyl ($-CHClC\equiv CH$), 3-chloropropynyl ($-C\equiv C-CH_2Cl$), and the like.

The term "alkoxyalkyl" refers to an alkyl group substitued with one alkoxy group, wherein the alkoxy and the alkyl are as defined herein. In some embodiments, alkoxyalkyl refers to $C_{1-6}$ alkoxy $C_{1-6}$ alkyl; in other embodiments, alkoxyalkyl refers to $C_{1-4}$ alkoxy $C_{1-4}$ alkyl; in still some embodiments, the alkoxyalkyl refers to $C_{1-4}$ alkoxy $C_{1-3}$ alkyl; In some embodiments, alkoxyalkyl refers to $C_{1-3}$ alkoxy $C_{1-3}$ alkyl. Examples of alkoxy groups include, but are not limited to: methoxymethyl, ethoxymethyl, *n*-propoxymethyl, isopropoxymethyl, methoxyethyl, methoxy-*n*-propyl, methoxyisopropyl, ethoxyethyl, ethoxy-*n*-propyl, ethoxyisopropyl, (*n*-propoxy)ethyl, (isopropoxy)ethyl, (*n*-propoxy)-*n*-propyl, (*n*-propoxy)isopropyl, (isopropoxy)-*n*-propyl, (isopropoxy)isopropyl, and the like.

The term "carboxyalkyl" refers to an alkyl group substitued with one or more carboxy groups, wherein the carboxy group and the alkyl group are as defined herein. In some embodiments, the carboxyalkyl is $C_{1-6}$ carboxyalkyl, referring to a $C_{1-6}$ alkyl group substituted with one or more carboxy groups; in other embodiments, the carboxyalkyl is $C_{1-4}$ carboxyalkyl, referring to a $C_{1-4}$ alkyl group substituted with one or more carboxy groups; in other embodiments, the carboxyalkyl is $C_{1-3}$ carboxyalkyl, referring to a $C_{1-3}$ alkyl group substituted with one or more carboxy groups. Examples of carboxyalkyl groups include, but are not limited to, carboxymethyl ($-CH_2COOH$), 2-carboxyethyl ($-(CH_2)_2COOH$), 3-carboxypropyl ($-(CH_2)_3COOH$), and the like.

The term "alkamino" or "alkylamino" refers to an amino group substituted with one or two alkyl groups, comprising "*N*-alkylamino" and "*N,N*-dialkylamino", wherein the alkyl group and the amino group are as defined herein. In some embodiments, the alkylamino refers to $C_{1-6}$ alkylamino, that is, alkylamino having 1 to 6 carbon atoms; in other embodiments, the alkylamino refers to $C_{1-4}$ alkylamino, that is, alkylamino having 1 to 4 carbon atoms; in yet other embodiments, the alkylamino refers to $C_{1-3}$ alkylamino, that is, alkylamino having 1 to 3 carbon atoms. Suitable alkylamino groups may be monoalkylamino or dialkylamino groups. Such examples include, but are not limited to: *N*-methylamino (methylamino, $-NHCH_3$), *N*-ethylamino (ethylamino, $-NHCH_2CH_3$), *N,N*-dimethylamino (dimethylamino, $-N(CH_3)_2$), *N,N*-diethylamino (diethylamino, $-N(CH_2CH_3)_2$), and the like.

The term "mercaptoalkyl" refers to an alkyl group substitued with one or more mercapto groups, wherein the alkyl group is as defined herein. In some embodiments, mercaptoalkyl refers to $C_{1-6}$ mercaptoalkyl, that is, $C_{1-6}$ alkyl group substituted with one or more mercapto groups; preferably, $C_{1-6}$ mercaptoalkyl is a $C_{1-6}$ alkyl substituted with one mercapto group. In other embodiments, mercaptoalkyl refers to $C_{1-4}$ mercaptoalkyl. In other embodiments, mercaptoalkyl refers to $C_{1-3}$ mercaptoalkyl. Examples of mercaptoalkyl groups include, but are not limited to, mercaptomethyl ($-CH_2SH$), 2-mercaptoethyl ($-(CH_2)_2SH$), 3-mercaptopropyl ($-(CH_2)_3SH$), 2,3-dimercaptopropyl ($-CH_2CH(SH)CH_2(SH)$), and the like.

The term "alkoxy" means an alkyl group attached to the rest of the molecule via an oxygen atom, wherein the alkyl group is as defined herein. Unless otherwise specified, the alkoxy group contains 1-12 carbon atoms. In some embodiments, the alkoxy group contains 1-6 carbon atoms, referring to $C_{1-6}$ alkoxy; in still other embodiments, the

alkoxy group contains 1-4 carbon atoms, referring to $C_{1-4}$ alkoxy; in yet other embodiments, the alkoxy group contains 1-3 carbon atoms, referring to $C_{1-3}$ alkoxy. The alkoxy group may be optionally substituted with one or more substituents disclosed herein. Some non-limiting examples of the alkoxy group include, but are not limited to, methoxy (MeO, $-OCH_3$), ethoxy (EtO, $-OCH_2CH_3$), 1-propoxy (*n*-PrO, *n*-propoxy, $-OCH_2CH_2CH_3$), 2-propoxy (*i*-PrO, *i*-propoxy, $-OCH(CH_3)_2$), 1-butoxy (*n*-BuO, *n*-butoxy, $-OCH_2CH_2CH_2CH_3$), 2-methyl-l-propoxy (*i*-BuO, *i*-butoxy, $-OCH_2CH(CH_3)_2$), 2-butoxy (*s*-BuO, s-butoxy, $-OCH(CH_3)CH_2CH_3$), 2-methyl-2-propoxy (*t*-BuO, t-butoxy, $-OC(CH_3)_3$), 1-pentoxy (n-pentoxy, $-OCH_2CH_2CH_2CH_2CH_3$), 2-pentoxy ($-OCH(CH_3)CH_2CH_2CH_3$), 3-pentoxy ($-OCH(CH_2CH_3)_2$), 2-methyl-2-butoxy ($-OC(CH_3)_2CH_2CH_3$), 3-methyl-2-butoxy ($-OCH(CH_3)CH(CH_3)_2$), 3-methyl-1-butoxy ($-OCH_2CH_2CH(CH_3)_2$), 2-methyl-1-butoxy ($-OCH_2CH(CH_3)CH_2CH_3$), and the like.

The term "haloalkoxy" refers to an alkoxy group substituted with one or more halogen atoms, wherein the alkoxy group and halogen atoms are as defined herein. In some embodiments, haloalkoxy refers to a haloalkoxy group containing 1 to 6 carbon atoms, i.e., $C_{1-6}$ haloalkoxy; in other embodiments, haloalkoxy refers to a haloalkoxy group containing 1 to 4 carbon atoms, i.e., $C_{1-4}$ haloalkoxy; in yet other embodiments, haloalkoxy refers to a haloalkoxy group containing 1 to 3 carbon atoms, i.e., $C_{1-3}$ haloalkoxy. Some non-limiting examples of the haloalkoxy group include trifluoromethoxy ($-OCF_3$), monofluoromethoxy ($-OCH_2F$), 2-fluoroethoxy ($-OCH_2CH_2F$), and the like.

The term "carbocycle" or "carbocyclyl" refers to a saturated or partially unsaturated monocyclic, bicyclic, or tricyclic carbon ring system containing 3 to 12 ring atoms, wherein a $-CH_2$-group in the carbocycle may be optionally replaced by -C(=O)- (or -(CO)-). In one embodiment, the carbocyclyl contains 3 to 6 ring carbon atoms, referring to $C_{3-6}$ carbocyclyl; in other embodiments, the carbocyclyl is a saturated ring containing 3 to 6 ring carbon atoms, referring to $C_{3-6}$ cycloalkyl; in yet other embodiments, the $C_{3-6}$ cycloalkyl is a saturated monocyclic ring. In one embodiment, the carbocyclyl contains 7 to 12 ring carbon atoms, referring to $C_{7-12}$ carbocyclyl; in other embodiments, the carbocyclyl is a saturated ring containing 7 to 12 ring carbon atoms, referring to $C_{7-12}$ carbocyclyl. Examples of carbocyclyl include, but are not limited to, cyclopropyl, cyclobutyl, cyclopentyl, cyclopentenyl, cyclohexyl, cyclohexenyl, octahydro-1*H*-indenyl, octahydro-bicyclo[3.3.0]octanyl, etc. Examples of carbocycles where a $-CH_2$- group is replaced by -C(=O)- include, but are not limited to, cyclopentanone and cyclobutanone.

The term "cycloalkyl" refers to a monovalent saturated monocyclic or bicyclic carbon ring system containing 3 to 12 carbon atoms, wherein a $-CH_2$- group in the carbon ring may be optionally replaced by -C(=O)- (or -(CO)-). In some embodiments, the cycloalkyl contains 3 to 12 ring carbon atoms, i.e., $C_{3-12}$ cycloalkyl. In other embodiments, the cycloalkyl contains 3 to 10 ring carbon atoms, i.e., $C_{3-10}$ cycloalkyl. In other embodiments, the cycloalkyl contains 7 to 12 ring carbon atoms, i.e., $C_{7-12}$ cycloalkyl. In other embodiments, the $C_{7-12}$ cycloalkyl is a bicyclic system. In other embodiments, the cycloalkyl contains 7 to 10 ring carbon atoms, i.e., $C_{7-10}$ cycloalkyl. In other embodiments, the $C_{7-10}$ cycloalkyl is a bicyclic system. In other embodiments, the cycloalkyl contains 3 to 6 ring carbon atoms, i.e., $C_{3-6}$ cycloalkyl. In other embodiments, the $C_{3-6}$ cycloalkyl is a monocyclic system. In other embodiments, the cycloalkyl contains 3 to 5 ring carbon atoms, i.e., $C_{3-5}$ cycloalkyl. Examples of cycloalkyl include, but are not limited to, cyclopropyl, cyclobutyl, cyclopentyl, cyclohexyl, octahydro-1*H*-indenyl, octahydro-bicyclo[3.3.0]octanyl, etc. Examples of carbocycles where a $-CH_2$- group is replaced by -C(=O)- include, but are not limited to, cyclopentanone and cyclobutanone.

The term "heterocycle" or "heterocyclyl" refers to a saturated or partially unsaturated monocyclic, bicyclic, or tricyclic system containing 3 to 12 ring atoms, wherein at least one ring atom is selected from nitrogen, sulfur, and oxygen atoms; and wherein the heterocycle or heterocyclyl is non-aromatic and contains no aromatic rings. When a heterocycle is attached to the rest of the molecule via a single attachment point, it is referred to as a monovalent heterocyclyl group. Unless otherwise specified, the heterocyclyl group may be carbon or nitrogen linked, and a $-CH_2$- group can be optionally replaced by a -C(=O)- group. The sulfur can be optionally oxygenized to *S*-oxide. The nitrogen can be optionally oxygenized to *N*-oxide. In some embodiments, the heterocycle or heterocyclyl is composed of 3 to 12 atoms, referring to a 3-12 membered heterocycle or 3-12 membered heterocyclyl group. In some embodiments, the heterocycle or heterocyclyl is composed of 3 to 10 atoms, referring to a 3-10 membered heterocycle or 3-10 membered heterocyclyl group. In other embodiments, the heterocycle or heterocyclyl is composed of 3 to 9 atoms, referring to a 3-9 membered heterocycle or 3-9 membered heterocyclyl group. In other embodiments, the heterocycle or heterocyclyl is composed of 5 to 9 atoms, referring to a 5-9 membered heterocycle or 5-9 membered heterocyclyl group. In other embodiments, the heterocycle or heterocyclyl is composed of 3 to 7 atoms, referring to a 3-7 membered heterocycle or 3-7 membered heterocyclyl group. In other embodiments, the 3-7-membered heterocycle or 3-7-membered heterocyclyl group is a 3-7-membered monocyclic heterocycle or 3-7-membered monocyclic heterocyclyl group. In other embodiments, the heterocycle or heterocyclyl is composed of 3 to 6 atoms, referring to a 3-6 membered heterocycle or 3-6 membered heterocyclyl group. In other embodiments, the 3-6-membered heterocycle or 3-6 membered heterocyclyl group is a 3-6 membered monocyclic heterocycle or 3-6 membered monocyclic heterocyclyl group. In other embodiments, the heterocycle or heterocyclyl is composed of 7 to 12 atoms, referring to a 7-12 membered heterocycle or 7-12 membered heterocyclyl group. In other embodiments, the 7-12 membered heterocycle or 7-12 membered heterocyclyl group is a 7-12 membered bicyclic heterocycle or 7-12 membered bicyclic heterocyclyl group. In other embodiments, the heterocycle or heterocyclyl is composed of 7 to 10 atoms, referring to a 7-10 membered heterocycle or 7-10 membered heterocyclyl group. In other

embodiments, the 7-10 membered heterocycle or 7-10 membered heterocyclyl group is a 7-10 membered bicyclic heterocycle or 7-10 membered bicyclic heterocyclyl group. In other embodiments, the heterocycle or heterocyclyl is composed of 5 to 6 atoms, referring to a 5-6 membered heterocycle or 5-6 membered heterocyclyl group. Examples of the heterocycle include, but are not limited to: oxirane, aziridine, azetidine, oxetane, pyrrolidine, tetrahydrofuran, tetrahydrothiophene, thiazolidine, pyrazolidine, pyrazoline, oxazolidine, imidazolidine, piperidine, piperazine, morpholine, 3,8-diazabicyclo[3.2.1]octane, 3,6-diazabicyclo[3.1.1]heptane, and 2,5-diazabicyclo[2.2.2]octane. Examples of the heterocyclyl group include, but are not limited to: oxiranyl, aziridinyl, azetidinyl, oxetanyl, thietanyl, pyrrolidinyl, tetrahydrofuranyl, tetrahydrothiophenyl, thiazolidinyl, pyrazolidinyl, pyrazolinyl, oxazolidinyl, imidazolidinyl, piperidinyl, piperazinyl, or morpholinyl, etc.

The term "aryl" refers to monocyclic, bicyclic and tricyclic carbocyclic ring systems having a total of 6-14 ring members, or 6-12 ring members, or 6-10 ring members, wherein at least one ring in the system is aromatic, wherein each ring in the system contains 3 to 7 ring members. In some embodiments, the aryl group contains 6 to 12 ring atoms, referring to $C_{6-12}$ aryl or 6-12-membered aryl. In some embodiments, the aryl group contains 6 to 10 ring atoms, referring to $C_{6-10}$ aryl or 6-10-membered aryl. Examples of the aryl group may include, but are not limited to: phenyl, naphthyl, 1,2,3,4-tetrahydronaphthalenyl and anthryl.

The term "heteroaryl" or "heteroaromatic ring" refers to a monovalent monocyclic, bicyclic, or tricyclic system containing 5 to 14 ring atoms, or 5 to 12 ring atoms, or 5 to 10 ring atoms, or 5 to 6 ring atoms, wherein at least one ring is aromatic and at least one ring contains one or more ring heteroatoms selected from nitrogen, oxygen, and sulfur. The heteroaryl group is generally, but not necessarily bonded to the parent molecule through an aromatic ring of the heteroaryl group. When a $-CH_2-$ group is present in the heteroaryl group, the $-CH_2-$ group may be optionally replaced by -C(=O)-. Unless otherwise specified, the heteroaryl group may be attached to the rest of the molecule (e.g., the core structure in a general formula) through any chemically feasible atom, which may be a carbon or nitrogen atom. The term "heteroaryl" may be used interchangeably with "heteroaromatic ring" or "heteroaromatic compound". In some embodiments, the heteroaryl is a heteroaromatic group containing 5 to 12 ring atoms, referring to a 5-12-membered heteroaryl; in other embodiments, the heteroaryl is a heteroaromatic group containing 5 to 10 ring atoms, referring to a 5-10-membered heteroaryl; in yet other embodiments, the heteroaryl is a heteroaromatic group containing 5 to 6 ring atoms, referring to a 5-6-membered heteroaryl. Examples of heteroaryl include, but are not limited to: furyl, imidazolyl, isoxazolyl, oxazolyl, pyrrolyl, pyrazolyl, pyridyl, pyrimidinyl, pyridazinyl, pyrazinyl, thienyl, thiazolyl, triazolyl, tetrazolyl, benzopyridyl, benzimidazolyl, benzopyrazolyl, benzopyrrolyl (indolyl), etc.

The term "alkylthio" refers to an alkyl group attached to the rest of the molecule through a sulfur atom, wherein the alkyl group is as defined herein. In some embodiments, the alkylthio is $C_{1-6}$ alkylthio, referring to an alkylthio group containing 1 to 6 carbon atoms; in other embodiments, the alkylthio is $C_{1-4}$ alkylthio, referring to an alkylthio group containing 1 to 4 carbon atoms; in yet other embodiments, the alkylthio is $C_{1-3}$ alkylthio, referring to an alkylthio group containing 1 to 3 carbon atoms. Examples of alkylthio include, but are not limited to: methylthio ($-SCH_3$), ethylthio ($-SCH_2CH_3$), etc.

The term "haloalkylthio" refers to an alkylthio group substituted with one or more halogen atoms, wherein the alkylthio group is as defined herein. In some embodiments, the haloalkylthio is $C_{1-6}$ haloalkylthio, referring to a $C_{1-6}$ alkylthio substituted with one or more halogens; in other embodiments, the haloalkylthio is $C_{1-4}$ haloalkylthio, referring to a $C_{1-4}$ alkylthio substituted with one or more halogens; in yet other embodiments, the haloalkylthio is $C_{1-3}$ haloalkylthio, referring to a $C_{1-3}$ alkylthio substituted with one or more halogens. Examples of haloalkylthio include, but are not limited to: trifluoromethylthio ($-SCF_3$), 2,2,2-trifluoroethylthio ($-SCH_2CF_3$), monofluoromethylthio ($-SCH_2F$), etc.

The term "arylalkyl" refers to an alkyl group substituted with one aryl group, wherein the aryl and alkyl groups are as defined herein. In some embodiments, the arylalkyl is ($C_{6-10}$ aryl)-$C_{1-6}$ alkyl or (6-10-membered aryl)-$C_{1-6}$ alkyl; in other embodiments, the arylalkyl is ($C_{6-10}$ aryl)-$C_{1-4}$ alkyl or (6-10-membered aryl)-$C_{1-4}$ alkyl; in yet other embodiments, the arylalkyl is ($C_{6-10}$ aryl)-$C_{1-3}$ alkyl or (6-10-membered aryl)-$C_{1-3}$ alkyl; in other embodiments, the arylalkyl is phenyl-$C_{1-6}$ alkyl; in other embodiments, the arylalkyl is phenyl-$C_{1-4}$ alkyl; in yet other embodiments, the arylalkyl is phenyl-$C_{1-3}$ alkyl. Examples of arylalkyl include, but are not limited to: benzyl, phenethyl, naphthylmethyl, etc.

The term "heteroarylalkyl" refers to an alkyl group substituted with one heteroaryl group, wherein the heteroaryl and alkyl groups are as defined herein. In some embodiments, the heteroarylalkyl is (5-12-membered heteroaryl)-$C_{1-6}$ alkyl; in other embodiments, the heteroarylalkyl is (5-12-membered heteroaryl)-$C_{1-4}$ alkyl; in yet other embodiments, the heteroarylalkyl is (5-12-membered heteroaryl)-$C_{1-3}$ alkyl; in other embodiments, the heteroarylalkyl is (5-6-membered heteroaryl)-$C_{1-6}$ alkyl; in other embodiments, the heteroarylalkyl is (5-6-membered heteroaryl)-$C_{1-4}$ alkyl; in yet other embodiments, the heteroarylalkyl is (5-6-membered heteroaryl)-$C_{1-3}$ alkyl. Examples of heteroarylalkyl include, but are not limited to: pyrimidinylmethyl, pyridinylmethyl, pyridinylethyl, pyrazolylmethyl, etc.

The term "heterocyclylalkyl" refers to an alkyl group substituted with one heterocyclyl group, wherein the heterocyclyl and alkyl groups are specifically as defined herein. In some embodiments, the heterocyclylalkyl is (3-6-membered heterocyclyl)-$C_{1-6}$ alkyl; in other embodiments, the heterocyclylalkyl is (3-6-membered heterocyclyl)-$C_{1-4}$ alkyl; in other embodiments, the heterocyclylalkyl is (3-6-membered heterocyclyl)-$C_{1-3}$ alkyl. Examples of heterocycly-

lalkyl include, but are not limited to: piperidinylmethyl, piperidinylethyl, pyrrolidinylmethyl, etc.

The term "cycloalkylalkyl" refers to an alkyl group substituted with one cycloalkyl group. In some embodiments, the cycloalkylalkyl is ($C_{3-6}$ cycloalkyl)-$C_{1-6}$ alkyl; in other embodiments, the cycloalkylalkyl is ($C_{3-6}$ cycloalkyl)-$C_{1-4}$ alkyl; in yet other embodiments, the cycloalkylalkyl is ($C_{3-6}$ cycloalkyl)-$C_{1-3}$ alkyl. Examples of cycloalkylalkyl include, but are not limited to: cyclopropylmethyl, cyclopentylmethyl, cyclohexylmethyl, cyclohexylethyl, etc.

The term "cycloalkyl-O-alkyl" refers to an alkyl group substituted with a cycloalkyloxy group (cycloalkyl-O-). In some embodiments, the cycloalkyl-O-alkyl is ($C_{3-12}$ cycloalkyl)-O-$C_{1-6}$ alkyl; in other embodiments, the cycloalkyl-O-alkyl is ($C_{3-6}$ cycloalkyl)-O-$C_{1-6}$ alkyl; in other embodiments, the cycloalkyl-O-alkyl is ($C_{3-6}$ cycloalkyl)-O-$C_{1-4}$ alkyl; in yet other embodiments, the cycloalkyl-O-alkyl is ($C_{3-6}$ cycloalkyl)-O-$C_{1-3}$ alkyl. Examples of cycloalkyl-O-alkyl include, but are not limited to: -$CH_2$O-cyclopropyl, -$CH_2$O-cyclobutyl, -$(CH_2)_2$O-cyclobutyl, -$CH_2$O-cyclopentyl, -$(CH_2)_2$O-cyclopentyl, etc.

The term "heterocyclyl-O-alkyl" refers to an alkyl group substituted with a heterocyclyloxy group (heterocyclyl-O-). In some embodiments, the heterocyclyl-O-alkyl is (3-12-membered heterocyclyl)-O-$C_{1-6}$ alkyl; in other embodiments, the heterocyclyl-O-alkyl is (3-6-membered heterocyclyl)-O-$C_{1-4}$ alkyl; in other embodiments, the heterocyclyl-O-alkyl is (7-12-membered heterocyclyl)-O-$C_{1-4}$ alkyl; in yet other embodiments, the heterocyclyl-O-alkyl is (3-6-membered heterocyclyl)-O-$C_{1-3}$ alkyl. Examples of heterocyclyl-O-alkyl include, but are not limited to: -$CH_2$O-azetidinyl, -$CH_2$O-oxetanyl, -$CH_2$O-tetrahydrofuranyl, -$CH_2$O-spiro[2.3]hexanyl, -$CH_2$O-spiro[3.3]heptanyl, etc.

The term "halogen" refers to F (fluoro), Cl (chloro), Br (bromo), or I (iodo).

The term "oxo" refers to =O.

The term "cyano" refers to -CN or -C=N.

The term "mercapto" refers to -SH.

The term "hydroxy" refers to -OH.

The term "carboxy" refers to -C(=O)OH.

The term "amino" refers to -$NH_2$.

The term "composed of j-k atoms" or "j-k membered" refers to a cyclic group comprising j to k ring atoms, wherein the ring atoms include carbon atoms and/or heteroatoms such as O, N, S, or P; j and k each independently represent any non-zero natural number with k > j; and "j-k" includes j, k, and every natural number therebetween. For example: "composed of 3-8 atoms" or "3-8-membered", "composed of 3-6 atoms" or "3-6 membered", "composed of 5-10 atoms" or "5-10-membered", or "composed of 5-6 atoms" or "5-6 membered" indicates a cyclic group comprising 3-8 (i.e., 3,4,5,6,7,8), 3-6 (i.e., 3,4,5,6), 5-10 (i.e., 5,6,7,8,9,10), or 5-6 (i.e., 5,6) ring atoms, respectively, wherein the ring atoms include carbon atoms and/or heteroatoms such as O, N, S, or P.

As described herein, a ring system formed by substituent $(R)_q$ attached to the central ring via a single bond indicates that q R substituents may substitute at any substitutable position or chemically feasible location on said ring. For example, Formula (a) represents a naphthalene ring substituted by n $R^2$ groups, when n is greater than 1, each $R^2$ may be independently selected from identical or different substituent groups.

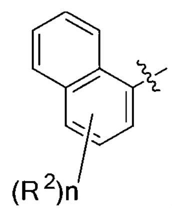

Formula a

The term "prodrug" refers to a compound that is transformed *in vivo* into a compound of Formula (I) or (I-1). Such a transformation can be affected, for example, by hydrolysis of the prodrug form in blood or enzymatic transformation to the parent form in blood or tissue. Prodrugs of the compounds disclosed herein may be, for example, esters. Some common esters which have been utilized as prodrugs are phenyl esters, aliphatic ($C_{1-24}$) esters, acyloxymethyl esters, carbonates, carbamates and amino acid esters. For example, a compound disclosed herein that contains a hydroxy group may be acylated at this position in its prodrug form. Other prodrug forms include phosphates, such as, those phosphate compounds derived from the phosphonation of a hydroxy group on the parent compound.

A "metabolite" is a product produced through metabolism in the body of a specified compound or salt thereof. The metabolites of a compound may be identified using routine techniques known in the art and their activities determined using tests such as those described herein. Such products may result for example from oxidation, reduction, hydrolysis, amidation, deamidation, esterification, deesterification, enzyme cleavage, and the like, of the administered compound. Accordingly, the invention includes metabolites of compounds disclosed herein, including metabolites produced by contacting a compound disclosed herein with a mammal for a sufficient time period.

A "pharmaceutically acceptable salts" refers to organic or inorganic salts of a compound disclosed herein. Pharmaceutically acceptable salts are well known in the art, for example: S. M. Berge et al., describe pharmaceutically

acceptable salts in detail in J. Pharmaceutical Sciences, 1977, 66: 1-19, which is incorporated herein by reference. Some non-limiting examples of pharmaceutically acceptable and nontoxic salts include salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid and malonic acid or by using other methods used in the art such as ion exchange. This invention also envisions the quaternization of any basic nitrogen-containing groups of the compounds disclosed herein. Water or oil soluble or dispersable products may be obtained by such quaternization. Further pharmaceutically acceptable salts include, when appropriate, nontoxic ammonium, quaternary ammonium, and amine cations formed using counterions such as halide, hydroxide, carboxylate, sulfate, phosphate, nitrate, $C_1$-$C_8$ sulfonate or aryl sulfonate.

The term "solvate" refers to an association or complex of one or more solvent molecules and a compound disclosed herein. Some non-limiting examples of the solvent that form solvates include water, isopropanol, ethanol, methanol, dimethylsulfoxide (DMSO), ethyl acetate, acetic acid, ethanolamine or mixtures thereof. The term "hydrate" refers to the complex where the solvent molecule is water.

The term "hydrate" can be used when said solvent is water. In one embodiment, one solvent molecule is associated with one molecule of the compounds disclosed herein, such as a hydrate; in another embodiment, more than one solvent molecule may be associated with one molecule of the compounds disclosed herein, such as a dihydrate; in still another embodiment, less than one solvent molecule may be associated with one molecule of the compounds disclosed herein, such as a hemihydrate. Furthermore, all the solvates of the invention retain the biological effectiveness of the non-hydrate form of the compounds disclosed herein.

As used herein, the term "treat", "treating" or "treatment" of any disease or disorder refers in some embodiments, to ameliorating the disease or disorder (i.e., slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (e.g., stabilization of a discernible symptom), physiologically, (e.g., stabilization of a physical parameter), or both. In yet another embodiment, "treat", "treating" or "treatment" refers to preventing or delaying the onset or development or progression of the disease or disorder.

The term "preventing" or "prevention" refers to a reduction in risk of acquiring a disease or disorder (i.e., causing at least one of the clinical symptoms of the disease not to develop in a subject that may be exposed to or predisposed to the disease but does not yet experience or display symptoms of the disease).

The term "therapeutically effective amount" refers to an amount of a compound sufficient to elicit a therapeutic effect when administered to a subject for treating a disease. The therapeutically effective amount may vary depending on the compound, the disease and its severity, as well as the condition, age, body weight, and sex of the subject to be treated.

Unless otherwise specified, all suitable isotopic variations, stereoisomers, tautomers, solvates, metabolites, pharmaceutically acceptable salts, and prodrugs of the compounds of the present invention are encompassed within the scope of the invention.

In the structures disclosed herein, when the stereochemistry of any specific chiral atom is unspecified, all stereoisomers of such structure are considered part of the invention and are included as compounds disclosed herein. When stereochemistry is indicated by a solid wedge or dashed line denoting a specific configuration, the stereoisomer of the structure is thereby explicitly defined.

Nitrogen oxides of the compounds of the invention are also included within the scope of the invention. Nitrogen oxides of the compounds of the invention can be prepared by oxidation of the corresponding nitrogen-containing basic substance using a conventional oxidizing agent (e.g., hydrogen peroxide) at elevated temperatures in the presence of an acid such as acetic acid, or by reaction with a peracid in a suitable solvent, such as peracetic acid in methylene chloride, ethyl acetate or methyl acetate, or with 3-chloroperoxybenzoic acid in chloroform or methylene chloride.

The compound of formula (I) or (I-1) may exist in the form of a salt.

Any formula given herein is also intended to represent isotopically unenriched forms as well as isotopically enriched forms of the compounds. Any formula given herein is also intended to represent isotopically unenriched forms as well as isotopically enriched forms of the compounds. Examples of isotopes that can be incorporated into compounds of the invention include isotopes of hydrogen, carbon, nitrogen, oxygen, phosphorus, sulfur, fluorine, chlorine, and iodine, such as $^{2}H$, $^{3}H$, $^{11}C$, $^{13}C$, $^{14}C$, $^{15}N$, $^{17}O$, $^{18}O$, $^{18}F$, $^{31}P$, $^{32}P$, $^{35}S$, $^{36}Cl$, $^{125}I$, respectively.

## DESCRIPTION OF COMPOUNDS OF THE INVENTION

The present invention provides a compound, or a pharmaceutical composition thereof, useful as a KRAS inhibitor, particularly a KRAS G12D inhibitor. The invention further relates to the use of said compound or pharmaceutical composition in the manufacture of a medicament for treating diseases and/or disorders by inhibiting KRAS activity.

The superior properties of the compounds of the present invention, such as half-life, clearance rate, selectivity,

bioavailability, chemical stability, metabolic stability, membrane permeability, and solubility may lead to reduced side effects, broadened therapeutic index, or improved tolerability.

In one aspect, the present invention provides a compound having Formula (I) or a stereoisomer, a tautomer, an *N*-oxide, a solvate, a metabolite, a pharmaceutically acceptable salt or a prodrug thereof,

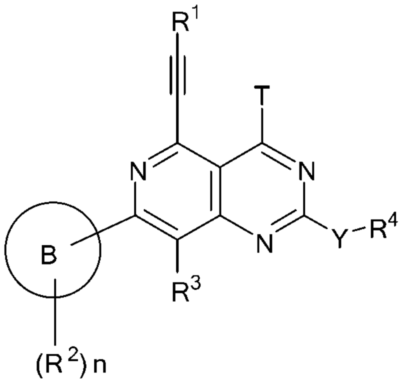

(I),

wherein, the $R^1$, $R^2$, $R^3$, $R^4$, Y, T, ring B and n are each as defined herein.

In some embodiments, $R^1$ is -H, -D, -CN, $-NH_2$, -C(=O)H, -C(=O)OH, $-C(=O)OR^{6a}$, $-C(=O)NR^6R^7$, $-NR^6C(=O)R^7$, $-NR^{6a}C(=O)NR^6R^7$, $-C(=O)R^{6a}$, $-C(=O)OR^{6a}$, $-NR^6S(=O)_2R^7$, $-S(=O)_2NR^6R^7$, $-NR^{6a}S(=O)_2NR^6R^7$, $-NR^6R^7$, $-C_{1-6}$ alkyl $NR^6C(=O)R^7$, $-C_{1-6}$ alkyl $NR^6R^7$, $-C_{1-6}$ alkyl-$C(=O)OR^6$, $-C_{1-6}$ alkyl-$C(=O)NR^6R^7$, $C_{1-6}$ alkyl, $C_{1-6}$ cyanoalkyl, $C_{1-6}$ hydroxyalkyl, $C_{1-6}$ haloalkyl, $C_{1-6}$ alkoxy $C_{1-6}$ alkyl, ($C_{3-12}$ cycloalkyl)-$C_{1-6}$ alkyl, (3-12 membered heterocyclyl)-$C_{1-6}$ alkyl, ($C_{6-10}$ aryl)-$C_{1-6}$ alkyl, (5-12 membered heteroaryl)-$C_{1-6}$ alkyl, ($C_{3-12}$ cycloalkyl)-O-$C_{1-6}$ alkyl, (3-12 membered heterocyclyl)-O-$C_{1-6}$ alkyl, $C_{1-6}$ mercaptoalkyl, $C_{6-12}$ aryl, 5-12 membered heteroaryl, $C_{3-12}$ cycloalkyl or 3-12 membered heterocyclyl; wherein the $C_{1-6}$ alkoxy $C_{1-6}$ alkyl, ($C_{3-12}$ cycloalkyl)-$C_{1-6}$ alkyl, (3-12 membered heterocyclyl)-$C_{1-6}$ alkyl, ($C_{6-10}$ aryl)-$C_{1-6}$ alkyl, (5-12 membered heteroaryl)-$C_{1-6}$ alkyl, ($C_{3-12}$ cycloalkyl)-O-$C_{1-6}$ alkyl, (3-12 membered heterocyclyl)-O-$C_{1-6}$ alkyl, $C_{6-12}$ aryl, 5-12 membered heteroaryl, $C_{3-12}$ cycloalkyl and 3-12 membered heterocyclyl are each independently optionally substituted with 1, 2, 3 or 4 substituents selected from D, -OH, -F, -Cl, -Br, -I, CN, $-C(=O)OR^6$, $-NR^6R^7$, $-C(=O)NR^6R^7$, $-NR^6C(=O)R^7$ or $C_{1-6}$ alkyl; wherein, the $R^6$, $R^7$ and $R^{6a}$ are each as defined herein.

In some embodiments, T is

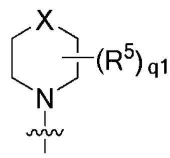

;

X is a bond, -O-, -S-, -NH-, $-CH_2-$, $-CH_2-NH-$, $-CH_2-NH-CH_2-$, $-(CH_2)_3-$, $-(CH_2)_2-$, $-NH-CH_2-$, $-O-CH_2-$, $-CH_2-O-$, $-CH_2-S-$ or $-S-CH_2-$;

q1 is 0, 1, 2, 3, 4, 5, 6, 7 or 8;

with the proviso that T is not

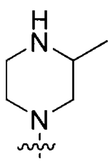

,

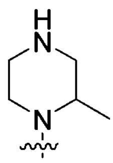

,

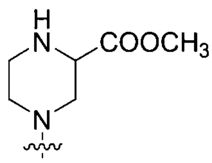

,

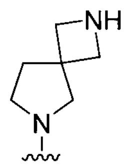

,

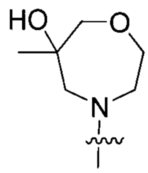

or

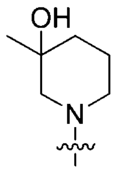,

wherein, the

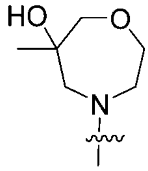

includes stereoisomers or mixtures of stereoisomers thereof;
wherein, the $R^5$ is as defined herein.

In some embodiments, each $R^5$ is independently H, -D, -OH, -F, -Cl, -Br, -I, -CN, -SH, -COOH, oxo, -NHC(=O)H, -C(=O)$R^{6c}$, -C(=O)O$R^{6c}$, -C(=O)N$R^{6b}R^{7b}$, -N$R^{6b}$C(=O)$R^{7b}$, -N$R^{6b}$S(=O)$_2R^{7b}$, -S(=O)$_2$N($R^{7b}$)$_2$, -N$R^{6b}$C(=O)N($R^{7b}$)$_2$, -N$R^{6b}$S(=O)$_2$N($R^{7b}$)$_2$, -OS(=O)$_2$N($R^{7b}$)$_2$, -S(=O)$_2R^{6c}$, -$C_{1-4}$ alkylene -S(=O)$_2$N($R^{7b}$)$_2$, $C_{1-6}$ alkyl, $C_{1-6}$ alkylthio, $C_{1-6}$ alkoxy, $C_{2-6}$ alkenyl, $C_{2-6}$ alkynyl, $C_{1-6}$ cyanoalkyl, $C_{1-6}$ hydroxyalkyl, $C_{1-6}$ haloalkyl, -NH($C_{1-6}$ alkyl), -N($C_{1-6}$ alkyl)$_2$, $C_{2-6}$ haloalkenyl, $C_{2-6}$ haloalkynyl, $C_{1-6}$ haloalkoxy, $C_{1-6}$ haloalkylthio, $C_{6-10}$ aryl, 5-10 membered heteroaryl, $C_{3-6}$ cycloalkyl or 3-6 membered heterocyclyl, wherein the $C_{1-6}$ alkyl, $C_{1-6}$ alkylthio, $C_{1-6}$ alkoxy, $C_{2-6}$ alkenyl, $C_{2-6}$ alkynyl, -NH($C_{1-6}$ alkyl), -N($C_{1-6}$ alkyl)$_2$, $C_{6-10}$ aryl, 5-10 membered heteroaryl, $C_{3-6}$ cycloalkyl and 3-6 membered heterocyclyl are each independently optionally substituted with 1, 2, 3 or 4 $R^8$, wherein, the $R^{6b}$, $R^{7c}$, $R^{6c}$ and $R^8$ are each as defined herein.

In some embodiments, two $R^5$ attached to the same carbon atom together form

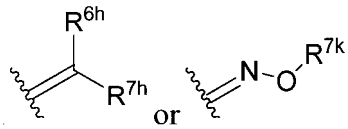,

wherein, the $R^{6h}$, $R^{7h}$ and $R^{7k}$ are each as defined herein.

In some embodiments, two $R^5$ attached to the same carbon atom together with the carbon atom to which they are attached form a $C_{3-6}$ cycloalkyl or 3-6 membered heterocyclyl, wherein the $C_{3-6}$ cycloalkyl and 3-6 membered heterocyclyl are each independently optionally substituted with 1, 2, 3 or 4 $R^8$, wherein, the $R^8$ is as defined herein;

In some embodiments, two $R^5$ attached to two adjacent atoms together with the two adjacent atoms to which they are attached form a $C_{3-6}$ cycloalkyl or 3-6 membered heterocyclyl, wherein the $C_{3-6}$ cycloalkyl and 3-6 membered heterocyclyl are each independently optionally substituted with 1, 2, 3 or 4 $R^8$, wherein, the $R^8$ is as defined herein.

In some embodiments, each $R^8$ is independently -D, -OH, -F, -Cl, -Br, -I, -CN, -$NH_2$, -C(=O)OH, -C(=O)$NH_2$, oxo, $C_{1-6}$ alkyl, $C_{1-6}$ alkoxy, $C_{1-6}$ hydroxyalkyl, $C_{6-10}$ aryl, 6-10 membered heteroaryl, -C(=O)O$C_{1-6}$ alkyl, -C(=O)NH$C_{1-6}$ alkyl or -C(=O)N($C_{1-6}$ alkyl)$_2$.

In some embodiments, ring B is $C_{6-12}$ aryl or 5-12 membered heteroaryl.

In some embodiments, each $R^2$ is independently -D, -OH, -F, -Cl, -Br, -I, -CN, -SH, -$CH_2$C(=O)N$R^{6b}R^{7b}$, -C(=O)$R^{6c}$, -C(=O)O$R^{6c}$, -C(=O)N$R^{6b}R^{7b}$, -N$R^{6b}$C(=O)$R^{7b}$, -N$R^{6b}R^{7b}$, $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, $C_{6-12}$ aryl, 5-12 membered heteroaryl, $C_{3-6}$ cycloalkyl or 3-6 membered heterocyclyl, wherein the $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, $C_{6-12}$ aryl, 5-12 membered heteroaryl, $C_{3-6}$ cycloalkyl and 3-6 membered heterocyclyl are each independently optionally substituted with 1, 2, 3 or 4 substituents selected from -D, -OH, -F, -Cl, -Br, -I, -CN, -$NH_2$, -C(=O)H, -C(=O)OH, $C_{1-6}$ alkyl, $C_{1-6}$ alkoxy, $C_{3-6}$ cycloalkyl and 3-6 membered heterocyclyl, wherein, the $R^{6b}$, $R^{7b}$ and $R^{6c}$ are each as defined herein.

In some embodiments, $R^3$ is -H, -D, -OH, -SH, -F, -Cl, -Br, -I, -CN, methyl, ethyl, n-propyl, i-propyl, n-butyl or $C_{1-4}$ haloalkyl.

In some embodiments, Y is bond, O or S.

In some embodiments, $R^4$ is 3-10 membered heterocyclyl, -L-(3-12 membered heterocyclyl), -L-($C_{3-12}$ cycloalk-

yl), -L-(5-12 membered heteroaryl) or -L-($C_{6-10}$ aryl); wherein the 3-10 membered heterocyclyl, -L-($C_{3-12}$ cycloalkyl), -L-(5-12 membered heteroaryl) and -L-($C_{6-10}$ aryl) are each independently optionally substituted with 1, 2, 3 or 4 $R^{9a}$; and the -L-(3-12 membered heterocyclyl) is optionally substituted with 1, 2, 3 or 4 $R^{9b}$, wherein, the L, $R^{9a}$ and $R^{9b}$ are each as defined herein.

In some embodiments, L is $C_{1-6}$ alkylene.

In some embodiments, wherein the $R^{9a}$ is -D, -OH, -F, -Cl, -Br, -I, -CN, -$NR^{6d}R^{7d}$, -C(=O)$NR^{6d}R^{7d}$, -$CH_2NR^{6d}R^{7d}$, -$CH_2OC$(=O)$NR^{6d}R^{7d}$, $C_{1-6}$ alkyl, $C_{1-6}$ alkoxy, $C_{1-6}$ haloalkyl, $C_{1-6}$ haloalkoxy, ($C_{6-10}$ aryl)-$C_{1-6}$ alkyl, (5-12 membered heteroaryl)-$C_{1-6}$ alkyl, (3-6 membered heterocyclyl)-$C_{1-6}$ alkyl, ($C_{3-6}$ cycloalkyl)-$C_{1-6}$ alkyl, $C_{3-6}$ cycloalkyl or 3-6 membered heterocyclyl, wherein the $C_{1-6}$ alkyl, $C_{1-6}$ alkoxy, $C_{1-6}$ haloalkyl, $C_{1-6}$ haloalkoxy, ($C_{6-10}$ aryl)-$C_{1-6}$ alkyl, (5-12 membered heteroaryl)-$C_{1-6}$ alkyl, (3-6 membered heterocyclyl)-$C_{1-6}$ alkyl, ($C_{3-6}$ cycloalkyl)-$C_{1-6}$ alkyl, $C_{3-6}$ cycloalkyl and 3-6 membered heterocyclyl are each independently optionally substituted with 1, 2, 3 or 4 substituents selected from -D, -F, -Cl, -Br, -I, -OH, -CN, -$NR^{6e}R^{7e}$, -C(=O)$C_{1-6}$ alkyl and $C_{1-6}$ alkyl, wherein, the $R^{6d}$ and $R^{7d}$ are each as defined herein.

In some embodiments, wherein the $R^{9b}$ are each independently -D, -OH, -F, -Cl, -Br, -I, -CN, -$NR^{6d}R^{7d}$, -C(=O)$NR^{6d}R^{7d}$, -$CH_2NR^{6d}R^{7d}$, -$CH_2OC$(=O)$NR^{6d}R^{7d}$, $C_{1-6}$ alkyl, $C_{1-6}$ alkoxy, $C_{1-6}$ haloalkyl, $C_{1-6}$ haloalkoxy, ($C_{6-10}$ aryl)-$C_{1-6}$ alkyl, (5-12 membered heteroaryl)-$C_{1-6}$ alkyl, (3-6 membered heterocyclyl)-$C_{1-6}$ alkyl, ($C_{3-6}$ cycloalkyl)-$C_{1-6}$ alkyl, $C_{3-6}$ cycloalkyl or 3-6 membered heterocyclyl, wherein the $C_{1-6}$ alkyl, $C_{1-6}$ alkoxy, $C_{1-6}$ haloalkyl, $C_{1-6}$ haloalkoxy, ($C_{6-10}$ aryl)-$C_{1-6}$ alkyl, (5-12 membered heteroaryl)-$C_{1-6}$ alkyl, (3-6 membered heterocyclyl)-$C_{1-6}$ alkyl, ($C_{3-6}$ cycloalkyl)-$C_{1-6}$ alkyl, $C_{3-6}$ cycloalkyl and 3-6 membered heterocyclyl are each independently optionally substituted with 1, 2, 3 or 4 substituents selected from -D, -F, -Cl, -Br, -I, -OH, -CN, -$NR^{6e}R^{7e}$, -C(=O)$C_{1-6}$ alkyl and $C_{1-6}$ alkyl, wherein, the $R^{6d}$ and $R^{7d}$ are each as defined herein.

In some embodiments, two $R^{9b}$ attached to the same carbon atom together form

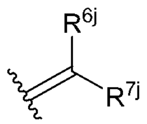

,

wherein, the $R^{6j}$ and $R^{7j}$ are each as defined herein.

In some embodiments, $R^6$, $R^7$, $R^{6b}$, $R^{7b}$, $R^{6d}$, $R^{7d}$, $R^{6c}$ and $R^{7e}$ are each independently -H, -D or $C_{1-6}$ alkyl, wherein the $C_{1-6}$ alkyl is independently optionally substituted with 1, 2, 3 or 4 substituents selected from -D, -OH, -F, -Cl, -Br, -I, -CN, -C(=O)H, -C(=O)OH, -$NR^{6g}R^{7g}$, $C_{1-6}$ alkoxy, $C_{6-12}$ aryl, $C_{3-6}$ cycloalkyl and 3-6 membered heterocyclyl;

In some embodiments, $R^6$ and $R^7$, together with the same N atom to which they are attached, form a 4-6 membered heterocyclic ring; wherein the 4-6 membered heterocyclic ring is optionally substituted with 1, 2, 3 or 4 substituents selected from -D, -OH, -F, -Cl, -Br, -I, -CN, -$NH_2$, $C_{1-6}$ alkyl, $C_{1-6}$ alkylamino, $C_{3-6}$ cycloalkyl, 3-6 membered heterocyclyl, $C_{1-6}$ alkoxy, $C_{1-6}$ cyanoalkyl, $C_{1-6}$ hydroxyalkyl, $C_{1-6}$ haloalkoxy or $C_{1-6}$ haloalkyl.

In some embodiments, $R^{6b}$ and $R^{7b}$ , together with the same N atom to which they are attached, form a 4-6 membered heterocyclic ring; wherein the 4-6 membered heterocyclic ring is optionally substituted with 1, 2, 3 or 4 substituents selected from -D, -OH, -F, -Cl, -Br, -I, -CN, -$NH_2$, $C_{1-6}$ alkyl, $C_{1-6}$ alkylamino, $C_{3-6}$ cycloalkyl, 3-6 membered heterocyclyl, $C_{1-6}$ alkoxy, $C_{1-6}$ cyanoalkyl, $C_{1-6}$ hydroxyalkyl, $C_{1-6}$ haloalkoxy or $C_{1-6}$ haloalkyl.

In some embodiments, $R^{6d}$ and $R^{7d}$, together with the same N atom to which they are attached, form a 4-6 membered heterocyclic ring; wherein the 4-6 membered heterocyclic ring is optionally substituted with 1, 2, 3 or 4 substituents selected from -D, -OH, -F, -Cl, -Br, -I, -CN, -$NH_2$, $C_{1-6}$ alkyl, $C_{1-6}$ alkylamino, $C_{3-6}$ cycloalkyl, 3-6 membered heterocyclyl, $C_{1-6}$ alkoxy, $C_{1-6}$ cyanoalkyl, $C_{1-6}$ hydroxyalkyl, $C_{1-6}$ haloalkoxy or $C_{1-6}$ haloalkyl.

In some embodiments, $R^{6e}$ and $R^{7e}$, together with the N atom to which they are attached, form a 4-6 membered heterocyclic ring; wherein the 4-6 membered heterocyclic ring is optionally substituted with 1, 2, 3 or 4 substituents selected from -D, -OH, -F, -Cl, -Br, -I, -CN, -$NH_2$, $C_{1-6}$ alkyl, $C_{1-6}$ alkylamino, $C_{3-6}$ cycloalkyl, 3-6 membered heterocyclyl, $C_{1-6}$ alkoxy, $C_{1-6}$ cyanoalkyl, $C_{1-6}$ hydroxyalkyl, $C_{1-6}$ haloalkoxy and $C_{1-6}$ haloalkyl.

In some embodiments, $R^{6a}$, $R^{6c}$, $R^{6g}$, $R^{7g}$, $R^{6h}$, $R^{7h}$, $R^{7k}$, $R^{6j}$ and $R^{7j}$ are each independently -H, -D or $C_{1-6}$ alkyl;

In some embodiments, n is 0, 1, 2, 3, 4, 5, 6 or 7.

In some embodiments, $R^1$ is -H, -D, -CN, -$NH_2$, -C(=O)H, -C(=O)OH, -C(=O)$OR^{6a}$, -C(=O)$NR^6R^7$, -$NR^6C$(=O)$R^7$, -$NR^{6a}C$(=O)$NR^6R^7$, -C(=O)$R^{6a}$, -C(=O)$OR^{6a}$, -$NR^6S(=O)_2R^7$, -$S(=O)_2NR^6R^7$, -$NR^{6a}S(=O)_2NR^6R^7$, -$NR^6R^7$, -$C_{1-4}$ alkyl $NR^6C$(=O)$R^7$, -$C_{1-4}$ alkyl $NR^6R^7$, -$C_{1-4}$ alkyl-C(=O)$OR^6$, -$C_{1-4}$ alkyl-C(=O)$NR^6R^7$, $C_{1-6}$ alkyl, $C_{1-4}$ cyanoalkyl, $C_{1-4}$ hydroxyalkyl, $C_{1-4}$ haloalkyl, $C_{1-4}$ alkoxy $C_{1-4}$ alkyl, ($C_{3-6}$ cycloalkyl)-$C_{1-4}$ alkyl, ($C_{7-12}$ cycloalkyl)-$C_{1-4}$ alkyl, (3-6 membered heterocyclyl)-$C_{1-4}$ alkyl, (7-12 membered heterocyclyl)-$C_{1-4}$ alkyl, phenyl-$C_{1-4}$ alkyl, (5-6 membered heteroaryl)-$C_{1-4}$ alkyl, ($C_{3-6}$ cycloalkyl)-O-$C_{3-4}$ alkyl, ($C_{7-12}$ cycloalkyl)-O-$C_{1-4}$ alkyl, (3-6 membered heterocyclyl)-O-$C_{1-6}$ alkyl, (3-7 membered heterocyclyl)-O-$C_{1-6}$ alkyl, (7-12 membered heterocyclyl)-O-$C_{1-4}$ alkyl, $C_{1-4}$ mercaptoalkyl, $C_{6-10}$ aryl, 5-12 membered heteroaryl, $C_{3-6}$ cycloalkyl or 3-6 membered heterocyclyl; wherein the $C_{1-4}$ alkoxy $C_{1-4}$ alkyl, ($C_{3-6}$ cycloalkyl)-$C_{1-4}$ alkyl, ($C_{7-12}$ cycloalkyl)-$C_{1-4}$ alkyl, (3-6 membered heterocyclyl)-$C_{1-4}$ alkyl, (7-12 membered heterocyclyl)-$C_{1-4}$

alkyl, phenyl-$C_{1-4}$ alkyl, (5-6 membered heteroaryl)-$C_{1-4}$ alkyl, ($C_{3-6}$ cycloalkyl)-O-$C_{1-4}$ alkyl, ($C_{7-12}$ cycloalkyl)-O-$C_{1-4}$ alkyl, (3-6 membered heterocyclyl)-O-$C_{1-6}$ alkyl, (3-7 membered heterocyclyl)-O-$C_{1-6}$ alkyl, (7-12 membered heterocyclyl)-O-$C_{1-4}$ alkyl, $C_{6-10}$ aryl, 5-12 membered heteroaryl, $C_{3-6}$ cycloalkyl and 3-6 membered heterocyclyl are each independently optionally substituted with 1, 2, 3 or 4 substituents selected from D, -OH, -F, -Cl, -Br, -I, CN, -C(=O)$OR^6$, -$NR^6R^7$, -C(=O)$NR^6R^7$, -$NR^6$C(=O)$R^7$ and $C_{1-4}$ alkyl; wherein, the $R^6$, $R^7$ and $R^{6a}$ are each as defined herein.

In other embodiments, $R^1$ is -H, -D, -CN, -$NH_2$, -C(=O)H, -C(=O)OH, -C(=O)$OR^{6a}$, -C(=O)$NR^6R^7$, -$NR^6$C(=O)$R^7$, -$NR^{6a}$C(=O)$NR^6R^7$, -C(=O)$R^{6a}$, -C(=O)$OR^{6a}$, -$NR^6$S(=O)$_2R^7$, -S(=O)$_2NR^6R^7$, -$NR^{6a}$S(=O)$_2NR^6R^7$, -$NR^6R^7$, -$CH_2NR^6$C(=O)$R^7$, -$CH_2NR^6R^7$, -$(CH_2)_2NR^6R^7$, -$CH_2$C(=O)$OR^6$, -$(CH_2)_2$C(=O)$OR^6$, -$(CH_2)_3$(=O)$OR^6$, -$CH_2$C(=O)$NR^6R^7$, -$(CH_2)_2$C(=O)$NR^6R^7$, -$(CH_2)_3$C(=O)$NR^6R^7$, -$CH_3$, -$CH_2CH_3$, -$(CH_2)_2CH_3$, -$(CH_2)_2CH(CH_3)_2$, -$(CH_2)_3CH_3$, -$C(CH_3)_3$, -$CH(CH_3)_2$, -$CH_2CH(CH_3)_2$, -$CH_2CN$, -$(CH_2)_2CN$, -$(CH_2)_3CN$, -$CH(CH_3)CN$, -$C(CH_3)_2CN$, -$CH_2OH$, -$(CH_2)_2OH$, -$(CH_2)_3OH$, -CH(OH)$CH_3$, -$(CH_2)_2CHF_2$, -$(CH_2)_2CF(CF_3)_2$, -$CF_3$, -$CHF_2$, -$CH_2F$, -$(CH_2)_2F$, -$(CH_2)_2Cl$, -$CH_2CF_3$, -$CH_2OCH_3$, -$(CH_2)_2OCH_3$, -$(CH_2)_2OCH_2CH_3$, -$CH_2OCH_2CH_3$, -$CH_2OC(CH_3)_3$, -$CH_2$-cyclopropyl, -$CH_2$-cyclobutyl, -$CH_2$-cyclopentyl, -$CH_2$-cyclohexyl, -$(CH_2)_2$-cyclopentyl, -$(CH_2)_3$-cyclopentyl, -$(CH_2)_2$-cyclohexyl, -$CH_2$-phenyl, -$CH_2$-imidazolyl, -$CH_2$-pyrazolyl, -$CH_2$O-cyclopropyl, -$CH_2$O-cyclobutyl, -$(CH_2)_2$O-cyclobutyl, -$CH_2$O-cyclopentyl, -$(CH_2)_2$O-cyclopentyl, -$CH_2$O-azetidinyl, -$CH_2$O-oxetanyl, -$CH_2$O-tetrahydrofuranyl, -$CH_2$O-pyrrolidinyl, -$CH_2$O-spiro[2.3]hexanyl, -$CH_2$O-spiro[3.3]heptanyl, -$CH_2SH$, -$(CH_2)_2SH$, phenyl, naphthyl, pyridinyl, pyrimidinyl, cyclopropyl, cyclobutyl, cyclopentyl, cyclohexyl, oxiranyl, oxetanyl, tetrahydropyranyl, azetidinyl or pyrrolidinyl; wherein the -$CH_2OCH_3$, -$(CH_2)_2OCH_3$, -$(CH_2)_2OCH_2CH_3$, -$CH_2OCH_2CH_3$, -$CH_2OC(CH_3)_3$, -$CH_2$-cyclopropyl, -$CH_2$-cyclobutyl, -$CH_2$-cyclopentyl, -$CH_2$-cyclohexyl, -$(CH_2)_2$-cyclopentyl, -$(CH_2)_3$-cyclopentyl, -$(CH_2)_2$-cyclohexyl, -$CH_2$-phenyl, -$CH_2$-imidazolyl, -$CH_2$-pyrazolyl, -$CH_2$O-cyclopropyl, -$CH_2$O-cyclobutyl, -$(CH_2)_2$O-cyclobutyl, -$CH_2$O-cyclopentyl, -$(CH_2)_2$O-cyclopentyl, -$CH_2$O-azetidinyl, -$CH_2$O-oxetanyl, -$CH_2$O-tetrahydrofuranyl, -$CH_2$O-pyrrolidinyl, -$CH_2$O-spiro[2.3]hexanyl, -$CH_2$O-spiro[3.3]heptanyl, phenyl, naphthyl, pyridinyl, pyrimidinyl, cyclopropyl, cyclobutyl, cyclopentyl, cyclohexyl, oxiranyl, oxetanyl, tetrahydropyranyl, azetidinyl and pyrrolidinyl are each independently optionally substituted with 1, 2, 3 or 4 substituents selected from D, -OH, -F, -Cl, -Br, -I, CN, -C(=O)$OR^6$, -$NR^6R^7$, -C(=O)$NR^6R^7$, -$NR^6$C(=O)$R^7$, methyl, ethyl, *n*-propyl, isopropyl, *n*-butyl, isobutyl and *tert*-butyl; wherein $R^6$, $R^7$ and $R^{6a}$ are each as defined herein.

In some embodiments, T is

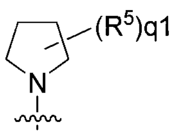, 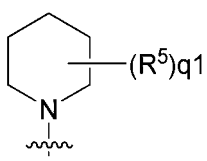, 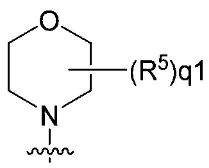, 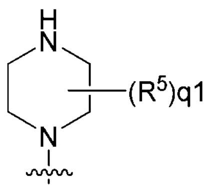,

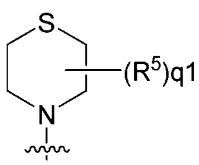, 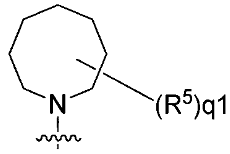, 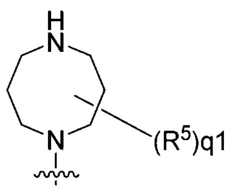, 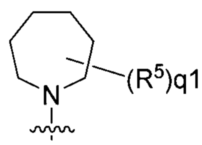, 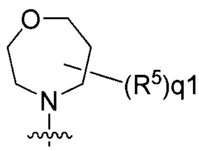,

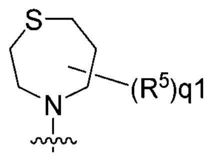 or 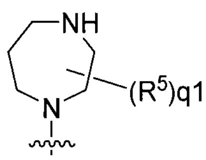,

wherein, the $R^5$ and q1 are each as defined herein.

In some embodiments, each $R^5$ is independently -H, -D, -OH, -F, -Cl, -Br, -I, -CN, -SH, -COOH, oxo, -NHC(=O)H, -C(=O)$R^{6c}$, -C(=O)$OR^{6c}$, -C(=O)$NR^{6b}R^{7b}$, -$NR^{6b}$C(=O)$R^{7b}$, -$NR^{6b}$S(=O)$_2R^{76}$, -S(=O)$_2N(R^{7b})_2$, -$NR^{6b}$C(=O)$N(R^{7b})_2$, -$NR^{6b}$S(=O)$_2N(R^{7b})_2$, -OS(=O)$_2N(R^{7b})_2$, -S(=O)$_2R^{6c}$, -$C_{1-4}$ alkylene-S(=O)$_2N(R^{7b})_2$, $C_{1-4}$ alkyl, $C_{1-4}$ alkylthio, $C_{1-4}$ alkoxy, $C_{2-4}$ alkenyl, $C_{2-4}$ alkynyl, $C_{1-4}$ cyanoalkyl, $C_{1-4}$ hydroxyalkyl, $C_{1-4}$ haloalkyl, -NH($C_{1-4}$ alkyl), -N($C_{1-4}$ alkyl)$_2$, $C_{2-4}$ haloalkenyl, $C_{2-4}$ haloalkynyl, $C_{1-4}$ haloalkoxy, $C_{1-4}$ haloalkylthio, 6-10 membered aryl, 5-10 membered heteroaryl, $C_{3-6}$ cycloalkyl or 3-6 membered heterocyclyl; wherein the $C_{1-4}$ alkyl, $C_{1-4}$ alkylthio, $C_{1-4}$ alkoxy, $C_{2-4}$ alkenyl, $C_{2-4}$ alkynyl, -NH($C_{1-4}$ alkyl), -N($C_{1-4}$ alkyl)$_2$, 6-10 membered aryl, 5-10 membered heteroaryl, $C_{3-6}$ cycloalkyl and 3-6 membered heterocyclyl are each independently optionally substituted with 1, 2, 3 or 4 $R^8$, wherein, the $R^{6b}$, $R^{7c}$, $R^{6c}$ and $R^8$ are each

as defined herein.

In some embodiments, two $R^5$ attached to the same carbon atom together form

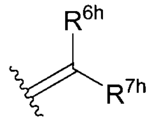 or 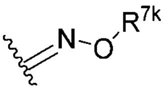 ,

wherein, the $R^{6h}$, $R^{7h}$ and $R^{7k}$ are each as defined herein.

In some embodiments, two $R^5$ attached to the same carbon atom together with the carbon atom to which they are attached form a $C_{3-6}$ cycloalkyl or 3-6 membered heterocyclyl, wherein the $C_{3-6}$ cycloalkyl and 3-6 membered heterocyclyl are each independently optionally substituted with 1, 2, 3 or 4 $R^8$, wherein, the $R^8$ is as defined herein.

In some embodiments, two $R^5$ attached to two adjacent atoms together with the two adjacent atoms to which they are attached form a $C_{3-6}$ cycloalkyl or 3-6 membered heterocyclyl, wherein the $C_{3-6}$ cycloalkyl and 3-6 membered heterocyclyl are each independently optionally substituted with 1, 2, 3 or 4 $R^8$, wherein, the $R^8$ is as defined herein.

In some embodiments, each $R^8$ is independently -D, -OH, -F, -Cl, -Br, -I, -CN, $-NH_2$, -C(=O)OH, $-C(=O)NH_2$, oxo, $C_{1-4}$ alkyl, $C_{1-4}$ alkoxy, $C_{1-4}$ hydroxyalkyl, $C_{6-10}$ aryl, 6-10 membered heteroaryl, $-C(=O)OC_{1-4}$ alkyl, $-C(=O)NHC_{1-4}$ alkyl or $-C(=O)N(C_{1-4}$ alkyl$)_2$;

In some embodiments, $R^{6h}$, $R^{7h}$ and $R^{7k}$ are each independently -H, -D or $C_{1-4}$ alkyl.

In some embodiments, each $R^5$ is independently -H, -D, -OH, -F, -Cl, -Br, -I, -CN, -SH, -COOH, oxo, -NH(C=O)H, $-C(=O)R^{6c}$, $-C(=O)OR^{6c}$, $-C(=O)NR^{6b}R^{7b}$, $-NR^{6b}C(=O)R^{7b}$, $-NR^{6b}S(=O)_2R^{7b}$, $-S(=O)_2N(R^{7b})_2$, $-NR^{6b}C(=O)N(R^{7b})_2$, $-NR^{6b}S(=O)_2N(R^{7b})_2$, $-OS(=O)_2N(R^{7b})_2$, $-S(=O)_2R^{6c}$, $-CH_2S(=O)_2N(R^{7b})_2$, $-(CH_2)_2S(=O)_2N(R^{7b})_2$, $-CH_3$, $-CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-S(CH_2)_2CH_3$, $-SCH_2CH(CH_3)_2$, $-SCH(CH_3)_2$, $-OCH_3$, $-OCH_2CH_3$, $-O(CH_2)_2CH_3$, $-OCH_2CH(CH_3)_2$, $-OCH(CH_3)_2$, $-CH=CH_2$, $-CH=CHCH_3$, $-CH_2CH=CH_2$, -C≡CH, $-C≡CCH_3$, $-CH_2C≡CH$, $-CH_2CN$, $-(CH_2)_2CN$, $-(CH_2)_3CN$, $-CH_2OH$, $-(CH_2)_2OH$, $-(CH_2)_3OH$, $-CH(OH)CH_3$, $-CF_3$, $-CHF_2$, $-CH_2F$, $-(CH_2)_2F$, $-(CH_2)_2Cl$, $-CH_2CF_3$, $-NHCH_3$, $-NH(CH_2CH_3)$, $-NH((CH_2)_2CH_3)$, $-NH((CH_2)_3CH_3)$, $-NH(CH(CH_3)_2)$, $-N(CH_3)_2$, $-N(CH_2CH_3)_2$, $-N((CH_2)_2CH_3)_2$, $-CHFCH=CH_2$, -CH=CHF, -CH=CHCl, $-CH=CHCH_2F$, $-C≡CCH_2F$, $-OCF_3$, $-OCH_2F$, $-OCHF_2$, $-OCH_2CF_3$, $-OCH_2CHF_2$, $-SCF_3$, $-SCH_2F$, $-SCHF_2$, $-SCH_2CF_3$, $-SCH_2CHF_2$, phenyl, furyl, imidazolyl, isoxazolyl, oxazolyl, pyrrolyl, pyrazolyl, pyridinyl, pyrimidinyl, pyridazinyl, pyrazinyl, thiophenyl, thiazolyl, triazolyl, tetrazolyl, benzopyridinyl, benzimidazolyl, benzopyrrolyl, benzopyrazolyl, cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl, wherein the $-CH_3$, $-CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-S(CH_2)_2CH_3$, $-SCH_2CH(CH_3)_2$, $-SCH(CH_3)_2$, $-OCH_3$, $-OCH_2CH_3$, $-O(CH_2)_2CH_3$, $-OCH_2CH(CH_3)_2$, $-OCH(CH_3)_2$, $-CH=CH_2$, $-CH=CHCH_3$, $-CH_2CH=CH_2$, -C≡CH, $-C≡CCH_3$, $-CH_2C≡CH$, $-CH_2CN$, $-(CH_2)_2CN$, $-(CH_2)_3CN$, $-CH_2OH$, $-(CH_2)_2OH$, $-(CH_2)_3OH$, $-CH(OH)CH_3$, $-CHF_2$, $-CH_2F$, $-(CH_2)_2F$, $-(CH_2)_2Cl$, $-CH_2CF_3$, $-NHCH_3$, $-NH(CH_2CH_3)$, $-NH((CH_2)_2CH_3)$, $-NH((CH_2)_3CH_3)$, $-NH(CH(CH_3)_2)$, $-N(CH_3)_2$, $-N(CH_2CH_3)_2$, $-N((CH_2)_2CH_3)_2$, $-CHFCH=CH_2$, -CH=CHF, -CH=CHCl, $-CH=CHCH_2F$, $-C≡CCH_2F$, $-OCH_2F$, $-OCHF_2$, $-OCH_2CF_3$, $-OCH_2CHF_2$, $-SCH_2F$, $-SCHF_2$, $-SCH_2CF_3$, $-SCH_2CHF_2$, phenyl, furyl, imidazolyl, isoxazolyl, oxazolyl, pyrrolyl, pyrazolyl, pyridinyl, pyrimidinyl, pyridazinyl, pyrazinyl, thiophenyl, thiazolyl, triazolyl, tetrazolyl, benzopyridinyl, benzimidazolyl, benzopyrrolyl, benzopyrazolyl, cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl are each independently optionally substituted with 1, 2, 3 or 4 $R^8$, wherein, the $R^{6b}$, $R^{7c}$, $R^{6c}$ and $R^8$ are each as defined herein.

In some embodiments, two $R^5$ attached to the same carbon atom together with the carbon atom to which they are attached form a cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein the cyclopropyl, cyclobutyl, cyclopentyl, cyclohexyl, oxiranyl, aziridinyl, azetidinyl, oxetanyl, thietanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl are each independently optionally substituted with 1, 2, 3 or 4 $R^8$,wherein the $R^8$ is as defined herein.

In some embodiments, two $R^5$ attached to two adjacent atoms together with the two adjacent atoms to which they are attached form a cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl are each independently optionally substituted with 1, 2, 3 or 4 $R^8$,wherein the $R^8$ is as defined herein.

In some embodiments, each $R^8$ is independently -D, -OH, -F, -Cl, -Br, -I, -CN, $-NH_2$, -C(=O)OH, $-C(=O)NH_2$, oxo,

$-CH_3$, $-CH_2CH_3$, $-(CH_2)_2CH_3$, $-(CH_2)_3CH_3$, $-CH(CH_3)_2$, $-CH_2CH(CH_3)_2$, $-C(CH_3)_3$, $-OCH_3$, $-OCH_2CH_3$, $-O(CH_2)_2CH_3$, $-CH_2OH$, $-(CH_2)_2OH$, $-(CH_2)_3OH$, $-CH(OH)CH_3$, phenyl, furyl, imidazolyl, isoxazolyl, oxazolyl, pyrrolyl, pyrazolyl, pyridinyl, pyrimidinyl, pyridazinyl, pyrazinyl, thiophenyl, thiazolyl, triazolyl, tetrazolyl, benzopyridinyl, benzimidazolyl, benzopyrrolyl, benzopyrazolyl, $-C(=O)OCH_3$, $-C(=O)OCH_2CH_3$, $-C(=O)O(CH_2)_2CH_3$, $-C(=O)OCH(CH_3)_2$, $-C(=O)NHCH_3$, $-C(=O)NHCH_2CH_3$, $-C(=O)N(CH_3)_2$ or $-C(=O)N(CH_3)CH_2CH_3$.

In some embodiments, $R^{6h}$, $R^{7h}$ and $R^{7k}$ are each independently -H, -D, methyl, ethyl, *n*-propyl, isopropyl, *n*-butyl, isobutyl or *tert-butyl.*

In some embodiments, T is

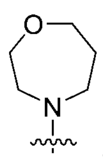 , 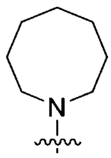 , 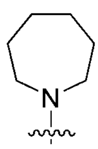 , 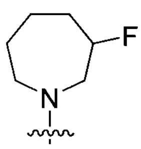 , 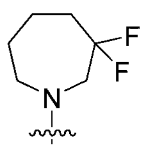 , 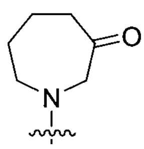 ,

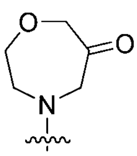 , 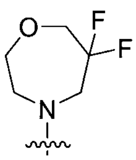 , 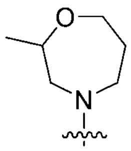 , 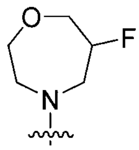 , 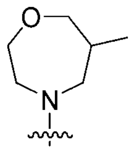 , 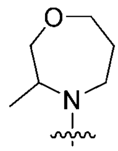 , 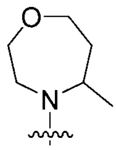 , 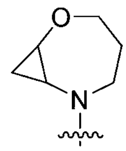 ,

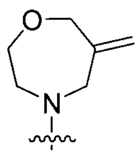 , 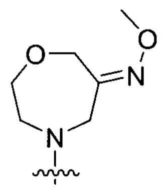 , 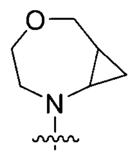 or 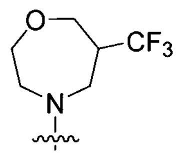 .

In some embodiments, ring B is one of the following sub-structures:

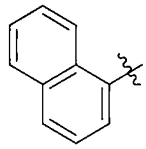 , 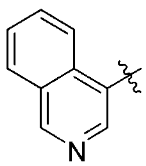 , 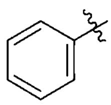 , 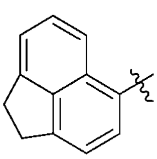 , 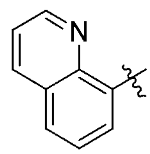 , 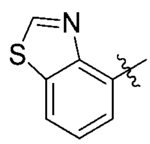 , 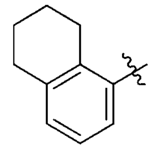 ,

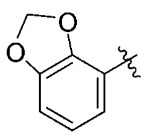 , 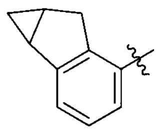 , 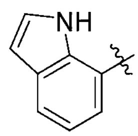 , 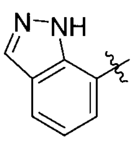 , 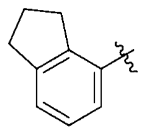 , 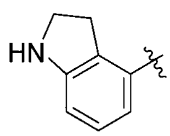 , 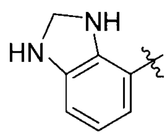 ,

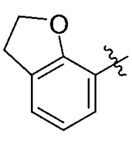 , 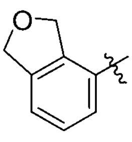 , 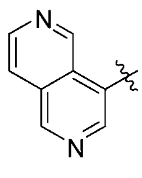 , 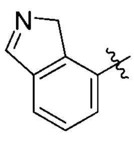 , 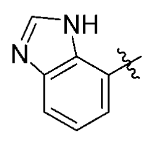 , 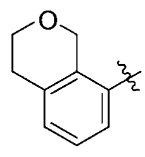 , 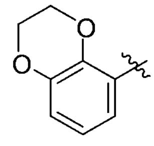 or

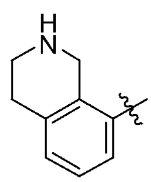 .

In some embodiments, each $R^2$ is independently -D, -OH, -F, -Cl, -Br, -I, -CN, -SH, $-CH_2C(=O)NR^{6b}R^{7b}$, $-C(=O)R^{6c}$, $-C(=O)OR^{6c}$, $-C(=O)NR^{6b}R^{7b}$, $-NR^{6b}C(=O)R^{7b}$, $-NR^{6b}R^{7b}$, $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, $C_{6-10}$ aryl, 5-12 membered heteroaryl, $C_{3-6}$ cycloalkyl or 3-6 membered heterocyclyl, wherein the $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, $C_{6-10}$ aryl, 5-12 membered heteroaryl, $C_{3-6}$ cycloalkyl and 3-6 membered heterocyclyl are each independently optionally substituted with 1, 2, 3 or 4 substituents selected from -D, -OH, -F, -Cl, -Br, -I, -CN, $-NH_2$, -C(=O)H, -C(=O)OH, $C_{1-4}$ alkyl, $C_{1-4}$ alkoxy, $C_{3-6}$ cycloalkyl and 3-6 membered heterocyclyl; wherein, the $R^{6b}$, $R^{7b}$ and $R^{6c}$ are each as defined herein.

In some embodiments, each $R^2$ is independently -D, -OH, -F, -Cl, -Br, -I, -CN, -SH, $-CH_2C(=O)NR^{6b}R^{6b}$, $-C(=O)R^{6c}$, $-C(=O)OR^{6c}$, $-C(=O)NR^{6b}R^{7b}$, $-NR^{6b}C(=O)R^{7b}$, $-NR^{6b}R^{7b}$, $-CH_3$, $-CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-CH=CH_2$, $-CH=CHCH_3$, $-CH_2CH=CH_2$, -C=CH, $-C\equiv CCH_3$, $-CH_2C\equiv CH$, $-C\equiv CCH_2OH$, $-C\equiv C(CH_2)_2OH$, $-OCH_3$, $-OCH_2CH_3$, $-O(CH_2)_2CH_3$, $-CH_2CN$, $-(CH_2)_2CN$, $-(CH_2)_3CN$, $-CH_2OH$, $-(CH_2)_2OH$, $-(CH_2)_3OH$, $-CH(OH)CH_3$, $-(CH_2)_2F$, $-CH_2CHF_2$, $-CF_3$, $-CH_2CF_3$, $-CHF_2$, $-CH_2F$, $-(CH_2)_2Cl$, -CH=CHF, -CH=CHCl, $-CH=CHCH_2F$, $-C\equiv CCH_2F$, $-C\equiv C(CH_2)_2F$, -C=CF, $-OCF_3$, $-OCHF_2$, $-OCH_2CHF_2$, $-OCH_2CF_3$, $-OCHClCHCl_2$, $-OCH_2CH_2F$, $-SCF_3$, $-SCH_2CF_3$, $-SCH_2CHF_2$, phenyl, naphthyl, pyridinyl, pyrimidinyl, cyclopropyl, cyclobutyl, cyclopentyl, cyclohexyl, pyrrolidinyl, oxazolidinyl, tetrahydrofuranyl, piperidyl or piperazinyl, wherein the $-CH_3$, $-CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-CH=CH_2$, $-CH=CHCH_3$, $-CH_2CH=CH_2$, -C=CH, $-C\equiv CCH_3$, $-CH_2C\equiv CH$, $-C\equiv CCH_2OH$, $-C\equiv C(CH_2)_2OH$, $-OCH_3$, $-OCH_2CH_3$, $-O(CH_2)_2CH_3$, $-CH_2CN$, $-(CH_2)_2CN$, $-(CH_2)_3CN$, $-CH_2OH$, $-(CH_2)_2OH$, $-(CH_2)_3OH$, $-CH(OH)CH_3$, $-(CH_2)_2F$, $-CH_2CHF_2$, $-CH_2CF_3$, $-CHF_2$, $-CH_2F$, $-(CH_2)_2Cl$, -CH=CHF, -CH=CHCl, $-CH=CHCH_2F$, $-C\equiv CCH_2F$, $-C\equiv C(CH_2)_2F$, $-OCHF_2$, $-OCH_2CHF_2$, $-OCH_2CF_3$, $-OCHClCHCl_2$, $-OCH_2CH_2F$, $-SCH_2CF_3$, $-SCH_2CHF_2$, phenyl, naphthyl, pyridinyl, pyrimidinyl, cyclopropyl, cyclobutyl, cyclopentyl, cyclohexyl, pyrrolidinyl, oxazolidinyl, tetrahydrofuranyl, piperidyl and piperazinyl are each independently optionally substituted with 1, 2, 3 or 4 substituents selected from -D, -OH, -F, -Cl, -Br, -I, -CN, $-NH_2$, -C(=O)H, -C(=O)OH, methyl, ethyl, n-propyl, isopropyl, methoxy, ethoxy, n-propoxy, i-propoxy, cyclopropyl, cyclobutyl, cyclopentyl, cyclohexyl, pyrrolidinyl, oxazolidinyl, tetrahydrofuranyl, piperidyl and piperazinyl; wherein $R^{6b}$, $R^{6c}$ and $R^{7b}$ are as defined herein; wherein, the $R^{6b}$, $R^{7b}$ and $R^{6c}$ are each as defined herein.

In some embodiments,

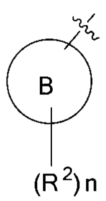 is 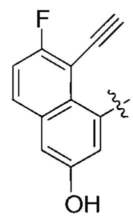, 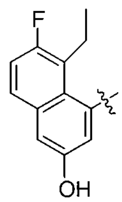, 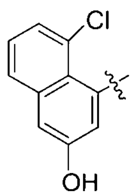, 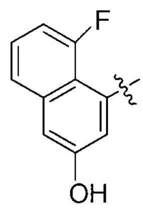, 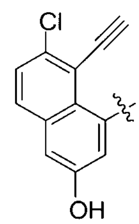,

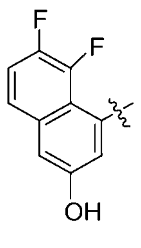 or 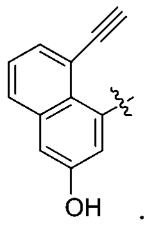.

In some embodiments, $R^4$ is a 3-6 membered heterocyclyl,

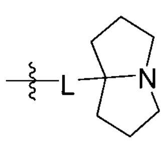, 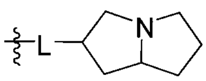,

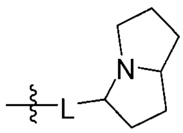, 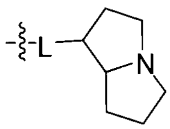, 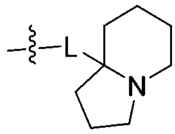, 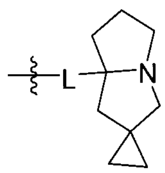,

-L-pyrrolidinyl, -L-piperidyl, -L-morpholinyl, -L-oxetanyl, -L-oxiranyl, -L-tetrahydrofuranyl, -L-octahydroindolizinyl, -L-cyclopropyl, -L-cyclopentyl, -L-octahydropentalenyl, -L-octahydro-1*H*-indenyl, -L-decalinyl, -L-pyridinyl, -L-pyrazolyl or -L-phenyl; wherein the 3-6 membered heterocyclyl, -L-cyclopropyl, -L-cyclopentyl, -L-octahydropentalenyl, -L-octahydro-1*H*-indenyl, -L-decalinyl, -L-pyridinyl, -L-pyrazolyl and -L-phenyl are each independently optionally substituted with 1, 2, 3 or 4 $R^{9a}$; and

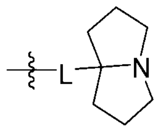, 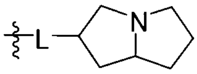, 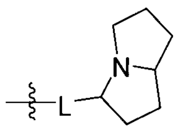, 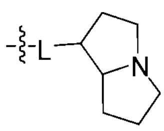, 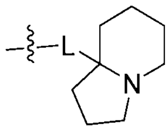, 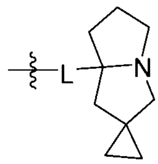,

-L-pyrrolidinyl, -L-piperidinyl, -L-morpholinyl, -L-oxetanyl, -L-oxiranyl, -L-tetrahydrofuranyl and -L-octahydroindolizinyl are each optionally substituted with 1, 2, 3 or 4 $R^{9b}$,wherein, the L, $R^{9a}$ and $R^{9b}$ are each as defined herein.

In some embodiments, L is $C_{1-4}$ alkylene.

In some embodiments, the $R^{9a}$ is -D, -OH, -F, -Cl, -Br, -I, -CN, $-NR^{6d}R^{7d}$, $-C(=O)NR^{6d}R^{7d}$, $-CH_2NR^{6d}R^{7d}$, $-CH_2OC(=O)NR^{6d}R^{7d}$, $C_{1-4}$ alkyl, $C_{1-4}$ alkoxy, $C_{1-4}$ haloalkyl, $C_{1-4}$ haloalkoxy, phenyl-$C_{1-4}$ alkyl, (5-6 membered heteroaryl)-$C_{1-4}$ alkyl, (3-6 membered heterocyclyl)-$C_{1-4}$ alkyl, ($C_{3-6}$ cycloalkyl)-$C_{1-4}$ alkyl, $C_{3-6}$ cycloalkyl or 3-6 membered heterocyclyl, wherein the $C_{1-4}$ alkyl, $C_{1-4}$ alkoxy, $C_{1-4}$ haloalkyl, $C_{1-4}$ haloalkoxy, phenyl-$C_{1-4}$ alkyl, (5-6 membered heteroaryl)-$C_{1-4}$ alkyl, (3-6 membered heterocyclyl)-$C_{1-4}$ alkyl, ($C_{3-6}$ cycloalkyl)-$C_{1-4}$ alkyl, $C_{3-6}$ cycloalkyl and 3-6 membered heterocyclyl are each independently optionally substituted with 1, 2, 3 or 4 substituents selected from -D, -F, -Cl, -Br, -I, -OH, -CN, $-NR^{6e}R^{7e}$, $-C(=O)C_{1-4}$ alkyl and $C_{1-4}$ alkyl, wherein, the $R^{6d}$, $R^{7d}$, $R^{6e}$ and $R^{7e}$ are each as defined herein.

In some embodiments, $R^{9b}$ is -D, -OH, -F, -Cl, -Br, -I, -CN, $-NR^{6d}R^{7d}$, $-C(=O)NR^{6d}R^{7d}$, $-CH_2NR^{6d}R^{7d}$, $-CH_2O-C(=O)NR^{6d}R^{7d}$, $C_{1-4}$ alkyl, $C_{1-4}$ alkoxy, $C_{1-4}$ haloalkyl, $C_{1-4}$ haloalkoxy, phenyl-$C_{1-4}$ alkyl, (5-6 membered heteroaryl)-$C_{1-4}$ alkyl, (3-6 membered heterocyclyl)-$C_{1-4}$ alkyl, ($C_{3-6}$ cycloalkyl)-$C_{1-4}$ alkyl, $C_{3-6}$ cycloalkyl or 3-6 membered heterocyclyl, wherein the $C_{1-4}$ alkyl, $C_{1-4}$ alkoxy, $C_{1-4}$ haloalkyl, $C_{1-4}$ haloalkoxy, phenyl-$C_{1-4}$ alkyl, (5-6 membered heteroaryl)-$C_{1-4}$ alkyl, (3-6 membered heterocyclyl)-$C_{1-4}$ alkyl, ($C_{3-6}$ cycloalkyl)-$C_{1-4}$ alkyl, $C_{3-6}$ cycloalkyl and 3-6 membered heterocyclyl are each independently optionally substituted with 1, 2, 3 or 4 substituents selected from -D, -F, -Cl, -Br, -I, -OH, -CN, $-NR^{6e}R^{7e}$, $-C(=O)C_{1-4}$ alkyl and $C_{1-4}$ alkyl, wherein, the $R^{6d}$ and $R^{7d}$ are each as defined herein.

In some embodiments, two $R^{9b}$ attached to the same carbon atom together form

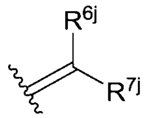

or, wherein, the $R^{6j}$ and $R^{7j}$ are each as defined herein. In some embodiments, $R^{6j}$ and $R^{7j}$ each are independently -H, -D or $C_{1-4}$ alkyl.

In some embodiments,

$R^4$ is piperidyl, piperazinyl, pyrrolidinyl, imidazolidinyl,

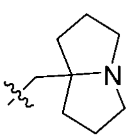, 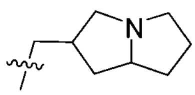,

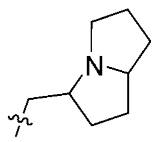, 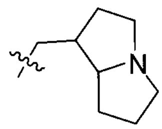, 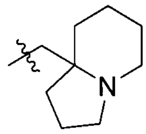, 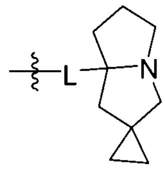,

$-CH_2$-pyrrolidinyl, $-CH_2$-morpholinyl, $-(CH_2)_2$-morpholinyl, $-CH_2$-oxetanyl, $-CH_2$-oxiranyl, $-CH_2$-tetrahydrofuranyl, $-CH_2$-octahydroindolizinyl, $-CH_2$-cyclopropyl, $-CH_2$-cyclopentyl, $-CH_2$-octahydropentalenyl, $-CH_2$-octahydro-1*H*-indenyl, $-CH_2$-decahydronaphthalenyl, $-CH_2$-pyridinyl, $-(CH_2)_2$-pyridinyl, $-CH_2$-pyrazolyl, $-(CH_2)_2$-pyrazolyl or $-CH_2$-phenyl, wherein the piperidyl, piperazinyl, pyrrolidinyl, imidazolidinyl, $-CH_2$-cyclopropyl, $-CH_2$-cyclopentyl, $-CH_2$-octahydropentalenyl, $-CH_2$-octahydro-1*H*-indenyl, $-CH_2$-decahydronaphthalenyl, $-CH_2$-pyridinyl, $-(CH_2)_2$-pyridinyl, $-CH_2$-pyrazolyl, $-(CH_2)_2$-pyrazolyl and $-CH_2$-phenyl are each independently optionally substituted with 1, 2, 3 or 4 $R^{9a}$; and the

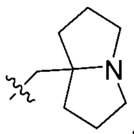, 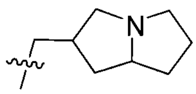, 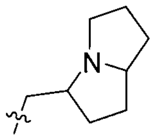, 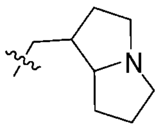, 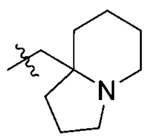,

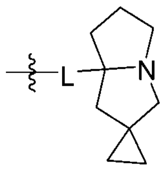,

$-CH_2$-pyrrolidinyl, $-CH_2$-morpholinyl, $-(CH_2)_2$-morpholinyl, $-CH_2$-oxetanyl, $-CH_2$-oxiranyl, $-CH_2$-tetrahydrofuranyl and $-CH_2$-octahydroindolizinyl are each optionally substituted with 1, 2, 3 or 4 $R^{9b}$, wherein, the L, $R^{9a}$ and $R^{9b}$ are each as defined herein.

In some embodiments, $R^{9a}$ is -D, -OH, -F, -Cl, -Br, -I, -CN, $-NR^{6d}R^{7d}$ $-C(=O)NR^{6d}R^{7d}$, $-CH_2NR^{6d}R^{7d}$, $-CH_2O-C(=O)NR^{6d}R^{7d}$, methyl, ethyl, *n*-propyl, isopropyl, *n*-butyl, isobutyl, *tert*-butyl, methoxy, ethoxy, isopropoxy, $-CHF_2$, $-CF_3$, $-OCF_3$, benzyl, pyridinylmethyl, pyrazolylmethyl, morpholinylmethyl, pyrrolidinylmethyl, piperazinylmethyl, azetidinylmethyl, piperidinylmethyl, tetrahydropyranylmethyl, cyclopropylmethyl, cyclopentylmethyl, cyclohexylmethyl, cyclopentyl, cyclohexyl, morpholinyl, piperidinyl, pyrrolidinyl, piperazinyl or azetidinyl, wherein the methyl, ethyl, *n*-propyl, isopropyl, *n*-butyl, isobutyl, *tert-butyl,* methoxy, ethoxy, isopropoxy, $-CHF_2$, benzyl, pyridinylmethyl, pyrazolylmethyl, morpholinylmethyl, pyrrolidinylmethyl, piperazinylmethyl, azetidinylmethyl, piperidinylmethyl, tetrahydropyranylmethyl, cyclopropylmethyl, cyclopentylmethyl, cyclohexylmethyl, cyclopentyl, cyclohexyl, morpholinyl, piperidinyl, pyrrolidinyl, piperazinyl and azetidinyl are each independently optionally substituted with 1, 2, 3 or 4 substituents selected from -D, -F, -Cl, -Br, -I, -OH, -CN, $-NH_2$, $-NHCH_3$, $-N(CH_3)_2$, $-NHCH_2CH_3$, $-C(=O)CH_3$, $-C(=O)CH_2CH_3$, methyl, ethyl, *n*-propyl and isopropyl;

In some embodiments, $R^{9b}$ is -D, -OH, -F, -Cl, -Br, -I, -CN, $-NR^{6d}R^{7d}$, $-C(=O)NR^{6d}R^{7d}$, $-CH_2NR^{6d}R^{7d}$, $-CH_2O-C(=O)NR^{6d}R^{7d}$, methyl, ethyl, *n*-propyl, isopropyl, *n*-butyl, isobutyl, *tert-butyl,* methoxy, ethoxy, isopropoxy, $-CHF_2$, $-CF_3$, $-OCF_3$, benzyl, pyridinylmethyl, pyrazolylmethyl, morpholinylmethyl, pyrrolidinylmethyl, piperazinylmethyl, azetidinylmethyl, piperidinylmethyl, tetrahydropyranylmethyl, cyclopropylmethyl, cyclopentylmethyl, cyclohexylmethyl, cyclopentyl, cyclohexyl, morpholinyl, piperidinyl, pyrrolidinyl, piperazinyl or azetidinyl, wherein the methyl, ethyl, *n*-propyl, isopropyl, *n*-butyl, isobutyl, *tert*-butyl, methoxy, ethoxy, isopropoxy, $-CHF_2$, benzyl, pyridinylmethyl, pyrazolylmethyl, morpholinylmethyl, pyrrolidinylmethyl, piperazinylmethyl, azetidinylmethyl, piperidinylmethyl, tetrahydropyranylmethyl, cyclopropylmethyl, cyclopentylmethyl, cyclohexylmethyl, cyclopentyl, cyclohexyl, morpholinyl, piperidinyl, pyrrolidinyl, piperazinyl and azetidinyl are each independently optionally substituted with 1, 2, 3 or 4 substituents selected from -D, -F, -Cl, -Br, -I, -OH, -CN, $-NH_2$, $-NHCH_3$, $-N(CH_3)_2$, $-NHCH_2CH_3$, $-C(=O)CH_3$, $-C(=O)CH_2CH_3$, methyl, ethyl, n-propyl and isopropyl, wherein, the $R^{6d}$ and $R^{7d}$ are each as defined herein.

In some embodiments, two $R^{9b}$ attached to the same carbon atom together form

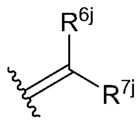.

In some embodiments, $R^{6j}$ and $R^{7j}$ each are independently -H, -D, methyl, ethyl, *n*-propyl, isopropyl, *n*-butyl, isobutyl or *tert-butyl.*

In some embodiments, $R^6$, $R^7$, $R^{6b}$, $R^{7b}$, $R^{6a}$, $R^{7d}$, $R^{6c}$ and $R^{7c}$ are each independently -H, -D or $C_{1-4}$ alkyl, wherein the $C_{1-4}$ alkyl is independently optionally substituted with 1, 2, 3 or 4 substituents selected from -D, -OH, -F, -Cl, -Br, -I, -CN, -C(=O)H, -C(=O)OH, $-NR^{6g}R^{7g}$, $C_{1-4}$ alkyl, $C_{6-10}$ aryl, $C_{3-6}$ cycloalkyl and 3-6 membered heterocyclyl.

In some embodiments, $R^6$ and $R^7$ are taken together with the N atom to which they are attached to form a 4-6 membered heterocyclic ring; wherein the 4-6 membered heterocyclic ring is optionally substituted with 1, 2, 3 or 4 substituents selected from -D, -OH, -F, -Cl, -Br, -I, -CN, $-NH_2$, $C_{1-4}$ alkyl, $C_{1-4}$ alkylamino, $C_{3-6}$ cycloalkyl, 3-6 membered heterocyclyl, $C_{1-4}$ alkoxy, $C_{1-4}$ cyanoalkyl, $C_{1-4}$ hydroxyalkyl, $C_{1-4}$ haloalkoxy or $C_{1-4}$ haloalkyl.

In some embodiments, $R^{6b}$ and $R^{7b}$, together with the same N atom to which they are attached, form a 4-6 membered heterocyclic ring; wherein the 4-6 membered heterocyclic ring is optionally substituted with 1, 2, 3 or 4 substituents selected from -D, -OH, -F, -Cl, -Br, -I, -CN, $-NH_2$, $C_{1-4}$ alkyl, $C_{1-4}$ alkylamino, $C_{3-6}$ cycloalkyl, 3-6 membered heterocyclyl, $C_{1-4}$ alkoxy, $C_{1-4}$ cyanoalkyl, $C_{1-4}$ hydroxyalkyl, $C_{1-4}$ haloalkoxy or $C_{1-4}$ haloalkyl.

In some embodiments, $R^{6d}$ and $R^{7d}$, together with the same N atom to which they are attached, form a 4-6 membered heterocyclic ring; wherein the 4-6 membered heterocyclic ring is optionally substituted with 1, 2, 3 or 4 substituents selected from -D, -OH, -F, -Cl, -Br, -I, -CN, $-NH_2$, $C_{1-4}$ alkyl, $C_{1-4}$ alkylamino, $C_{3-6}$ cycloalkyl, 3-6 membered heterocyclyl, $C_{1-4}$ alkoxy, $C_{1-4}$ cyanoalkyl, $C_{1-4}$ hydroxyalkyl, $C_{1-4}$ haloalkoxy or $C_{1-4}$ haloalkyl.

In some embodiments, $R^{6e}$ and $R^{7e}$, together with the same N atom to which they are attached, form a 4-6 membered heterocyclic ring; wherein the 4-6 membered heterocyclic ring is optionally substituted with 1, 2, 3 or 4 substituents selected from -D, -OH, -F, -Cl, -Br, -I, -CN, $-NH_2$, $C_{1-4}$ alkyl, $C_{1-4}$ alkylamino, $C_{3-6}$ cycloalkyl, 3-6 membered heterocyclyl, $C_{1-4}$ alkoxy, $C_{1-4}$ cyanoalkyl, $C_{1-4}$ hydroxyalkyl, $C_{1-4}$ haloalkoxy or $C_{1-4}$ haloalkyl.

In some embodiments, $R^{6a}$, $R^{6c}$, $R^{6g}$ and $R^{7g}$ each are independently -H, -D or $C_{1-4}$ alkyl.

In some embodiments, $R^4$ is

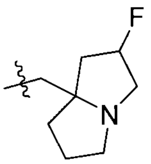 , 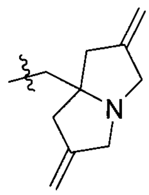 , 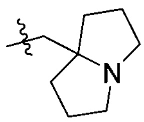 , 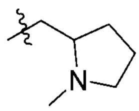 , 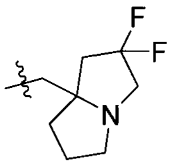 ,

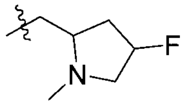 or 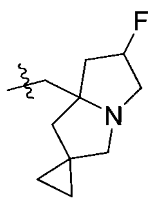 .

In some embodiments, $R^4$ is

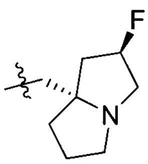 , 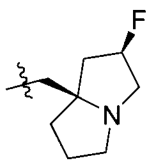 , 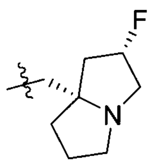 , 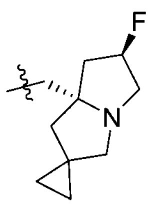 , 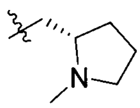 ,

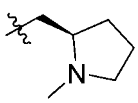 or 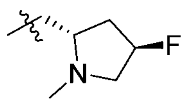 .

In some embodiments, $R^6$, $R^7$, $R^{6b}$, $R^{7b}$, $R^{6d}$, $R^{7d}$ , $R^{6e}$ and $R^{7e}$ each are independently -H, -D, methyl, ethyl, *n*-propyl, isopropyl, *n*-butyl, isobutyl or *tert*-butyl, wherein the methyl, ethyl, *n*-propyl, isopropyl, *n*-butyl, isobutyl and tert-butyl are each independently optionally substituted with 1, 2, 3 or 4 substituents selected from -D, -OH, -F, -Cl, -Br, -I, -CN, C(=O)H, -C(=O)OH, $-NR^{6g}R^{7g}$, methoxy, ethoxy, n-propoxy, isopropoxy, isobutoxy, cyclopropyl, cyclobutyl, cyclopentyl,

cyclohexyl, phenyl, oxiranyl, oxetanyl, azetidinyl and pyrrolidinyl;

In some embodiments, $R^6$ and $R^7$ together with the same nitrogen atom to which they are attached form pyrrolidine, piperazine, piperidine, morpholinyl, oxazolidine or imidazolidinel, wherein the pyrrolidine, piperazine, piperidine, morpholinyl, oxazolidine and imidazolidine are each independently optionally substituted with 1, 2, 3 or 4 substituents selected from -D, -OH, -F, -Cl, -Br, -I, -CN, $-NH_2$, methyl, ethyl, *n*-propyl, isopropyl, *n*-butyl, isobutyl, *tert*-butyl, methylamino, dimethylamino, ethylamino, cyclopropyl, cyclopentyl, pyrrolidinyl, methoxy, ethoxy, isopropoxy, cyanomethyl, hydroxymethyl, hydroxyethyl, trifluoromethoxy, monofluoromethyl, difluoromethyl, trifluoromethyl or 1,2-dichloroethyl.

In some embodiments, $R^{6b}$ and $R^{7b}$ together with the same nitrogen atom to which they are attached form pyrrolidine, piperazine, piperidine, morpholinyl, oxazolidine or imidazolidine, wherein the pyrrolidine, piperazine, piperidine, morpholinyl, oxazolidine and imidazolidine are each independently optionally substituted with 1, 2, 3 or 4 substituents selected from -D, -OH, -F, -Cl, -Br, -I, -CN, $-NH_2$, methyl, ethyl, n-propyl, isopropyl, n-butyl, isobutyl, tert-butyl, methylamino, dimethylamino, ethylamino, cyclopropyl, cyclopentyl, pyrrolidinyl, methoxy, ethoxy, isopropoxy, cyanomethyl, hydroxymethyl, hydroxyethyl, trifluoromethoxy, fluoromethyl, difluoromethyl, trifluoromethyl or 1,2-dichloroethyl.

In some embodiments, $R^{6d}$ and $R^{7d}$ together with the same nitrogen atom to which they are attached form pyrrolidine, piperazine, piperidine, morpholinyl, oxazolidine or imidazolidine, wherein the pyrrolidine, piperazine, piperidine, morpholinyl, oxazolidine and imidazolidine are each independently optionally substituted with 1, 2, 3 or 4 substituents selected from -D, -OH, -F, -Cl, -Br, -I, -CN, $-NH_2$, methyl, ethyl, *n*-propyl, isopropyl, *n*-butyl, isobutyl, *tert-butyl,* methylamino, dimethylamino, ethylamino, cyclopropyl, cyclopentyl, pyrrolidinyl, methoxy, ethoxy, isopropoxy, cyanomethyl, hydroxymethyl, hydroxyethyl, trifluoromethoxy, fluoromethyl, difluoromethyl, trifluoromethyl or 1,2-dichloroethyl.

In some embodiments, $R^{6e}$ and $R^{7e}$ together with the nitrogen atom to which they are attached form pyrrolidine, piperazine, piperidine, morpholinyl, oxazolidine or imidazolidine, wherein the pyrrolidine, piperazine, piperidine, morpholinyl, oxazolidine and imidazolidine are each independently optionally substituted with 1, 2, 3 or 4 substituents selected from -D, -OH, -F, -Cl, -Br, -I, -CN, $-NH_2$, methyl, ethyl, *n*-propyl, isopropyl, *n*-butyl, isobutyl, *tert*-butyl, methylamino, dimethylamino, ethylamino, cyclopropyl, cyclopentyl, pyrrolidinyl, methoxy, ethoxy, isopropoxy, cyanomethyl, hydroxymethyl, hydroxyethyl, trifluoromethoxy, fluoromethyl, difluoromethyl, trifluoromethyl or 1,2-dichloroethyl.

In some embodiments, $R^{6a}$, $R^{6c}$, $R^{6g}$ and $R^{7g}$ are each independently -H, -D, methyl, ethyl, *n*-propyl, isopropyl, *n*-butyl, isobutyl or *tert*-butyl.

In some embodiments, the compound of the present invention is a compound of Formula (I-1), or a stereoisomer, tautomer, nitrogen oxide, solvate, metabolite, pharmaceutically acceptable salt, or prodrug thereof,

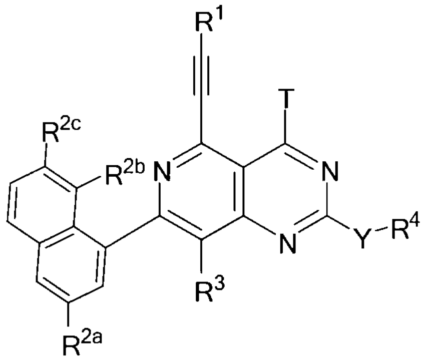

(I-1),

wherein, the $R^1$, $R^3$, $R^4$, Y and T are as defined herein;
$R^{2a}$, $R^{2b}$ and $R^{2c}$ are each as defined herein.

In some embodiments, $R^{2a}$, $R^{2b}$ and $R^{2c}$ are each independently -D, -OH, -F, -Cl, -Br, -I, -CN, -SH, $-CH_2C(=O)NR^{6b}R^{7b}$, $-C(=O)R^{6c}$, $-C(=O)OR^{6c}$, $-C(=O)NR^{6b}R^{7b}$, $-NR^{6g}C(=O)R^{7g}$, $-NR^{6b}R^{7b}$, $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, $C_{6-12}$ aryl, 5-12 membered heteroaryl, $C_{3-6}$ cycloalkyl or 3-6 membered heterocyclyl, wherein the $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, $C_{6-12}$ aryl, 5-12 membered heteroaryl, $C_{3-6}$ cycloalkyl and 3-6 membered heterocyclyl are each independently optionally substituted with 1, 2, 3 or 4 substituents selected from -D, -OH, -F, -Cl, -Br, -I, -CN, $-NH_2$, -C(=O)H, -C(=O)OH, $C_{1-6}$ alkyl, $C_{1-6}$ alkoxy, $C_{3-6}$ cycloalkyl and 3-6 membered heterocyclyl;

$R^{6b}$, $R^{7b}$ and $R^{6c}$ are each as defined herein. In some embodiments, $R^{2a}$, $R^{2b}$ and $R^{2c}$ are each independently -D, -OH, -F, -Cl, -Br, -I, -CN, -SH, $-CH_2C(=O)NR^{6b}R^{7b}$, $-C(=O)R^{6c}$, $-C(=O)OR^{6c}$, $-C(=O)NR^{6b}R^{7b}$, $-NR^{6b}C(=O)R^{7b}$, $-NR^{6b}R^{7b}$, $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, $C_{6-10}$ aryl, 5-12 membered heteroaryl, $C_{3-6}$ cycloalkyl or 3-6 membered heterocyclyl, wherein the $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, $C_{6-10}$ aryl, 5-12 membered heteroaryl, $C_{3-6}$ cycloalkyl and 3-6 membered heterocyclyl are each independently optionally substituted with 1, 2, 3 or 4 substituents selected from -D, -OH, -F, -Cl, -Br, -I, -CN, $-NH_2$, -C(=O)H, -C(=O)OH, $C_{1-4}$ alkyl, $C_{1-4}$ alkoxy, $C_{3-6}$ cycloalkyl and 3-6 membered heterocyclyl;

$R^{6b}$, $R^{7b}$ and $R^{6c}$ are each as defined herein.

In some embodiments, $R^{2a}$, $R^{2b}$ and $R^{2c}$ are each independently -D, -OH, -F, -Cl, -Br, -I, -CN, -SH, $-CH_2C(=O)NR^{6d}R^{7b}$, $-C(=O)R^{6c}$, $-C(=O)OR^{6c}$, $-C(=O)NR^{6b}R^{7b}$, $-NR^{6b}C(=O)R^{7b}$, $-NR^{6b}R^{7b}$, $-CH_3$, $-CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-CH=CH_2$, $-CH=CHCH_3$, $-CH_2CH=CH_2$, $-C\equiv CH$, $-C\equiv CCH_3$, $-CH_2C\equiv CH$, $-C\equiv CCH_2OH$, $-C\equiv C(CH_2)_2OH$, $-OCH_3$, $-OCH_2CH_3$, $-O(CH_2)_2CH_3$, $-CH_2CN$, $-(CH_2)_2CN$, $-(CH_2)_3CN$, $-CH_2OH$, $-(CH_2)_2OH$, $-(CH_2)_3OH$, $-CH(OH)CH_3$, $-(CH_2)_2F$, $-CH_2CHF_2$, $-CF_3$, $-CH_2CF_3$, $-CHF_2$, $-CH_2F$, $-(CH_2)_2Cl$, -CH=CHF, -CH=CHCl, $-CH=CHCH_2F$, $-C\equiv CCH_2F$, $-C\equiv C(CH_2)_2F$, $-C\equiv CF$, $-OCF_3$, $-OCHF_2$, $-OCH_2CHF_2$, $-OCH_2CF_3$, $-OCHClCHCl_2$, $-OCH_2CH_2F$, $-SCF_3$, $-SCH_2CF_3$, $-SCH_2CHF_2$, phenyl, naphthyl, pyridinyl, pyrimidinyl, cyclopropyl, cyclobutyl, cyclopentyl, cyclohexyl, pyrrolidinyl, oxazolidinyl, tetrahydrofuranyl, piperidyl or piperazinyl, wherein the $-CH_3$, $-CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-CH=CH_2$, $-CH=CHCH_3$, $-CH_2CH=CH_2$, $-C\equiv CH$, $-C\equiv CCH_3$, $-CH_2C\equiv CH$, $-C\equiv CCH_2OH$, $-C\equiv C(CH_2)_2OH$, $-OCH_3$, $-OCH_2CH_3$, $-O(CH_2)_2CH_3$, $-CH_2CN$, $-(CH_2)_2CN$, $-(CH_2)_3CN$, $-CH_2OH$, $-(CH_2)_2OH$, $-(CH_2)_3OH$, $-CH(OH)CH_3$, $-(CH_2)_2F$, $-CH_2CHF_2$, $-CH_2CF_3$, $-CHF_2$, $-CH_2F$, $-(CH_2)_2Cl$, -CH=CHF, -CH=CHCl, $-CH=CHCH_2F$, $-C\equiv CCH_2F$, $-C\equiv C(CH_2)_2F$, $-OCHF_2$, $-OCH_2CHF_2$, $-OCH_2CF_3$, $-OCHClCHCl_2$, $-OCH_2CH_2F$, $-SCH_2CF_3$, $-SCH_2CHF_2$, phenyl, naphthyl, pyridinyl, pyrimidinyl, cyclopropyl, cyclobutyl, cyclopentyl, cyclohexyl, pyrrolidinyl, oxazolidinyl, tetrahydrofuranyl, piperidyl and piperazinyl are each independently optionally substituted with 1, 2, 3 or 4 substituents selected from -D, -OH, -F, -Cl, -Br, -I, -CN, $-NH_2$, -C(=O)H, -C(=O)OH, methyl, ethyl, *n*-propyl, isopropyl, methoxy, ethoxy, *n*-propoxy, *i*-propoxy, cyclopropyl, cyclobutyl, cyclopentyl, cyclohexyl, pyrrolidinyl, oxazolidinyl, tetrahydrofuranyl, piperidyl and piperazinyl.

In some embodiments, the compound of the present invention is a compound of the following structure, or a stereoisomer, a tautomer, an nitrogen oxide, a solvate, a metabolite, a pharmaceutically acceptable salt, or a prodrug thereof,

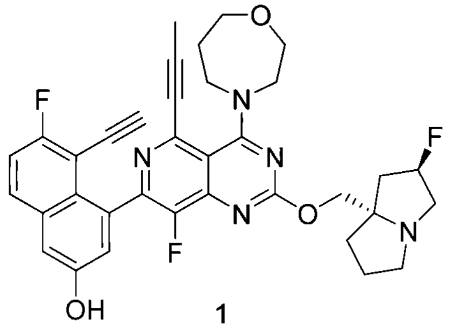
1
,
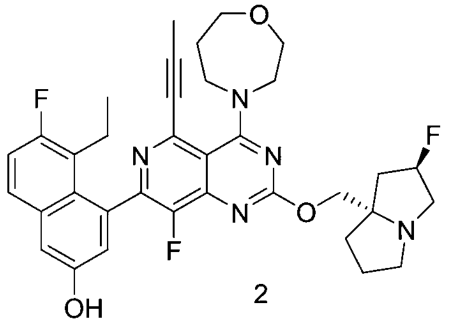
2
,
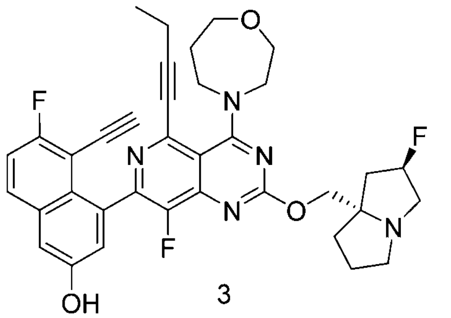
3
,
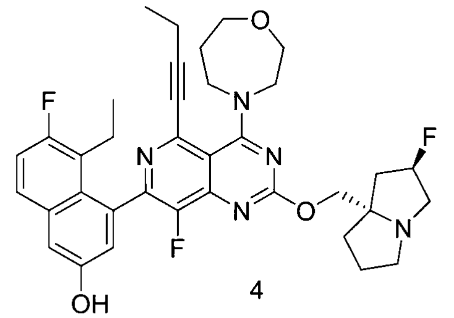
4
,

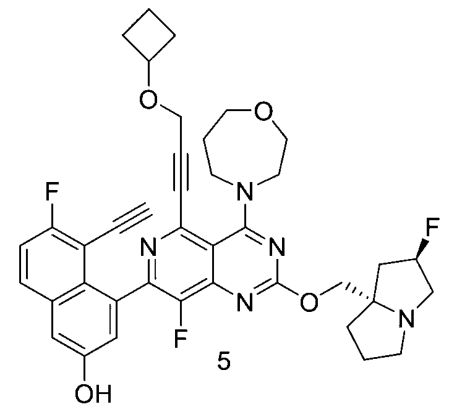

5

,

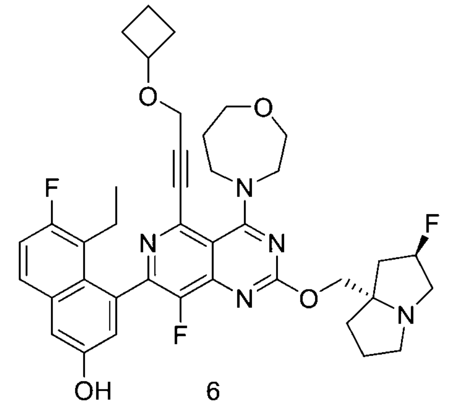

6

,

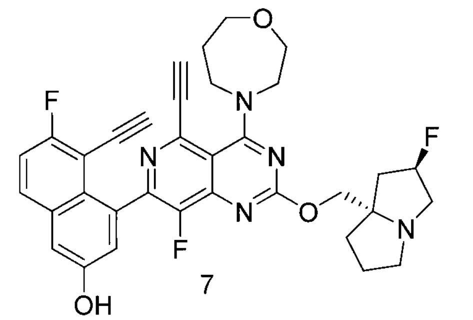

7

,

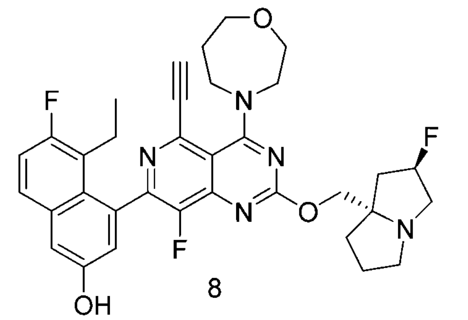

8

,

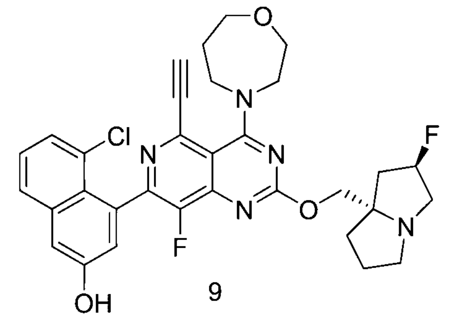

9

,

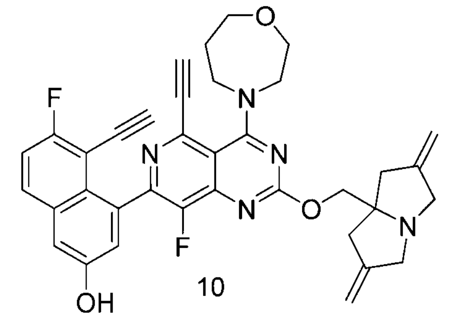

10

,

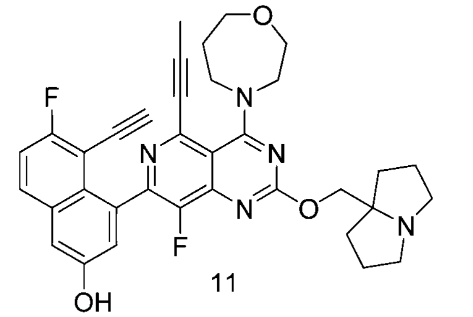

11

,

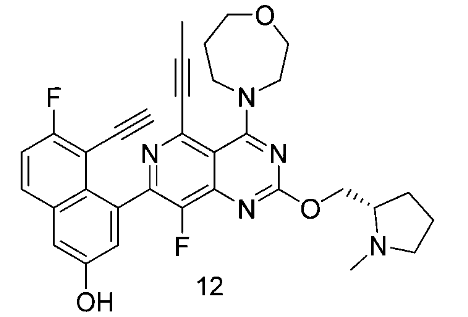

12

,

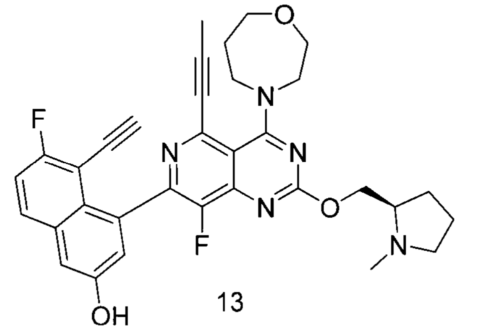

13

,

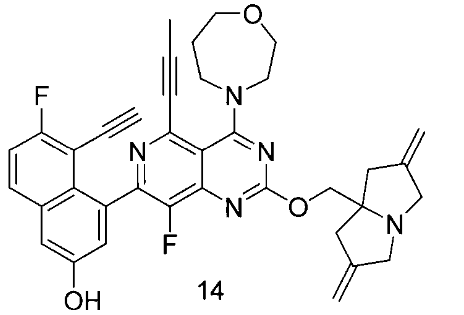

14

,

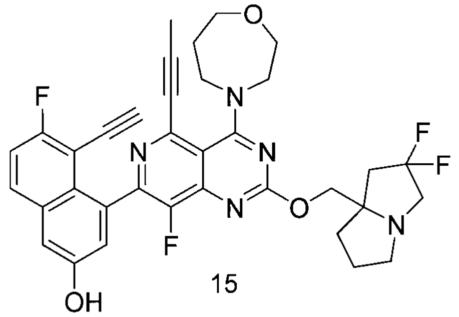

15

,

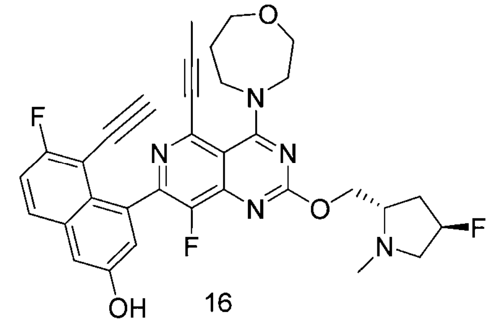

16

,

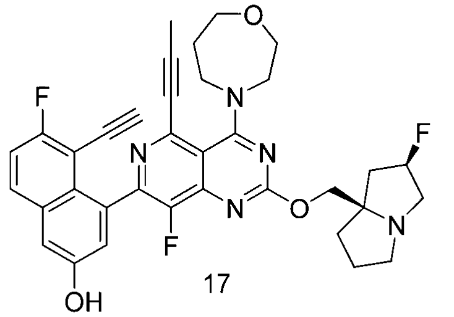

17

,

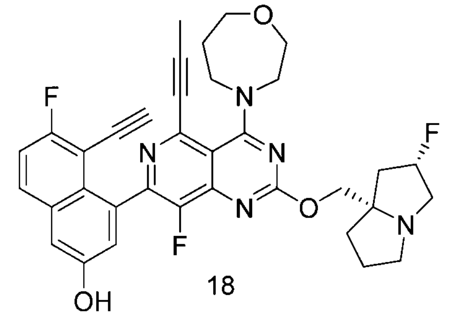

18

,

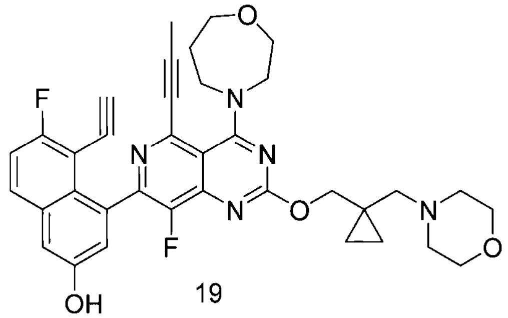

19

,

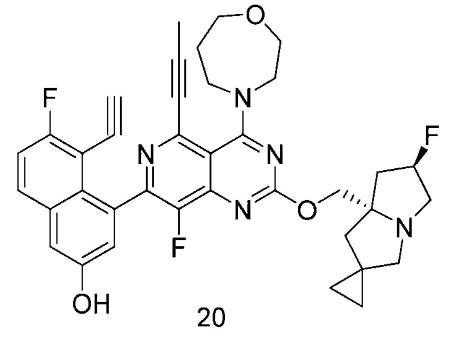

20

,

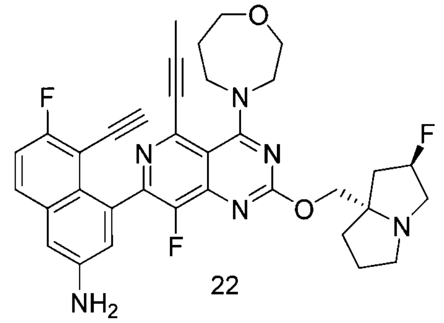

22,

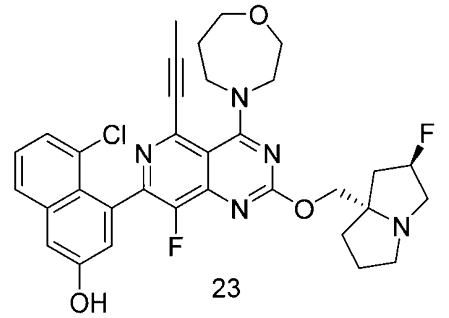

23,

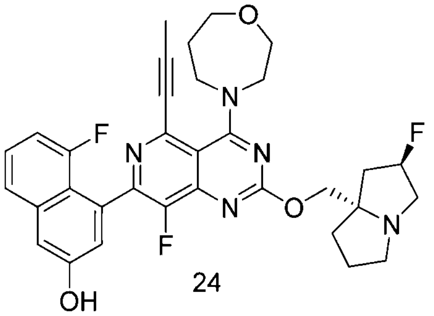

24,

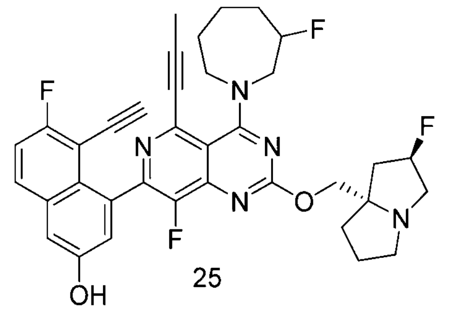

25,

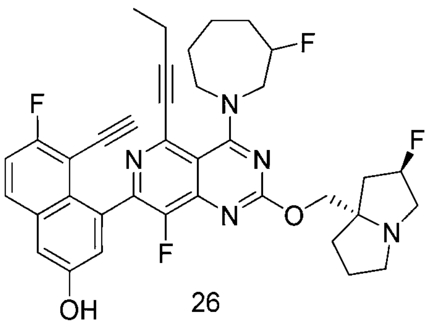

26,

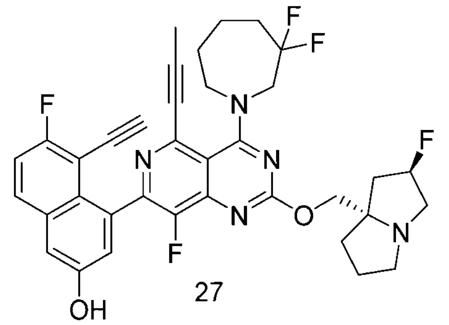

27,

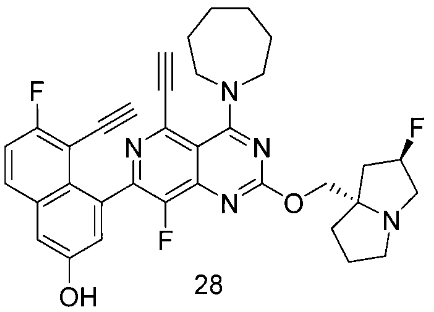

28,

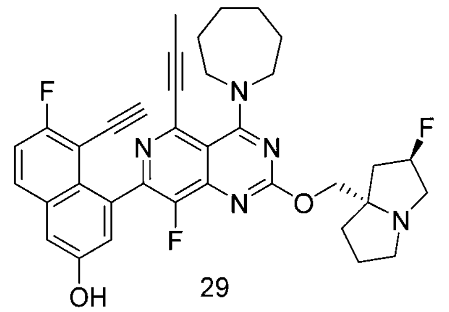

29,

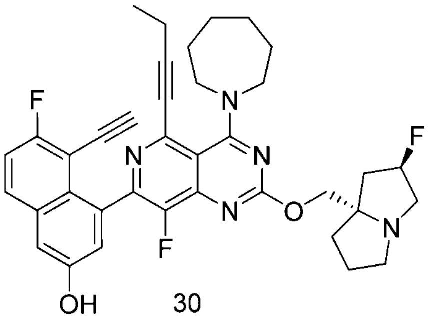

30,

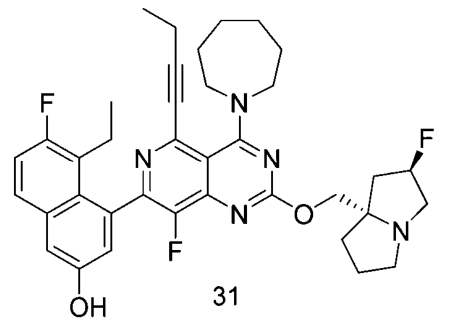

31,

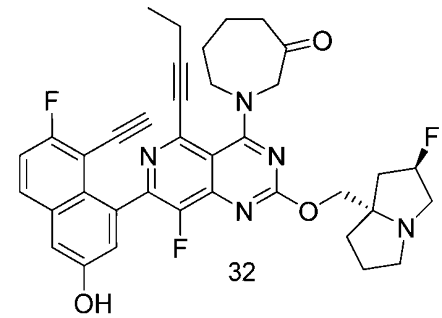
32
,

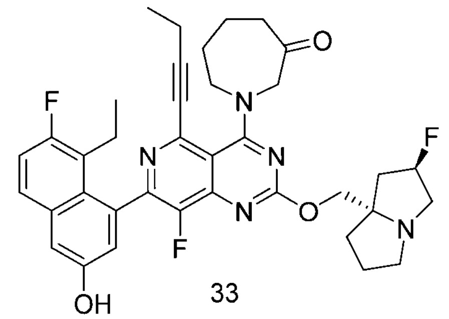
33
,

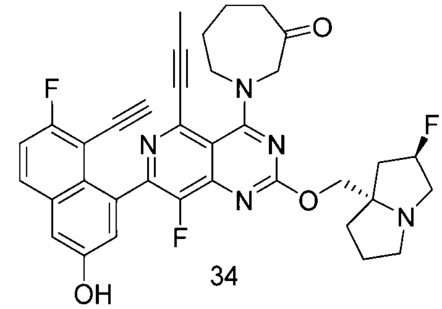
34
,

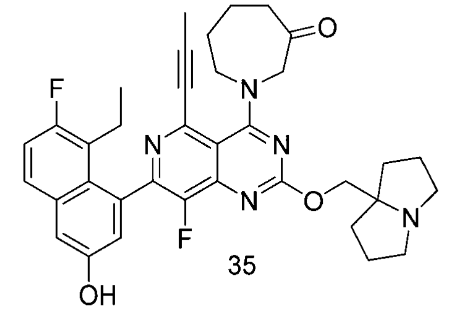
35
,

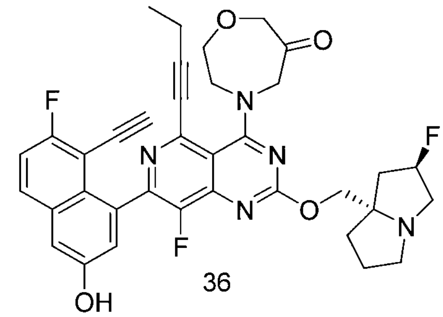
36
,

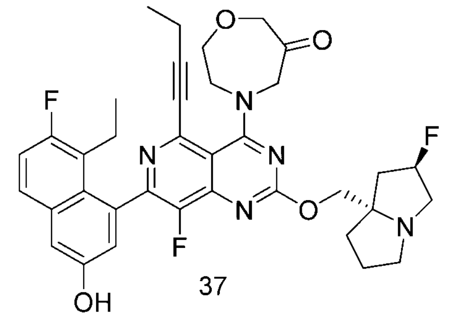
37
,

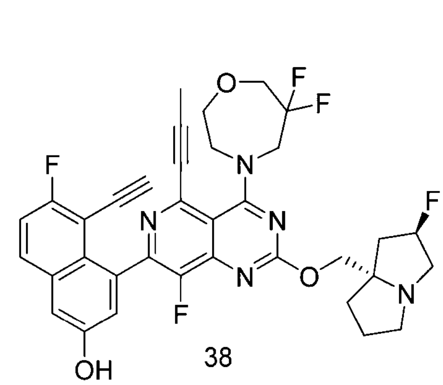
38
,

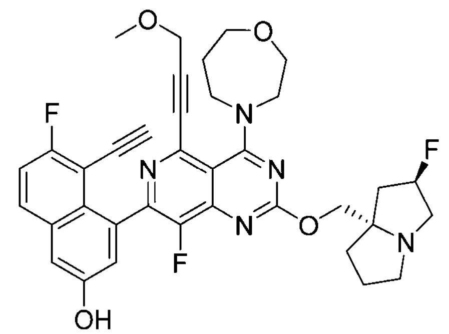
39
,

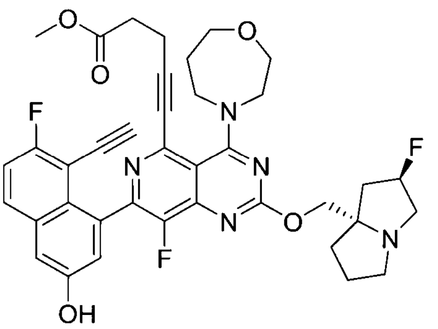

40

,

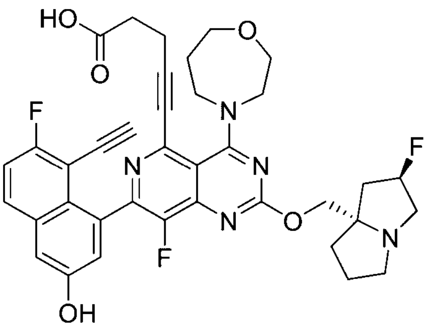

41

,

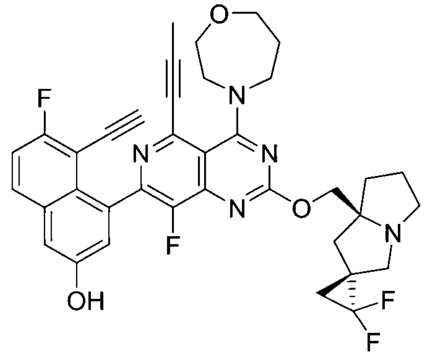

42

,

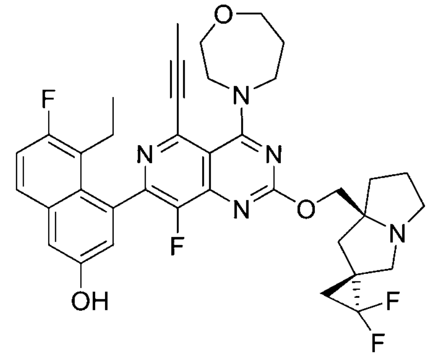

43

,

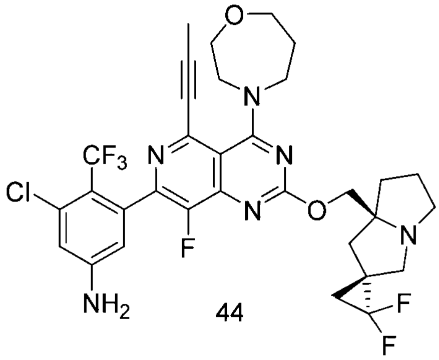

44

,

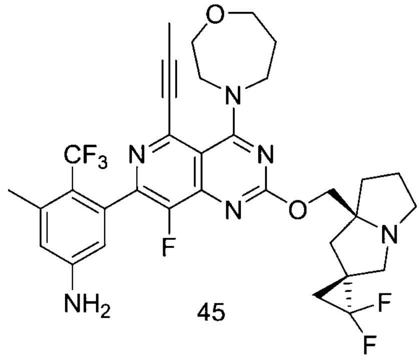

45

,

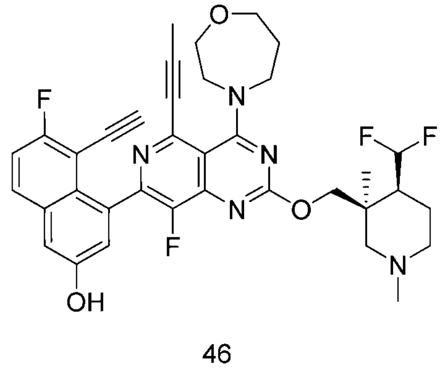

46

,

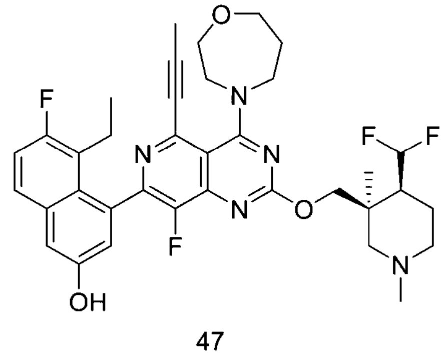

47

,

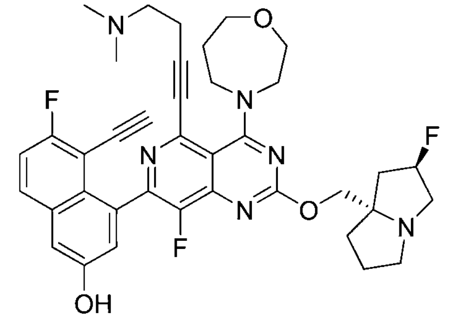

48

,

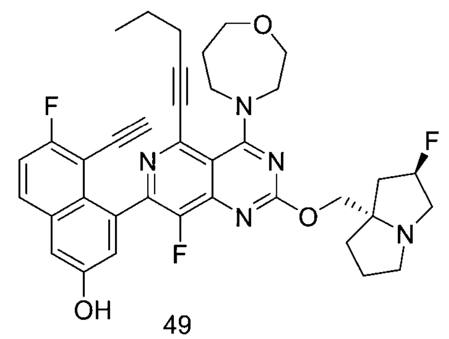

49

,

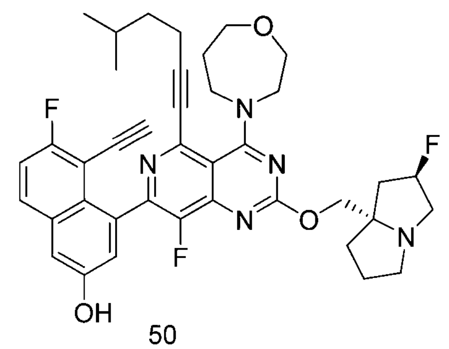

50

,

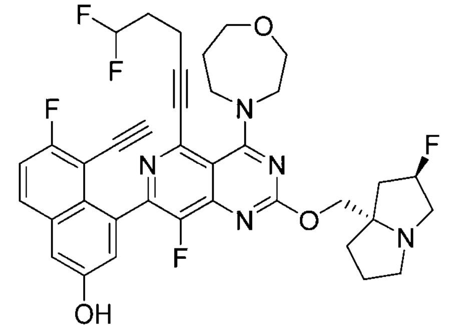

51

,

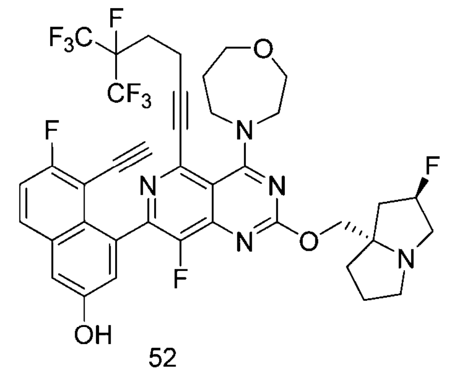

52

,

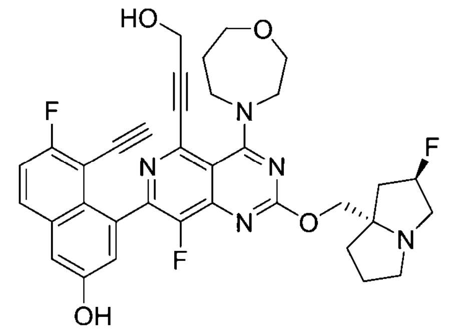

53

,

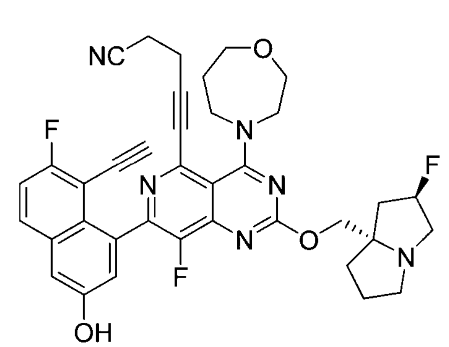

54

,

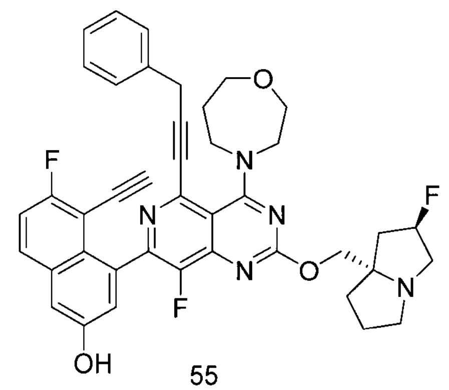

55

,

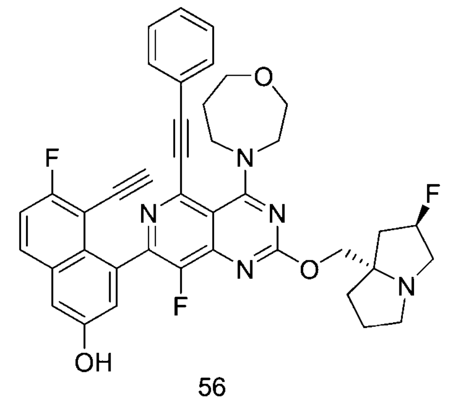

56

,

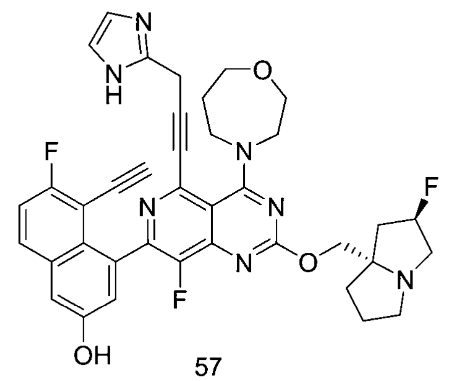

57

,

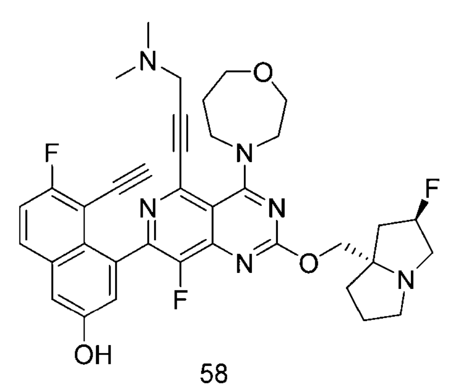

58

,

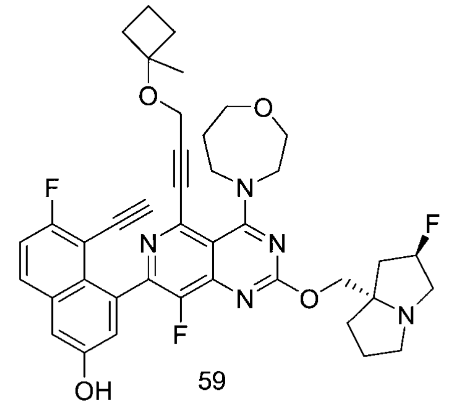

59

,

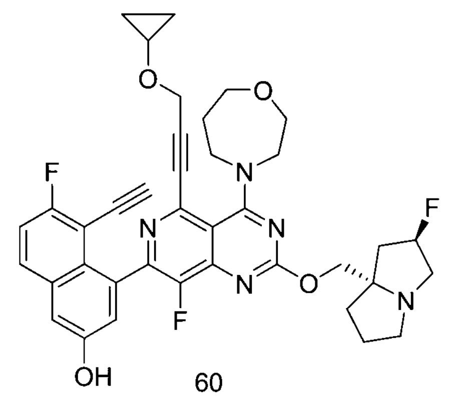

60

,

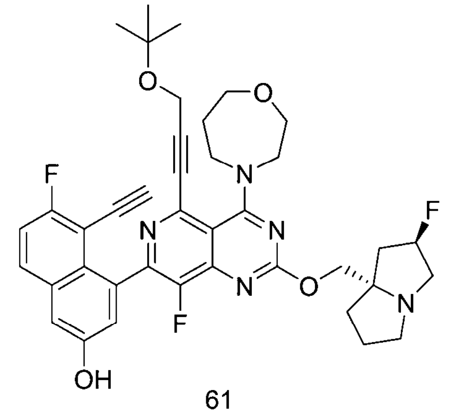

61

,

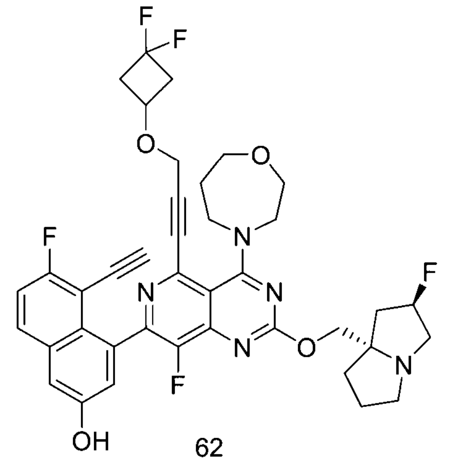

62 ,

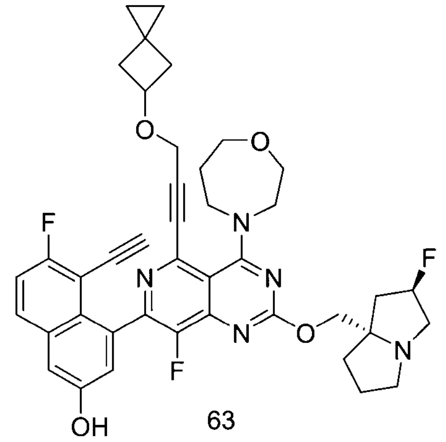

63 ,

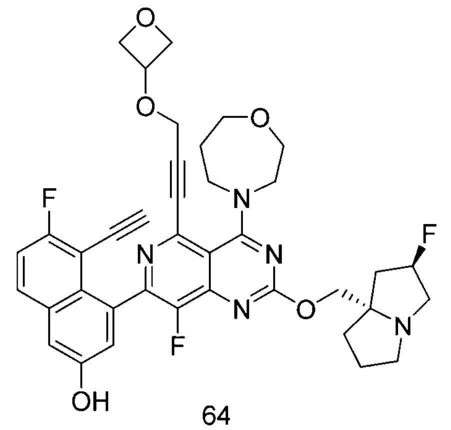

64 ,

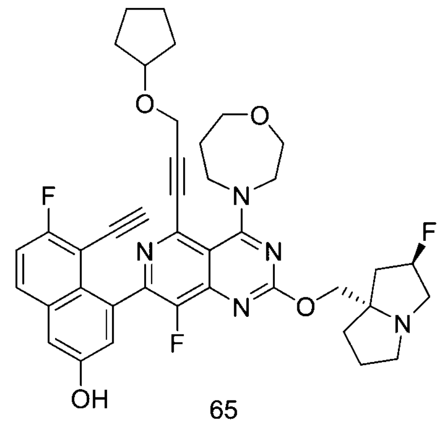

65 ,

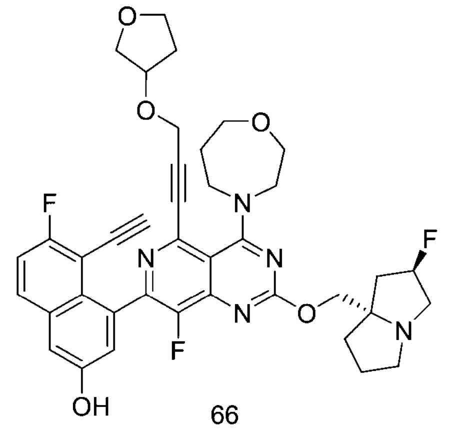

66 ,

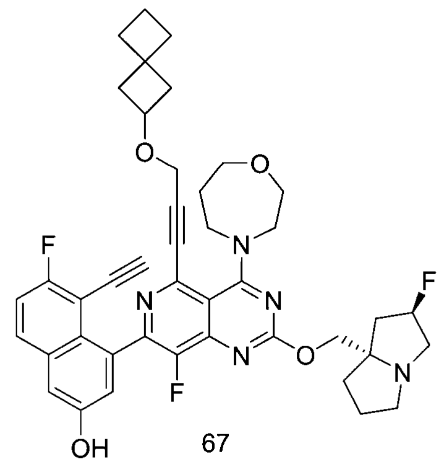

67 ,

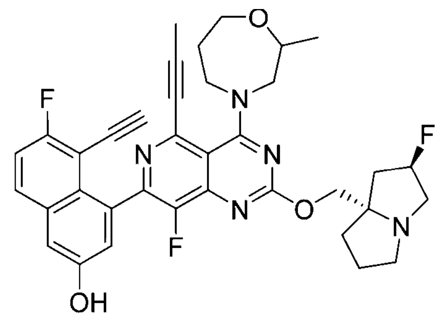

68

,

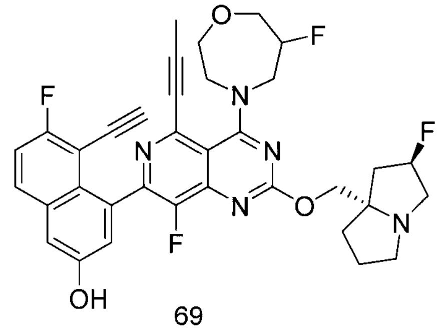

69

,

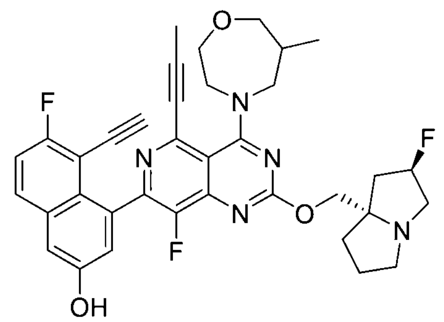

70

,

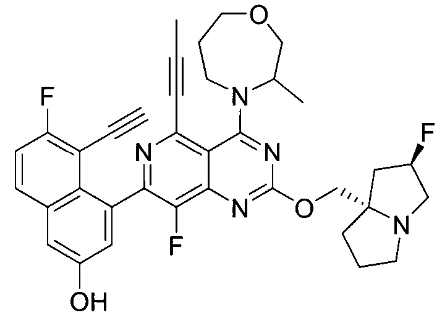

71

,

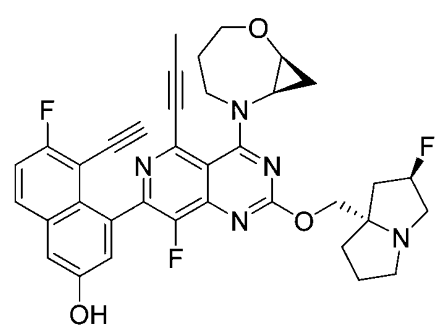

72

,

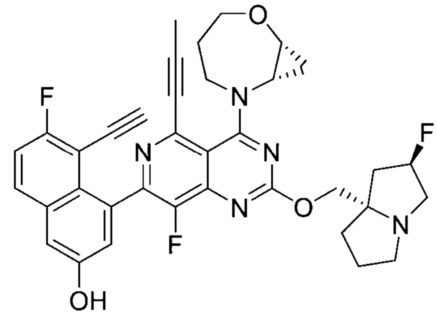

73

,

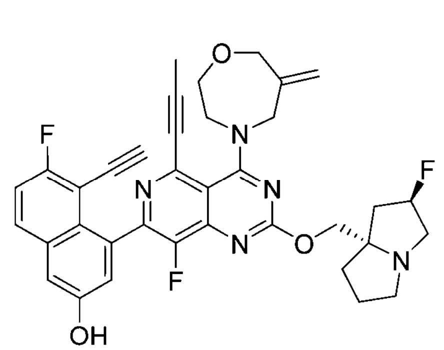

74

,

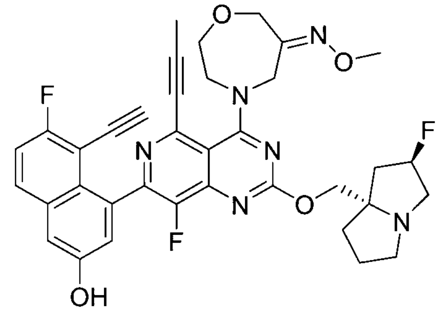

75

,

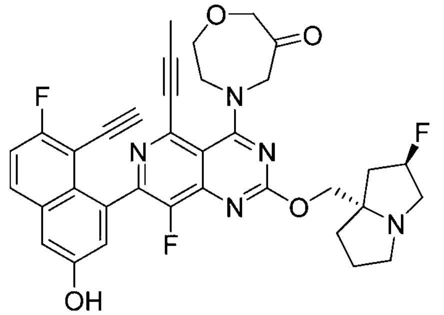

76 ,

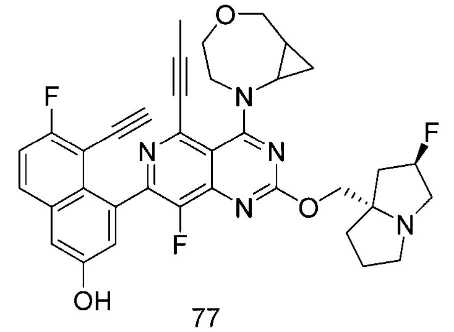

77 ,

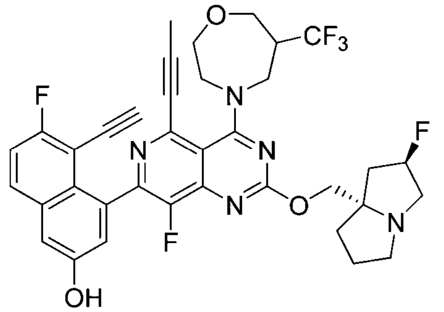

78 ,

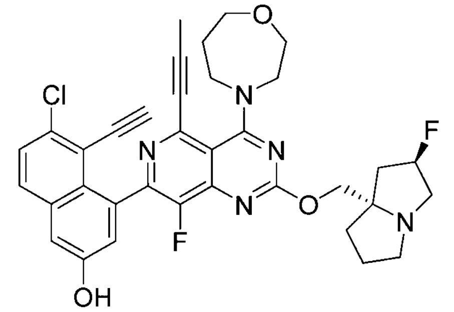

79 ,

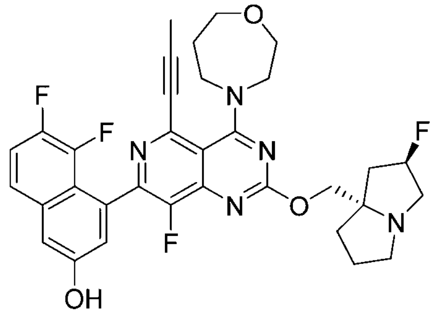

80 ,

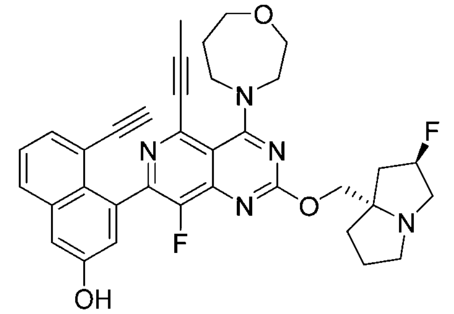

81 ,

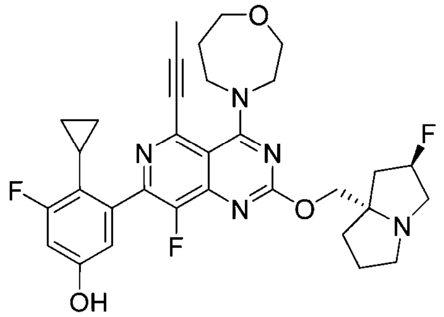

82 ,

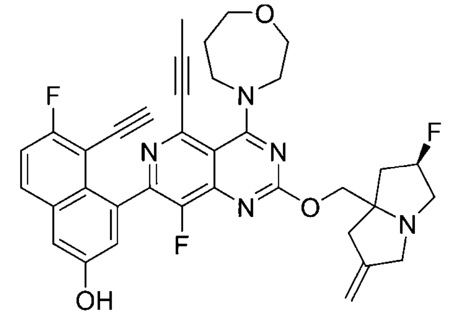

83 ,

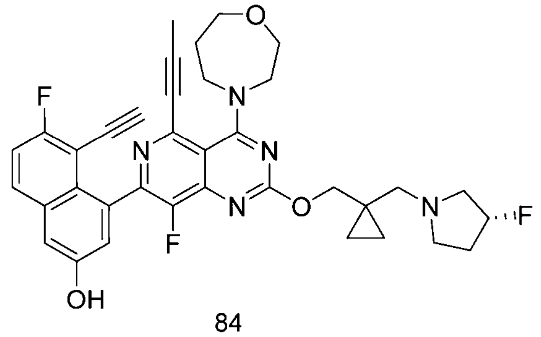

84

,

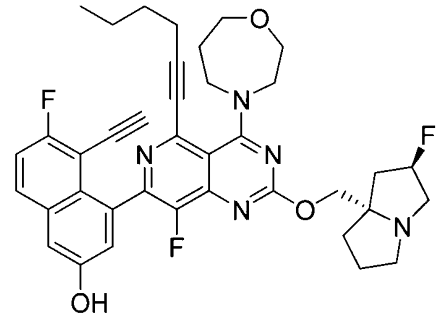

85

,

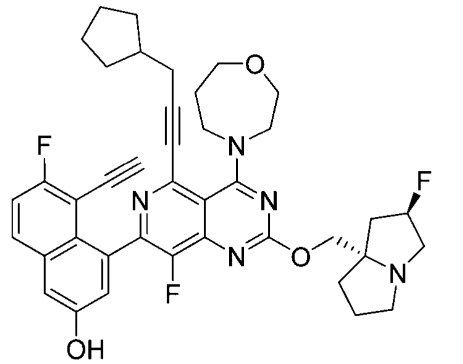

86

,

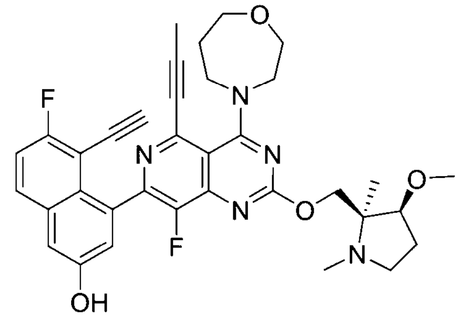

87

or

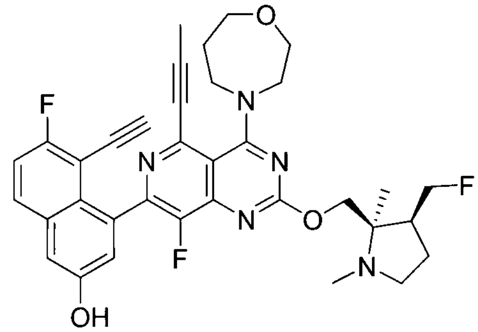

88

.

In other aspect, provided herein is a pharmaceutical composition comprising the compound disclosed herein.

In some embodiments, the pharmaceutical compositions of the present invention further comprise pharmaceutically acceptable adjuvant.

In some embodiments, the adjuvants of the present invention include, but are not limited to, carriers, excipients, diluents, vehicles, or combinations thereof. In some embodiments, the pharmaceutical composition may be in a liquid, solid, semi-solid, gel, or spray dosage form.

In other aspect, the present invention provides a use of the pharmaceutical composition of the present invention in the preparation of a medicament for preventing, treating, or alleviating a disease associated with KRAS wild-type, or KRAS G12A, KRAS G12C, KRAS G12D, KRAS G12R, KRAS G12S, KRAS G12V, KRAS G13D or KRAS Q61H mutation.

In some embodiments, the disease associated with KRAS wild-type, or KRAS G12A, KRAS G12C, KRAS G12D, KRAS G12R, KRAS G12S, KRAS G12V, KRAS G13D or KRAS Q61H mutation according to the present invention is cancer.

In some embodiments, the cancer of the present invention is cardiac cancer: sarcoma (angiosarcoma, fibrosarcoma, rhabdomyosarcoma, liposarcoma, etc.), myxoma, rhabdomyoma, fibroma, lipoma and teratoma, etc.; lung cancer: bronchogenic carcinoma (squamous cell, undifferentiated small cell, undifferentiated large cell, adenocarcinoma), non-small cell lung cancer, small cell lung cancer, alveolar (bronchiolar) carcinoma, bronchial adenoma,

sarcoma, lymphoma, chondromatous hamartoma, mesothelioma, etc.; gastrointestinal cancer: esophageal cancer (squamous cell carcinoma, adenocarcinoma, leiomyosarcoma, lymphoma), gastric cancer (lymphoma, leiomyosarcoma), pancreatic cancer (ductal adenocarcinoma, insulinoma, glucagonoma, gastrinoma, carcinoid tumor, VIPoma), small intestinal cancer (adenocarcinoma, lymphoma, carcinoid tumor, Kaposi's sarcoma, leiomyoma, hemangioma, lipoma, neurofibroma, fibroma), large intestinal cancer (adenocarcinoma, tubular adenoma, villous adenoma, hamartoma, leiomyoma), etc.; genitourinary cancer: renal cancer (adenocarcinoma, Wilms tumor, lymphoma), bladder and urethral cancer (squamous cell carcinoma, transitional cell carcinoma, adenocarcinoma), prostate cancer (adenocarcinoma, sarcoma), testicular cancer (seminoma, teratoma, embryonal carcinoma, teratocarcinoma, choriocarcinoma, sarcoma, interstitial cell carcinoma, fibroma, fibroadenoma, adenomatoid tumor, lipoma), etc.; liver cancer: hepatocellular carcinoma, cholangiocarcinoma, hepatoblastoma, angiosarcoma, hepatocellular adenoma, hemangioma, etc.; biliary tract cancer: gallbladder cancer, ampullary cancer, cholangiocarcinoma, etc.; bone cancer: osteogenic sarcoma, fibrosarcoma, malignant fibrous histiocytoma, chondrosarcoma, Ewing sarcoma, malignant lymphoma (reticulum cell sarcoma), multiple myeloma, malignant giant cell tumor, chordoma, osteochondroma, chondroblastoma, chondromyxoid fibroma, osteoid osteoma and giant cell tumor, etc.; nervous system cancer: skull tumor (osteoma, hemangioma, granuloma, xanthoma, Paget disease), brain cancer (astrocytoma, medulloblastoma, glioma, ependymoma, germinoma (pinealoma), glioblastoma multiforme, oligodendroglioma, schwannoma, retinoblastoma, congenital tumor), spinal cord neurofibroma, meningioma, glioma, sarcoma), etc.; gynecological cancer: uterine cancer (endometrial carcinoma, granulosa cell tumor, Sertoli-Leydig cell tumor, dysgerminoma, malignant teratoma), vulvar cancer (squamous cell carcinoma, carcinoma in situ, adenocarcinoma, fibrosarcoma, melanoma), vaginal cancer (clear cell carcinoma, squamous cell carcinoma, sarcoma botryoides (embryonal rhabdomyosarcoma)), fallopian tube carcinoma, ovarian cancer, breast cancer, etc.; hematologic cancer: acute or chronic myeloid leukemia, acute lymphoblastic leukemia, chronic lymphocytic leukemia, Hodgkin disease, non-Hodgkin lymphoma (malignant lymphoma), etc.; skin cancer: melanoma, basal cell carcinoma, squamous cell carcinoma, Kaposi's sarcoma, dysplastic nevus, lipoma, hemangioma, dermatofibroma, keloid, psoriasis, etc.; and adrenal cancer: neuroblastoma, etc.

In other aspect, the present invention further provides a method for preventing or treating a disease associated with KRAS, comprising administering to a patient a therapeutically effective amount of the compound according to the present invention or a pharmaceutical composition thereof.

In other aspect, the present invention further provides the compound or pharmaceutical composition thereof of the present invention for use in treating a disease associated with KRAS wild-type, or KRAS G12A, KRAS G12C, KRAS G12D, KRAS G12R, KRAS G12S, KRAS G12V, KRAS G13D or KRAS Q61H mutation.

In other aspect, the present invention relates to a method for the preparation, isolation, and purification of the compound of Formula (I) or (I-1).

Unless otherwise stated, all stereoisomers, tautomers, N-oxides, hydrates, solvates, metabolites, salts and pharmaceutically acceptable prodrugs of the compounds disclosed herein are within the scope of the invention.

In certain embodiments, the salt is a pharmaceutically acceptable salt. The term "pharmaceutically acceptable" refers to that the substance or composition must be compatible chemically and/or toxicologically, with the other ingredients comprising a formulation, and/or the mammal being treated therewith.

The compounds disclosed herein also include salts of the compounds which are not necessarily pharmaceutically acceptable salts, and which may be useful as intermediates for preparing and/or purifying compounds of Formula (I) or (I-1), and/or for separating enantiomers of compounds of Formula (I) or (I-1).

**Pharmaceutical Compositions, Administration, and Use of the Compound of the Present Invention**

Characteristics of the pharmaceutical composition of the present invention include a compound having the structure of Formula (I) or (I-1), a compound disclosed herein, or an embodiment compound, and a pharmaceutically acceptable carrier. The amount of the compound in the pharmaceutical composition of the present invention is effective to treat or alleviate a KRAS-mediated disease in a patient.

It will also be appreciated that certain of the compounds disclosed herein can exist in free form for treatment, or where appropriate, as a pharmaceutically acceptable derivative thereof. Some non-limiting examples of the pharmaceutically acceptable derivative include pharmaceutically acceptable prodrugs, salts, esters, salts of such esters, or any other adducts or derivatives which upon administration to a patient in need is capable of providing, directly or indirectly, a compound as otherwise described herein, or a metabolite or residue thereof.

As described herein, the pharmaceutical composition of the present invention further comprises a pharmaceutically acceptable excipient, including, but not limited to, any solvent, diluent, or other liquid vehicle; dispersing or suspending agent; surfactant; tonicity agent; thickening agent; emulsifier; preservative; solid binder; or lubricant; and the like, suitable for a particular target dosage form. As described in the following: In Remington: The Science and Practice of Pharmacy, 21st edition, 2005, ed. D.B. Troy, Lippincott Williams& Wilkins, Philadelphia, and Encyclopedia of Pharmaceutical Technology, eds. J. Swarbrick and J. C. Boylan, 1988-1999, Marcel Dekker, New York, based on a

synthesis of the literature herein, it is demonstrated that various excipients are applicable in the formulation of pharmaceutically acceptable compositions through industry-standard preparation methods. Except where conventional excipients are incompatible with the compound of the present invention-such as instances producing any adverse biological effects or detrimental interactions with other components of the pharmaceutical composition-their use remains encompassed within the scope of the present invention.

Some non-limiting examples of materials which can serve as pharmaceutically acceptable carriers include ion exchangers; aluminium; aluminum stearate; lecithin; serum proteins such as human serum albumin; buffer substances such as phosphates; glycine; sorbic acid; potassium sorbate; partial glyceride mixtures of saturated vegetable fatty acids; water; salts or electrolytes such as protamine sulfate, disodium hydrogen phosphate, potassium hydrogen phosphate, sodium chloride and zinc salts; colloidal silica; magnesium trisilicate; polyvinyl pyrrolidone; polyacrylates; waxes; polyethylene-polyoxypropylene-block polymers; wool fat; sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols such as propylene glycol and polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol; and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants.

In preparing the pharmaceutical composition provided by the present invention, the active ingredient is typically mixed with an excipient, diluted by the excipient, or encapsulated within a carrier in the form of, for example, a capsule, sachet, paper, or other container. When employed as a diluent, the excipient may be a solid, semi-solid, or liquid material that functions as a vehicle, carrier, or medium for the active ingredient. Suitable carriers include, but are not limited to, magnesium carbonate, magnesium stearate, talc, sugar, lactose, pectin, dextrin, starch, gelatin, tragacanth, methylcellulose, sodium carboxymethylcellulose, low-melting waxes, cocoa butter, and the like. Accordingly, the compositions may be formulated as tablets, pills, powders, lozenges, sachets, cachets, elixirs, suspensions, emulsions, solutions, syrups, aerosols (in solid form or in a liquid medium), ointments containing up to 10% by weight of the active compound, soft and hard gelatin capsules, suppositories, sterile injectable solutions, and sterile packaged powders. In one embodiment, the composition is formulated for oral administration. In one embodiment, the composition is formulated as a tablet or capsule.

The compound or the pharmaceutical composition of the present invention may be administered in the form of oral dosage forms such as tablets, capsules each of which includes a sustained-release or time-release formula, pills, powders, granules, elixirs, tinctures, suspensions, syrups and emulsifiers. They may also be administered intravenously (bolus or infusion), intraperitoneally, subcutaneously or intramuscularly, all dosage forms used are well-known to one of ordinary skill in the pharmaceutical arts. They can be administered alone, but will generally be administered together with a single pharmaceutical carrier based on the chosen mode of administration and standard pharmaceutical practice.

The compound or the pharmaceutical composition of the present invention may be administered intranasally, for topical use with a suitable intranasal vehicle, or transdermally by the use of a transdermal patch. When administered in the form of a transdermal delivery system, the dosage administered throughout the dosage is continuous rather than intermittent.

The compound or the pharmaceutical composition of the invention may also be administered in the form of liposomal delivery systems, such as small, monolayer, large, single-layered vesicles and multilamellar vesicles. Liposomes can be formed by different phospholipids, such as cholesterol, stearylamine, or phosphatidylcholine.

The compound or the pharmaceutical composition of the present invention are also conjugated to soluble polymers that serve as targeted drug carriers. Such polymers may encompass polyvinylpyrrolidone, pyran copolymer, polyhydroxypropylmethacrylamide-phenol, polyhydroxyethylaspartamide phenol, or polyethylene oxide-polylysine substituted with palmitoyl residues. Moreover, the compounds of the invention can be coupled to a class of biodegradable polymers, used to complete controlled release of drugs. For example, polylactic acid, polyglycolic acid, copolymers of polylactic acid and polyglycolic acid, polyepsilon caprolactone, polyhydroxybutyric acid, polyorthoesters, polyacetals, polydihydropyrans, polycyanoacrylates, and crosslinked or amphipathic blocking copolymers of hydrogels.

The dosage regimen of the compound or the pharmaceutical composition of this invention will vary with known factors such as the pharmacokinetic profile of the particular agent and its mode and route of administration; the race, age, sex, health status, medical condition and weight of the recipient; The nature and extent of symptoms; the type of concurrent treatment; the frequency of treatment; the route of administration; the renal and hepatic function of the patient; and the effect desired. A physician or veterinarian can make a decision and prescribe an effective amount of medication to prevent, counteract or prevent the development of cancer.

The compounds and compositions described herein may be administered alone or in combination with other compounds or other therapeutic agents. The compounds or compositions of the present invention may be administered concurrently or sequentially with other therapeutic agents via the same or different routes of administration. The

compounds of the present invention may be included together with other therapeutic agents in a single formulation or in separate formulations.

When the compounds of the present invention are administered together with other therapeutic agents, the amount of each component in a typical daily dose and typical dosage form may generally be reduced relative to the usual dose administered alone, taking into account the additive or synergistic effects of the therapeutic agents when administered in combination.

The compound of the present invention, or a pharmaceutically acceptable salt thereof, or a hydrate thereof, or a pharmaceutical composition thereof, is effective for preventing, treating, or alleviating disorders-particularly cancer-in a patient mediated by KRAS wild-type, or by KRAS G12A, KRAS G12C, KRAS G12D, KRAS G12R, KRAS G12S, KRAS G12V, KRAS G13D or KRAS Q61H mutations.

n some embodiments, the compound of the present invention or pharmaceutical composition thereof is effective for preventing, treating, or alleviating cancer conditions in patients including, but not limited to: sarcoma (angiosarcoma, fibrosarcoma, rhabdomyosarcoma, liposarcoma), myxoma, rhabdomyoma, fibroma, lipoma and teratoma, etc.; lung cancer: bronchogenic carcinoma (squamous cell, undifferentiated small cell, undifferentiated large cell, adenocarcinoma), non-small cell lung cancer, small cell lung cancer, alveolar (bronchiolar) carcinoma, bronchial adenoma, sarcoma, lymphoma, chondromatous hamartoma, mesothelioma, etc.; gastrointestinal cancer: esophageal cancer (squamous cell carcinoma, adenocarcinoma, leiomyosarcoma, lymphoma), gastric cancer (lymphoma, leiomyosarcoma), pancreatic cancer (ductal adenocarcinoma, insulinoma, glucagonoma, gastrinoma, carcinoid tumor, VIPoma), small intestinal cancer (adenocarcinoma, lymphoma, carcinoid tumor, Kaposi's sarcoma, leiomyoma, hemangioma, lipoma, neurofibroma, fibroma), large intestinal cancer (adenocarcinoma, tubular adenoma, villous adenoma, hamartoma, leiomyoma), etc.; genitourinary cancer: renal cancer (adenocarcinoma, Wilms tumor, lymphoma), bladder and urethral cancer (squamous cell carcinoma, transitional cell carcinoma, adenocarcinoma), prostate cancer (adenocarcinoma, sarcoma), testicular cancer (seminoma, teratoma, embryonal carcinoma, teratocarcinoma, choriocarcinoma, sarcoma, interstitial cell carcinoma, fibroma, fibroadenoma, adenomatoid tumor, lipoma), etc.; liver cancer: hepatocellular carcinoma, cholangiocarcinoma, hepatoblastoma, angiosarcoma, hepatocellular adenoma, hemangioma, etc.; biliary tract cancer: gallbladder cancer, ampullary cancer, cholangiocarcinoma, etc.; bone cancer: osteogenic sarcoma, fibrosarcoma, malignant fibrous histiocytoma, chondrosarcoma, Ewing sarcoma, malignant lymphoma (reticulum cell sarcoma), multiple myeloma, malignant giant cell tumor, chordoma, osteochondroma, chondroblastoma, chondromyxoid fibroma, osteoid osteoma and giant cell tumor, etc.; nervous system cancer: skull tumor (osteoma, hemangioma, granuloma, xanthoma, Paget disease), brain cancer (astrocytoma, medulloblastoma, glioma, ependymoma, germinoma (pinealoma), glioblastoma multiforme, oligodendroglioma, schwannoma, retinoblastoma, congenital tumor), spinal cord neurofibroma, meningioma, glioma, sarcoma), etc.; gynecological cancer: uterine cancer (endometrial carcinoma, granulosa cell tumor, Sertoli-Leydig cell tumor, dysgerminoma, malignant teratoma), vulvar cancer (squamous cell carcinoma, carcinoma in situ, adenocarcinoma, fibrosarcoma, melanoma), vaginal cancer (clear cell carcinoma, squamous cell carcinoma, sarcoma botryoides (embryonal rhabdomyosarcoma)), fallopian tube carcinoma, ovarian cancer, breast cancer, etc.; hematologic cancer: acute or chronic myeloid leukemia, acute lymphoblastic leukemia, chronic lymphocytic leukemia, Hodgkin disease, non-Hodgkin lymphoma (malignant lymphoma), etc.; skin cancer: melanoma, basal cell carcinoma, squamous cell carcinoma, Kaposi's sarcoma, dysplastic nevus, lipoma, hemangioma, dermatofibroma, keloid, psoriasis, etc.; and adrenal cancer: neuroblastoma, etc.

## GENERAL SYNTHETIC PROCEDURES

To describe the present invention, the invention will be further illustrated by the following examples with respect to its technical solution. The following examples are provided solely to illustrate specific embodiments of the present invention, enabling those skilled in the art to understand the invention, but are not to be construed as limiting the scope of protection thereof. In the specific embodiments of the present invention, technical means or methods not otherwise specified are conventional technical means or methods in the art.

Unless further defined, the definitions of substituents are as described herein in the present invention. The following non-limiting schemes and examples are presented to further exemplify the invention.

Persons skilled in the art will recognize that the chemical reactions described may be readily adapted to prepare a number of other compounds disclosed herein, and alternative methods for preparing the compounds disclosed herein are deemed to be within the scope disclosed herein. For example, the synthesis of non-exemplified compounds according to the invention may be successfully performed by modifications apparent to those skilled in the art, e.g., by appropriately protecting interfering groups, by utilizing other suitable reagents known in the art other than those described, and/or by making routine modifications of reaction conditions. Alternatively, other reactions disclosed herein or known in the art will be recognized as having applicability for preparing other compounds disclosed herein.

In the examples described below, unless otherwise indicated all temperatures are set forth in degrees Celsius (°C), room temperature in the examples refers to 15 °C - 30 °C; in some examples, room temperature is 20 °C - 30 °C.

Reagents were purchased from commercial suppliers such as Aldrich Chemical Company, Arco Chemical Company and Alfa Chemical Company, and were used without further purification. Unless otherwise specified, general reagents were purchased from Shantou XiLong Chemical Factory, Guangdong Guanghua Reagent Chemical Factory Co. Ltd., Guangzhou Reagent Chemical Factory, Tianjin YuYu Fine Chemical Ltd., Tianjin Fuchen Chemical Reagent Factory, Wuhan Xinhua Yuan Technology Development Co., Ltd., Qingdao Tenglong Reagent Chemical Ltd., and Qingdao Ocean Chemical Factory.

Anhydrous THF, dioxane, toluene, and ether were obtained by refluxing the solvent with sodium. Anhydrous $CH_2Cl_2$ and $CHCl_3$ were obtained by refluxing the solvent with $CaH_2$. EtOAc, PE, hexane, DMAC and DMF were treated with anhydrous $Na_2SO_4$ prior use.

The reactions set forth below were done generally under a positive pressure of nitrogen or argon or with a drying tube (unless otherwise stated) in anhydrous solvents, and the reaction flasks were typically fitted with rubber septa for the introduction of substrates and reagents via syringe. Glassware was oven dried and/or heat dried. Glassware was oven dried and/or heat dried.

Column chromatography was conducted using a silica gel column. Silica gel (300-400 mesh) was purchased from Qingdao Ocean Chemical Factory.

The $^1$H NMR spectra were recorded using a Bruker 400 MHz or 600 MHz nuclear magnetic resonance spectrometer. $^1$H NMR spectra were obtained by using $CDCl_3$, DMSO-*d*$_6$, $CD_3OD$ or acetone-*d*$_6$ solvent (reported in ppm), with TMS (0 ppm) or chloroform (7.26 ppm) as the reference standard. When peak multiplicities are reported, the following abbreviations are used: s (singlet), d (doublet), t (triplet), q (quartet), m (multiplet), br (broadened), br s (broadened singlet, br.s), dd (doublet of doublets), dt (doublet of triplets). Coupling constant *J*, when given, was reported in Hertz (Hz).

Low-resolution mass spectrometry (MS) data were acquired using an Agilent 6120 quadrupole HPLC-MS system (column: Zorbax SB-C18, 2.1 × 30 mm, 3.5 μm, 6 min, flow rate: 0.6 mL/min). The mobile phases consisted of a gradient of 5% to 95% phase A (0.1% formic acid in $CH_3CN$) in phase B (0.1% formic acid in $H_2O$), and an ESI source was used, the peak of HPLC was recorded with UV-Vis detection at 210/254 nm.

Pure compounds were purified using either an Agilent 1260 preparative HPLC system or a Calesep pump 250 preparative HPLC system (column: NOVASEP 50/80 mm DAC) with UV detection at 210 nm/254 nm.

The following abbreviations are used throughout the specification:

| | | | |
|---|---|---|---|
| $(Boc)_2O$ | Di-tert-butyl dicarbonate | TMPMgCl-LiCl | 2,2,6,6-Tetramethylpiperidinylmagnesium chloride lithium chloride complex |
| TBAF | Tetrabutylammonium fluoride | Conc. $H_2SO_4$ | Concentrated sulfuric acid |
| Xphos Pd G3 | | | |
| Methanesulfonato(2-dicyclohexylphosphino-2',4 ',6'-tri-i-propyl-1,1'-biphenyl)(2'-amino-1,1'-biph enyl-2-yl)palladium(II) | | | |
| DMAP | N,N-dimethylaminopyridine | TFA, $CF_3COOH$ | Trifluoroacetic acid |
| THF | tetrahydrofuran | DMSO-*d*$_6$ | Dimethyl sulfoxide-*d*$_6$ |
| $Pd(PPh_3)_2Cl_2$ | | MeOH, $CH_3OH$ | methanol |
| Bis(triphenylphosphine)palladium(II) chloride | | | |
| TMSOTf | Trimethylsilyl trifluoromethanesulfonate | PE | Petroleum ether |
| CuI | cuprous iodide | EA | ethyl acetate |
| TEA | triethylamine | DIPEA | *N,N*- diisopropylethylamine |
| $NH_3$/MeOH | Ammonia in methanol | $POCl_3$ | Phosphorus oxychloride |
| $K_3PO_4 \cdot 7H_2O$ | Potassium phosphate heptahydrate | DCM | dichloromethane |
| ACN | acetonitrile | rt | room temperature |
| CsF | Caesium fluoride | M, mol/L | Mole/liter |
| DMF | N,N-dimethylformamide | H | hour, hours |
| THF/$H_2O$ | A mixture of tetrahydrofuran and water | °C | degrees Celsius |
| equiv. | equivalent | MTBE | Methyl tert-Butyl Ether |
| TMS | Trimethylsilyl group | DABCO | Triethylenediamine |

(continued)

| | | | |
|---|---|---|---|
| Xphos Pd G4 | | HCl/dioxane, HCl/1,4-dioxane | A soultion of |
| Methanesulfonato(2-dicyclohexylphosphino-2',4 ',6'-tri-i-propyl-1,1'-biphenyl)(2'-methylamino-1, 1'-biphenyl-2-yl)palladium(II) | | | hydrogen chloride in 1,4-dioxane |
| $K_3PO_4{\cdot}7H_2O$ | Potassium phosphate heptahydrate- | MOM | methoxymethyl |
| $Et_2Zn$ | Diethylzinc | equiv. | equivalent |
| TIPS | Triisopropylsilyl | | |

The compounds, pharmaceutical compositions, and uses provided by the present invention will be further illustrated below with reference to the examples.

## Scheme 1

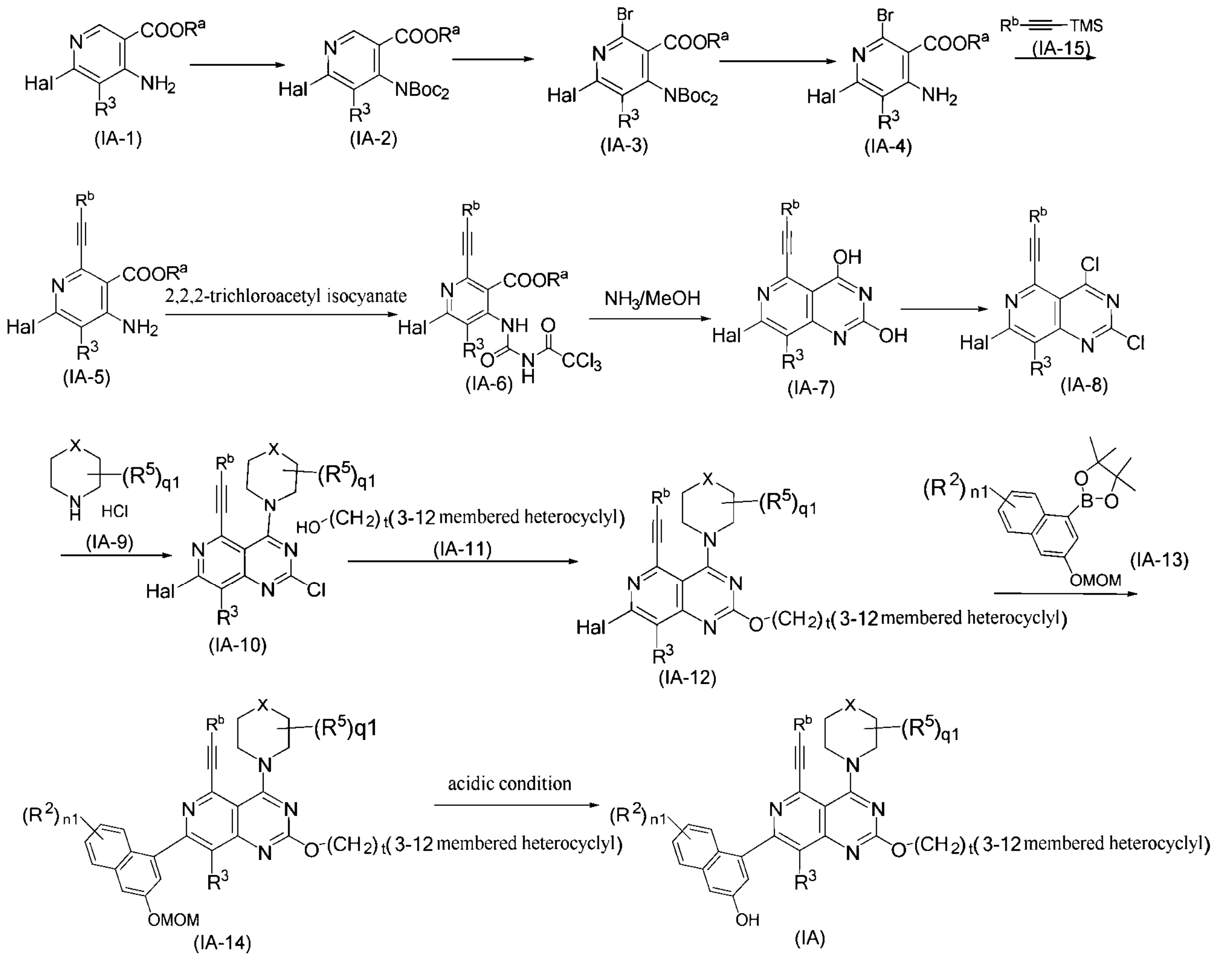


Compound (IA) can be synthesized with reference to the method described in **Scheme 1.** Wherein X, q1, $R^1$, $R^2$, $R^3$ and $R^5$ are as defined herein; n1 is an integer from 1 to 6; Hal is halogen, preferably Cl or Br; $R^a$ is $C_{1-4}$ alkyl, preferably methyl or ethyl; $R^b$ is $C_{1-6}$ alkyl, $C_{1-6}$ cyanoalkyl, $C_{1-6}$ hydroxyalkyl, $C_{1-6}$ haloalkyl, $C_{1-6}$ alkoxy-$C_{1-6}$ alkyl, ($C_{3-12}$ cycloalkyl)-$C_{1-6}$ alkyl, (3-12 membered heterocyclyl)-$C_{1-6}$ alkyl, ($C_{6-10}$ aryl)-$C_{1-6}$ alkyl, (5-12 membered heteroaryl)-$C_{1-6}$ alkyl, ($C_{3-12}$ cycloalkyl)-O-$C_{1-6}$ alkyl, (3-12 membered heterocyclyl)-O-$C_{1-6}$ alkyl or $C_{1-6}$ mercaptoalkyl; t is an integer from 1 to 6, preferably 1 or 2; and the 3-12 membered heterocyclyl is as defined herein and optionally substituted with substituents described herein. Compound (IA-1) can react with di-tert-butyl dicarbonate under suitable conditions (e.g., in the presence of DMAP and imidazole in dichloromethane) to afford compound (IA-2); compound (IA-2) can first react with

TMPMgCl·LiCl and subsequently react with 1,2-dibromotetrachloroethane to give compound (IA-3); deprotection of the amino group in compound (IA-3) under acidic conditions yields compound (IA-4); compound (IA-4) undergoes coupling with compound (IA-15) under suitable conditions (e.g., in the presence of $Pd(PPh_3)_2Cl_2$ and CuI) to give compound (IA-5); compound (IA-5) can react with 2,2,2-trichloroacetyl isocyanate to form Compound (IA-6), which can react with a solution of ammonia in methanol to afford compound (IA-7); compound (IA-7) can react with phosphorus oxychloride in the presence of DIPEA to give compound (IA-8); compound (IA-8) can react with compound (IA-9) under suitable conditions (e.g., in the presence of DIPEA) to yield compound (IA-10); compound (IA-10) can couple with compound (IA-11) under appropriate conditions (e.g., in the presence of DIPEA with heating) to provide compound (IA-12); compound (IA-12) can react with compound (IA-13) in the presence of a suitable catalyst (e.g., XPhos Pd G3) to afford compound (IA-14); compound (IA-14) can react under acidic conditions (e.g., with TMSOTf or concentrated sulfuric acid) to give compound (IA).

**Scheme 2**

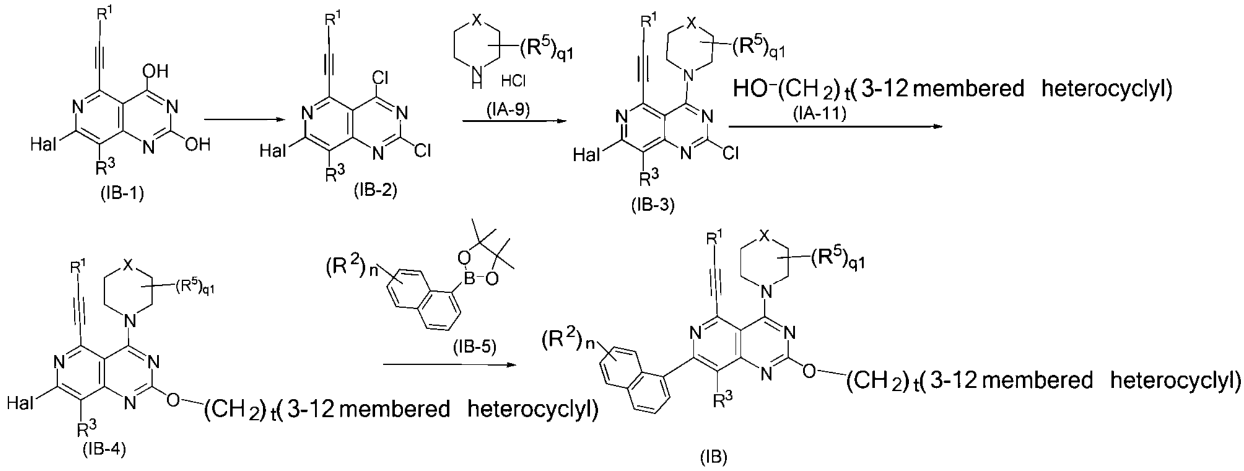

(IB-1) (IB-2) (IB-3) (IB-4) (IB)

Compound (IB) can be synthesized with reference to the method described in **Scheme 2.** Wherein X, q1, $R^1$, $R^2$, $R^3$ and $R^5$ are as defined herein; Hal is halogen, preferably Cl or Br; t is an integer from 1 to 6, preferably 1 or 2; the 3-12-membered heterocyclyl is as defined herein and optionally substituted with substituents described herein. Compound (IB-1) can react with phosphorus oxychloride in the presence of DIPEA to give compound (IB-2); compound (IB-2) can react with compound (IA-9) under suitable conditions (e.g., in the presence of DIPEA) to give compound (IB-3); compound (IB-3) can react with compound (IA-11) under appropriate conditions (e.g., in the presence of DIPEA with heating ) to provide compound (IB-4); compound (IB-4) can react with compound (IB-5) in the presence of a suitable catalyst (e.g., XPhos Pd G3) to give compound (IB).

**Example**

**Synthesis of Intermediate Compound M1**

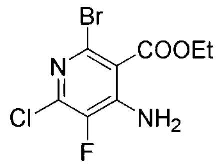

**M1**

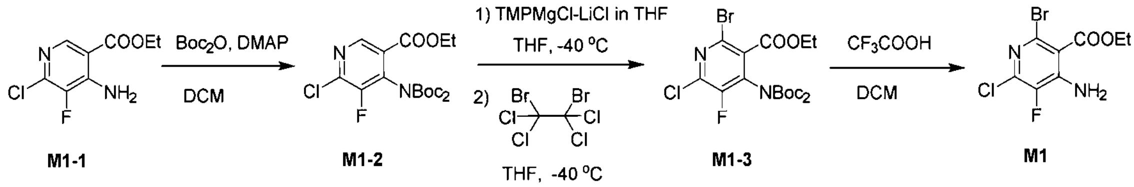


Step 1: Synthesis of Compound **M1-2**

Into a 1000 mL single-necked flask were added compound **M1-1** (10 g, 45.74 mmol), DMAP (1.12 g, 9.15 mmol) and DCM (200 mL). The mixture was stirred at 30 °C, and di-tert-butyl dicarbonate (21.96 g, 100.63 mmol) was added dropwise. After complete addition, the mixture was stirred at 30 °C for 18 h. The reaction was stopped, imidazole (3.11 g, 45.74 mmol) was added into the mixture. After stirring for 0.5 h, the mixture was washed with saturated ammonium chloride solution (100 mL × 3). The mixture were separated, and the organic phase was further washed with saturated sodium chloride solution (100 mL × 2). The combined organic phases were dried over anhydrous sodium sulfate and concentrated to obtain 17.4 g of yellow solid **M1-2** with a yield of 90.82%, which was used directly in the subsequent step. $^1$H NMR (599 MHz, $CDCl_3$): δ 8.79 (s, 1H), 4.37 (q, *J* = 7.1 Hz, 2H), 1.41 (s, 18H), 1.38 (t, *J* = 7.1 Hz, 3H).

Step 2: Synthesis of Compound **M1-3**

Into a 500 mL single-necked flask were added compound **M1-2** (10 g, 23.88 mmol) and anhydrous THF (100 mL), the mixture was stirred and cooled to -40 °C, and TMPMgCl·LiCl (35.82 mL, 35.82 mmol, 1 M in THF) was added dropwise at -40 °C. After stirring at -40 °C for 4 h, a solution of 1,2-dibromotetrachloroethane (9.33 g, 28.66 mmol) in THF (30 mL) was added dropwise into the reaction mixture. Stirring continued at -40 °C for an additional 4 h. The reaction was stopped, and the mixture was quenched with saturated ammonium chloride solution (100 mL), then extracted with ethyl acetate (EA, 100 mL × 2). The combined organic phases were dried over anhydrous sodium sulfate and concentrated. The residue was purified by column chromatography using PE/DCM (v/v = 100/1-1/1) as eluent to obtain 8.5 g of a yellow solid **M1-3** with a yield of 71.5%. $^1$H NMR (599 MHz, $CDCl_3$): δ 4.40 (q, *J* = 7.2 Hz, 2H), 1.42 (s, 18H), 1.37 (t, *J* = 7.2 Hz, 3H).

Step 3: Synthesis of Compound **M1**

To a 250 mL single-necked flask were added compound **M1-3** (5 g, 10.05 mmol), DCM (50 mL), and TFA (14.97 mL, 200.1 mmol). The mixture was stirred at 30 °C for 5 h. The reaction was stopped, and saturated aqueous sodium carbonate solution was added to adjust the pH to neutral. The mixture was then extracted with DCM (30 mL × 3). The combined organic phases were dried over anhydrous sodium sulfate and concentrated to obtain 2.9 g of a pale brown solid **M1** with a yield of 97%. $^1$H NMR (599 MHz, $CDCl_3$): δ 6.07 (s, 2H), 4.41 (q, *J* = 7.1 Hz, 2H), 1.41 (t, *J* = 7.1 Hz, 3H); LC-MS (ESI, pos. ion) m/z: 297.1 $[M+H]^+$.

**Synthesis of Intermediate Compound M2**

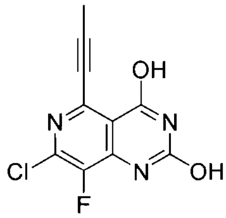

**M2**

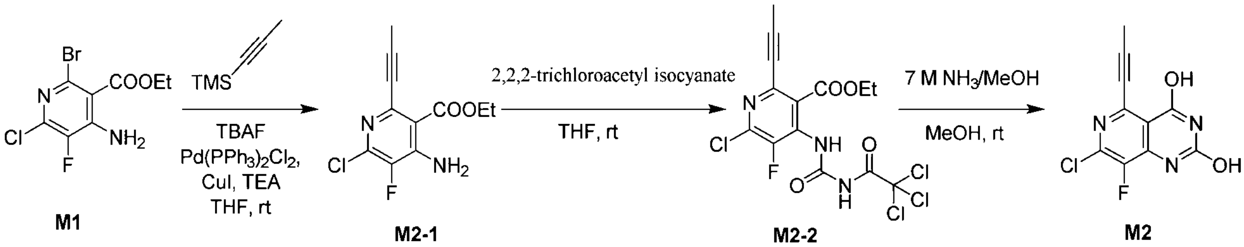


Step 1: Synthesis of Compound **M2-1**

Into a 50 mL two-necked flask were added compound **M1** (1.0 g, 3.36 mmol), $Pd(PPh_3)_2Cl_2$ (0.472 g, 0.67 mmol) and CuI (0.128 g, 0.67 mmol). The mixture was purged with nitrogen three times. Then, 1-(trimethylsilyl)propyne (0.566 g, 5.04 mmol), TEA (2.33 mL, 16.8 mmol) and anhydrous THF (10 mL) were added. The mixture was purged with nitrogen three times again. TBAF (5.04 mL, 5.04 mmol, 1.0 M in THF) was added dropwise under stirring. After completion of the addition, the mixture was stirred at room temperature (25 °C) for 6 h. The reaction was stopped and filtered through a celite pad. The filtrate was concentrated and purified by column chromatography using PE/DCM (v/v = 100/1-2/1) as eluent to obtain 0.523 g of a yellow solid **M2-1** with a yield of 60.6%. LC-MS (ESI, pos. ion) m/z: 257.3 $[M+H]^+$.

Step 2: Synthesis of Compound **M2-2**

Into a 50 mL single-necked flask were added compound **M2-1** (0.52 g, 2.03 mmol) and anhydrous THF (6 mL). The mixture was stirred to dissolve compound **M2-1** under a nitrogen atmosphere. Then, 2,2,2-trichloroacetyl isocyanate (0.497 g, 2.64 mmol) was added. The resulting mixture was stirred at room temperature (25 °C) for 1 h. The reaction was stopped and concentrated. The residue was used directly in the next step. The yield was calculated as 100%. LC-MS (ESI, pos. ion) m/z: 445.9 $[M+H]^+$.

Step 3: Synthesis of Compound **M2**

Into the compound **M2-2** (0.885 g, 1.99 mmol) obtained in the previous step was added methanol (6 mL), and the mixture was stirred to dissolve compound **M2-2.** Then, a solution of ammonia in methanol (2.84 mL, 19.99 mmol, 7 M) was added. The resulting mixture was stirred at room temperature for 2 h. The reaction was stopped and concentrated. The crude material was triturated with MTBE (10 mL) for 0.5 h and filtered to give 0.48 g of a white solid **M2.** The combined yield for step 2 and step 3 was 95%. LC-MS (ESI, pos. ion) m/z: 254.1 $[M+H]^+$.

**Synthesis of Intermediate Compound M3**

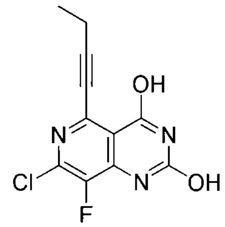

M3

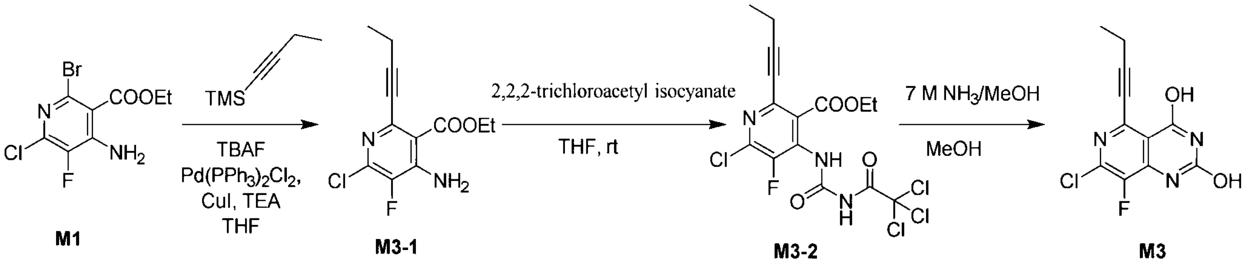


M1 M3-1 M3-2 M3

Step 1: Synthesis of Compound **M3-1**

Into a 50 mL single-necked flask were added compound **M1** (705.1 mg, 2.37 mmol), (but-1-yn-1-yl)trimethylsilane (448.89 mg, 3.56 mmol), $Pd(PPh_3)_2Cl_2$ (332.70 mg, 0.47 mmol), CuI(90.27 mg, 0.47 mmol), TEA (1.64 mL, 11.85 mmol), and anhydrous THF (10 mL). The mixture was purged with nitrogen three times. Tetrabutylammonium fluoride in THF (2.84 mL, 2.84 mmol, 1 M) was added dropwise at 25 °C. After completion of the addition, the mixture was stirred at room temperature (30 °C) for 6 h. The reaction was stopped and filtered through a celite pad. The filtrate was dried over anhydrous sodium sulfate and concentrated. The residue was purified by column chromatography using PE/DCM (v/v = 100/1-2/1) as eluent to obtain 354 mg of a yellow solid **M3-1** with a yield of 55.2%. LC-MS (ESI, pos. ion) m/z: 271.3 $[M+H]^+$; $^1H$ NMR (400 MHz, $CDCl_3$): δ 6.25 (s, 2H), 4.46 - 4.34 (m, 2H), 2.46 (q, *J* = 7.5 Hz, 2H), 1.46 - 1.37 (m, 3H), 1.25 (m, 3H).

Step 2: Synthesis of Compound **M3-2**

Into a 50 mL single-necked flask were added compound **M3-1** (350 mg, 1.29 mmol) and anhydrous THF (6 mL). The mixture was stirred to dissolve the compound **M3-1.** Then, 2,2,2-trichloroacetyl isocyanate (365 mg, 1.94 mmol) was added. The resulting mixture was stirred at room temperature (30 °C) for 1 h. The reaction was stopped and concentrated. The residue was used directly in the next step. The yield was calculated as 100%. LC-MS (ESI, pos. ion) m/z: 460.0 $[M+H]^+$.

Step 3: Synthesis of Compound **M3**

Into the compound **M3-2** obtained in the previous step was added methanol (6 mL), and the mixture was stirred to dissolve the compound **M3-2.** Then, a solution of ammonia in methanol (1.84 mL, 12.9 mmol, 7 M) was added. The resulting mixture was stirred at room temperature (30 °C) for 2 h. The reaction was stopped and concentrated. The crude material was triturated with MTBE (10 mL) for 0.5 h and filtered to give 320 mg of a white solid **M3.** The combined yield for step 2 and step 3 was 92.5%. LC-MS (ESI, pos. ion) m/z: 268.1 $[M+H]^+$.

**Synthesis of Intermediate Compound M6**

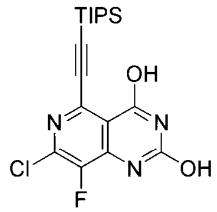

M6

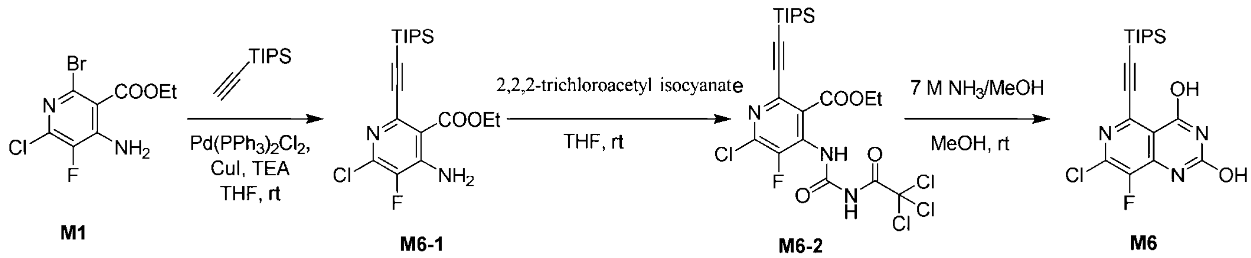


M1 M6-1 M6-2 M6

Step 1: Synthesis of Compound **M6-1**

Into a 500 mL two-necked flask were added compound **M1** (9 g, 30.25 mmol), $Pd(PPh_3)_2Cl_2$ (2.17 g, 3.03 mmol) and CuI (0.58 g, 3.03 mmol). The mixture was purged with nitrogen three times. Then, 2,2,2-trichloroacetyl isocyanate (7.32 g, 39.33 mmol), TEA (12.58 mL, 90.75 mmol) and anhydrous THF (180 mL) were added. The mixture was purged with nitrogen three times again, and stirred at room temperature for 2 h. The reaction was stopped and the mixture was filtered. The filtrate was concentrated and purified by column chromatography using PE/EA (v/v = 80/1-30/1) as eluent to obtain 10.45 g of a yellow solid **M6-1** with a yield of 86.6%. $^1H$ NMR (400 MHz, $CDCl_3$): δ 6.14 (s, 2H), 4.44 (q, *J* = 7.1 Hz, 2H), 1.40 (t, *J* = 7.1 Hz, 3H), 1.18-1.07 (m, 21H).

Step 2: Synthesis of Compound **M6-2**

Into a 500 mL single-necked flask were added compound **M6-1** (10.34 g, 25.92 mmol) and anhydrous THF (200 mL). The mixture was stirred to dissolve compound **M6-1.** Then, 2,2,2-trichloroacetyl isocyanate (6.17 g, 31.10 mmol) was added. The resulting mixture was stirred at room temperature for 0.5 h. The reaction was stopped and concentrated. The residue was used directly in the next step. The yield was calculated as 100%.

Step 3: Synthesis of Compound **M6**

Into the compound **M6-2** (15.22 g, 25.92 mmol) obtained in the previous step was added methanol (200 mL), and the mixture was stirred to dissolve compound **M6-2.** Then a solution of ammonia in methanol (18.51 mL, 129.55 mmol, 7 M) was added into the mixture. The resulting mixture was stirred at room temperature for 1.5 h. The reaction was stopped and concentrated. The crude material was triturated with PE/EA (100 mL/10 mL) for 0.5 h and filtered to give 8.72 g of a white solid M6. The combined yield for step 2 and step 3 was 85%. LC-MS (ESI, pos. ion) m/z: 396.4 $[M+H]^+$.

**Example 1: Synthesis of Compound 1**

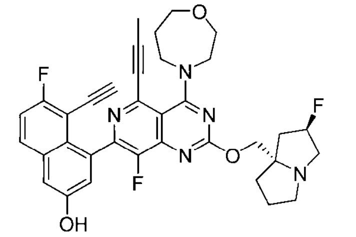

1

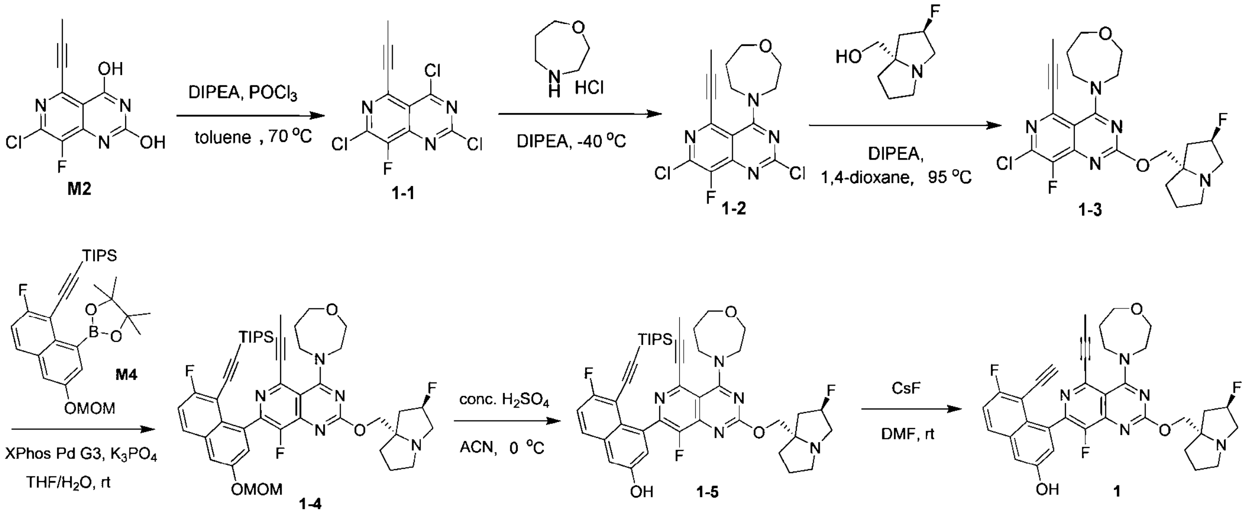


Step 1: Synthesis of Compound **1- 1**

Into a 100 mL single-necked flask were added compound **M2** (1.00 g, 3.94 mmol) and anhydrous toluene (10 mL). Then, phosphoryl chloride (1.08 mL, 11.82 mmol) and DIPEA (3.26 mL, 19.7 mmol) were added. The mixture was heated to 70 °C and stirred for 1 h. The reaction was stopped and the mixture was cooled. The mixture was concentrated and used directly in the next step. The yield was calculated as 100%.

Step 2: Synthesis of Compound **1- 2**

Into the compound **1-1** obtained in the previous step was added anhydrous dichloromethane (15 mL). The mixture was purged with nitrogen three times and stirred to dissolve compound **1-1.** The mixture was cooled to -40 °C and stirred for 5 minutes, then DIPEA (3.27 mL, 19.8 mmol) and homomorpholine hydrochloride (0.49 g, 3.56 mmol) were added sequencially. After stirring at -40 °C for 0.5 h, the reaction was stopped and the mixture was quenched with saturated ammonium chloride solution (30 mL). The mixture was warmed to room temperature and extracted with DCM (30 mL × 2). The organic phase was separated, dried over anhydrous sodium sulfate, and concentrated. The residue was purified by column chromatography using PE/EA (v/v = 3/2) as eluent to obtain 360 mg of a yellow solid compound **1-2.** The combined yield for step 1 and step 2 was 25.6%. LC-MS (ESI, pos. ion) m/z: 355.1 $[M+H]^+$.

Step 3: Synthesis of Compound 1- **3**

Into a 50 mL single-necked flask were added compound **1-2** (355 mg, 1.0 mmol), ((2*R*,7a*S*)-2-fluorohexahydro-1*H*-pyrrolizin-7a-yl)methanol (240 mg, 1.5 mmol), DIPEA (0.33 mL, 2 mmol) and anhydrous 1,4-dioxane (8 mL). The mixture was purged with nitrogen three times. The mixture was heated to 95 °C and stirred for 22 h. The reaction was stopped and cooled to room temperature. The reaction solution was concentrated, diluted with EA (30 mL), and washed with saturated ammonium chloride solution (30 mL). The phases were separated and extracted with EA (20 mL × 2). The combined organic phases were dried over anhydrous sodium sulfate and concentrated. The residue was purified by column chromatography using DCM/MeOH (v/v = 97/3) as eluent to obtain 281 mg of a yellow solid compound **1-3** with a yield of 58.83%. LC-MS (ESI, pos. ion) m/z: 478.2 $[M+H]^+$.

Step 4: Synthesis of Compound **1- 4**

Into a 25 mL single-necked flask were added compound **1-3** (200 mg, 0.42 mmol, 1 equiv.), **M4** (0.17 g, 0.34

mmol, Energy Chemical), XPhos Pd G3 (36 mg, 0.042 mmol), $K_3PO_4 \cdot 7H_2O$ (140 mg, 0.42 mmol) and $THF/H_2O$ (v/v = 5 mL/0.8 mL). The mixture was purged with nitrogen three times and stirred at room temperature for 2.5 h. The reaction was monitored by TLC, indicating significant remaining starting material **1-3.** Additional portions of **M4** (0.8 equiv.), XPhos Pd G3 (0.1 equiv.) and $K_3PO_4 \cdot 7H_2O$ (1.5 equiv.) were added. Stirring was continued for 1.5 h. The reaction remained incomplete, and the addition step was repeated until compound **1-3** was substantially consumed. Saturated ammonium chloride solution (50 mL) was added. The mixture was extracted with DCM (30 mL × 3). The combined organic phases were dried over anhydrous sodium sulfate and concentrated. The residue was purified by column chromatography using DCM/MeOH (v/v = 50/1) as eluent to obtain 244 mg of a red solid compound **1-4** with a yield of 70.4%. LC-MS (ESI, pos. ion) m/z: 829.0 $[M+H]^+$.

Step 5: Synthesis of Compound **1- 5**

Into a 25 mL single-necked flask were added compound **1-4** (235 mg, 0.28 mmol) and acetonitrile (5 mL). The mixture was cooled to 0 °C and stirred for 5 minutes. Concentrated sulfuric acid (0.04 mL, 0.74 mmol, 98% purity) was added dropwise. After stirring for 45 minutes, the reaction was stopped and quenched with saturated aqueous sodium bicarbonate solution (20 mL). The mixture was extracted with DCM (20 mL × 3). The combined organic phases were dried over anhydrous sodium sulfate and concentrated. The residue was purified by column chromatography using DCM/MeOH (v/v = 50/1) as eluent to obtain 184 mg of a red solid compound **1-5** with a yield of 82.7%, LC-MS (ESI, pos. ion) m/z: 784.4 $[M+H]^+$.

Step 6: Synthesis of Compound **1**

Into a 25 mL single-necked flask were added compound **1-5** (184 mg, 0.23 mmol), cesium fluoride (520 mg, 3.45 mmol) and DMF (1 mL). The mixture was purged with nitrogen three times and stirred at room temperature for 15 h. The reaction was stopped, and water (20 mL) was added, precipitating a solid. Filtration afforded the crude solid. The crude product was purified by column chromatography using DCM/MeOH (v/v = 30/1) as eluent to obtain 100 mg of a red solid compound **1-5,** with a yield of 67.9%. LC-MS (ESI, pos. ion) m/z: 628.3 $[M+H]^+$; HRMS (ESI): 628.2530 $[M+H]^+$; $^1H$ NMR (400 MHz, $CD_3OD$): δ 7.84 (dd, *J* = 9.0, 5.7 Hz, 1H), 7.38 - 7.27 (m, 2H), 7.23 (s, 1H), 5.30 (d, *J* = 54.0 Hz, 1H), 4.35 - 4.17 (m, 4H), 4.16 - 4.01 (m, 2H), 3.99 - 3.84 (m, 2H), 3.79 (t, *J* = 5.3 Hz, 2H), 3.41 - 3.36 (m, 1H), 3.29 - 3.18 (m, 2H), 3.09 - 2.94 (m, 1H), 2.39 - 2.19 (m, 2H), 2.16 (s, 3H), 2.15 - 2.08 (m, 2H), 2.08 - 1.81 (m, 5H). $^{19}F$ NMR (376 MHz, $CD_3OD$): δ -111.52 (1F), -140.76 (1F), -173.59 (1F).

**Example 2: Synthesis of Compound 2**

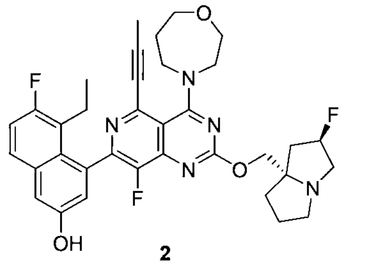

2

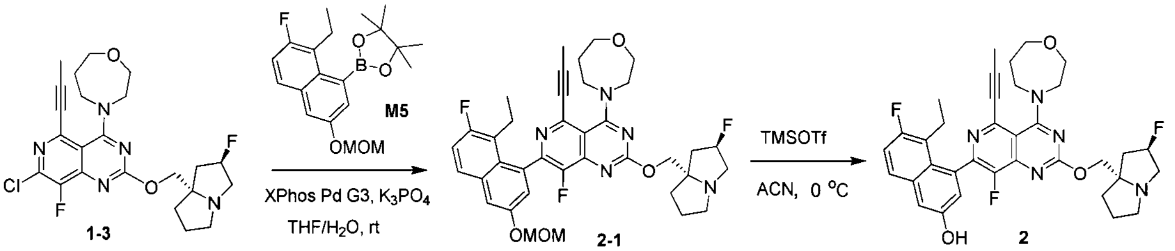


1-3 2-1 2

Step 1: Synthesis of Compound **2- 1**

Into a 25 mL single-necked flask were added compound **1-3** (80 mg, 0.17 mmol, 1 equiv.), **M5** (0.061 g, 0.17

mmol, Bide Pharm), XPhos Pd G3 (22 mg, 0.026 mmol), $K_3PO_4 \cdot 7H_2O$ (86 mg, 0.26 mmol) and THF/$H_2O$ (v/v = 3 mL/0.5 mL). The mixture was purged with nitrogen three times and stirred at room temperature for 15 h, then there was still some compound **1-3** remained. Additional portions of **M5** (1 equiv.), XPhos Pd G3 (0.1 equiv.) and $K_3PO_4 \cdot 7H_2O$ (1.5 equiv.) were added. Stirring was continued for 1.5 h. The reaction remained incomplete, and the addition step was repeated until compound **1-3** was substantially consumed. The reaction was stopped. The reaction mixture was diluted with DCM (20 mL) and washed with saturated ammonium chloride solution (30 mL). The aqueous layer was extracted with DCM (20 mL × 2). The combined organic phases were dried over anhydrous sodium sulfate and concentrated. The residue was purified by column chromatography using DCM/MeOH (v/v = 97/3) as eluent to obtain 85 mg of a yellow solid compound **2-1** with a yield 75.1%. LC-MS (ESI, pos. ion) m/z: 676.3 $[M+H]^+$.

Step 2: Synthesis of Compound **2**

Into a 25 mL single-necked flask were added compound **2-1** (85 mg, 0.13 mmol) and acetonitrile (3 mL). The mixture was cooled to 0 °C and stirred for 5 minutes, then TMSOTF (0.047 mL, 0.26 mmol) was added. After stirring for 15 minutes, the reaction was stopped and the mixture was quenched with saturated sodium bicarbonate (20 mL). The resulting mixture was extracted with DCM (20 mL × 2). The combined organic phases were dried over anhydrous sodium sulfate and concentrated. The residue was purified by preparative TLC using DCM/MeOH (v/v = 12/1) as eluent to obtain 30 mg of a yellow solid compound **2** with a yield 25.2%. LC-MS (ESI, pos. ion) m/z: 632.2 $[M+H]^+$; HRMS(ESI): 632.2852 $[M+H]^+$; $^1H$ NMR (400 MHz, $CD_3OD$): δ 7.68 (dd, *J* = 9.0, 5.8 Hz, 1H), 7.34 - 7.29 (m, 1H), 7.29 - 7.19 (m, 1H), 7.08 (s, 1H), 5.46 (d, *J* = 52.6 Hz, 1H), 4.63 - 4.42 (m, 3H), 4.33 - 4.05 (m, 4H), 4.01 - 3.86 (m, 2H), 3.83 - 3.75 (m, 2H), 3.69 - 3.54 (m, 3H), 2.61 - 2.35 (m, 3H), 2.33 - 2.25 (m, 2H), 2.25 - 2.19 (m, 2H), 2.18 (s, 3H), 2.13 - 1.99 (m, 3H), 0.83 (t, *J* = 7.4 Hz, 3H). $^{19}F$ NMR (376 MHz, $CD_3OD$): δ -120.90 (1F), -140.63 (1F), -173.92 (1F).

**Example 3: Synthesis of Compound 3**

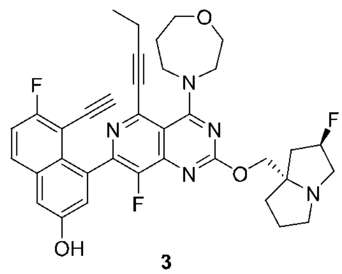

**3**

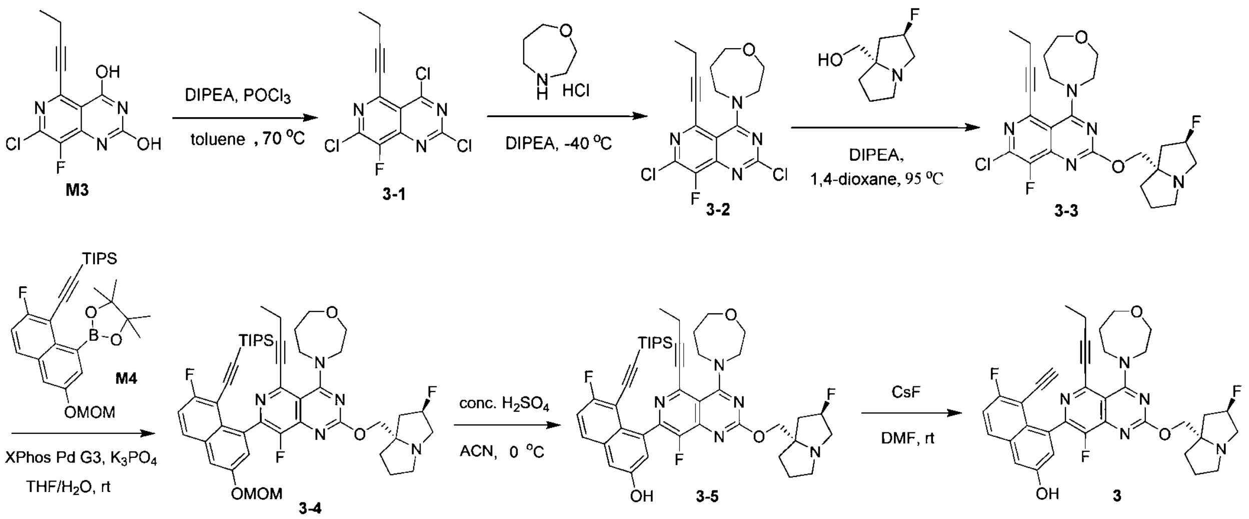


Step 1: Synthesis of Compound **3- 1**

Into a 100 mL single-necked flask were added compound **M3** (1.3 g, 4.86 mmol) and anhydrous toluene (11 mL).

Then, phosphoryl chloride (1.33 mL, 14.58 mmol) and DIPEA (4.02 mL, 24.3 mmol) were added. The mixture was heated to 70 °C and stirred for 1 h. The reaction was stopped and cooled. The mixture was concentrated and used directly in the next step. The yield was calculated as 100%.

Step 2: Synthesis of Compound **3- 2**

Into the compound **3-1** obtained in the previous step was added anhydrous dichloromethane (10 mL). The mixture was purged with nitrogen three times and stirred to dissolve compound **3-1.** The mixture was cooled to -40 °C and stirred for 5 minutes. DIPEA (4.02 mL, 24.3 mmol) was added, followed by homomorpholine hydrochloride (0.67 g, 4.86 mmol). After stirring at -40 °C for 1.5 h, the reaction was stopped and quenched with saturated ammonium chloride solution (30 mL). The mixture was warmed to room temperature and extracted with DCM (30 mL × 2). The organic phase was separated, dried over anhydrous sodium sulfate, and concentrated. The residue was purified by column chromatography using PE/EA (v/v = 65/35) as eluent to obtain 588 mg of a yellow solid compound **3-2.** The combined yield for step 1 and step 2 was 32.8%. LC-MS (ESI, pos. ion) m/z: 369.1 $[M+H]^+$.

Step 3: Synthesis of Compound **3- 3**

Into a 50 mL single-necked flask were added compound **3-2** (584 mg, 1.58 mmol), ((2*R*,7a*S*)-2-fluorohexahydro-1H-pyrrolizin-7a-yl)methanol (380 mg, 2.37 mmol), DIPEA (0.52 mL, 3.16 mmol) and anhydrous 1,4-dioxane (8 mL). The mixture was purged with nitrogen three times. The mixture was heated to 95 °C and stirred for 20 h. The reaction was stopped and cooled to room temperature. The reaction solution was concentrated, diluted with EA (30 mL), and washed with saturated ammonium chloride solution (30 mL). The phases were separated and extracted with EA (20 mL × 2). The combined organic phases were dried over anhydrous sodium sulfate and concentrated. The residue was purified by column chromatography using DCM/MeOH (v/v = 97/3) as eluent to obtain 380 mg of a yellow solid compound **3-3** with a yield of 48.8%. LC-MS (ESI, pos. ion) m/z: 492.3 $[M+H]^+$.

Step 4: Synthesis of Compound **3- 4**

Into a 50 mL single-necked flask were added compound **3-3** (200 mg, 0.41 mmol, 1 equiv.), **M4** (0.21 g, 0.61 mmol, Energy Chemical), XPhos Pd G3 (35 mg, 0.041 mmol), $K_3PO_4 \cdot 7H_2O$ (210 mg, 0.61 mmol) and $THF/H_2O$ (v/v = 5 mL/1.2 mL). The system was purged with nitrogen three times and stirred at room temperature for 1.5 h. The reaction was monitored by TLC, indicating significant remaining starting material **3-3.** Additional portions **of M4** (1 equiv.), XPhos Pd G3 (0.1 equiv.) and $K_3PO_4 \cdot 7H_2O$ (1.5 equiv.) were added. Stirring was continued for 1.5 h. The reaction remained incomplete, and the addition step was repeated until compound **3-3** was substantially consumed. The reaction was stopped and saturated ammonium chloride solution (30 mL) was added. The mixture was extracted with DCM (20 mL × 3). The combined organic phases were dried over anhydrous sodium sulfate and concentrated. The residue was purified by column chromatography using DCM/MeOH (v/v = 50/1) as eluent to obtain 432 mg of a red oil compound **3-4** with a yield of 100%. LC-MS (ESI, pos. ion) m/z: 843.0 $[M+H]^+$.

Step 5: Synthesis of Compound **3- 5**

Into a 50 mL single-necked flask were added compound **3-4** (342 mg, 0.41 mmol) and acetonitrile (6 mL). The mixture was cooled to 0 °C and stirred for 5 minutes. Concentrated sulfuric acid (0.033 mL, 0.61 mmol, 98% purity) was added dropwise. After stirring for 30 minutes, the reaction was stopped and quenched with saturated aqueous sodium bicarbonate solution (20 mL). The mixture was extracted with DCM (20 mL × 2). The combined organic phases were dried over anhydrous sodium sulfate and concentrated. The residue was purified by column chromatography using DCM/MeOH (v/v = 50/1) as eluent to obtain 160 mg of a yellow solid compound **3-5** with a yield of 49.4%, LC-MS (ESI, pos. ion) m/z: 799.1 $[M+H]^+$.

Step 6: Synthesis of Compound **3**

Into a 25 mL single-necked flask were added compound **3-5** (160 mg, 0.20 mmol), cesium fluoride (460 mg, 3.0 mmol) and DMF (0.8 mL). The mixture was purged with nitrogen three times and stirred at room temperature for 22 h. The reaction was stopped, and water (20 mL) was added, precipitating a solid. Filtration afforded the crude solid. The crude product was purified by column chromatography using DCM/MeOH (v/v = 25/1) as eluent to obtain 103 mg of a yellow solid compound **3** with a yield of 80.06%. LC-MS (ESI, pos. ion) m/z: 642.9 $[M+H]^+$; HRMS (ESI): 642.2685 $[M+H]^+$; $^1H$ NMR (400 MHz, $CD_3OD$): δ 7.92 - 7.77 (m, 1H), 7.41 - 7.27 (m, 2H), 7.24 (s, 1H), 5.31 (d, *J* = 53.8 Hz, 1H), 4.42 - 4.17 (m, 4H), 4.15 - 4.01 (m, 2H), 4.00 - 3.84 (m, 2H), 3.82 - 3.69 (m, 2H), 3.42 - 3.36 (m, 1H), 3.28 - 3.15 (m, 2H), 3.09 - 2.97 (m, 1H), 2.55

(q, *J* = 7.4 Hz, 2H), 2.43 - 1.69 (m, 9H), 1.26 (t, *J* = 7.2 Hz, 3H). $^{19}$F NMR (376 MHz, $CD_3OD$): δ -111.53 (1F), -140.75 (1F), -173.58 (1F).

**Example 4: Synthesis of Compound 4**

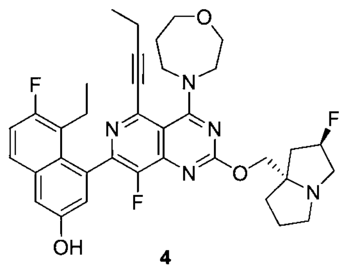

**4**

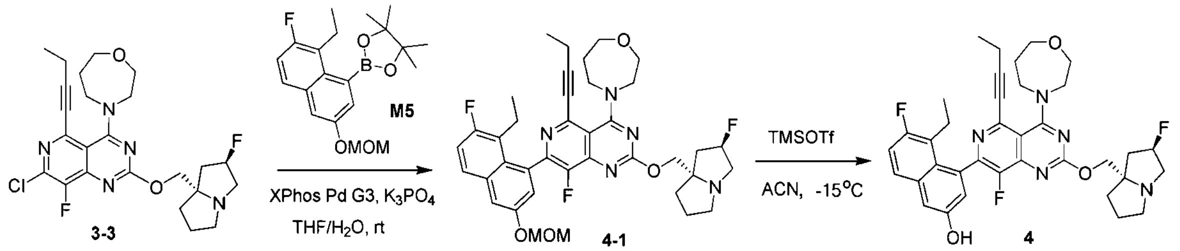


Step 1: Synthesis of Compound **4- 1**

Into a 25 mL single-necked flask were added compound **3-3** (80 mg, 0.16 mmol, 1 equiv.), **M5** (0.058 g, 0.16 mmol, Bide Pharm), XPhos Pd G3 (20 mg, 0.024 mmol), $K_3PO_4 \cdot 7H_2O$ (110 mg, 0.32 mmol) and $THF/H_2O$ (v/v = 3 mL/0.5 mL). The mixture was purged with nitrogen three times and stirred at room temperature for 3.5 h. The reaction was monitored by TLC, indicating significant remaining starting material **3-3.** Additional portions of **M5** (1 equiv.), XPhos Pd G3 (0.15 equiv.) and $K_3PO_4 \cdot 7H_2O$ (1.5 equiv.) were added. Stirring was continued for 1.5 h, the starting material **3-3** was substantially consumed. The reaction was stopped. The reaction mixture was diluted with DCM (20 mL) and washed with saturated ammonium chloride solution (30 mL). The aqueous layer was extracted with DCM (20 mL × 2). The combined organic phases were dried over anhydrous sodium sulfate and concentrated. The residue was purified by column chromatography using DCM/MeOH (v/v = 97/3) as eluent to obtain 96 mg of a yellow solid compound **4-1** with a yield 85.6%. LC-MS (ESI, pos. ion) m/z: 690.2 [M+H]$^+$.

Step 2: Synthesis of Compound **4**

Into a 25 mL single-necked flask were added compound **4-1** (96 mg, 0.14 mmol) and acetonitrile (4 mL). The mixture was cooled to -15 °C and stirred for 5 minutes. TMSOTf (0.051 mL, 0.28 mmol) was added. After stirring for 30 minutes, the reaction was stopped and quenched with saturated sodium bicarbonate (20 mL). The mixture was extracted with DCM (20 mL × 2). The combined organic phases were dried over anhydrous sodium sulfate and concentrated. The residue was purified twice by preparative TLC using DCM/MeOH (v/v = 15/1) as eluent to obtain 34 mg of a yellow solid compound **4** with a yield 37.8%. LC-MS (ESI, pos. ion) m/z: 646.6 [M+H]$^+$; HRMS(ESI): 646.3031 [M+H]$^+$;'H NMR (400 MHz, $CD_3OD$): δ 7.67 (dd, *J* = 9.0, 5.9 Hz, 1H), 7.33 - 7.29 (m, 1H), 7.28 - 7.20 (m, 1H), 7.08 (s, 1H), 5.41 (d, *J* = 53.5 Hz, 1H), 4.53 - 4.34 (m, 2H), 4.30 - 4.05 (m, 4H), 4.02 - 3.83 (m, 2H), 3.83 - 3.72 (m, 2H), 3.68 - 3.40 (m, 3H), 3.25 - 3.13 (m, 1H), 2.56 (q, *J* = 7.5 Hz, 2H), 2.50 - 2.32 (m, 3H), 2.32 - 2.20 (m, 2H), 2.19 - 2.09 (m, 3H), 2.07 - 1.95 (m, 2H), 1.28 - 1.24 (m, 3H), 0.84 (t, *J* = 7.3 Hz, 3H). $^{19}$F NMR (376 MHz, $CD_3OD$): δ -120.91 (1F), -140.55 (1F), -173.84 (1F).

**Example 5: Synthesis of Compound 5**

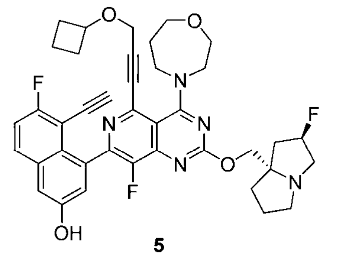

5

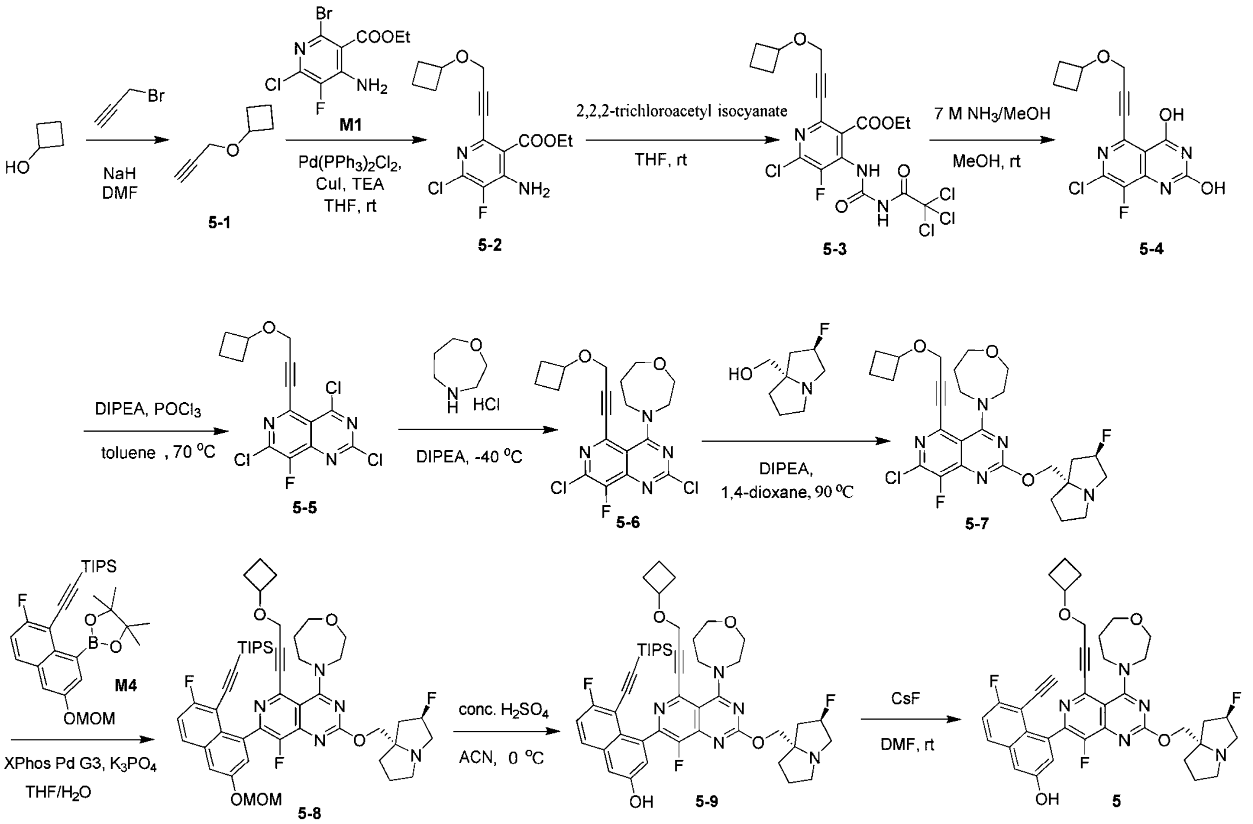


Step 1: Synthesis of Compound **5- 1**

Into a 100 mL two-neck flask were added NaH (3.74 g, 93.6 mmol, purity: 60% ) and anhydrous DMF (30 mL). The mixture was purged with nitrogen three times and the mixture was cooled to 0°C with stirring. Cyclobutanol (4.5 g, 62.4 mmol) was added dropwise slowly, followed by stirring at 0 °C for 1 h. 3-Bromo-1-(trimethylsilyl)-1-propyne (10.73 g, 56.16 mmol) was then added, and stirring continued at 0 °C for 0.5 h. The temperature was raised to 30 °C and stirring maintained for 12 h. The reaction was stopped and cooled. The mixture was quenched with saturated ammonium chloride solution (100 mL) and extracted with ethyl acetate (50 mL × 3). The combined organic phases were washed with water (50 mL × 5), dried over anhydrous sodium sulfate, and concentrated to obtain 7.6 g of a viscous liquid, which was directly used in the next step.

Step 2: Synthesis of Compound **5-2**

Into a 50 mL single-necked flask were added compound **M1** (4.05 g, 9.53 mmol), the compound **5-1** (7.6 g) from the previous step, $Pd(PPh_3)_2Cl_2$ (1.338 g, 1.91 mmol), CuI(363 mg, 1.91 mmol), TEA (13.21 mL, 95.3 mmol) and anhydrous THF (30 mL). The mixture was purged with nitrogen three times. The mixture was stirred at 35 °C for 1.5 h. The reaction was stopped and filtered through a celite pad. The filtrate was diluted with dichloromethane (100 mL), washed with saturated ammonium chloride solution (20 mL × 3) followed by saturated brine (20 mL × 3), dried over anhydrous sodium sulfate, filtered, and concentrated. The residue was purified by column chromatography using PE/EA (v/v = 100/1-5/1) as eluent to obtain 2.0 g of a yellow oil compound **5-2** with a yield 9.9%. LC-MS (ESI, pos. ion) m/z: 327.0 $[M+H]^+$

Step 3: Synthesis of Compound **5-3**

Into a 100 mL single-necked flask were added compound **5-2** (2.0 g, 6.12 mmol) and anhydrous THF (20 mL). The mixture was stirred to dissolve compound **5-2.** Then, 2,2,2-trichloroacetyl isocyanate (1.38 g, 7.34 mmol) was added. The resulting mixture was stirred at room temperature for 1 h. The reaction was stopped and concentrated to obtain a brown solid. The residue was used directly in the next step. The yield was calculated as 100%.

Step 4: Synthesis of Compound **5- 4**

Into the compound **5-2** obtained in the previous step was added methanol (20 mL), and the mixture was stirred to dissolve compound **5-2.** Then, a solution of ammonia in methanol (8.21 mL, 57.5 mmol, 7 M) was added. The mixture was stirred at room temperature for 4 h. The reaction was then terminated and filtered under vacuum. The resulting solid was washed with petroleum ether (20 mL) to obtain 1.84 g of a white solid compound **5-4** with a yield 98.92%. LC-MS (ESI, pos. ion) m/z: 324.1 $[M+H]^+$.

Step 5: Synthesis of Compound **5-5**

Into a 100 mL single-necked flask were added compound **5-4** (0.9 g, 2.78 mmol) and anhydrous toluene (20 mL). Then, phosphoryl chloride (1.55 mL, 16.68 mmol) and DIPEA (2.30 mL, 13.90 mmol) were added. The mixture was heated to 70 °C and stirred for 1 h under $N_2$. The reaction was stopped and cooled. The mixture was concentrated and used directly in the next step. The yield was calculated as 100%.

Step 6: Synthesis of Compound **5-6**

Into the compound **5-5** obtained in the previous step was added anhydrous dichloromethane (10 mL). The mixture was purged with nitrogen three times and stirred to dissolve compound **3-1.** The mixture was cooled to -40 °C and stirred for 5 minutes. DIPEA (2.30 mL, 13.9 mmol) was added, followed by homomorpholine hydrochloride (0.383 g, 2.78 mmol). After stirring at -40 °C for 0.5 h, the reaction was stopped and the mixture was diluted with dichloromethane (100 mL). The organic phase was washed with saturated ammonium chloride solution (100 mL × 2). The organic layer was separated, dried over anhydrous sodium sulfate, and concentrated. The residue was purified by column chromatography using PE/EA (v/v = 100/1-9/1) as eluent to obtain 700 mg of a yellow solid compound **5-6** with a yield 59.2% over two steps. LC-MS (ESI, pos. ion) m/z: 425.1 $[M+H]^+$.

Step 7: Synthesis of Compound **5-7**

Into a 50 mL single-necked flask were added compound **5-6** (700 mg, 1.65 mmol), ((2*R*,7a*S*)-2-fluorohexahydro-1H-pyrrolizin-7a-yl)methanol (657 mg, 4.13 mmol), DIPEA (1.37 mL, 8.25 mmol) and anhydrous 1,4-dioxane (10 mL). The mixture was purged with nitrogen three times. The mixture was heated to 90 °C and stirred for 12 h. The reaction was stopped and cooled to room temperature. The reaction solution was concentrated, diluted with DCM (50 mL), and washed with saturated ammonium chloride solution (20 mL × 3). The phases were separated. The organic phases were dried over anhydrous sodium sulfate and concentrated. The residue was purified by column chromatography using PE/EA (v/v = 100/1-1/1) as eluent to obtain 645 mg of a yellow solid compound **5-7** with a yield of 71.5%. LC-MS (ESI, pos. ion) m/z: 548.3 $[M+H]^+$.

Step 8: Synthesis of Compound **5-8**

Into a 50 mL single-necked flask were added compound **5-7** (350 mg, 0.64 mmol), **M4** (656 mg, 1.28 mmol), Xphos Pd G3 (135.4 mg, 0.16 mmol), $K_3PO_4 \cdot 7H_2O$ (191 mg, 0.90 mmol) and THF/$H_2O$ (v/v = 6 mL/0.5 mL). The mixture was purged with nitrogen three times. The mixture was heated to 30 °C and stirred for 6 h. The reaction was stopped and added saturated ammonium chloride solution (30 mL). The mixture was extracted with DCM (20 mL × 3). The combined organic phases were dried over anhydrous sodium sulfate and concentrated. The residue was purified by column chromatography using DCM/MeOH (v/v = 100/1-19/1) as eluent to obtain 323 mg of a tan solid compound **5-8** with a yield of 56.3%. LC-MS (ESI, pos. ion) m/z: 899.3 $[M+H]^+$.

Step 9: Synthesis of Compound **5-9**

Into a 50 mL single-necked flask were added compound **5-8** (323 mg, 0.36 mmol) and acetonitrile (10 mL). The mixture was cooled to 0 °C and stirred for 5 minutes. Concentrated sulfuric acid (0.096 mL, 1.8 mmol, 98% purity) was

added dropwise. After stirring for 30 minutes, the reaction was stopped and the mixture quenched with saturated aqueous sodium bicarbonate solution (20 mL). The mixture was extracted with DCM (20 mL × 2). The combined organic phases were dried over anhydrous sodium sulfate and concentrated to obtain 300 mg of a brown solid compound **5-9** with a yield of 97.7%, LC-MS (ESI, pos. ion) m/z: 855.2 $[M+H]^+$.

Step 10: Synthesis of Compound **5**

Into a 25 mL single-necked flask were added **5-9** (264 mg, 0.35 mmol), cesium fluoride (532 mg, 3.5 mmol), and DMF (2 mL). The mixture was purged with nitrogen three times and stirred at room temperature for 4 h. The reaction was stopped, and water (8 mL) was added, precipitating a solid. Filtration afforded a crude solid. The crude product was purified by column chromatography using DCM/MeOH (v/v = 12/1) as eluent to obtain 105 mg of a brown solid compound **5** with a yield of 42.8%. LC-MS (ESI, pos. ion) m/z: 698.3 $[M+H]^+$; HRMS (ESI): 698.2950 $[M+H]^+$; $^1$H NMR (400 MHz, $CD_3OD$): δ 7.84 (dd, *J* = 9.1, 5.7 Hz, 1H), 7.37 - 7.27 (m, 2H), 7.27 - 7.23 (m, 1H), 5.34 (d, *J* = 53.5 Hz, 1H), 4.43 - 4.30 (m, 4H), 4.29 - 4.04 (m, 5H), 3.97 - 3.85 (m, 2H), 3.82 - 3.74 (m, 2H), 3.50 - 3.33 (m, 3H), 3.15 - 3.05 (m, 1H), 2.46 - 2.13 (m, 6H), 2.13 - 1.99 (m, 4H), 2.00 - 1.83 (m, 3H), 1.77 - 1.45 (m, 2H). $^{19}$F NMR (376 MHz, $CD_3OD$): δ -111.40 - -111.50 (1F), -139.53 (1F), -173.27 - -174.07 (1F).

**Example 6: Synthesis of Compound 6**

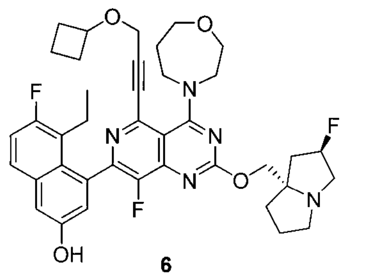

6

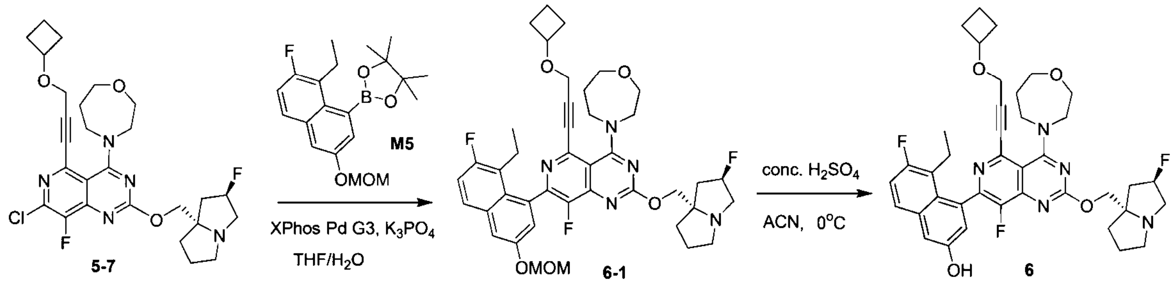


Step 1: Synthesis of Compound **6-1**

Into a 25 mL single-necked flask were added compound **5-7** (300 mg, 0.55 mmol), **M5** (0.297 g, 0.83 mmol), Xphos Pd G3 (116.4 mg, 0.14 mmol), $K_3PO_4 \cdot 7H_2O$ (191 mg, 0.90 mmol) and THF/$H_2O$ (v/v = 6 mL/0.5 mL). The mixture was purged with nitrogen three times. The mixture was heated to 30 °C and stirred for 6 h. The reaction was stopped diluted with dichloromethane (20 mL), washed with saturated ammonium chloride solution (30 mL) and extracted with dichloromethane (20 mL × 2). The combined organic phases were dried over anhydrous sodium sulfate and concentrated. The residue was purified by column chromatography using DCM/MeOH (v/v = 10011-1911) as eluent to obtain 245 mg of a tan solid compound **6-1** with a yield of 60.0%. LC-MS (ESI, pos. ion) m/z: 747.0 $[M+H]^+$.

Step 2: Synthesis of Compound **6**

Into a 50 mL single-necked flask were added **6-1** (245 mg, 0.33 mmol) and acetonitrile (5 mL). The mixture was cooled to 0 °C and stirred for 5 minutes. Concentrated sulfuric acid (0.088 mL, 1.56 mmol, 98% purity) was added dropwise. After stirring at 0 °C for 30 minutes, the reaction was stopped and the mixture was quenched with saturated aqueous sodium bicarbonate solution (20 mL). The mixture was extracted with DCM (20 mL × 2). The combined organic phases were dried over anhydrous sodium sulfate and concentrated. The residue was purified by column chromatography

using DCM/MeOH (v/v = 14/1) as eluent to obtain 110 mg of a yellow solid compound **6** with a yield of 47.7%, LC-MS (ESI, pos. ion) m/z: 702.3 $[M+H]^+$; HRMS(ESI): 702.3264 $[M+H]^+$; $^1H$ NMR (400 MHz, $CD_3OD$): δ 7.66 (dd, *J* = 9.0, 5.9 Hz, 1H), 7.32 - 7.28 (m, 1H), 7.27 - 7.20 (m, 1H), 7.11 - 7.07 (m, 1H), 5.31 (d, *J* = 54.0 Hz, 1H), 4.40 - 4.25 (m, 4H), 4.25 - 4.04 (m, 5H), 3.96 - 3.83 (m, 2H), 3.81 - 3.73 (m, 2H), 3.40 - 3.32 (m, 1H), 3.30 - 3.18 (m, 2H), 3.09 - 2.99 (m, 1H), 2.46 - 2.29 (m, 2H), 2.29 - 2.16 (m, 4H), 2.16 - 2.05 (m, 3H), 2.04 - 1.85 (m, 5H), 1.77 - 1.47 (m, 2H), 0.83 (t, *J* = 7.3 Hz, 3H). $^{19}F$ NMR (376 MHz, $CD_3OD$): δ -120.65 - -120.90 (1F), -139.04 (1F), -173.22 --173.83 (1F).

**Example 7: Synthesis of Compound 7**

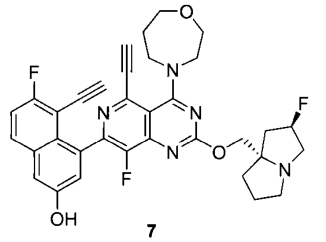

7

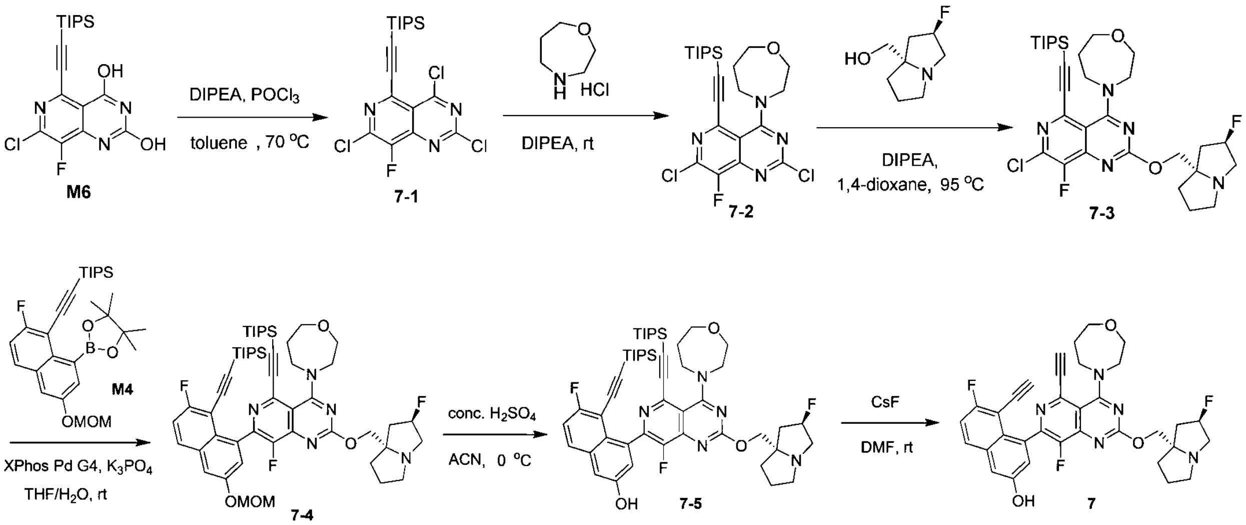


Step 1: Synthesis of Compound **7-1**

Into a 250 mL single-necked flask were added compound **M6** (2.0 g, 5.1 mmol) and anhydrous toluene (40 mL). Then, phosphoryl chloride (1.9 mL, 2.04 mmol) and DIPEA (3.4 mL, 20.4 mmol) were added. The mixture was heated to 100 °C and stirred for 1 h. The reaction was stopped and cooled. The mixture was concentrated and used directly in the next step. The yield was calculated as 100%.

Step 2: Synthesis of Compound **7-2**

Into the compound **7-1** obtained in the previous step was added anhydrous dichloromethane (30 mL). The mixture was purged with nitrogen three times and stirred to dissolve. DIPEA (5.3 g, 40.8 mmol) was added, followed by slow dropwise addition of homomorpholine hydrochloride (0.88 g, 6.4 mmol). After stirring for 1 h, the reaction was stopped and the mixture was quenched with saturated ammonium chloride solution (50 mL). The mixture was extracted with DCM (50 mL × 2). The organic phase was separated, dried over anhydrous sodium sulfate, and concentrated. The residue was purified by column chromatography using PE/EA (v/v = 10/1) as eluent to obtain 1.73 g of a yellow solid compound **7-2.** The combined yield for step 1 and step 2 was 68.8%. LC-MS (ESI, pos. ion) m/z:497.3 $[M+H]^+$.

Step 3: Synthesis of Compound **7-3**

Into a 100 mL single-necked flask were added compound **7-2** (1 g, 2.01 mmol), ((2*R*,7a*S*)-2-fluorohexahydro-1*H*-pyrrolizin-7a-yl)methanol (480 mg, 3.01 mmol), DIPEA (0.66 mL, 4.02 mmol), and anhydrous 1,4-dioxane (25 mL). The mixture was purged with nitrogen three times. The mixture was heated to 95 °C and stirred for 20 h. The reaction was stopped and cooled to room temperature. The reaction solution was concentrated, diluted with EA (30 mL), and washed with saturated ammonium chloride solution (30 mL). The phases were separated and extracted with EA (20 mL × 2). The combined organic phases were dried over anhydrous sodium sulfate and concentrated. The residue was purified by column chromatography using DCM/MeOH (v/v = 50/1) as eluent to obtain 860 mg of a yellow solid compound **7-3** with a yield of 68.9%. LC-MS (ESI, pos. ion) m/z: 620.5 $[M+H]^+$.

Step 4: Synthesis of Compound **7-4**

Into a 50 mL single-necked flask were added compound **7-3** (200 mg, 0.32 mmol, 1 equiv.), **M4** (0.33 g, 0.64 mmol, Jiangsu Aikon Biopharma), Xphos Pd G4 (83 mg, 0.096 mmol), $K_3PO_4 \cdot 7H_2O$ (430 mg, 1.28 mmol) and THF/$H_2O$ (v/v = 5.1 mL/1.7 mL). The mixture was purged with nitrogen three times and stirred at room temperature for 18 h. The reaction was quenched and the mixture was diluted with ethyl acetate (15 mL). The mixture was washed with saturated sodium chloride solution (20 mL) and extracted with ethyl acetate (20 mL × 2). The combined organic phases were dried over anhydrous sodium sulfate and concentrated. The residue was purified by column chromatography using DCM/MeOH (v/v = 30/1) as eluent to obtain 282 mg of a yellow solid compound **7-4** with a yield of 90.1 %.

Step 5: Synthesis of Compound **7-5**

Into a 50 mL single-necked flask were added compound **7-4** (220 mg, 0.23 mmol) and acetonitrile (3 mL). The mixture was cooled to 0 °C and stirred for 5 minutes. Concentrated sulfuric acid (0.069 mL, 0.69 mmol, 98% purity) was added dropwise. After stirring for 60 minutes, the reaction was stopped and the mixture was quenched with saturated aqueous sodium bicarbonate solution (20 mL). The mixture was extracted with DCM (20 mL × 2). The combined organic phases were dried over anhydrous sodium sulfate and concentrated. The residue was purified by column chromatography using DCM/MeOH (v/v = 20/1) as eluent to obtain 155 mg of a yellow solid compound **7-5** with a yield of 73.8%, LC-MS (ESI, pos. ion) m/z: 463.8 $[M+2H]^{2+}$.

Step 6: Synthesis of Compound **7**

Into a 25 mL single-necked flask were added compound **7-5** (150 mg, 0.16 mmol), cesium fluoride (489 mg, 3.2 mmol) and DMF (1 mL). The mixture was purged with nitrogen three times and stirred at room temperature for 3.5 h. The reaction was stopped, and water (20 mL) was added, then there was precipitated a solid. The mixture was filtered to afforded the crude solid. The crude product was purified by column chromatography using DCM/MeOH (v/v = 25/1) as eluent to obtain 64 mg of a yellow solid compound **7** with a yield of 64.3%. LC-MS (ESI, pos. ion) m/z: 614.3$[M+H]^+$; HRMS (ESI): 614.2385 $[M+H]^+$; $^1H$ NMR (400 MHz, $CD_3OD$) δ 7.88 (dd, *J* = 9.1, 5.7 Hz, 1H), 7.41 - 7.25 (m, 3H), 5.45 - 5.25 (m, 1H), 4.66 - 4.46 (m, 1H), 4.41 - 4.21 (m, 4H), 4.20 - 4.05 (m, 2H), 4.01 - 3.89 (m, 2H), 3.86 - 3.77 (m, 2H), 3.44 - 3.35 (m, 2H), 3.32 - 3.25 (m, 2H), 3.14 - 3.03 (m, 1H), 2.34 - 1.94 (m, 8H). $^{19}F$ NMR (376 MHz, $CD_3OD$) δ -114.48 (1F), -138.98 (1F), -173.66 (1F).

**Example 8: Synthesis of Compound 8**

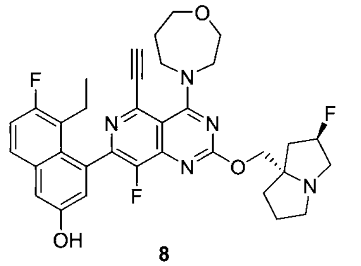

8

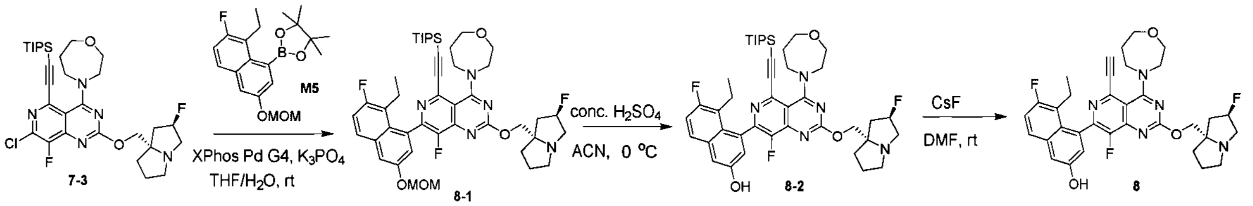


Step 1: Synthesis of Compound **8-1**

Into a 50 mL single-necked flask were added compound **7-3** (200 mg, 0.32 mmol, 1 equiv.), **M5** (0.35 g, 0.96 mmol), Xphos Pd G4 (110 mg, 0.13 mmol), $K_3PO_4 \cdot 7H_2O$ (433 mg, 1.28 mmol) and THF/$H_2O$ (v/v = 6 mL/2 mL). The mixture was purged with nitrogen three times and stirred at room temperature for 18 h. The reaction was quenched and diluted with ethyl acetate (15 mL). The mixture was washed with saturated sodium chloride solution (20 mL) and extracted with ethyl acetate (20 mL). The combined organic phases were dried over anhydrous sodium sulfate and concentrated. The residue was purified by column chromatography using DCM/MeOH (v/v = 30/1) as eluent to obtain 204 mg of a yellow solid compound **8-1** with a yield of 77.3%. LC-MS (ESI, pos. ion) m/z: 818.7 $[M+H]^+$.

Step 2: Synthesis of Compound **8-2**

Into a 50 mL single-necked flask were added compound **8-1** (204 mg, 0.25 mmol) and acetonitrile (5 mL). The mixture was cooled to 0 °C and stirred for 5 minutes. Concentrated sulfuric acid (0.075 mL, 0.75 mmol, 98% w/w) was added dropwise. After stirring at 0 °C for 120 minutes, the reaction was stopped and quenched with saturated aqueous sodium bicarbonate solution (20 mL). The mixture was extracted with DCM (15 mL $\times$ 2). The combined organic phases were dried over anhydrous sodium sulfate and concentrated. The residue was purified by column chromatography using DCM/MeOH (v/v = 20/1) as eluent to obtain 165 mg of a yellow solid compound **8-2** with a yield of 85.4%, LC-MS (ESI, pos. ion) m/z: 774.7 $[M+H]^+$.

Step 3: Synthesis of Compound **8**

Into a 25 mL single-necked flask were added compound **8-2** (165 mg, 0.21 mmol), cesium fluoride (260 mg, 1.68 mmol) and DMF (1 mL). The mixture was purged with nitrogen three times and stirred at room temperature for 4 h. The reaction was stopped, and water (20 mL) was added, precipitating a solid. Filtration afforded the crude solid. The crude product was purified by column chromatography using DCM/MeOH (v/v = 20/1) as eluent to obtain 85 mg of a yellow solid compound **8** with a yield of 64.5%. LC-MS (ESI, pos. ion) m/z: 618.4$[M+H]^+$; HRMS (ESI): 618.2726 $[M+H]^+$; $^1H$ NMR (400 MHz, $CD_3OD$) $\delta$ 7.69 (dd, $J$ = 9.0, 5.8 Hz, 1H), 7.35 - 7.31 (m, 1H), 7.26 - 7.22 (m, 1H), 7.13 - 7.09 (m, 1H), 5.34 (d, $J$ = 53.5 Hz, 1H), 4.64 - 4.42 (m, 1H), 4.41 - 4.28 (m, 3H), 4.27 - 4.05 (m, 4H), 4.00 - 3.86 (m, 2H), 3.84 - 3.75 (m, 2H), 3.43 - 3.34 (m, 1H), 3.30 - 3.21 (m, 1H), 3.12 - 3.02 (m, 1H), 2.47 - 2.24 (m, 4H), 2.17 - 1.88 (m, 6H), 0.86 (t, $J$ = 7.4 Hz, 3H). $^{19}F$ NMR (376 MHz, $CD_3OD$) $\delta$ -120.83 (1F), -138.55 (1F), -173.68 (1F).

**Example 9: Synthesis of Compound 9**

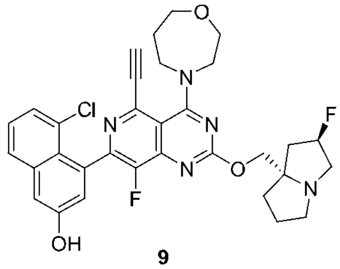

9

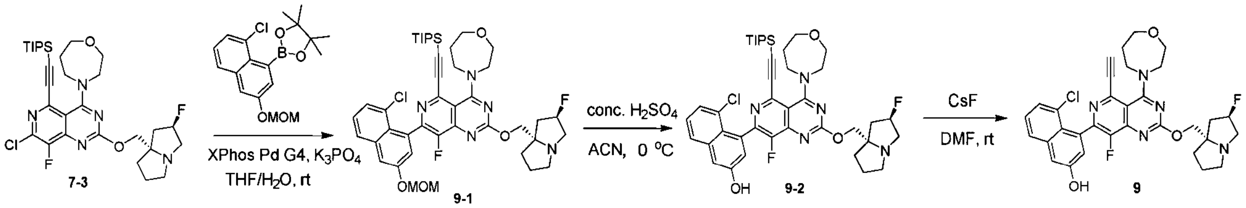


Step 1: Synthesis of Compound **9-1**

Into a 50 mL single-necked flask were added compound **7-3** (250 mg, 0.40 mmol, 1 equiv.), 2-(8-chloro-3-(methoxymethoxy)naphthalen-1-yl)-4,4,5,5-tetramethyl-1,3,2-dioxaborolane (0.42 g, 1.20 mmol, Aikon Biotech), XPhos Pd G4 (137 mg, 0.16 mmol), $K_3PO_4 \cdot 7H_2O$ (541 mg, 1.6 mmol) and THF/$H_2O$ (v/v = 6 mL/2 mL). The reaction mixture was purged with nitrogen three times and stirred at room temperature for 24 h. The reaction was quenched, diluted with dichloromethane (15 mL), and washed with saturated ammonium chloride solution (20 mL). The aqueous layer was extracted with dichloromethane (20 mL × 2). The combined organic phases were dried over anhydrous sodium sulfate and concentrated. The residue was purified by column chromatography using DCM/MeOH (v/v = 50/1) as eluent to obtain 200 mg of a yellow solid compound **9-1** with a yield of 61.5%. LC-MS (ESI, pos. ion) m/z: 806.4 $[M+H]^+$.

Step 2: Synthesis of Compound **9-2**

Into a 50 mL single-necked flask were added compound **9-1** (200 mg, 0.25 mmol) and acetonitrile (5 mL). The mixture was cooled to 0 °C and stirred for 5 minutes. Concentrated sulfuric acid (0.041 mL, 0.75 mmol, 98% w/w) was added dropwise. After stirring at 0 °C for 90 minutes, the reaction was stopped and quenched with saturated aqueous sodium bicarbonate solution (20 mL). The mixture was extracted with DCM (15 mL × 2). The combined organic phases were dried over anhydrous sodium sulfate and concentrated. The residue was purified by column chromatography using DCM/MeOH (v/v = 30/1) as eluent to obtain 120 mg of a yellow solid compound **9-2** with a yield of 63.5%, LC-MS (ESI, pos. ion) m/z: 762.7 $[M+H]^+$.

Step 3: Synthesis of Compound **9**

Into a 25 mL single-necked flask were added compound **9-2** (120 mg, 0.16 mmol), cesium fluoride (190 mg, 1.28 mmol) and DMF (1 mL). The mixture was purged with nitrogen three times and stirred at room temperature for 5 h. The reaction was stopped, and water (20 mL) was added, precipitating a solid. Filtration afforded the crude solid. The crude product was purified by column chromatography using DCM/MeOH (v/v = 20/1) as eluent to obtain 62 mg of a yellow solid compound **9** with a yield of 65.0%. LC-MS (ESI, pos. ion) m/z: 606.4$[M+H]^+$; HRMS (ESI): 606.2089 $[M+H]^+$; $^1H$ NMR (400 MHz, $CD_3OD$) δ 7.75 (dd, *J* = 11.0, 6.4 Hz, 1H), 7.40 - 7.32 (m, 3H), 7.24 - 7.21 (m, 1H), 5.45 - 5.25 (m, 1H), 4.43 - 4.23 (m, 5H), 4.22 - 4.00 (m, 3H), 3.98 - 3.84 (m, 2H), 3.83 - 3.75 (m, 2H), 3.48 - 3.37 (m, 1H), 3.31 - 3.28 (m, 1H), 3.07 (d, *J* = 5.6 Hz, 1H), 2.34 - 1.87 (m, 8H). $^{19}F$ NMR (376 MHz, $CD_3OD$) δ -139.16 (1F), -173.66 (1F).

**Example 10: Synthesis of Compound 10**

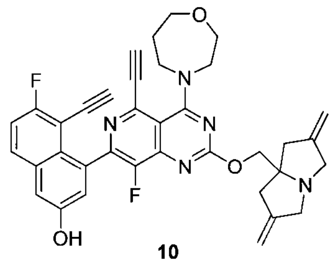

10

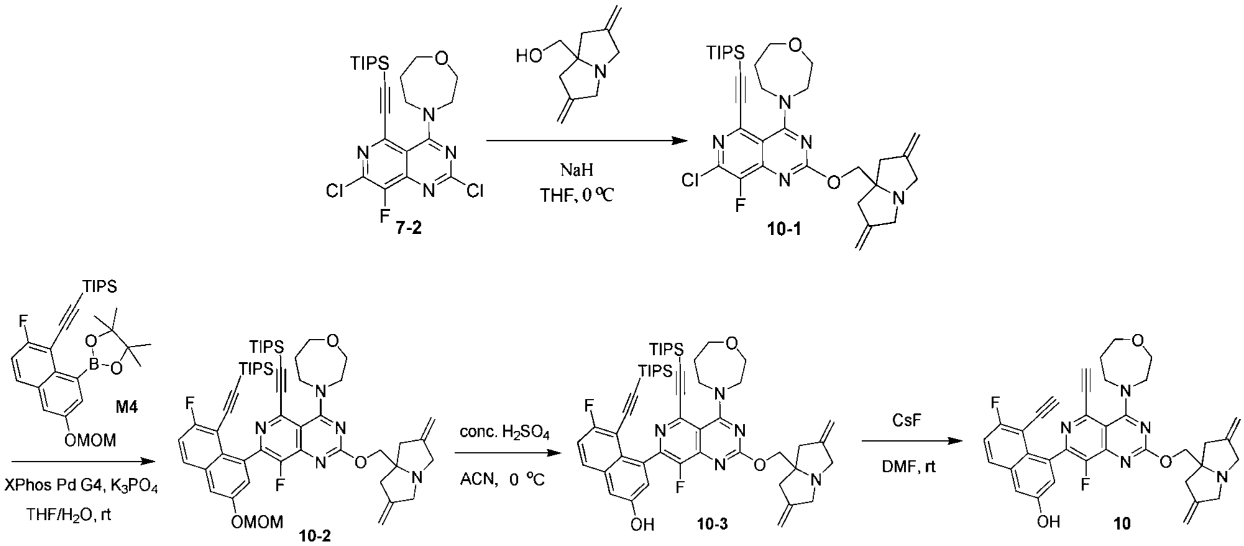


7-2 10-1 M4 10-2 10-3 10

Step 1: Synthesis of Compound **10-1**

Into a 100 mL two-necked flask was charged sodium hydride (0.070 g, 1.76 mmol, 60% w/w). The flask was purged with nitrogen, followed by addition of THF (7 mL). The mixture was cooled to 0 °C and stirred for 5 min. A solution of (2,6-dimethylenetetrahydro-1*H*-pyrrolizin-7a(5*H*)-yl)methanol (0.073 g, 0.44 mmol, Aikon Biotech) in THF (3 mL) was added dropwise slowly while maintaining 0 °C. A solution of compound **7-2** (220 mg, 0.44 mmol) in THF (5 mL) was added dropwise slowly. Stirring was stopped, and the reaction was quenched with saturated ammonium chloride solution (30 mL). The mixture was separated into layers, and the aqueous phase was extracted with ethyl acetate (20 mL × 2). The combined organic phases were dried over anhydrous sodium sulfate and concentrated. The residue was purified by column chromatography using DCM/MeOH (v/v = 50/1) as eluent to obtain 160 mg of a yellow solid compound **10-1** with a yield 57.7%. LC-MS (ESI, pos. ion) m/z: 626.5 $[M+H]^+$.

Step 2: Synthesis of Compound **10-2**

Into a 50 mL two-necked flask were added compound **10-1** (160 mg, 0.33 mmol, 1 equiv.), **M4** (0.59 g, 1.16 mmol), Xphos Pd G4 (113 mg, 0.13 mmol), $K_3PO_4 \cdot 7H_2O$ (447 mg, 1.32 mmol) and $THF/H_2O$ (v/v = 5.1 mL/1.7 mL). The mixture was purged with nitrogen three times and stirred at room temperature for 18 h. The reaction was stopped and the mixture was diluted with EA (15 mL). The mixture was washed with saturated ammonium chloride solution (20 mL) and extracted with EA (20 mL × 2). The combined organic phases were dried over anhydrous sodium sulfate and concentrated. The residue was purified by column chromatography using DCM/MeOH (v/v = 30/1) as eluent to obtain 258 mg of a yellow solid compound **10-2** with a yield of 93.4%. LC-MS (ESI, pos. ion) m/z: 976.8 $[M+H]^+$.

Step 3: Synthesis of Compound **10-3**

Into a 50 mL single-necked flask were added compound **10-2** (303 mg, 0.31 mmol) and acetonitrile (5 mL). The mixture was cooled to 0 °C and stirred for 5 minutes. Concentrated sulfuric acid (0.051 mL, 0.93 mmol, 98% w/w) was added dropwise. After stirring at 0 °C for 120 minutes, the reaction was stopped and quenched with saturated aqueous sodium bicarbonate solution (20 mL). The mixture was extracted with DCM (15 mL × 2). The combined organic phases

were dried over anhydrous sodium sulfate and concentrated. The residue was purified by column chromatography using DCM/MeOH (v/v = 20/1) as eluent to obtain 243 mg of a yellow solid compound **10-3** with a yield of 84%, LC-MS (ESI, pos. ion) m/z: 932.8 $[M+H]^+$.

Step 4: Synthesis of Compound **10**

Into a 25 mL single-necked flask were added compound **10-3** (243 mg, 0.26 mmol), cesium fluoride (320 mg, 2.08 mmol), and DMF (1 mL). The mixture was purged with nitrogen three times and stirred at room temperature for 12 h. The reaction was stopped, and water (20 mL) was added, precipitating a solid. Filtration afforded the crude solid. The crude product was purified by column chromatography using DCM/MeOH (v/v = 20/1) as eluent to obtain 113 mg of a yellow solid compound **10** with a yield of 70.0%. LC-MS (ESI, pos. ion) m/z: 620.5$[M+H]^+$; HRMS (ESI): 620.2456 $[M+H]^+$; $^1H$ NMR (400 MHz, $CD_3OD$) δ 7.91 - 7.79 (m, 1H), 7.39 - 7.18 (m, 3H), 5.10 - 4.93 (m, 4H), 4.41 - 4.19 (m, 4H), 4.19 - 4.03 (m, 2H), 4.00 - 3.85 (m, 2H), 3.86 - 3.71 (m, 4H), 3.44 - 3.22 (m, 4H), 2.86 - 2.70 (m, 2H), 2.68 - 2.50 (m, 2H), 2.23 - 1.91 (m, 2H). $^{19}F$ NMR (376 MHz, $CD_3OD$) δ -111.43 (1F), -138.73 (1F).

**Example 11: Synthesis of Compound 11**

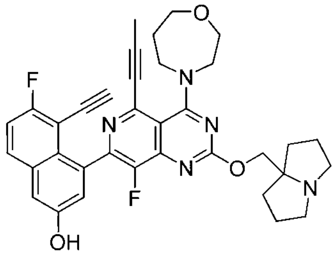

**11**

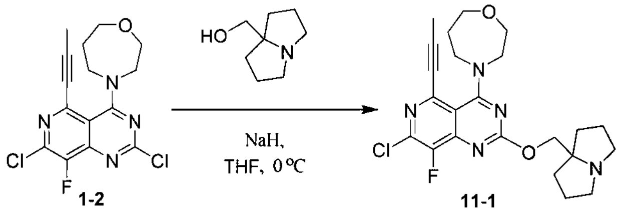


1-2

11-1

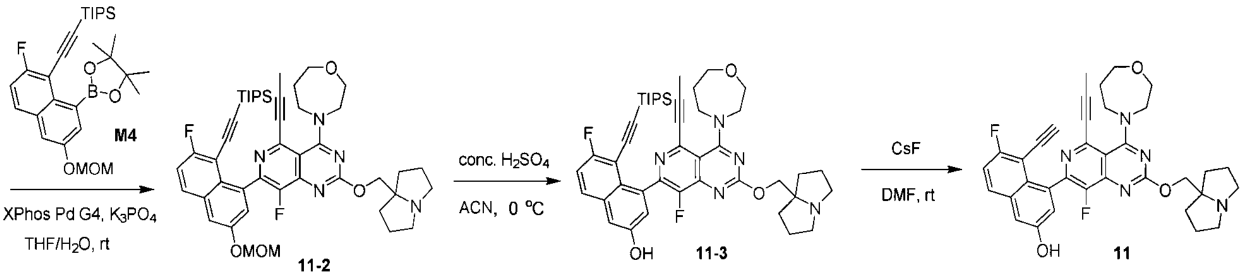


M4

11-2

11-3

11

Step 1: Synthesis of Compound **11-1**

Into a 100 mL two-necked flask was charged sodium hydride (0.18 g, 4.52 mmol, 60% w/w). The flask was purged with nitrogen, followed by addition of THF (7 mL). The mixture was cooled to 0 °C and stirred for 5 min. A solution of (hexahydro-1*H*-pyrrolizin-7a-yl)methanol (0.16 g, 1.13 mmol, Shaoyuan Technology) in THF (3 mL) was added dropwise slowly while maintaining 0 °C for 25 min. A solution of compound **1-2** (400 mg, 1.13 mmol) in THF (5 mL) was added dropwise slowly. The mixture was reacted at 0 °Cfor 12 h. Stirring was stopped, and the reaction was quenched with saturated ammonium chloride solution (20 mL). The mixture was separated into layers, and the aqueous phase was extracted with DCM (20 mL × 2). The combined organic phases were dried over anhydrous sodium sulfate and concentrated. The residue was purified by column chromatography using DCM/MeOH (v/v = 50/1) as eluent to obtain 160 mg of a yellow solid compound **11-1** with a yield 30.9%. LC-MS (ESI, pos. ion) m/z: 460.4 $[M+H]^+$.

Step 2: Synthesis of Compound **11-2**

Into a 50 mL two-necked flask were added compound **11-1** (180 mg, 0.39 mmol, 1 equiv.), **M4** (0.50 g, 0.98 mmol), Xphos Pd G4 (100 mg, 0.12 mmol), $K_3PO_4 \cdot 7H_2O$ (528 mg, 1.56 mmol) and THF/$H_2O$ (v/v = 5.1 mL/1.7 mL). The mixture was purged with nitrogen three times and stirred at room temperature for 12 h. The reaction was stopped and added saturated ammonium chloride solution (20 mL). The mixture was extracted with DCM (20 mL × 3). The combined organic phases were dried over anhydrous sodium sulfate and concentrated. The residue was purified by column chromatography using DCM/MeOH (v/v = 50/1) as eluent to obtain 160 mg of a yellow solid compound **11-2** with a yield of 50.5%. LC-MS (ESI, pos. ion) m/z: 810.4 $[M+H]^+$.

Step 3: Synthesis of Compound **11-3**

Into a 25 mL single-necked flask were added compound **11-2** (153 mg, 0.19 mmol) and acetonitrile (5 mL). The mixture was cooled to 0 °C and stirred for 5 minutes. Concentrated sulfuric acid (0.057 mL, 0.57 mmol, 98% purity) was added dropwise. After stirring at 0 °C for 120 minutes, the reaction was stopped and quenched with saturated aqueous sodium bicarbonate solution (20 mL). The mixture was extracted with DCM (20 mL × 3). The combined organic phases were dried over anhydrous sodium sulfate and concentrated. The residue was purified by column chromatography using DCM/MeOH (v/v = 30/1) as eluent to obtain 88 mg of a yellow solid compound **11-3** with a yield of 60.8%, LC-MS (ESI, pos. ion) m/z: 766.7 $[M+H]^+$.

Step 4: Synthesis of Compound **11**

Into a 25 mL single-necked flask were added compound **11-3** (83 mg, 0.11 mmol), cesium fluoride (100 mg, 0.66 mmol) and DMF (1 mL). The mixture was purged with nitrogen three times and stirred at room temperature for 15 h. The reaction was stopped, and water (20 mL) was added, precipitating a solid. The mixture was filetered to afford a crude product. The crude product was purified by column chromatography using DCM/MeOH (v/v = 20/1) as eluent to obtain 20 mg of a red solid compound **1-5** with a yield of 30.3%. LC-MS (ESI, pos. ion) m/z: 610.3 $[M+H]^+$; HRMS (ESI): 610.2602 $[M+H]^+$; $^1$H NMR (400 MHz, $CD_3OD$) δ 7.89 (dd, *J* = 9.2, 5.7 Hz, 1H), 7.40 - 7.30 (m, 2H), 7.28 - 7.23 (m, 1H), 4.74 - 4.54 (m, 4H), 4.37 - 4.22 (m, 2H), 4.22 - 4.10 (m, 2H), 4.01 - 3.90 (m, 2H), 3.88 - 3.77 (m, 2H), 3.78 - 3.67 (m, 2H), 3.40 (s, 1H), 2.41 - 2.30 (m, 2H), 2.26 - 2.03 (m, 11H). $^{19}$F NMR (376 MHz, $CD_3OD$) δ -111.47 (1F), -141.27 (1F).

**Example 12: Synthesis of Compound 12**

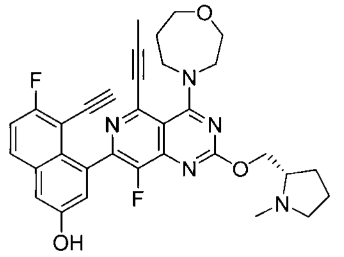

12

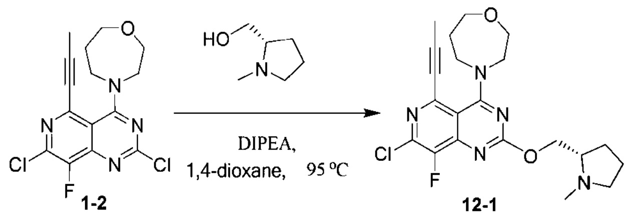


1-2 12-1

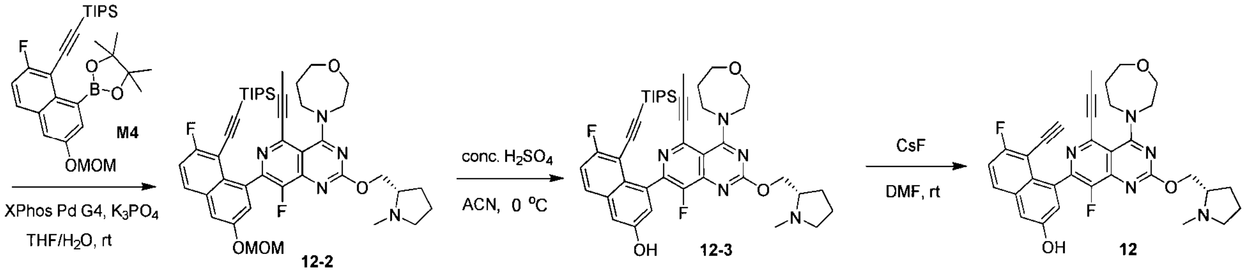


12-2 12-3 12

Step 1: Synthesis of Compound **12-1**

Into a 100 mL single-necked flask were added compound **1-2** (300 mg, 0.84 mmol), *N*-methyl-L-prolinol (190 mg, 1.68 mmol, Saen Chemical Technology), DIPEA (0.49 mL, 2.94 mmol), and anhydrous 1,4-dioxane (10 mL). The mixture was purged with nitrogen three times. The mixture was heated to 95 °C and stirred for 36 h. The reaction was stopped and cooled to room temperature. The reaction solution was concentrated, diluted with DCM (20 mL), and washed with saturated ammonium chloride solution (30 mL). The phases were separated and extracted with DCM (20 mL × 2). The combined organic phases were dried over anhydrous sodium sulfate and concentrated. The residue was purified by column chromatography using DCM/MeOH (v/v = 50/1) as eluent to obtain 210 mg of a yellow solid compound **12-1** with a yield of 57.3%. LC-MS (ESI, pos. ion) m/z: 434.2 $[M+H]^+$.

Step 2: Synthesis of Compound **12-2**

Into a 50 mL two-necked flask were added compound **12-1** (199.6 mg, 0.46 mmol, 1 equiv.), **M4** (0.59 g, 1.15 mmol), Xphos Pd G4 (120 mg, 0.14 mmol), $K_3PO_4 \cdot 7H_2O$ (620 mg, 1.84 mmol) and $THF/H_2O$ (v/v = 5.1 mL/1.7 mL). The mixture was purged with nitrogen three times and stirred at room temperature for 12 h. The reaction was stopped and added saturated ammonium chloride solution (20 mL). The mixture was extracted with DCM (20 mL × 3). The combined organic phases were dried over anhydrous sodium sulfate and concentrated. The residue was purified by column chromatography using DCM/MeOH (v/v = 30/1) as eluent to obtain 190 mg of a yellow solid compound **12-2** with a yield of 52.7%. LC-MS (ESI, pos. ion) m/z: 784.4 $[M+H]^+$.

Step 3: Synthesis of Compound **12-3**

Into a 25 mL single-necked flask were added compound **12-2** (188 mg, 0.24 mmol) and acetonitrile (5 mL). The mixture was cooled to 0 °C and stirred for 5 minutes. Concentrated sulfuric acid (0.072 mL, 0.72 mmol, 98% purity) was added dropwise. After stirring at 0 °C for 60 minutes, the reaction was stopped and quenched with saturated aqueous sodium bicarbonate solution (20 mL). The mixture was extracted with DCM (20 mL × 2). The combined organic phases were dried over anhydrous sodium sulfate and concentrated. The residue was purified by column chromatography using DCM/MeOH (v/v = 20/1) as eluent to obtain 92 mg of a yellow solid compound **12-3** with a yield of 51.8%, LC-MS (ESI, pos. ion) m/z: 740.2 $[M+H]^+$.

Step 4: Synthesis of Compound **12**

Into a 25 mL single-necked flask were added compound **12-3** (90 mg, 0.12 mmol), cesium fluoride (109 mg, 0.72 mmol) and DMF (1 mL). The mixture was purged with nitrogen three times and stirred at room temperature for 15 h. The reaction was stopped, and water (20 mL) was added, precipitating a solid. The mixture was filtered to afford a crude solid. The crude product was purified by column chromatography using DCM/MeOH (v/v = 20/1) as eluent to obtain 46 mg of a light yellow solid compound **12** with a yield of 64.8%. LC-MS (ESI, pos. ion) m/z: 584.1 $[M+H]^+$; HRMS (ESI): 584.2482 $[M+H]^+$; $^1$H NMR (400 MHz, $CD_3OD$) δ 7.93 - 7.84 (m, 1H), 7.44 - 7.30 (m, 2H), 7.27 - 7.22 (m, 1H), 4.71 - 4.66 (m, 1H), 4.41 - 4.08 (m, 4H), 4.00 - 3.68 (m, 6H), 3.46 - 3.37 (m, 1H), 3.28 - 3.20 (m, 2H), 3.10 (s, 3H), 2.48 - 2.32 (m, 2H), 2.26 - 2.02 (m, 7H). $^{19}$F NMR (376 MHz, $CD_3OD$) δ -111.46 (1F), -141.16 (1F).

**Example 13: Synthesis of Compound 13**

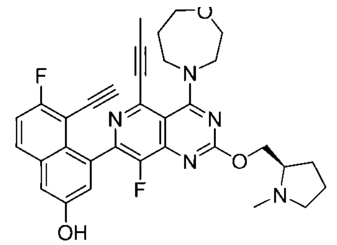

**13**

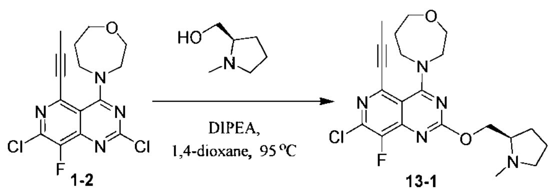


1-2

13-1

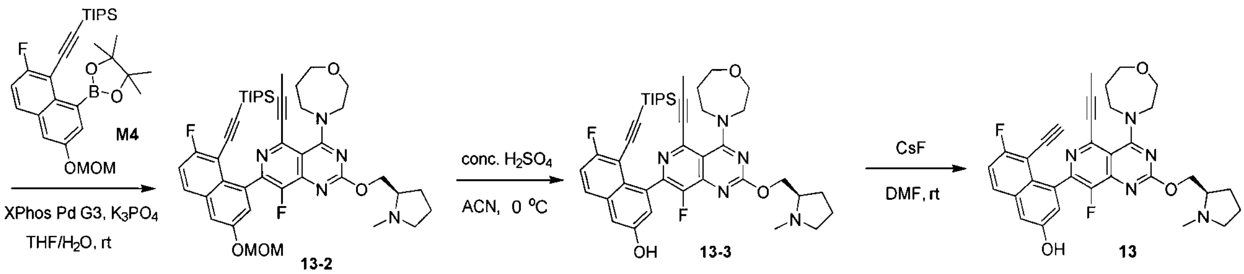


M4

13-2

13-3

13

Step 1: Synthesis of Compound **13**

The synthesis of compound **13** was carried out with reference to the synthesis method of compound **12,** and *N*-methyl-D-prolinol (purchased from Shaoyuan Technology) was used to replace *N*-methyl-L-prolinol to obtain compound **13** as a light yellow solid , LC-MS (ESI, pos. ion) m/z: 584.3 $[M+H]^+$; HRMS (ESI): 584.2417$[M+H]^+$; $^1$H NMR (400 MHz, $CD_3OD$) δ 7.88 (dd, *J* = 9.1, 5.7 Hz, 1H), 7.39 - 7.30 (m, 2H), 7.27 - 7.22 (m, 1H), 4.67 - 4.51 (m, 3H), 4.34 - 4.20 (m, 2H), 4.20 - 4.05 (m, 2H), 4.01 - 3.89 (m, 2H), 3.88 - 3.78 (m, 2H), 3.41 (s, 1H), 3.21 - 3.10 (m, 1H), 2.71 (s, 3H), 2.70 - 2.62 (m, 1H), 2.28 - 2.04 (m, 6H), 2.02 - 1.81 (m, 3H). $^{19}$F NMR (376 MHz, $CD_3OD$) δ -111.56 (1F), -140.92 (1F).

**Example 14: Synthesis of Compound 14**

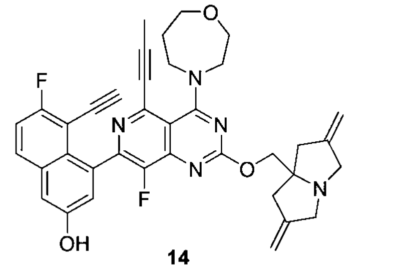

14

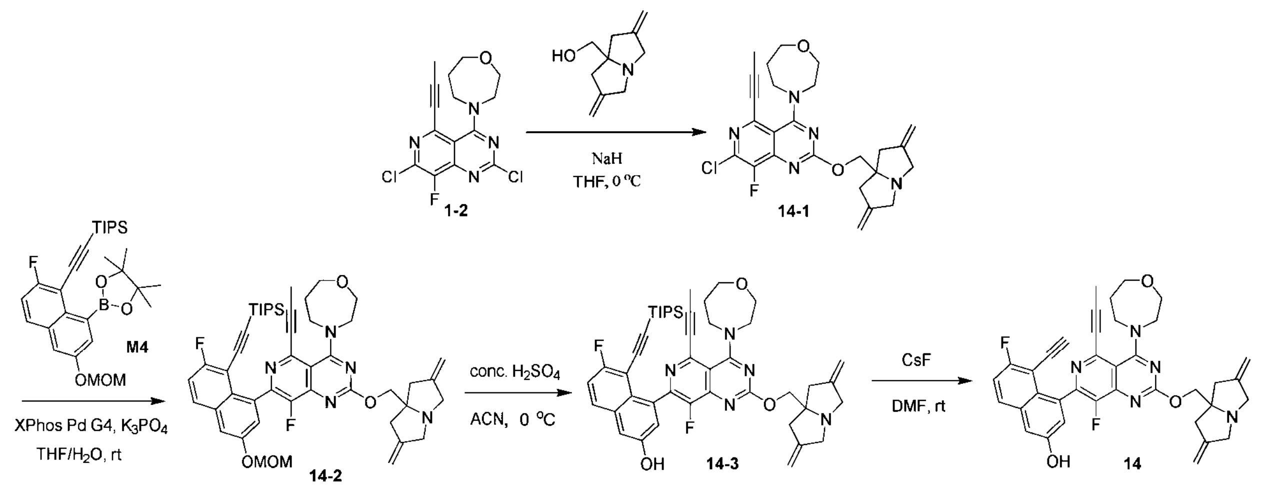


Step 1: Synthesis of Compound **14-1**

Into a 100 mL two-necked flask was added NaH (0.13 g, 3.36 mmol, 60% w/w). The flask was purged with nitrogen, followed by addition of THF (7 mL). The mixture was cooled to 0 °C and stirred for 5 min. A solution of (2,6-dimethylenetetrahydro-1*H*-pyrrolizin-7*a*(5*R*)-yl)methanol (0.17 g, 1.01 mmol) in THF (3 mL) was added dropwise slowly while maintaining 0 °C for 20 min. A solution of compound **1-2** (220 mg, 0.44 mmol) in THF (5 mL) was added dropwise slowly. The mixture was reacted by stirring at 0 °C for 12 h. Stirring was stopped, and the reaction was quenched with saturated ammonium chloride solution (20 mL). The mixture was separated into layers, and the aqueous phase was extracted with DCM (20 mL × 2). The combined organic phases were dried over anhydrous sodium sulfate and concentrated. The residue was purified by column chromatography using DCM/MeOH (v/v = 30/1) as eluent to obtain 230 mg of a yellow solid compound **14-1** with a yield 56%. LC-MS (ESI, pos. ion) m/z: 484.2 $[M+H]^+$.

Step 2: Synthesis of Compound **14-2**

Into a 50 mL two-necked flask were added compound **14-1** (184 mg, 0.38 mmol, 1 equiv.), **M4** (0.49 g, 0.95 mmol), Xphos Pd G4 (98 mg, 0.11 mmol), $K_3PO_4 \cdot 7H_2O$ (510 mg, 1.52 mmol) and THF/$H_2O$ (v/v = 5.1 mL/1.7 mL). The system was purged with nitrogen three times and stirred at room temperature for 18 h. The reaction was quenched and diluted with EA (15 mL). The mixture was washed with saturated ammonium chloride solution (20 mL) and extracted with EA (20 mL × 2). The combined organic phases were dried over anhydrous sodium sulfate and concentrated. The residue was purified by column chromatography using DCM/MeOH (v/v = 30/1) as eluent to obtain 258 mg of a yellow solid compound **14-2** with a yield of 54%. LC-MS (ESI, pos. ion) m/z: 834.4 $[M+H]^+$.

Step 3: Synthesis of Compound **14-3**

Into a 50 mL single-necked flask were added compound **14-2** (219 mg, 0.33 mmol) and acetonitrile (5 mL). The mixture was cooled to 0 °C and stirred for 5 minutes. Concentrated sulfuric acid (75.06 mg, 0.75 mmol, 98% w/w) was added dropwise. After stirring at 0 °C for 120 minutes, the reaction was stopped and quenched with saturated aqueous sodium bicarbonate solution (20 mL). The mixture was extracted with DCM (15 mL × 2). The combined organic phases were dried over anhydrous sodium sulfate and concentrated. The residue was purified by column chromatography using DCM/MeOH (v/v = 20/1) as eluent to obtain 56 mg of a yellow solid compound **14-3** with a yield of 29%. LC-MS (ESI, pos. ion) m/z: 790.1 $[M+H]^+$.

Step 4: Synthesis of Compound **14**

Into a 25 mL single-necked flask were added compound **14-3** (50 mg, 0.063 mmol), cesium fluoride (57.4 mg, 0.38 mmol), and DMF (1 mL). The mixture was purged with nitrogen three times and stirred at room temperature for 7 h. The reaction was stopped, and water (20 mL) was added, precipitating a solid. Filtration afforded the crude solid. The crude product was purified by column chromatography using DCM/MeOH (v/v = 20/1) as eluent to obtain 30 mg of a yellow solid compound 14 with a yield of 75%. LC-MS (ESI, pos. ion) m/z: 634.1[M+H]$^+$; HRMS (ESI): 634.2624 [M+H]$^+$; $^1$H NMR (400 MHz, $CD_3OD$) δ 7.87 (dd, *J* = 9.1, 5.7 Hz, 1H), 7.40 - 7.28 (m, 2H), 7.27 - 7.21 (m, 1H), 5.11 - 5.01 (m, 4H), 4.43 - 4.36 (m, 2H), 4.32 - 4.18 (m, 2H), 4.16 - 4.04 (m, 2H), 3.99 - 3.89 (m, 2H), 3.89 - 3.85 (m, 1H), 3.85 - 3.78 (m, 3H), 3.46 - 3.38 (m, 3H), 2.89 - 2.77 (m, 2H), 2.69 - 2.57 (m, 2H), 2.18 (s, 3H), 2.16 - 2.03 (m, 2H). $^{19}$F NMR (376 MHz, $CD_3OD$) δ -111.58 (1F), -140.74 (1F).

**Example 15: Synthesis of Compound 15**

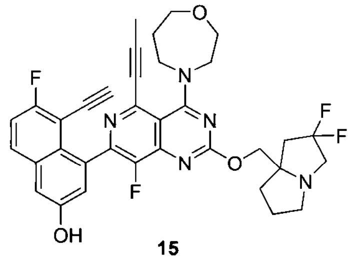

**15**

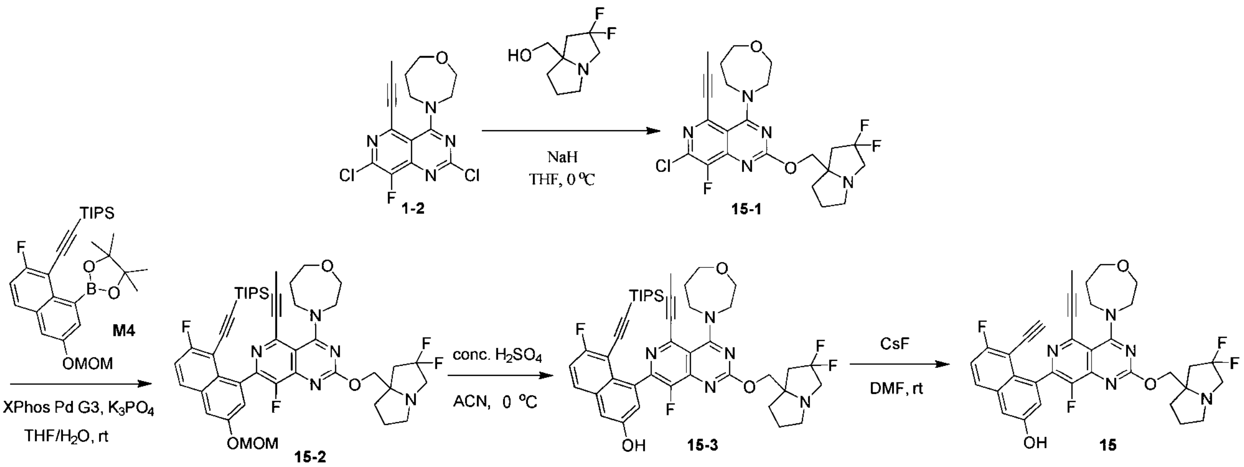


Step 1: Synthesis of Compound **15-1**

Into a 100 mL two-necked flask was added NaH (0.11 g, 2.7 mmol, 60% w/w). The flask was purged with nitrogen, followed by addition of THF (5 mL). The mixture was cooled to 0 °C and stirred for 5 min. A solution of (2,2-difluorotetrahydro-1*H*-pyrrolizin-7*a*(5H)-yl)methanol (0.19 g, 1.08 mmol, Shanghai Topchem Chemical) in THF (1.5 mL) was added dropwise slowly while maintaining 0 °C for 60 min. A solution of compound **1-2** (320 mg, 0.90 mmol) in THF (2 mL) was added dropwise slowly. The mixture was reacted at 0 °Cfor 1 h. Stirring was stopped, and the reaction was quenched with saturated ammonium chloride solution (20 mL). The mixture was separated into layers, and the aqueous phase was extracted with DCM (20 mL × 2). The combined organic phases were dried over anhydrous sodium sulfate and concentrated. The residue was purified by column chromatography using DCM/MeOH (v/v = 100/1) as eluent to obtain 203 mg of a yellow solid compound **15-1** with a yield 45%. LC-MS (ESI, pos. ion) m/z: 496.1[M+H]$^+$.

Step 2: Synthesis of Compound 15-2

Into a 50 mL two-necked flask were added compound **15-1** (198 mg, 0.40 mmol, 1 equiv.), **M4** (0.41 g, 0.80 mmol), Xphos Pd G3 (68 mg, 0.080 mmol), $K_3PO_4 \cdot 7H_2O$ (340 mg, 1.0 mmol) and $THF/H_2O$ (v/v = 4 mL/2 mL). The mixture was purged with nitrogen three times and stirred at room temperature for 2 h. Additional XPhos Pd G3 (0.2 equiv.) was added,

and stirring was continued at room temperature for 3 hours. The reaction was stoped and the mixture was diluted with DCM (20 mL). The mixture was washed with saturated ammonium chloride solution (20 mL) and extracted with DCM (20 mL × 2). The combined organic phases were dried over anhydrous sodium sulfate and concentrated. The residue was purified by column chromatography using DCM/MeOH (v/v = 100/1) as eluent to obtain 215 mg of a yellow solid compound **15-2** with a yield of 64%. LC-MS (ESI, pos. ion) m/z: 846.2 $[M+H]^+$.

Step 3: Synthesis of Compound **15-3**

Into a 50 mL single-necked flask were added compound **15-2** (215 mg, 0.25 mmol) and acetonitrile (5 mL). The mixture was cooled to 0 °C and stirred for 5 minutes. Concentrated sulfuric acid (0.14 mL, 2.5 mmol, 98% w/w) was added dropwise. After stirring at 0 °C for 30 minutes, the reaction was stopped and quenched with saturated aqueous sodium bicarbonate solution (20 mL). The mixture was extracted with DCM (15 mL × 2). The combined organic phases were dried over anhydrous sodium sulfate and concentrated. The residue was purified by column chromatography using DCM/MeOH (v/v = 10/1) as eluent to obtain 135 mg of a yellow solid compound **15-3** with a yield of 66%, LC-MS (ESI, pos. ion) m/z: 802.2 $[M+H]^+$.

Step 4: Synthesis of Compound **15**

Into a 25 mL single-necked flask were added compound **15-3** (135 mg, 0.17 mmol), cesium fluoride (210 mg, 1.36 mmol) and DMF (1 mL). The mixture was purged with nitrogen three times and stirred at room temperature for 13 h. The reaction was stopped, and water (10 mL) was added, precipitating a solid. Filtration afforded the crude solid. The crude product was purified by column chromatography using DCM/MeOH (v/v = 9/1) as eluent to obtain 60 mg of a yellow solid compound **15** with a yield of 55%, LC-MS (ESI, pos. ion) m/z: 646.1$[M+H]^+$; HRMS (ESI): 646.2478 $[M+H]^+$; $^1$H NMR (400 MHz, $CD_3OD$) δ 7.86 (dd, *J* = 9.1, 5.7 Hz, 1H), 7.38 - 7.28 (m, 2H), 7.26 - 7.20 (m, 1H), 4.63 (s, 1H), 4.35 - 4.18 (m, 4H), 4.16 - 4.04 (m, 2H), 3.99 - 3.85 (m, 2H), 3.84 - 3.77 (m, 2H), 3.53 - 3.36 (m, 2H), 3.24 - 3.07 (m, 2H), 2.68 - 2.50 (m, 1H), 2.41 - 2.25 (m, 1H), 2.21 - 1.96 (m, 7H), 1.96 - 1.84 (m, 2H). $^{19}$F NMR (376 MHz, $CD_3OD$) δ -99.21 (ddd, *J* = 283.9, 234.1, 33.4 Hz, 2F), -111.60 (s, 1F), -140.76 (d, *J*= 4.4 Hz, 1F).

**Example 16: Synthesis of Compound 16**

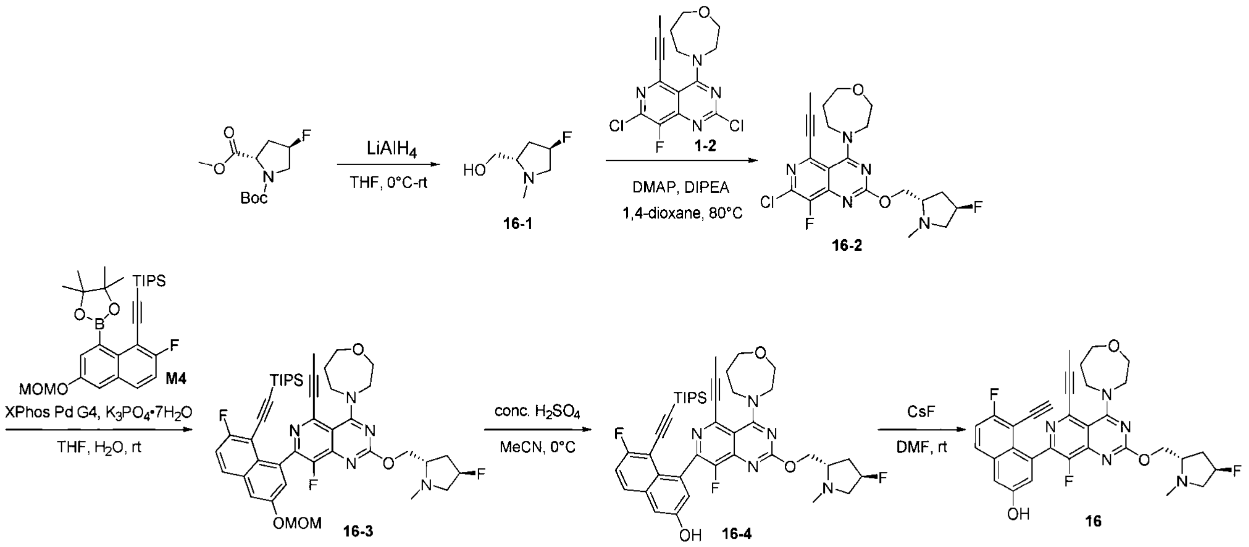


Step 1: Synthesis of Compound **16-1**

Into a 100 mL two-necked flask was added $LiAlH_4$ (0.46 g, 12.12 mmol). The mixture was purged with nitrogen three times and cooled to 0°C, followed by addition of THF (12.5 mL). The mixture was stirred at 0 °C for 5 min. A solution of N-Boc-trans-4-fluoro-L-proline methyl ester (1.00 g, 4.04 mmol, Shanghai Bide Pharmatech) in THF (2 mL) was added dropwise slowly. After completion of addition, the reaction mixture was warmed to room temperature and stirred for 16.5 h. The mixture was cooled to 0 °C and stirred for 5 min, then quenched by slow addition of sodium sulfate decahydrate. The

suspension was filtered through a Celite pad, concentrated, and dried by suction to obtain 445 mg of a pale yellow oil compound **16-1** with a yield of 82.6%. LC-MS (ESI, pos. ion) m/z: 134.1 $[M+H]^+$.

Step 2: Synthesis of Compound **16-2**

Into a 50 mL single-necked flask were charged compound **16-1** (130 mg, 1.01 mmol), **1-2** (300 mg, 0.84 mmol), 1,4-dioxane (6 mL), DIPEA (0.28 mL, 1.68 mmol) and DMAP (0.010 g, 0.084 mmol). The mixture was purged with nitrogen, heated to 80 °C, and stirred for 16 h. The reaction was stopped. The reaction mixture was diluted with EA (25 mL) and washed sequentially with saturated ammonium chloride solution (20 mL) and saturated sodium chloride solution (20 mL). The mixture were dried over anhydrous sodium sulfate and concentrated. The residue was purified by column chromatography using DCM/MeOH (v/v = 50/1) as eluent to obtain 56 mg of a yellow solid compound **16-2** with a yield 15%. LC-MS (ESI, pos. ion) m/z: 452.0 $[M+H]^+$.

Step 3: Synthesis of Compound **16-3**

Into a 25 mL two-necked flask were added compound **16-2** (56 mg, 0.12 mmol, 1 equiv.), **M4** (0.12 g, 0.24 mmol), Xphos Pd G4 (21 mg, 0.024 mmol), $K_3PO_4 \cdot 7H_2O$ (120 mg, 0.36 mmol) and THF/$H_2O$ (v/v = 2.1 mL/0.72 mL). The mixture was purged with nitrogen three times and stirred at room temperature for 2 h. Additional XPhos Pd G4 (0.2 equiv.) was added, and stirring was continued at room temperature for 2 h. The reaction was quenched and diluted with DCM (20 mL). The mixture was washed with saturated ammonium chloride solution (20 mL) and extracted with DCM (20 mL $\times$ 2). The combined organic phases were dried over anhydrous sodium sulfate and concentrated. The residue was purified by column chromatography using DCM/MeOH (v/v = 100/1) as eluent to obtain 54 mg of a yellow solid compound **16-3** with a yield of 54%. LC-MS (ESI, pos. ion) m/z: 401.8 $[M+2H]^{2+}$.

Step 4: Synthesis of Compound **16-4**

Into a 25 mL single-necked flask were added compound **16-3** (54 mg, 0.067 mmol) and acetonitrile (2 mL). The mixture was cooled to 0 °C and stirred for 5 minutes. Concentrated sulfuric acid (0.018 mL, 0.34 mmol, 98% purity) was added dropwise. After stirring at 0 °C for 30 min, the reaction was stopped and quenched with saturated aqueous sodium bicarbonate solution (15 mL). The mixture was extracted with DCM (15 mL $\times$ 2). The combined organic phases were dried over anhydrous sodium sulfate and concentrated to obtain 51 mg of a yellow solid compound **16-4,** which was used directly in the next step. The yield was calculated as 100%, LC-MS (ESI, pos. ion) m/z: 379.8 $[M+2H]^{2+}$.

Step 5: Synthesis of Compound **16**

Into a 25 mL single-necked flask were added compound **16-4** (51 mg, 0.067 mmol), cesium fluoride (81 mg, 0.54 mmol) and DMF (0.4 mL). The mixture was purged with nitrogen three times and stirred at room temperature for 18 h. The reaction was stopped, and water (10 mL) was added, precipitating a solid. Filtration afforded the crude solid. The crude product was purified by column chromatography using DCM/MeOH (v/v = 92/8) as eluent to obtain 20 mg of a yellow solid compound **16** with a yield of 49%, LC-MS (ESI, pos. ion) m/z: 602.2 $[M+H]^+$; HRMS (ESI): 602.2358 $[M+H]^+$; $^1$H NMR (400 MHz, $CD_3OD$) $\delta$ 7.86 (dd, *J* = 9.1, 5.7 Hz, 1H), 7.40 - 7.27 (m, 2H), 7.26 - 7.19 (m, 1H), 5.22 (d, *J* = 55.2 Hz, 1H), 4.63 - 4.47 (m, 2H), 4.34 - 4.03 (m, 4H), 3.99 - 3.86 (m, 2H), 3.85 - 3.74 (m, 2H), 3.68 - 3.49 (m, 1H), 3.41 (s, 1H), 2.91 - 2.71 (m, 1H), 2.64 (s, 3H), 2.44 - 2.26 (m, 1H), 2.22 - 1.96 (m, 7H). $^{19}$F NMR (376 MHz, $CD_3OD$) $\delta$ -111.56 (1F), -140.88 (1F), -171.92 (1F).

**Example 17: Synthesis of Compound 17**

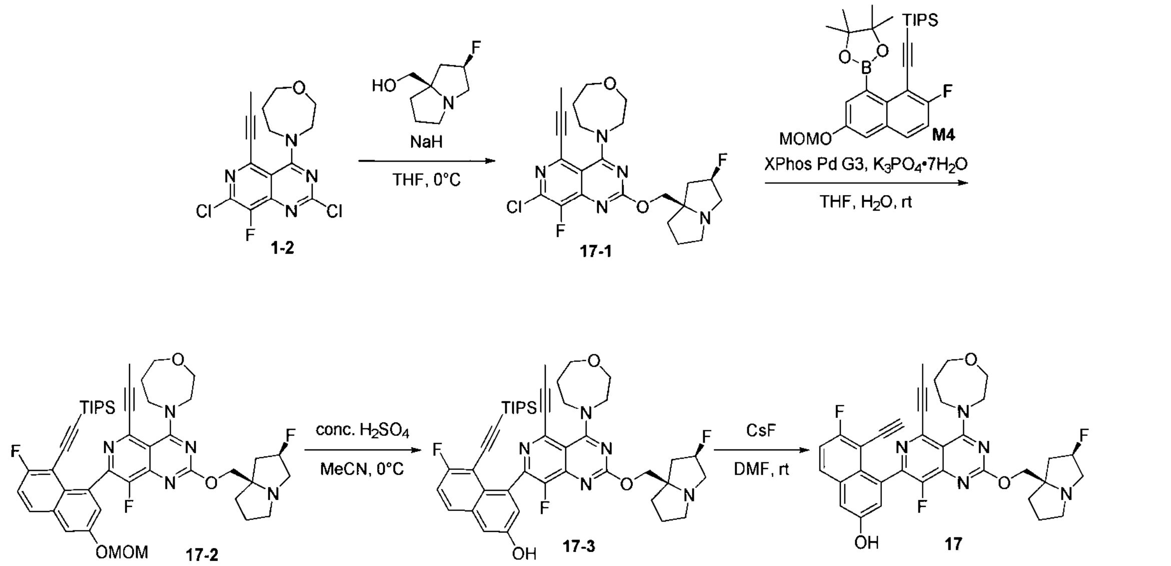


Step 1: Synthesis of Compound **17-1**

Into a 100 mL two-necked flask was added NaH (0.14 g, 3.39 mmol, 60% w/w). The mixture was purged with nitrogen, followed by addition of THF (3 mL). The mixture was cooled to 0 °C and stirred for 5 min, then a solution of [(2*R*,7*aR*)-2-fluorotetrahydro-1*H*-pyrrolizin-7a(5*H*)-yl]methanol (0.20 g, 1.24 mmol, Beijing Leyan Technology) in THF (1.5 mL) was added dropwise slowly. The reaction mixture was maintained at 0 °C for 60 min. A solution of compound **1-2** (400 mg, 1.13 mmol) in THF (5 mL) was added dropwise slowly. The mixture was reacted at 0 °Cfor 2.5 h. Stirring was stopped, and the reaction was quenched with saturated ammonium chloride solution (20 mL). The mixture was separated into layers, and the aqueous phase was extracted with DCM (30 mL × 2). The combined organic phases were dried over anhydrous sodium sulfate and concentrated. The residue was purified by column chromatography using DCM/MeOH (v/v = 30/1) as eluent to obtain 164 mg of a black oil compound **17-1** with a yield 30%. LC-MS (ESI, pos. ion) m/z: 477.9 $[M+H]^+$.

Step 2: Synthesis of Compound **17-2**

Into a 50 mL two-necked flask were added compound **17-1** (164 mg, 0.34 mmol, 1 equiv.), **M4** (0.35 g, 0.68 mmol), Xphos Pd G3 (58 mg, 0.068 mmol), $K_3PO_4 \cdot 7H_2O$ (290 mg, 0.85 mmol) and THF/$H_2O$ (v/v = 3.5 mL/1.7 mL). The mixture was purged with nitrogen three times and stirred at room temperature for 2 h. Additional XPhos Pd G3 (0.2 equiv.) was added, and stirring was continued at room temperature for 2 h. The reaction was quenched and diluted with DCM (20 mL). The mixture was washed with saturated ammonium chloride solution (20 mL) and extracted with DCM (20 mL × 2). The combined organic phases were dried over anhydrous sodium sulfate and concentrated. The residue was purified by column chromatography using DCM/MeOH (v/v = 98.3/1.7) as eluent to obtain 102 mg of a red oil compound **17-2** with a yield of 36%. LC-MS (ESI, pos. ion) m/z: 828.5 $[M+H]^+$.

Step 3: Synthesis of Compound **17-3**

Into a 50 mL single-necked flask were added compound **17-2** (102 mg, 0.12 mmol) and acetonitrile (2.5 mL). The mixture was cooled to 0 °C and stirred for 5 minutes. Concentrated sulfuric acid (0.039 mL, 0.72 mmol, 98% w/w) was added dropwise. After stirring at 0 °C for 60 minutes, the reaction was stopped and quenched with saturated aqueous sodium bicarbonate solution (20 mL). The mixture was extracted with DCM (20 mL × 2). The combined organic phases were dried over anhydrous sodium sulfate and concentrated. The residue was purified by column chromatography using DCM/MeOH (v/v = 10/1) as eluent to obtain 45 mg of a yellow solid compound **17-3** with a yield of 47%, LC-MS (ESI, pos. ion) m/z: 783.8 $[M+H]^+$.

Step 4: Synthesis of Compound **17**

Into a 25 mL single-necked flask were added compound **17-3** (45 mg, 0.057 mmol), cesium fluoride (69 mg, 0.46 mmol) and DMF (0.4 mL). The mixture was purged with nitrogen three times and stirred at room temperature for 10.5 h. The reaction was stopped, and water (8 mL) was added, precipitating a solid. Filtration afforded the crude solid. The crude product was purified by column chromatography using DCM/MeOH (v/v = 9/1) as eluent to obtain 21 mg of a yellow solid

compound **17** with a yield of 58%, LC-MS (ESI, pos. ion) m/z: 627.8 [M+H]+; HRMS (ESI): 628.2607 [M+H]+; $^1$H NMR (400 MHz, $CD_3OD$) δ 7.86 (dd, *J* = 9.1, 5.7 Hz, 1H), 7.38 - 7.28 (m, 2H), 7.26 - 7.20 (m, 1H), 5.37 (d, *J*= 52.9 Hz, 1H), 4.52 - 4.35 (m, 2H), 4.32 - 4.04 (m, 4H), 3.99 - 3.86 (m, 2H), 3.85 - 3.76 (m, 2H), 3.60 - 3.46 (m, 1H), 3.44 - 3.35 (m, 1H), 3.07 - 2.82 (m, 2H), 2.60 - 2.44 (m, 1H), 2.23 - 1.94 (m, 11H). $^{19}$F NMR (376 MHz, $CD_3OD$) δ -111.59 (1F), -140.86 (1F), -176.23 (1F).

**Example 18: Synthesis of Compound 18**

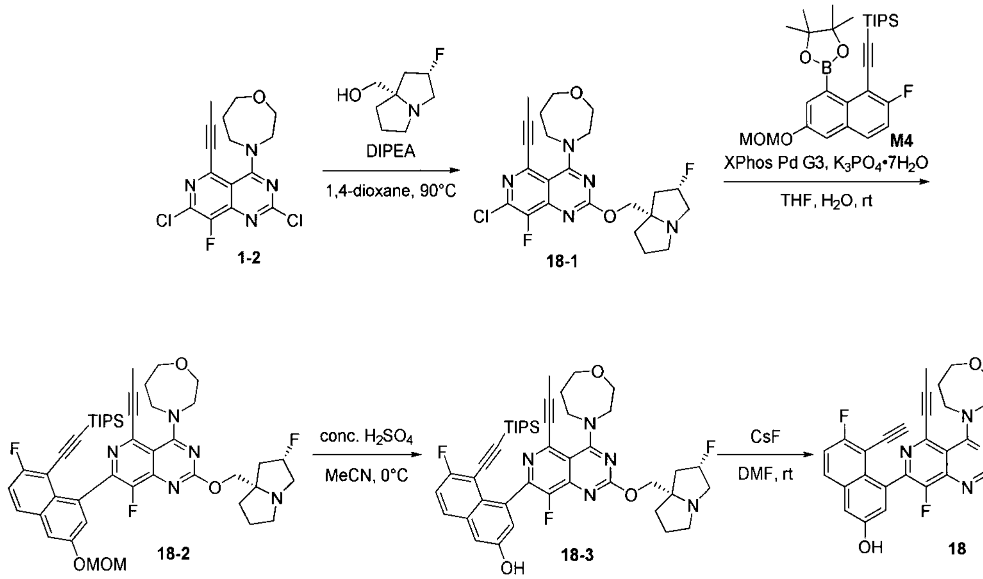


Step 1: Synthesis of Compound **18-1**

Into a 100 mL single-necked flask were added compound **1-2** (400 mg, 1.13 mmol), ((2*S*,7a*S*)-2-fluorotetrahydro-1H-pyrrolizin-7*a*(5*H*)-yl)methanol (180 mg, 1.13 mmol), DIPEA (0.37 mL, 2.26 mmol) and anhydrous 1,4-dioxane (6 mL). The mixture was purged with nitrogen three times. The mixture was heated to 90 °C and stirred for 15 h. The reaction was stopped and cooled to room temperature. The reaction solution was concentrated, diluted with EA (20 mL), and washed with saturated ammonium chloride solution (20 mL). The phases were separated and the aqueous phase was extracted with EA (20 mL × 2). The combined organic phases were dried over anhydrous sodium sulfate and concentrated. The residue was purified by column chromatography using DCM/MeOH (v/v = 20/1) as eluent to obtain 72 mg of a yellow solid compound **18-1** with a yield of 13.4%. LC-MS (ESI, pos. ion) m/z: 477.9 [M+H]+.

Step 2: Synthesis of Compound **18-2**

Into a 50 mL two-necked flask were added compound **18-1** (72 mg, 0.15 mmol, 1 equiv.), **M4** (0.15 g, 0.30 mmol), Xphos Pd G3 (25 mg, 0.030 mmol), $K_3PO_4 \cdot 7H_2O$ (150 mg, 0.45 mmol) and THF/$H_2O$ (v/v = 2.5 mL/0.9 mL). The mixture was purged with nitrogen three times and stirred at room temperature for 1 h. Additional XPhos Pd G3 (0.2 equiv.) was added, and stirring was continued at room temperature for 1 h. The reaction was quenched and diluted with DCM (20 mL). The mixture was washed with saturated ammonium chloride solution (20 mL) and extracted with DCM (20 mL × 2). The combined organic phases were dried over anhydrous sodium sulfate and concentrated. The residue was purified by column chromatography using DCM/MeOH (v/v = 97/3) as eluent to obtain 63 mg of a red oil compound **18-2** with a yield of 50.5%. LC-MS (ESI, pos. ion) m/z: 828.1 [M+H]+.

Step 3: Synthesis of Compound **18-3**

Into a 50 mL single-necked flask were added compound **18-2** (63 mg, 0.076 mmol) and acetonitrile (3 mL). The mixture was cooled to 0 °C and stirred for 5 minutes. Concentrated sulfuric acid (0.021 mL, 0.38 mmol, 98% w/w) was added dropwise. After stirring at 0 °C for 60 minutes, the reaction was stopped and quenched with saturated aqueous sodium bicarbonate solution (20 mL). The mixture was extracted with DCM (20 mL × 2). The combined organic phases were dried over anhydrous sodium sulfate and concentrated. The residue was purified by column chromatography using

DCM/MeOH (v/v = 10/1) as eluent to obtain 40 mg of a yellow solid compound **17-3** with a yield of 67%, LC-MS (ESI, pos. ion) m/z: 783.8 $[M+H]^+$.

Step 4: Synthesis of Compound **18**

Into a 25 mL single-necked flask were added compound **18-3** (40 mg, 0.051 mmol), cesium fluoride (62 mg, 0.41 mmol) and DMF (0.4 mL). The mixture was purged with nitrogen three times and stirred at room temperature for 4 h. The reaction was stopped, and water (8 mL) was added, precipitating a solid. Filtration afforded the crude solid. The crude product was purified by column chromatography using DCM/MeOH (v/v = 10/1) as eluent to obtain 20 mg of a yellow solid compound **18** with a yield of 62.4%, LC-MS (ESI, pos. ion) m/z: 627.9 $[M+H]^+$; HRMS (ESI): 628.2582 $[M+H]^+$; $^1H$ NMR (400 MHz, $CD_3OD$) δ 7.86 (dd, *J*= 9.1, 5.7 Hz, 1H), 7.37 - 7.28 (m, 2H), 7.26 - 7.19 (m, 1H), 5.38 (d, *J* = 52.3 Hz, 1H), 4.55 - 4.39 (m, 2H), 4.34 - 4.18 (m, 2H), 4.16 - 4.06 (m, 2H), 3.98 - 3.87 (m, 2H), 3.85 - 3.75 (m, 2H), 3.62 - 3.46 (m, 1H), 3.43 - 3.34 (m, 1H), 3.19 - 3.04 (m, 1H), 3.03 - 2.84 (m, 2H), 2.21 - 1.99 (m, 11H). $^{19}F$ NMR (376 MHz, $CD_3OD$) δ -111.58 (1F), -140.90 (1F), -176.34 (1F).

**Example 19: Synthesis of Compound 19**

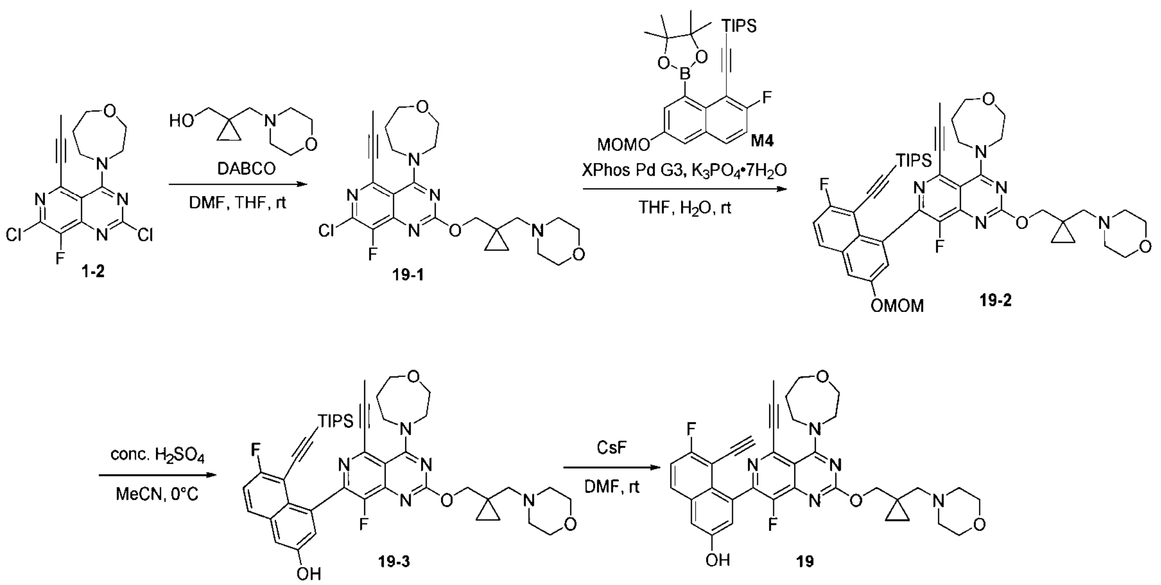


1-2 19-1 19-2 19-3 19

Step 1: Synthesis of Compound **19-1**

Into a 50 mL two-necked flask were added **1-2** (400 mg, 1.13 mmol), DMF (2 mL), THF (3.5 mL), cesium carbonate (1.1 g, 3.39 mmol), and (1-(morpholinomethyl)cyclopropyl)methanol (0.23 g, 1.36 mmol, Beijing Feilongrui Trading). The mixture was stirred at room temperature for 3 h, followed by addition of DABCO (0.013 g, 0.11 mmol). Stirring was continued at room temperature for 3.5 h. The reaction was stopped, and quenched with saturated ammonium chloride solution (20 mL). The mixture was separated into layers, and the aqueous phase was extracted with EA (30 mL × 2). The combined organic phases were dried over anhydrous sodium sulfate and concentrated. The residue was purified by column chromatography using DCM/MeOH (v/v = 98/2) as eluent to obtain 375 mg of a black oil compound **19-1** with a yield 68%. LC-MS (ESI, pos. ion) m/z: 490.3 $[M+H]^+$.

Step 2: Synthesis of Compound **19-2**

Into a 100 mL two-necked flask were added compound **19-1** (375 mg, 0.77 mmol, 1 equiv.), **M4** (0.59 g, 1.16 mmol), Xphos Pd G3 (130 mg, 0.15 mmol), $K_3PO_4 \cdot 7H_2O$ (780 mg, 2.31 mmol) and THF/$H_2O$ (v/v = 8 mL/4 mL). The mixture was purged with nitrogen three times and stirred at room temperature for 1 h. Additional XPhos Pd G3 (0.2 equiv.) was added, and stirring was continued at room temperature for 1 h. The reaction was stopped and the mixture was diluted with EA (20 mL). The resulting mixture was washed with saturated ammonium chloride solution (20 mL) and extracted with EA (20 mL × 2). The combined organic phases were dried over anhydrous sodium sulfate and concentrated. The residue

was purified by column chromatography using DCM/MeOH (v/v = 100/1) as eluent to obtain 295 mg of a black solid compound **19-2** with a yield of 46%. LC-MS (ESI, pos. ion) m/z: 840.4 [M+H]$^+$.

Step 3: Synthesis of Compound **19-3**

Into a 25 mL single-necked flask were added compound **19-2** (295 mg, 0.35 mmol) and acetonitrile (5 mL). The mixture was cooled to 0 °C and stirred for 5 minutes. Concentrated sulfuric acid (0.095 mL, 1.75 mmol, 98% w/w) was added dropwise. After stirring at 0 °C for 30 minutes, the reaction was stopped and quenched with saturated aqueous sodium bicarbonate solution (20 mL). The mixture was extracted with DCM (20 mL × 2). The combined organic phases were dried over anhydrous sodium sulfate and concentrated. The residue was purified by column chromatography using DCM/MeOH (v/v = 20/1) as eluent to obtain 162 mg of a yellow solid compound **19-3** with a yield of 58%, LC-MS (ESI, pos. ion) m/z: 796.3 [M+H]$^+$.

Step 4: Synthesis of Compound **19**

Into a 25 mL single-necked flask were added compound **19-3** (162 mg, 0.20 mmol), cesium fluoride (240 mg, 1.6 mmol) and DMF (0.8 mL). The mixture was purged with nitrogen three times and stirred at room temperature for 15 h. The reaction was stopped, and water (10 mL) was added, precipitating a solid. Filtration afforded the crude solid. The crude product was purified by column chromatography using DCM/MeOH (v/v = 10/1) as eluent to obtain 94 mg of a yellow solid compound **19** with a yield of 72%, LC-MS (ESI, pos. ion) m/z: 640.3 [M+H]$^+$; HRMS (ESI): 640.2742 [M+H]$^+$; $^1$H NMR (400 MHz, $CD_3OD$) δ 7.85 (dd, *J* = 9.0, 5.8 Hz, 1H), 7.37 - 7.27 (m, 2H), 7.26 - 7.20 (m, 1H), 4.43 (s, 2H), 4.32 - 4.17 (m, 2H), 4.16 - 4.02 (m, 2H), 4.00 - 3.86 (m, 2H), 3.84 - 3.76 (m, 2H), 3.70 - 3.57 (m, 4H), 3.39 (s, 1H), 2.61 - 2.35 (m, 6H), 2.19 - 2.01 (m, 5H), 0.71 (s, 2H), 0.51 (s, 2H). $^{19}$F NMR (376 MHz, $CD_3OD$) δ -111.55 (1F), -140.81 (1F).

**Example 20: Synthesis of Compound 20**

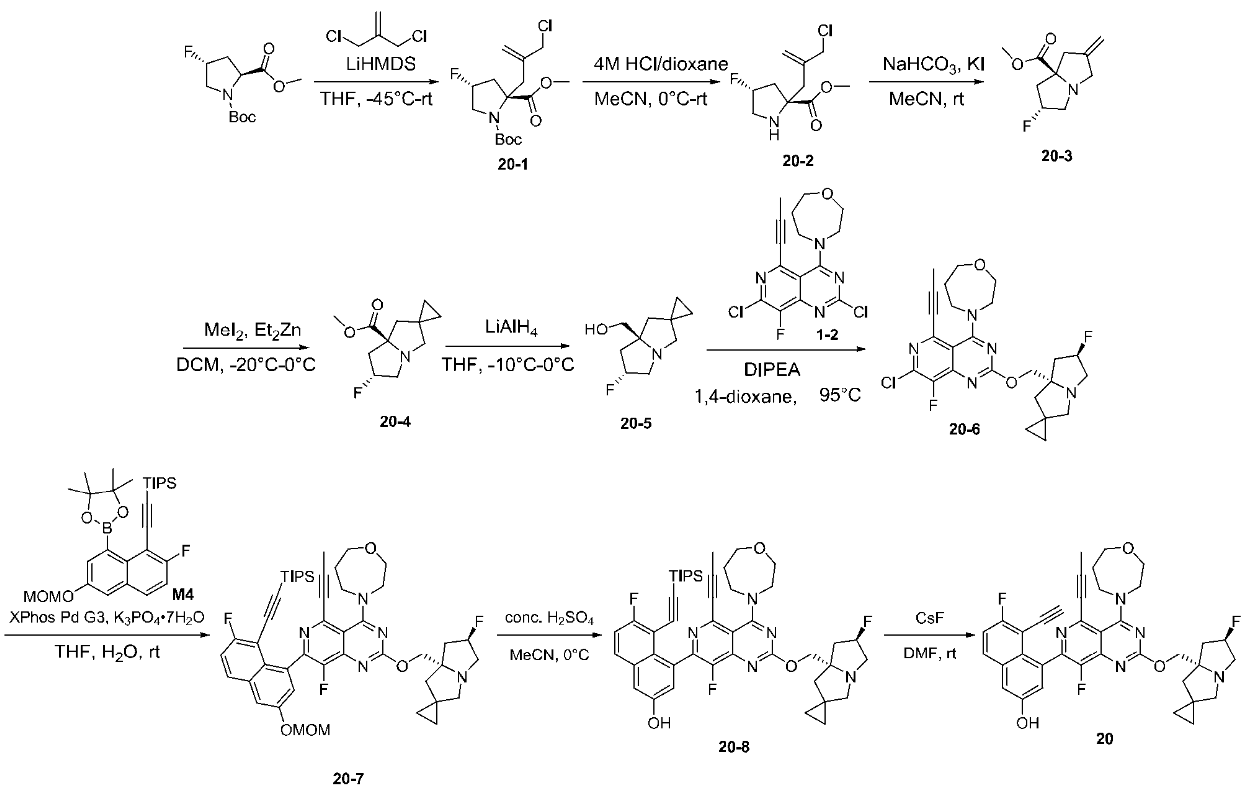


Step 1: Synthesis of Compound **20-1**

Into a 250 mL three-necked flask was added N-Boc-trans-4-fluoro-L-proline methyl ester (5.00 g, 20.2 mmol, Shanghai Bide Pharmatech). The mixture was cooled to -45 °C and stirred for 10 minutes, followed by slow dropwise

addition of LiHMDS (30.33 mL, 30.33 mmol, 1 M in THF). The reaction mixture was maintained at -45 °C for 60 minutes, then a solution of 3-chloro-2-(chloromethyl)prop-1-ene (3.03 g, 24.26 mmol) in anhydrous THF (15 mL) was added dropwise slowly. Upon completion of addition, the mixture was warmed gradually to room temperature (about 1 h). Stirring was discontinued, and the reaction was quenched with saturated ammonium chloride solution (20 mL). The mixture was separated into layers, and the aqueous phase was extracted with ethyl acetate (50 mL × 2). The combined organic phases were dried over anhydrous sodium sulfate and concentrated. The residue was purified by column chromatography using PE/EA (v/v = 20/1-5/1) as eluent to obtain 6.06 g of a yellow oil compound **20-1** with a yield 89%. LC-MS (ESI, pos. ion) m/z: 236.0 $[M\text{-}Boc+H]^+$.

Step 2: Synthesis of Compound **20-2**

Into a 250 mL single-necked flask were added compound **20-1** (6.06 g, 18.05 mmol) and DCM (100 mL). The mixture was cooled to 0 °C and stirred for 5 minutes. A solution of hydrogen chloride in dioxane (22.56 mL, 90.25 mmol, 4 M) was added dropwise. After completion of the addition, the reaction mixture was allowed to warm to room temperature and stirred for 14.5 h. Stirring was stopped, the reaction mixture was concentrated directly . The residue was dried by suction and used directly in the next step without further purification. The yield was assumed to be 100%. LC-MS (ESI, pos. ion) m/z: 236.0 $[M+H]^+$.

Step 3: Synthesis of Compound **20-3**

Into a 250 mL single-necked flask were added **20-2** (4.25 g, 18.03 mmol), acetonitrile (50 mL), sodium bicarbonate (7.57 g, 90.15 mmol) and potassium iodide (0.30g, 1.80 mmol). The mixture was stirred at room temperture for 2 h. Stirring was stopped, and the reaction mixture was filtered. The solid was washed with acetonitrile (50 mL) and concentrated. The residue was purified by column chromatography using PE/EA (v/v = 4/1-3/2) as eluent to obtain 3.00 g of a yellow oil compound **20-3** with a yield 83.5%. LC-MS (ESI, pos. ion) m/z: 200.1 $[M+H]^+$. $^1H$ NMR (400 MHz, $CDCl_3$) δ 5.29 - 5.12 (m, 1H), 4.95 (d, *J* = 13.7 Hz, 2H), 3.87 (d, *J* = 14.8 Hz, 1H), 3.75 (s, 3H), 3.59 - 3.40 (m, 1H), 3.27 - 3.16 (m, 1H), 2.98 - 2.86 (m, 2H), 2.85 - 2.73 (m, 1H), 2.57 - 2.48 (m, 1H), 2.21 - 1.93 (m, 1H).

Step 4: Synthesis of Compound **20-4**

Into a 100 mL single-necked flask was added compound **20-3** (400 mg, 2.01 mmol). The flask was purged with nitrogen three times. Anhydrous DCM (12 mL) was added, and the mixture was cooled to -20 °C and stirred for 10 minutes. Diethylzinc solution (10.05 mL, 10.05 mmol, 1 M in hexane) was added dropwise slowly. After stirring for 30 minutes. Diiodomethane (1.62 mL, 20.10 mmol) was slowly added dropwise. The reaction mixture was then transferred to 0 °C and stirred for 3 h. The reaction mixture was quenched with saturated aqueous ammonium chloride (20 mL). The aqueous phase was extracted with ethyl acetate (EA, 20 mL × 2). The combined organic layers were dried over anhydrous sodium sulfate, concentrated. The residue was purified by column chromatography using PE/EA (v/v = 7/3) as eluent to obtain 223 mg of a yellow oil compound **20-4** with a yield 52%. LC-MS (ESI, pos. ion) m/z: 214.4 $[M+H]^+$; $^1H$ NMR (400 MHz, $CDCl_3$) δ 5.37 - 5.16 (m, 1H), 3.77 (s, 3H), 3.68 - 3.52 (m, 1H), 3.19 (d, *J* = 10.6 Hz, 1H), 3.10 - 2.92 (m, 1H), 2.91 - 2.76 (m, 1H), 2.63 (d, *J* = 10.6 Hz, 1H), 2.25 - 2.02 (m, 2H), 1.89 (d, *J* = 12.9 Hz, 1H), 0.66 - 0.42 (m, 4H).

Step 5: Synthesis of Compound **20-5**

Into a 100 mL two-necked flask was added $LiAlH_4$ (0.79 g, 2.08 mmol). The mixture was purged with nitrogen three times and cooled to -10 °C, followed by addition of THF (2 mL). The mixture was stirred for 5 min. A solution of compound **20-4** (221 mg, 1.04 mmol) in THF (3 mL) was added dropwise slowly. After completion of addition, the reaction mixture was warmed to 0 °C and stirred for 3.5 h. The mixture was quenched by slow addition of sodium sulfate decahydrate. The suspension was filtered through a Celite pad, concentrated, and placed under high vacuum to obtain 162 mg of a light yellow oil compound **20.5** with a yield of 84%. LC-MS (ESI, pos. ion) m/z: 186.2 $[M+H]^+$.

Step 6: Synthesis of Compound **20-6**

Into a 100 mL single-necked flask were added compound **1-2** (300 mg, 0.84 mmol), **20-5** (160 mg, 0.84 mmol), DIPEA (0.42 mL, 2.52 mmol) and anhydrous 1,4-dioxane (5 mL). The mixture was purged with nitrogen three times. The mixture was heated to 95 °C and stirred for 11 h. The reaction was stopped and the mixture was cooled to room temperature. The reaction solution was concentrated, diluted with EA (20 mL), and washed with saturated ammonium chloride solution (20 mL). The phases were separated and extracted with EA (20 mL × 2). The combined organic phases were dried over anhydrous sodium sulfate and concentrated. The residue was purified by column chromatography using

DCM/MeOH (v/v = 30/1) as eluent to obtain 65 mg of a yellow solid compound **20-6** with a yield of 15.3%. LC-MS (ESI, pos. ion) m/z: 504.2 $[M+H]^+$.

Step 7: Synthesis of Compound **20-7**

Into a 25 mL single-necked flask were added compound **20-6** (65 mg, 0.13 mmol, 1 equiv.), **M4** (0.17 g, 0.33 mmol), Xphos Pd G3 (22 mg, 0.026 mmol), $K_3PO_4 \cdot 7H_2O$ (130 mg, 0.39 mmol) and $THF/H_2O$ (v/v = 2.5 mL/0.8 mL). The mixture was purged with nitrogen three times and stirred at room temperature for 1 h. Additional XPhos Pd G3 (0.2 equiv.) was added, and stirring was continued at room temperature for 1 h. The reaction was quenched and the mixture diluted with EA (20 mL). The mixture was washed with saturated ammonium chloride solution (20 mL) and extracted with EA (20 mL × 2). The combined organic phases were dried over anhydrous sodium sulfate and concentrated. The residue was purified by column chromatography using DCM/MeOH (v/v = 12/1) as eluent to obtain 65 mg of a red oil compound **18-2** with a yield of 59%. LC-MS (ESI, pos. ion) m/z: 854.3 $[M+H]^+$.

Step 8: Synthesis of Compound **20-8**

Into a 25 mL single-necked flask were added compound **20-7** (65 mg, 0.076 mmol) and acetonitrile (2 mL). The mixture was cooled to 0 °C and stirred for 5 minutes. Concentrated sulfuric acid (0.021 mL, 0.38 mmol, 98% purity) was added dropwise. After stirring at 0 °C for 60 min, the reaction was stopped and quenched with saturated aqueous sodium bicarbonate solution (15 mL). The mixture was extracted with DCM (20 mL × 2). The combined organic phases were dried over anhydrous sodium sulfate, concentrated and dried by suction. he crude product was used directly in the next step. The yield was calculated as 100%. LC-MS (ESI, pos. ion) m/z: 810.4 $[M+H]^+$.

Step 9: Synthesis of Compound **20**

Into a 25 mL single-necked flask were added compound **20-8** (61.6 mg, 0.076 mmol), cesium fluoride (69 mg, 0.46 mmol), and DMF (0.4 mL). The system was purged with nitrogen three times and stirred at room temperature for 3.5 h. The reaction was stopped, and water (10 mL) was added, precipitating a solid. Filtration afforded the crude solid. The crude product was purified by column chromatography using DCM/MeOH (v/v = 11/1) as eluent to obtain 34 mg of a yellow solid compound **20** with a yield of 68.4%, LC-MS (ESI, pos. ion) m/z: 654.3 $[M+H]^+$; HRMS (ESI): 654.2749 $[M+H]^+$; $^1H$ NMR (400 MHz, $CD_3OD$) δ 7.86 (dd, *J*= 9.1, 5.7 Hz, 1H), 7.38 - 7.28 (m, 2H), 7.26 - 7.21 (m, 1H), 5.42 (d, J= 51.9 Hz, 1H), 4.55 - 4.44 (m, 2H), 4.35 - 4.20 (m, 2H), 4.18 - 4.03 (m, 2H), 4.00 - 3.86 (m, 2H), 3.84 - 3.75 (m, 2H), 3.67 - 3.45 (m, 1H), 3.44 - 3.37 (m, 1H), 3.25 - 3.06 (m, 2H), 2.80 - 2.70 (m, 1H), 2.27 - 2.01 (m, 9H), 0.71 - 0.54 (m, 4H). $^{19}F$ NMR (376 MHz, $CD_3OD$) δ -111.59 (1F), -140.82 (1F), -175.79 (1F).

### Example 21: Synthesis of Compound 21

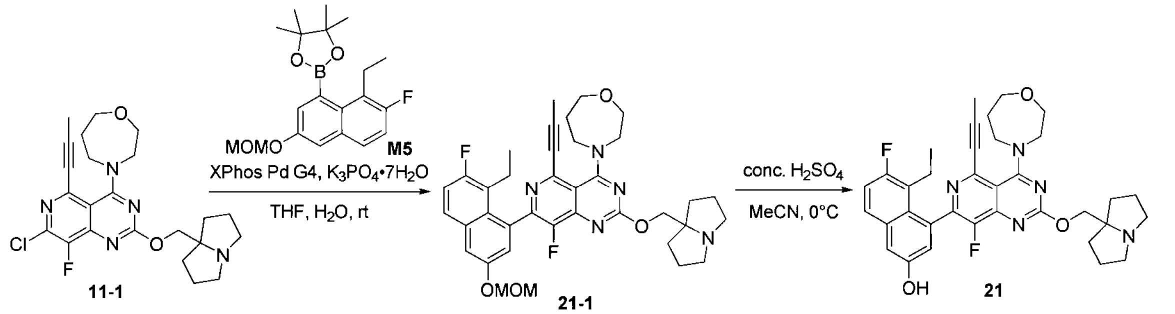


11-1 21-1 21

Step 1: Synthesis of Compound **21-1**

Into a 50 mL two-necked flask were added compound **11-1** (150 mg, 0.33 mmol, 1 equiv.), **M5** (0.30 g, 0.83 mmol), Xphos Pd G4 (85 mg, 0.099 mmol), $K_3PO_4 \cdot 7H_2O$ (450 mg, 1.32 mmol) and $THF/H_2O$ (v/v = 5.1 mL/1.7 mL). The mixture was purged with nitrogen three times and stirred at room temperature for 15 h. The reaction was quenched and diluted with DCM (20 mL). The mixture was washed with saturated ammonium chloride solution (20 mL) and extracted with EA (20 mL × 2). The combined organic phases were dried over anhydrous sodium sulfate and concentrated. The residue was purified by column chromatography using DCM/MeOH (v/v = 30/1) as eluent to obtain 42 mg of a yellow solid compound **21-1** with a

yield of 20%. LC-MS (ESI, pos. ion) m/z: 658.2 $[M+H]^+$.

Step 2: Synthesis of Compound **21**

Into a 50 mL single-necked flask were added compound **21-1** (40 mg, 0.061 mmol) and acetonitrile (5 mL). The mixture was cooled to 0 °C and stirred for 5 minutes. Concentrated sulfuric acid (18.3 mg, 0.18 mmol, 98% w/w) was added dropwise. After stirring at 0 °C for 1.5 h, the reaction was stopped and quenched with saturated aqueous sodium bicarbonate solution (20 mL). The mixture was extracted with DCM (20 mL × 2). The combined organic phases were dried over anhydrous sodium sulfate and concentrated. The residue was purified by column chromatography using DCM/MeOH (v/v = 20/1) as eluent to obtain 20 mg of a yellow solid compound **21** with a yield of 53%, LC-MS (ESI, pos. ion) m/z: 614.1 $[M+H]^+$; HRMS (ESI): 614.2985 $[M+H]^+$; $^1H$ NMR (400 MHz, $CD_3OD$) δ 7.70 (dd, *J* = 8.8, 5.8 Hz, 1H), 7.37 - 7.22 (m, 2H), 7.13 - 7.06 (m, 1H), 4.70 - 4.60 (m, 6H), 4.33 - 4.07 (m, 4H), 4.01 - 3.88 (m, 2H), 3.87 - 3.79 (m, 2H), 3.75 - 3.66 (m, 2H), 2.44 - 2.29 (m, 4H), 2.26 - 2.09 (m, 9H), 0.85 (t, *J* = 7.4 Hz, 3H). $^{19}F$ NMR (376 MHz, $CD_3OD$) δ -120.86 (1F), -140.73 (1F).

**Example 22: Synthesis of Compound 22**

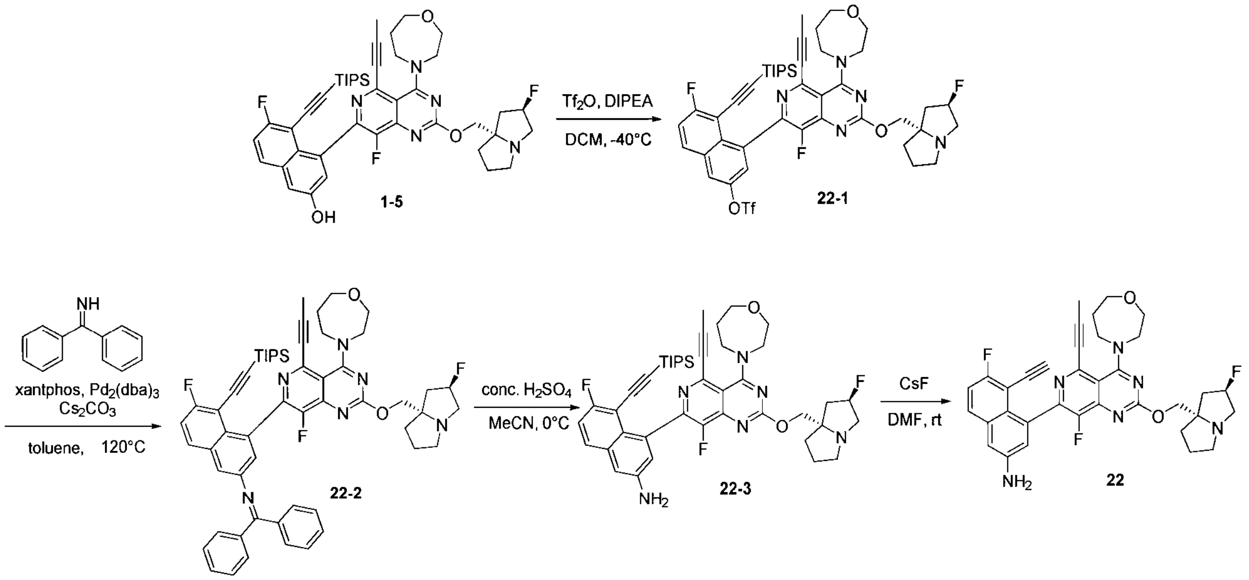


Step 1: Synthesis of Compound **22-1**

Into a 50 mL single-necked flask were added compound **1-5** (700 mg, 0.89 mmol), pyridine (0.28 g, 3.56 mmol) and DCM (15 mL). The mixture was purged with nitrogen three times, the mixture was cooled to 0 °C, and stirred for 5 minutes. $Tf_2O$ (1.00 g, 3.56 mmol) was slowly added dropwise. After completion of the addition, the reaction mixture was heated to 30 °C and stirred for 2.5 h. The reaction was stopped and quenched with saturated aqueous sodium bicarbonate solution (20 mL). The mixture was extracted with DCM (20 mL × 2). The combined organic phases were dried over anhydrous sodium sulfate and concentrated. The residue was purified by column chromatography using DCM/MeOH (v/v = 20/1) as eluent to obtain 140 mg of a brown solid compound **22-1** with a yield of 17%, LC-MS (ESI, pos. ion) m/z: 916.0 $[M+H]^+$.

Step 2: Synthesis of Compound **22-2**

Into a 25 mL two-necked flask were added compound **22-1** (140 mg, 0.15 mmol, 1.0 equiv.), benzophenone imine (140 mg, 0.75 mmol), $Pd_2(dba)_3$ (41 mg, 0.045 mmol), XantPhos (52 mg, 0.090 mmol), cesium carbonate (150 mg, 0.45 mmol), and toluene (3 mL). The mixture was purged with nitrogen three times and stirred at 120 °C for 15 h. Additional $Pd_2(dba)_3$ (0.3 equiv.) and XantPhos (0.6 equiv.) were added. Stirring was continued at 120 °C for 6 h. The reaction was stopped and the mixture was diluted with EA (20 mL). The mixture was washed with saturated aqueous ammonium chloride solution (20 mL) and extracted with ethyl acetate (20 mL × 2). The combined organic layers were dried over anhydrous sodium sulfate, concentrated, and purified by column chromatography using DCM/MeOH (v/v = 20:1) as the

eluent to obtain 70 mg of a yellow solid compound **22-2** with a yield of 48%. LC-MS (ESI, pos. ion) m/z: 947.3 $[M+H]^+$.

Step 3: Synthesis of Compound **22-3**

Into a 25 mL single-necked flask were added compound **22-2** (90 mg, 0.095 mmol) and acetonitrile (5 mL). The mixture was cooled to 0 °C and stirred for 5 minutes. Concentrated sulfuric acid (28.52 mg, 0.29 mmol, 98% w/w) was added dropwise. After stirring at 0 °C for 30 minutes, the reaction was stopped and quenched with saturated aqueous sodium bicarbonate solution (20 mL). The mixture was extracted with DCM (20 mL × 2). The combined organic phases were dried over anhydrous sodium sulfate and concentrated. The residue was purified by column chromatography using DCM/MeOH (v/v = 20/1) as eluent to obtain 70 mg of a yellow solid compound **22-3** with a yield of 94%, LC-MS (ESI, pos. ion) m/z: 392.2 $[M+2H]^{2+}$.

Step 4: Synthesis of Compound **22**

Into a 25 mL single-necked flask were added compound **22-3** (70 mg, 0.089 mmol), cesium fluoride (110 mg, 0.71 mmol) and DMF (1 mL). The mixture was purged with nitrogen three times and stirred at room temperature for 15 h. The reaction was stopped, and water (20 mL) was added, precipitating a solid. Filtration afforded the crude solid. The crude product was purified by column chromatography using DCM/MeOH (v/v = 20/1) as eluent to obtain 23 mg of a yellow solid compound **22** with a yield of 41%. LC-MS (ESI, pos. ion) m/z: 627.3 $[M+H]^+$; HRMS (ESI): 627.2686 $[M+H]^+$; $^1H$ NMR (400 MHz, $CD_3OD$) δ 7.77 (dd, *J* = 8.9, 5.7 Hz, 1H), 7.30 - 7.15 (m, 3H), 5.51 (d, *J* = 53.4 Hz, 1H), 4.60 - 4.50 (m, 2H), 4.36 - 4.21 (m, 2H), 4.22 - 4.07 (m, 2H), 4.01 - 3.89 (m, 2H), 3.89 - 3.79 (m, 2H), 3.77 - 3.61 (m, 3H), 3.43 - 3.34 (m, 2H), 2.67 - 2.41 (m, 2H), 2.39 - 2.02 (m, 9H). $^{19}F$ NMR (376 MHz, $CD_3OD$) δ -113.29 (1F), -141.12 (1F), -173.96 (1F).

Example 23: Synthesis of Compound 23

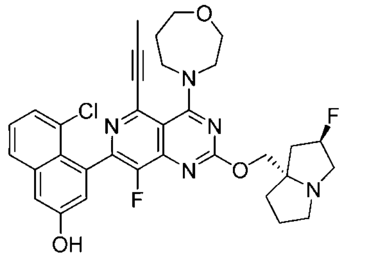

**23**

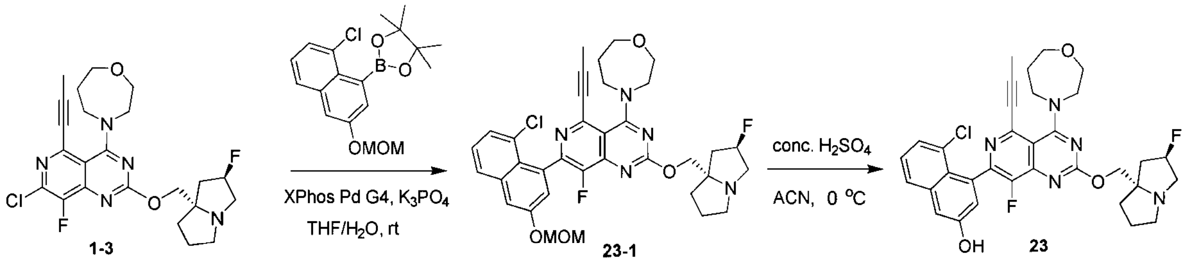


**1-3** **23-1** **23**

Step 1: Synthesis of Compound **23-1**

Into a 25 mL two-necked flask were added compound **1-3** (300 mg, 0.63 mmol, 1 equiv.), 2-(8-chloro-3-(methoxymethoxy)naphthalen-1-yl)-4,4,5,5-tetramethyl-1,3,2-dioxaborolane (0.549 g, 1.57 mmol, Aikon Biotech), XPhos Pd G4 (162 mg, 0.19 mmol), $K_3PO_4 \cdot 7H_2O$ (852 mg, 2.52 mmol) and $THF/H_2O$ (v/v = 5.1 mL/1.7 mL). The reaction mixture was purged with nitrogen three times and stirred at room temperature for 15 h. The reaction was added saturated ammonium chloride solution (20 mL), extracted with DCM (20 mL × 2). The combined organic phases were dried over anhydrous sodium sulfate and concentrated. The residue was purified by column chromatography using DCM/MeOH (v/v = 50/1) as eluent to obtain 220 mg of a yellow solid compound **23-1** with a yield of 53%. LC-MS (ESI, pos. ion) m/z: 664.3 $[M+H]^+$.

Step 2: Synthesis of Compound **23**

Into a 25 mL single-necked flask were added compound **23-1** (219 mg, 0.33 mmol) and acetonitrile (5 mL). The mixture was cooled to 0 °C and stirred for 5 minutes. Concentrated sulfuric acid (99 mg, 0.99 mmol, 98% purity) was added dropwise. After stirring for 1.5 h, the reaction was stopped and quenched with saturated aqueous sodium bicarbonate solution (20 mL). The mixture was extracted with DCM (20 mL × 2). The combined organic phases were dried over anhydrous sodium sulfate and concentrated. The residue was purified by column chromatography using DCM/MeOH (v/v = 30/1) as eluent to obtain 95 mg of a yellow solid compound **23** with a yield of 46%, LC-MS (ESI, pos. ion) m/z: 620.5 $[M+H]^+$; HRMS (ESI): 620.2251 $[M+H]^+$; $^1H$ NMR (400 MHz, $CD_3OD$) δ 7.80 - 7.71 (m, 1H), 7.41 - 7.30 (m, 3H), 7.25 - 7.16 (m, 1H), 5.46 - 5.25 (m, 1H), 4.42 - 4.29 (m, 2H), 4.28 - 4.06 (m, 4H), 3.99 - 3.83 (m, 2H), 3.84 - 3.74 (m, 2H), 3.49 - 3.34 (m, 3H), 3.15 - 3.04 (m, 1H), 2.49 - 2.25 (m, 2H), 2.22 - 1.91 (m, 9H). $^{19}F$ NMR (376 MHz, $CD_3OD$) δ -140.99 (1F), -173.66 (1F).

**Example 24: Synthesis of Compound 24**

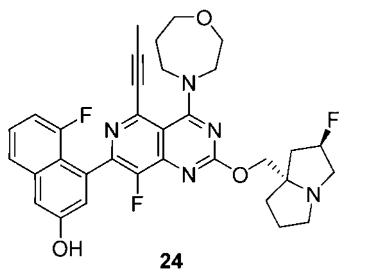

**24**

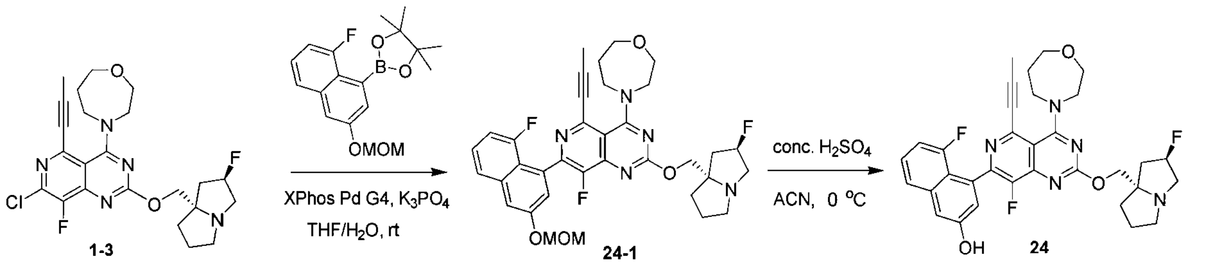


1-3 24-1 24

Step 1: Synthesis of Compound **24-1**

Into a 25 mL two-necked flask were added compound **1-3** (350 mg, 0.73 mmol, 1 equiv.), 2-(8-fluoro-3-(methoxymethoxy)naphthalen-1-yl)-4,4,5,5-tetramethyl-1,3,2-dioxaborolane (0.606 g, 1.82 mmol, Shanghai ZaiQi Bio-Tech), XPhos Pd G4 (188 mg, 0.22 mmol), $K_3PO_4 \cdot 7H_2O$ (988 mg, 2.92 mmol), and THF/$H_2O$ (v/v = 6 mL/2 mL). The reaction mixture was purged with nitrogen three times and stirred at room temperature for 15 h. The reaction was added saturated ammonium chloride solution (20 mL), extracted with DCM (20 mL × 2). The combined organic phases were dried over anhydrous sodium sulfate and concentrated. The residue was purified by column chromatography using DCM/MeOH (v/v = 30/1) as eluent to obtain 400 mg of a yellow solid compound **24-1** with a yield of 84%. LC-MS (ESI, pos. ion) m/z: 648.1 $[M+H]^+$.

Step 2: Synthesis of Compound **24**

Into a 25 mL single-necked flask were added compound **24-1** (400 mg, 0.62 mmol) and acetonitrile (10 mL). The mixture was cooled to 0 °C and stirred for 5 minutes. Concentrated sulfuric acid (186 mg, 1.86 mmol, 98% purity) was added dropwise. After stirring for 1.5 h, the reaction was stopped and quenched with saturated aqueous sodium bicarbonate solution (20 mL). The mixture was extracted with DCM (20 mL × 2). The combined organic phases were dried over anhydrous sodium sulfate and concentrated. The residue was purified by column chromatography using DCM/MeOH (v/v = 30/1) as eluent to obtain 210 mg of a yellow solid compound **24** with a yield of 50%, LC-MS (ESI, pos. ion) m/z: 604.1 $[M+H]^+$; HRMS (ESI): 604.2563 $[M+H]^+$; $^1H$ NMR (400 MHz, $CD_3OD$) δ 7.65 - 7.53 (m, 1H), 7.45 - 7.29 (m, 2H), 7.22 - 7.14 (m, 1H), 6.99 - 6.85 (m, 1H), 5.44 - 5.21 (m, 1H), 4.68 - 4.59 (m, 1H), 4.38 - 4.06 (m, 6H), 3.98 - 3.83 (m, 2H), 3.84 - 3.74 (m, 2H), 3.29 - 3.20 (m, 2H), 3.10 - 2.97 (m, 1H), 2.42 - 2.22 (m, 2H), 2.22 - 1.85 (m, 9H). $^{19}F$ NMR (376 MHz, $CD_3OD$) δ -115.94 (1F), -141.97 (1F), -173.62 (1F).

**Example 25: Synthesis of Compound 25**

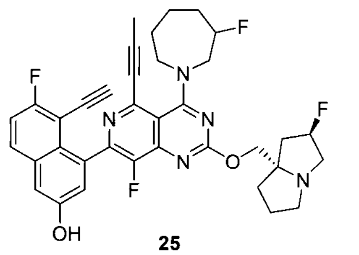

25

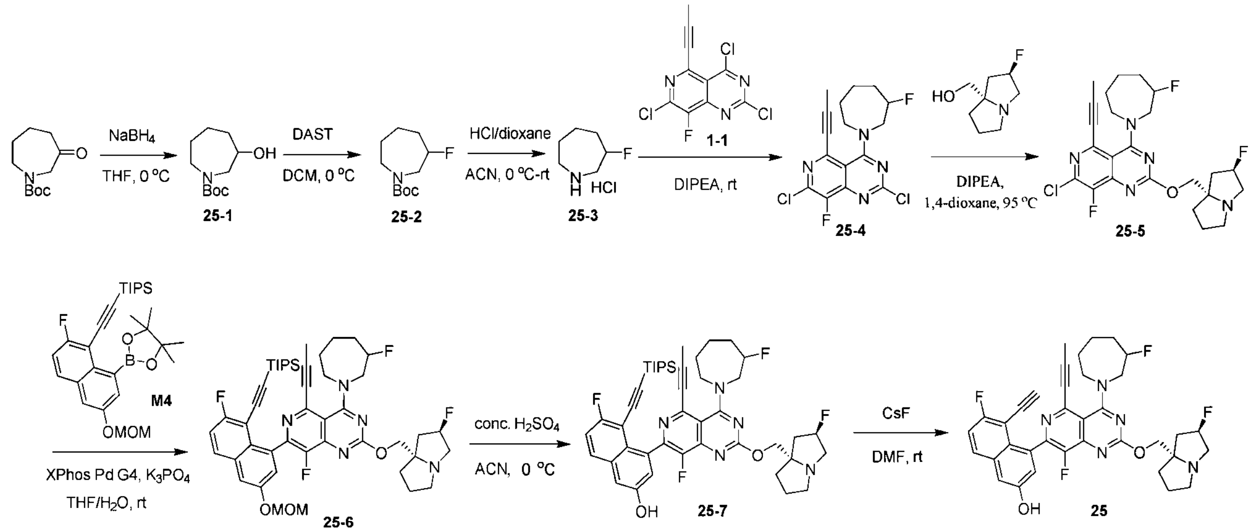


Step 1: Synthesis of Compound **25-1**

Into a 100 mL two-necked flask were added $NaBH_4$ (0.71 g, 18.76 mmol) and anhydrous THF (30 mL). The mixture was purged with nitrogen three times and stirred until dissolution was complete. After cooling to 0 °C, *N*-Boc-3-azepanone (2.0 g, 9.38 mmol, Shaoyuan Technology) was added. The reaction mixture was stirred at 0 °C for 1.5 h, then quenched by the addition of saturated aqueous ammonium chloride solution (20 mL). The mixture was allowed to warm to room temperature and extracted with EA (20 mL × 2). The organic layers were combined, dried over anhydrous sodium sulfate, and concentrated to obtain 2.0 g of a colorless clear oil compound **25-1** with a yield of 99%. $^1H$ NMR (400 MHz, $CDCl_3$) δ 3.90 - 3.78 (m, 1H), 3.72 - 3.54 (m, 1H), 3.55 - 3.38 (m, 2H), 3.38 - 3.27 (m, 1H), 3.10 - 2.97 (m, 1H), 1.85 - 1.73 (m, 1H), 1.73 - 1.52 (m, 3H), 1.50 - 1.21 (m, 11H).

Step 2: Synthesis of Compound **25-2**

Into a 250 mL two-necked flask were added compound **25-1** (2 g, 9.29 mmol) and anhydrous DCM (50 mL). The mixture was purged with nitrogen three times and stirred until dissolution was complete. After cooling to 0 °C, diethylaminosulfur trifluoride (DAST, 4.91 mL, 37.16 mmol) was added dropwise. The reaction mixture was stirred at 0 °C for 4 h, then quenched by the addition of saturated aqueous sodium bicarbonate solution (200 mL). The mixture was allowed to warm to room temperature and extracted with DCM (50 mL). The organic phase was separated, dried over anhydrous sodium sulfate, and concentrated. The residue was purified by column chromatography using PE/EA (v/v = 9/1) as the eluent to obtain 0.8 g of a yellow liquid compound **25-2** with a yield of 40.0%. $^1H$ NMR (400 MHz, $CDCl_3$) δ 4.97 - 4.62 (m, 1H), 4.09 - 3.71 (m, 1H), 3.68 - 3.54 (m, 1H), 3.38 - 3.00 (m, 2H), 1.97 - 1.59 (m, 6H), 1.45 (s, 9H).

Step 3: Synthesis of Compound **25-3**

Into a 50 mL single-necked flask were added compound **25-2** (0.8 g, 3.68 mmol) and DCM (15 mL). The mixture was stirred to dissolve. A solution of 4 M HCl in 1,4-dioxane (8 mL, 32 mmol) was added dropwise. The resulting mixture was stirred at room temperature for 15 h. The reaction was stopped and concentrated directly to obtain 0.5 g of a white solid

compound **25-3** with a yield of 88%.

Step 4: Synthesis of Compound **25-4**

Into a 50 mL two-necked flask were added compound **25-3** (0.24 g, 1.58 mmol) and DCM (4 mL). DIPEA (2.09 mL, 12.64 mmol) was added, and the mixture was stirred for 15 minutes. Into a 50 mL single-necked flask were added compound **1-1** (0.46 g, 1.58 mmol) and anhydrous DCM (4 mL). The mixture was purged with nitrogen three times and stirred until dissolution was complete. A solution of compound **25-3** was added dropwise to the mixture. The resulting mixture was stirred at room temperature for 12 h. The reaction was stopped and quenched by addition of saturated aqueous ammonium chloride solution (30 mL). The mixture was allowed to warm to room temperature and extracted with DCM (30 mL × 2). The organic phase was separated, dried over anhydrous sodium sulfate, and concentrated. The residue was purified by column chromatography using PE/EA (v/v = 3:1) as the eluent to obtain 360 mg of a yellow liquid compound **25-4** with a yield of 40.0%. LC-MS (ESI, pos. ion) m/z: 371.1 $[M+H]^+$.

Step 5: Synthesis of Compound **25-5**

Into a 100 mL single-necked flask were added compound **25-4** (360 mg, 0.97 mmol), ((2*R*,7a*S*)-2-fluorohexahydro-1*H*-pyrrolizin-7a-yl)methanol (280 mg, 1.75 mmol), DIPEA (0.48 mL, 2.91 mmol), and anhydrous 1,4-dioxane (15 mL). The mixture was purged with nitrogen three times. The mixture was heated to 95 °C and stirred for 20 h. The reaction was stopped and cooled to room temperature. The reaction solution was concentrated, diluted with EA (20 mL), and washed with saturated ammonium chloride solution (30 mL). The phases were separated and extracted with EA (20 mL × 2). The combined organic phases were dried over anhydrous sodium sulfate and concentrated. The residue was purified by column chromatography using DCM/MeOH (v/v = 50/1) as eluent to obtain 215 mg of a yellow solid compound **25-5** with a yield of 45%. LC-MS (ESI, pos. ion) m/z: 494.3 $[M+H]^+$.

Step 6: Synthesis of Compound **25-6**

Into a 25 mL single-necked flask were added compound **25-5** (195 mg, 0.39 mmol, 1 equiv.), **M4** (0.60 g, 1.17 mmol), Xphos Pd G4 (134 mg, 0.16 mmol), $K_3PO_4 \cdot 7H_2O$ (528 mg, 1.56 mmol) and $THF/H_2O$ (v/v = 5.1 mL/1.7 mL). The mixture was purged with nitrogen three times and stirred at room temperature for 15 h. The reaction was stopped and added saturated ammonium chloride solution (20 mL). The mixture was extracted with EA (30 mL × 3). The combined organic phases were dried over anhydrous sodium sulfate and concentrated. The residue was purified by column chromatography using DCM/MeOH (v/v = 30/1) as eluent to obtain 282 mg of a yellow solid compound **25-6** with a yield of 84%. LC-MS (ESI, pos. ion) m/z: 844.7 $[M+H]^+$.

Step 7: Synthesis of Compound **25-7**

Into a 25 mL single-necked flask were added compound **25-6** (282 mg, 0.33 mmol) and acetonitrile (5 mL). The mixture was cooled to 0 °C and stirred for 5 minutes. Concentrated sulfuric acid (100 mg, 1.00 mmol, 98% purity) was added dropwise. After stirring for 1.5 h, the reaction was stopped and quenched with saturated aqueous sodium bicarbonate solution (20 mL). The mixture was extracted with DCM (20 mL × 2). The combined organic phases were dried over anhydrous sodium sulfate and concentrated. The residue was purified by column chromatography using DCM/MeOH (v/v = 20/1) as eluent to obtain 152 mg of a yellow solid compound **25-7** with a yield of 46%, LC-MS (ESI, pos. ion) m/z: 800.7 $[M+H]^+$.

Step 8: Synthesis of Compound **25**

Into a 25 mL single-necked flask were added compound **25-7** (152 mg, 0.19 mmol), cesium fluoride (230 mg, 1.52 mmol) and DMF (1 mL). The mixture was purged with nitrogen three times and stirred at room temperature for 15 h. The reaction was stopped, and water (20 mL) was added, precipitating a solid. Filtration afforded the crude solid. The crude product was purified by column chromatography using DCM/MeOH (v/v = 20/1) as eluent to obtain 85 mg of a yellow solid compound **25** with a yield of 69%, LC-MS (ESI, pos. ion) m/z: 644.3 $[M+H]^+$; HRMS (ESI): 644.2678 $[M+H]^+$; $^1H$ NMR (400 MHz, $CD_3OD$) $\delta$ 7.86 (dd, *J* = 8.9, 5.9 Hz, 1H), 7.37 - 7.29 (m, 2H), 7.27 - 7.15 (m, 1H), 5.33 (d, *J*= 54.1 Hz, 1H), 5.03 (d, *J*= *45.9* Hz, 1H), 4.71 - 4.56 (m, 1H), 4.40 - 4.25 (m, 3H), 4.22 - 3.94 (m, 2H), 3.84 - 3.67 (m, 1H), 3.30 - 3.18 (m, 3H), 3.09 - 2.96 (m, 1H), 2.42 - 2.10 (m, 7H), 2.08 - 1.67 (m, 8H). $^{19}F$ NMR (376 MHz, $CD_3OD$) $\delta$ -111.60 (1F), -140.84 (1F), -173.57 (1F), -176.83 (1F).

**Example 26: Synthesis of Compound 26**

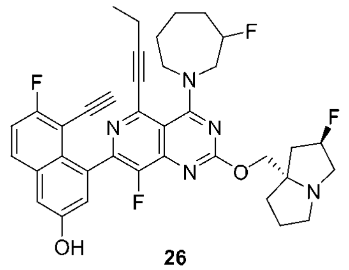

26

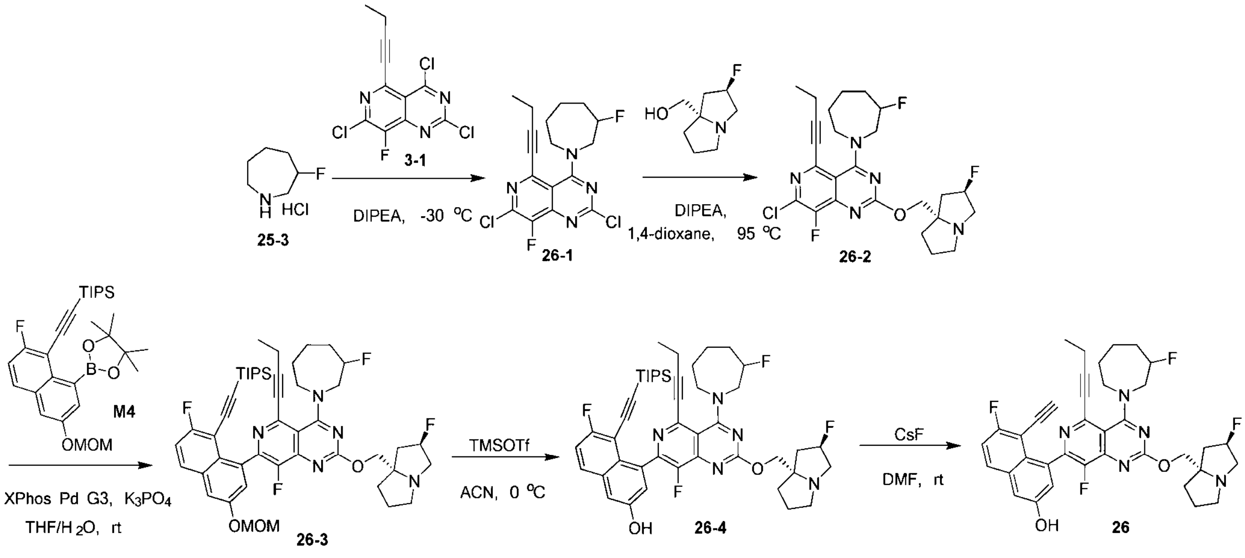


Step 1: Synthesis of Compound **26-1**

Into a 100 mL single-necked flask was added compound **3-1** (1.14 g, 3.74 mmol). The flask was purged with nitrogen three times. DCM (15 mL) was added for dissolution, and DIPEA (1.45 g, 11.22 mmol) was added. The mixture was cooled to -30 °C. Compound **25-3** (0.445 g, 2.90 mmol) was added. The reaction mixture was stirred at -30 °C for 30 min. The reaction was quenched by addition of saturated aqueous ammonium chloride solution (20 mL) and allowed to warm to room temperature. The mixture was extracted with DCM (20 mL × 2). The combined organic phase were dried over anhydrous sodium sulfate and concentrated. The residue was purified by column chromatography using PE/EA (v/v = 9/1) as the eluent to obtain 326 mg of a yellow solid compound 26-1 with a yield of 23%. LC-MS (ESI, pos. ion) m/z: 385.5 $[M+H]^+$.

Step 2: Synthesis of Compound **26-2**

Into a 100 mL single-necked flask were added compound **26-1** (326 mg, 0.85 mmol), ((2*R*,7a*S*)-2-fluorohexahydro-1*H*-pyrrolizin-7a-yl)methanol (270 mg, 1.7 mmol), DIPEA (0.33 g, 2.55 mmol) and anhydrous 1,4-dioxane (5 mL). The mixture was purged with nitrogen three times. The mixture was heated to 95 °C and stirred for 12 h. The reaction was stopped and cooled to room temperature. The reaction solution was concentrated, diluted with EA (20 mL), and washed with saturated ammonium chloride solution (30 mL). The phases were separated and extracted with EA (20 mL × 2). The combined organic phases were dried over anhydrous sodium sulfate and concentrated. The residue was purified by column chromatography using DCM/MeOH (v/v = 19/1) as eluent to obtain 340 mg of a yellow solid compound **26-2** with a yield of 79%. LC-MS (ESI, pos. ion) m/z: 508.2 $[M+H]^+$.

Step 3: Synthesis of Compound **26-3**

Into a 100 mL single-necked flask were added compound **26-2** (340 mg, 0.67 mmol, 1 equiv.), **M4** (0.69 g, 1.34

mmol), Xphos Pd G3 (110 mg, 0.13 mmol), $K_3PO_4 \cdot 7H_2O$ (680 mg, 2.01 mmol) and THF/$H_2O$ (v/v = 5 mL/1 mL). The mixture was purged with nitrogen three times and stirred at 30 °C for 15 h. The reaction was stopped and to the mixture was added saturated ammonium chloride solution (10 mL). The mixture was extracted with EA (20 mL × 3). The combined organic phases were dried over anhydrous sodium sulfate and concentrated. The residue was purified by column chromatography using DCM/MeOH (v/v = 49/1) as eluent to obtain 460 mg of a reddish-brown solid compound **26-3** with a yield of 80%. LC-MS (ESI, pos. ion) m/z: 858.4 $[M+H]^+$.

Step 4: Synthesis of Compound **26-4**

Into a 25 mL single-necked flask were added compound **26-3** (460 mg, 0.54 mmol) and acetonitrile (6 mL). The mixture was cooled to 0 °C. TMSOTf (0.29 mL, 1.62 mmol) was added dropwise. After stirring at 0 °C for 0.5 h, the reaction was stopped and quenched with saturated aqueous sodium bicarbonate solution (10 mL). The mixture was extracted with EA (20 mL × 2). The combined organic phases were dried over anhydrous sodium sulfate and concentrated. The residue was purified by column chromatography using DCM/MeOH (v/v = 49/1) as eluent to obtain 377 mg of a yellow solid compound **26-4** with a yield of 86%, LC-MS (ESI, pos. ion) m/z: 814.4 $[M+H]^+$.

Step 5: Synthesis of Compound **26**

Into a 25 mL single-necked flask were added compound **26-4** (377 mg, 0.46 mmol), cesium fluoride (350 mg, 2.3 mmol) and DMF (1 mL). The mixture was purged with nitrogen three times and stirred at room temperature for 5 h. The reaction was stopped, and water (20 mL) was added, precipitating a solid. Filtration afforded the crude solid. The crude product was purified by column chromatography using DCM/MeOH (v/v = 8/1) as eluent to obtain 200 mg of a yellow solid compound **26** with a yield of 66%, LC-MS (ESI, pos. ion) m/z: 658.3 $[M+H]^+$; HRMS (ESI): 658.2796$[M+H]^+$; $^1H$ NMR (400 MHz, $CD_3OD$) $\delta$ 7.88 (dd, *J* = 9.1, 5.8 Hz, 1H), 7.41 - 7.26 (m, 3H), 5.37 (d, *J*= 54.2 Hz, 1H), 5.04 (d, *J*= 45.5 Hz, 1H), 4.80 - 4.49 (m, 2H), 4.49 - 4.26 (m, 3H), 4.19 - 3.95 (m, 1H), 3.88 - 3.66 (m, 1H), 3.47 - 3.35 (m, 2H), 3.27 - 3.05 (m, 2H), 2.58 (q, *J* = 7.4 Hz, 2H), 2.47 - 1.68 (m, 12H), 1.14 - 1.06 (m, 3H). $^{19}F$ NMR (376 MHz, $CD_3OD$) $\delta$ -111.66 (1F), -140.97 (1F), -173.68 (1F), -176.96 (1F).

**Example 27: Synthesis of Compound 27**

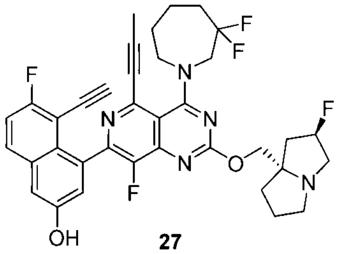

**27**

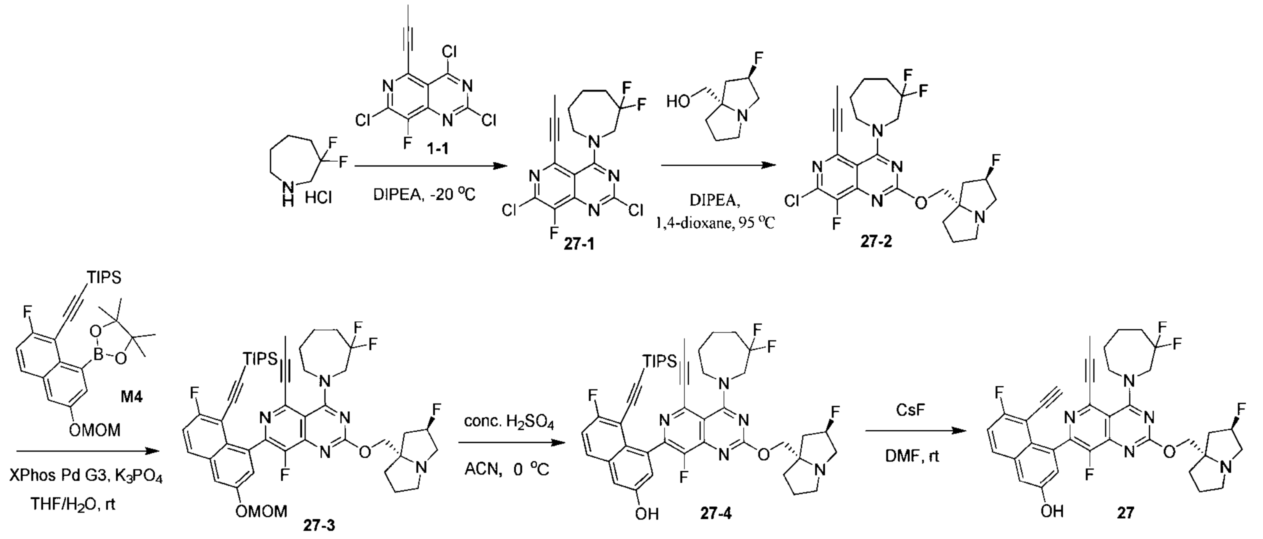


Step 1: Synthesis of Compound **27-1**

Into a 50 mL two-necked flask were added compound 3,3-difluoroazepane hydrochloride (0.43 g, 2.51 mmol, purchased from Acon Biotech) and DCM (4 mL). DIPEA (3.32 mL, 20.08 mmol) was added, and the mixture was stirred for 15 minutes. Into a separate 50 mL single-necked flask were added compound **1-1** (0.73 g, 1.58 mmol) and anhydrous DCM (4 mL), and the mixture was purged with nitrogen three times and stirred until dissolution was complete. The previously prepared 3, 3-difluoroazepane solution was then added dropwise. The resulting mixture was stirred at -20 °C for 12 h. The reaction was stopped and quenched by addition of saturated aqueous ammonium chloride solution (20 mL). The mixture was allowed to warm to room temperature and extracted with DCM (20 mL × 2). The organic phase was separated, dried over anhydrous sodium sulfate, and concentrated. The residue was purified by column chromatography using PE/EA (v/v = 50/1-10/1) as the eluent to obtain 179 mg of a yellow liquid compound **25-4** with a yield of 19%. LC-MS (ESI, pos. ion) m/z: 389.2 $[M+H]^+$.

Step 2: Synthesis of Compound **27-2**

Into a 100 mL single-necked flask were added compound **25-4** (348 mg, 0.89 mmol), ((2*R*,7a*S*)-2-fluorohexahydro-1*H*-pyrrolizin-7a-yl)methanol (260 mg, 1.60 mmol), DIPEA (0.44 mL, 2.67 mmol) and anhydrous 1,4-dioxane (20 mL). The mixture was purged with nitrogen three times. The mixture was heated to 95 °C and stirred for 17 h. The reaction was stopped and cooled to room temperature. The reaction solution was concentrated, diluted with EA (20 mL), and washed with saturated ammonium chloride solution (30 mL). The phases were separated and extracted with EA (20 mL × 2). The combined organic phases were dried over anhydrous sodium sulfate and concentrated. The residue was purified by column chromatography using DCM/MeOH (v/v = 50/1) as eluent to obtain 260 mg of a yellow solid compound **27-2** with a yield of 57%. LC-MS (ESI, pos. ion) m/z: 512.3 $[M+H]^+$.

Step 3: Synthesis of Compound **27-3**

Into a 25 mL single-necked flask were added compound **27-2** (260 mg, 0.51 mmol, 1 equiv.), **M4** (523 mg, 1.02 mmol), Xphos Pd G4 (129 mg, 0.15 mmol), $K_3PO_4 \cdot 7H_2O$ (518 mg, 1.53 mmol) and $THF/H_2O$ (v/v = 5.1 mL/1.7 mL). The mixture was purged with nitrogen three times and stirred at room temperature for 15 h. The reaction was stopped and added saturated ammonium chloride solution (20 mL). The mixture was extracted with DCM (20 mL × 2). The combined organic phases were dried over anhydrous sodium sulfate and concentrated. The residue was purified by column chromatography using DCM/MeOH (v/v = 50/1) as eluent to obtain 215 mg of a yellow solid compound **27-3** with a yield of 49%. LC-MS (ESI, pos. ion) m/z: 862.4 $[M+H]^+$.

Step 4: Synthesis of Compound **27-4**

Into a 25 mL single-necked flask were added compound **27-3** (215 mg, 0.25 mmol) and acetonitrile (5 mL). The mixture was cooled to 0 °C and stirred for 5 minutes. Concentrated sulfuric acid (75 mg, 0.75 mmol, 98% purity) was added dropwise. After stirring for 1.5 h, the reaction was stopped and quenched with saturated aqueous sodium bicarbonate

solution (20 mL). The mixture was extracted with DCM (20 mL × 2). The combined organic phases were dried over anhydrous sodium sulfate and concentrated. The residue was purified by column chromatography using DCM/MeOH (v/v = 30/1) as eluent to obtain 200 mg of a yellow solid compound **25-7** with a yield of 98%, LC-MS (ESI, pos. ion) m/z: 818.4 $[M+H]^+$.

Step 5: Synthesis of Compound **27**

Into a 25 mL single-necked flask were added compound **27-4** (200 mg, 0.24 mmol), cesium fluoride (290 mg, 1.92 mmol) and DMF (1 mL). The mixture was purged with nitrogen three times and stirred at room temperature for 15 h. The reaction was stopped, and water (20 mL) was added, precipitating a solid. Filtration afforded the crude solid. The crude product was purified by column chromatography using DCM/MeOH (v/v = 20/1) as eluent to obtain 130 mg of a yellow solid compound **27** with a yield of 80%, LC-MS (ESI, pos. ion) m/z: 662.3 $[M+H]^+$; HRMS (ESI): 662.2572 $[M+H]^+$; $^1$H NMR (400 MHz, $CD_3OD$) δ 7.88 (dd, *J*= 9.1, 5.8 Hz, 1H), 7.40 - 7.20 (m, 3H), 5.44 - 5.21 (m, 1H), 4.75 - 4.53 (m, 2H), 4.43 - 4.20 (m, 4H), 3.99 - 3.81 (m, 1H), 3.28 - 3.20 (m, 2H), 3.20 - 3.13 (m, 1H), 3.10 - 2.97 (m, 1H), 2.41 - 2.07 (m, 9H), 2.07 - 1.87 (m, 4H), 1.85 - 1.65 (m, 2H). $^{19}$F NMR (376 MHz, $CD_3OD$) δ -93.93 (2F), -111.44 (1F), -140.06 (1F), -173.60 (1F).

**Example 28: Synthesis of Compound 28**

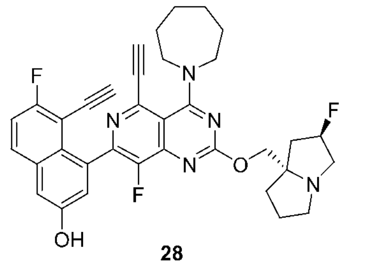

28

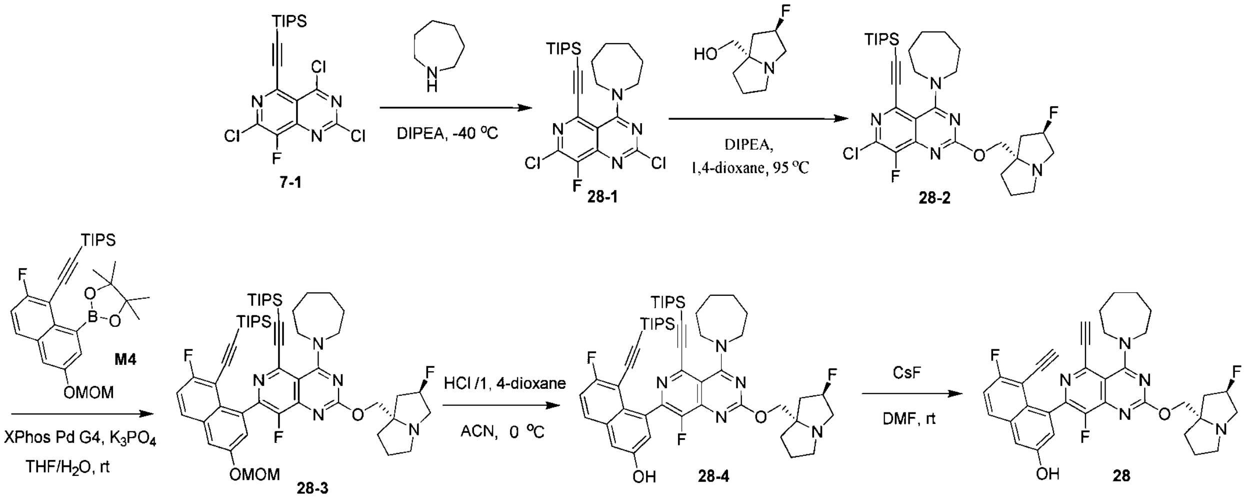


7-1 28-1 28-2 M4 28-3 28-4 28

Step 1: Synthesis of Compound **28-1**

Into a 100 mL single-necked flask were added compound **7-1** (1.09 g, 2.52 mmol) and anhydrous dichloromethane (10 mL). The mixture was purged with nitrogen three times, cooled to -40 °C, and stirred until dissolved. DIPEA (2.08 mL, 12.6 mmol) was added, followed by slow dropwise addition of homopiperidine (0.28 mL, 2.52 mmol). After stirring at -40 °C for 0.5 h, the reaction was stopped and quenched with saturated ammonium chloride solution (30 mL). The mixture was extracted with DCM (15 mL × 2). The organic phase was separated, dried over anhydrous sodium sulfate, and concentrated. The residue was purified by column chromatography using PE/EA (v/v = 25/1) as eluent to obtain 695 mg of a yellow solid compound **28-1** with a yield of 56%. LC-MS (ESI, pos. ion) m/z:495.2 $[M+H]^+$.

Step 2: Synthesis of Compound **28-2**

Into a 100 mL single-necked flask were added compound **28-1** (200 mg, 0.40 mmol), ((2*R*,7a*S*)-2-fluorohexahydro-1*H*-pyrrolizin-7a-yl)methanol (76 mg, 0.48 mmol), DIPEA (0.13 mL, 0.80 mmol) and anhydrous 1,4-dioxane (4 mL). The mixture was purged with nitrogen three times. The mixture was heated to 95 °C and stirred for 15 h. The reaction was stopped and cooled to room temperature. The reaction solution was concentrated, diluted with EA (20 mL), and washed with saturated ammonium chloride solution (30 mL). The phases were separated and extracted with EA (20 mL). The combined organic phases were dried over anhydrous sodium sulfate and concentrated. The residue was purified by column chromatography using DCM/MeOH (v/v = 20/1) as eluent to obtain 53 mg of a yellow solid compound **28-2** with a yield of 21%. LC-MS (ESI, pos. ion) m/z: 618.5 $[M+H]^+$.

Step 3: Synthesis of Compound **28-3**

Into a 25 mL two-necked flask were added compound **28-1** (53 mg, 0.086 mmol, 1 equiv.), **M4** (88 mg, 0.17 mmol), Xphos Pd G4 (15 mg, 0.017 mmol), $K_3PO_4 \cdot 7H_2O$ (73 mg, 0.21 mmol) and THF/$H_2O$ (v/v = 2 mL/0.4 mL). The mixture was purged with nitrogen three times and stirred at room temperature for 15 h. The reaction was quenched and diluted with DCM (20 mL). The mixture was washed with saturated sodium chloride solution (20 mL) and extracted with DCM (15 mL × 2). The combined organic phases were dried over anhydrous sodium sulfate and concentrated. The residue was purified by column chromatography using DCM/MeOH (v/v = 40/1) as eluent to obtain 47 mg of a yellow solid compound **28-4** with a yield of 57%. LC-MS (ESI, pos. ion) m/z: 968.4 $[M+H]^+$.

Step 4: Synthesis of Compound **28-4**

Into a 25 mL single-necked flask was added compound **28-3** (47 mg, 0.23 mmol) and DCM (2 mL). The mixture was cooled to 0 °C and stirred for 5 minutes. HCl/1,4-dioxane (0.12 mL, 0.48 mmol, 4 M) was added dropwise. The reaction mixture was transferred to room temperature and stirred for 30 min. The reaction was stopped and concentrated to obtain 44.8 mg of a yellow solid compound **28-4.** The yield was assumed to be 100%. No LCMS signal was detected.

Step 5: Synthesis of Compound **28**

Into a 25 mL single-necked flask were added compound **28-4** (44.8 mg, 0.048 mmol), cesium fluoride (110 mg, 0.72 mmol) and DMF (0.3 mL). The mixture was purged with nitrogen three times and stirred at room temperature for 16 h. The reaction was stopped, and water (10 mL) was added, precipitating a solid. Filtration afforded the crude solid. The crude product was purified by column chromatography using DCM/MeOH (v/v = 10/1) as eluent to obtain 20 mg of a red solid compound **28** with a yield of 67%, LC-MS (ESI, pos. ion) m/z: 612.3 $[M+H]^+$; HRMS (ESI): 612.2565 $[M+H]^+$; $^1H$ NMR (400 MHz, $CD_3OD$) δ 7.87 (dd, *J* = 9.1, 5.7 Hz, 1H), 7.39 - 7.29 (m, 2H), 7.28 - 7.22 (m, 1H), 5.40 (d, *J* = 52.5 Hz, 1H), 4.63 - 4.55 (m, 3H), 4.49 - 4.34 (m, 2H), 4.20 - 3.97 (m, 4H), 3.67 - 3.51 (m, 1H), 3.51 - 3.36 (m, 3H), 3.24 - 3.10 (m, 1H), 2.52 - 2.30 (m, 2H), 2.28 - 2.05 (m, 4H), 2.05 - 1.82 (m, 6H). $^{19}F$ NMR (376 MHz, $CD_3OD$) δ -114.46 (1F), -138.48 (1F), -173.78 (1F).

**Example 29: Synthesis of Compound 29**

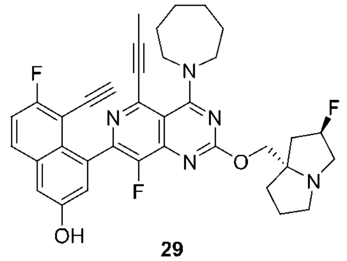

**29**

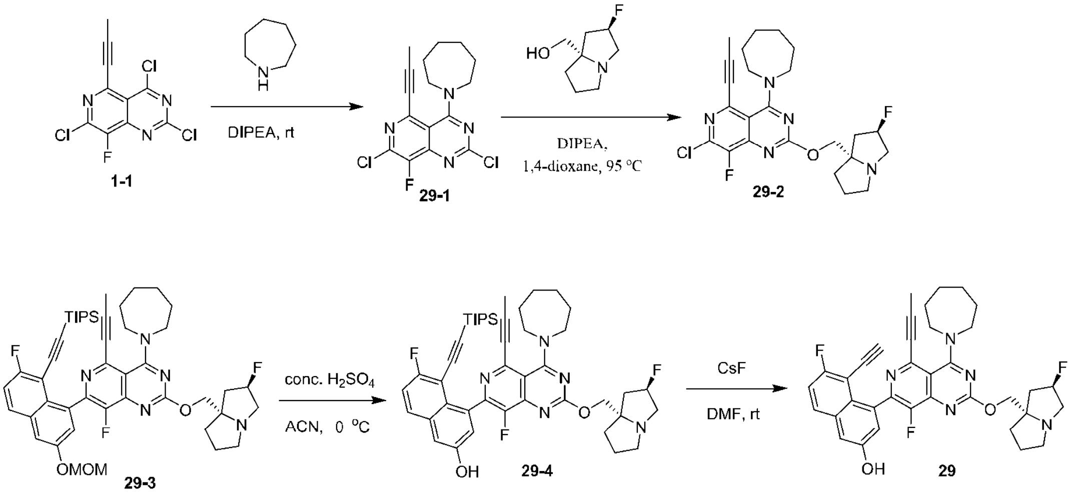


Step 1: Synthesis of Compound **29-1**

Into a 100 mL single-necked flask were added compound **1-1** (1.15 g, 3.96 mmol) and anhydrous dichloromethane (20 mL). The mixture was purged with nitrogen three times and stirred until dissolved. Homopiperidine (0.39 g, 3.96 mmol) was added dropwise, followed by dropwise addition of DIPEA (3.93 mL, 23.76 mmol). The reaction mixture was stirred at room temperature for 1.5 h. The reaction was quenched by addition of saturated aqueous ammonium chloride solution (30 mL) and extracted with DCM (30 mL × 2). The combined organic layers were dried over anhydrous sodium sulfate and concentrated. The residue was purified by column chromatography using PE/EA (v/v = 10/1) as the eluent to obtain 390 mg of a yellow solid compound **29-1** with a yield of 28%. LC-MS (ESI, pos. ion) m/z:353.1 $[M+H]^+$.

Step 2: Synthesis of Compound **29-2**

Into a 100 mL single-necked flask were added compound **29-1** (380 mg, 1.08 mmol), ((2*R*,7a*S*)-2-fluorohexahydro-1*H*-pyrrolizin-7a-yl)methanol (260 mg, 1.62 mmol), DIPEA (0.36 mL, 2.16 mmol), and anhydrous 1,4-dioxane (20 mL). The mixture was purged with nitrogen three times. The mixture was heated to 95 °C and stirred for 20 h. The reaction was stopped and cooled to room temperature. The reaction solution was concentrated, diluted with EA (20 mL), and washed with saturated ammonium chloride solution (30 mL). The phases were separated and extracted with EA (20 mL × 2). The combined organic phases were dried over anhydrous sodium sulfate and concentrated. The residue was purified by column chromatography using DCM/MeOH (v/v = 50/1) as eluent to obtain 215 mg of a yellow solid compound **29-2** with a yield of 42%. LC-MS (ESI, pos. ion) m/z: 476.4 $[M+H]^+$.

Step 3: Synthesis of Compound **29-3**

Into a 25 mL single-necked flask were added compound **29-2** (200 mg, 0.42 mmol, 1 equiv.), **M4** (430 mg, 0.84 mmol), Xphos Pd G4 (108 mg, 0.13 mmol), $K_3PO_4 \cdot 7H_2O$ (568 mg, 1.68 mmol) and THF/$H_2O$ (v/v = 6 mL/2 mL). The mixture was purged with nitrogen three times and stirred at room temperature for 15 h. The reaction was quenched and diluted with EA (15 mL). The mixture was washed with saturated sodium chloride solution (20 mL) and extracted with EA (20 mL × 2). The combined organic phases were dried over anhydrous sodium sulfate and concentrated. The residue was purified by column chromatography using DCM/MeOH (v/v = 30/1) as eluent to obtain 306 mg of a yellow solid compound **29-3** with a yield of 88%. LC-MS (ESI, pos. ion) m/z: 826.4 $[M+H]^+$.

Step 4: Synthesis of Compound **29-4**

Into a 25 mL single-necked flask were added compound **29-3** (306 mg, 0.37 mmol) and ACN (5 mL). The mixture was cooled to 0 °C and stirred for 5 minutes. Concentrated sulfuric acid (0.060 mL, 1.11 mmol, 98% w/w) was added dropwise. After stirring at 0 °C for 2 h, the reaction was stopped and quenched with saturated aqueous sodium bicarbonate solution (20 mL). The mixture was extracted with DCM (15 mL × 2). The combined organic phases were dried over anhydrous sodium sulfate and concentrated. The residue was purified by column chromatography using DCM/MeOH (v/v = 20/1) as eluent to obtain 160 mg of a yellow solid compound **29-4** with a yield of 55%, LC-MS (ESI, pos. ion) m/z: 782.7 $[M+H]^+$.

Step 5: Synthesis of Compound **29**

Into a 25 mL single-necked flask were added compound **29-4** (150 mg, 0.19 mmol), cesium fluoride (230 mg, 1.52 mmol), and DMF (1 mL). The mixture was purged with nitrogen three times and stirred at room temperature for 3 h. The reaction was stopped, and water (20 mL) was added, precipitating a solid. Filtration afforded the crude solid. The crude product was purified by column chromatography using DCM/MeOH (v/v = 20/1) as eluent to obtain 68 mg of a yellow solid compound **29** with a yield of 57%. LC-MS (ESI, pos. ion) m/z: 626.3 [M+H]$^+$; HRMS (ESI): 626.2739 [M+H]$^+$; $^1$H NMR (400 MHz, $CD_3OD$) δ 8.00 - 7.73 (m, 1H), 7.42 - 7.15 (m, 3H), 5.47 - 5.17 (m, 1H), 4.43 - 4.21 (m, 2H), 4.22 - 3.82 (m, 5H), 3.57 - 3.13 (m, 5H), 3.13 - 2.75 (m, 1H), 2.48 - 2.07 (m, 7H), 2.08 - 1.76 (m, 8H). $^{19}$F NMR (376 MHz, $CD_3OD$) δ -114.48 (1F), -140.98 (1F), -173.52 (1F).

**Example 30: Synthesis of Compound 30**

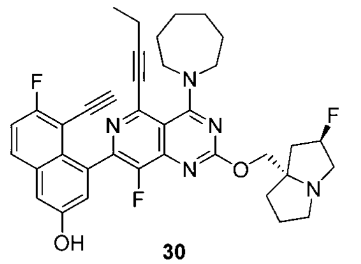

**30**

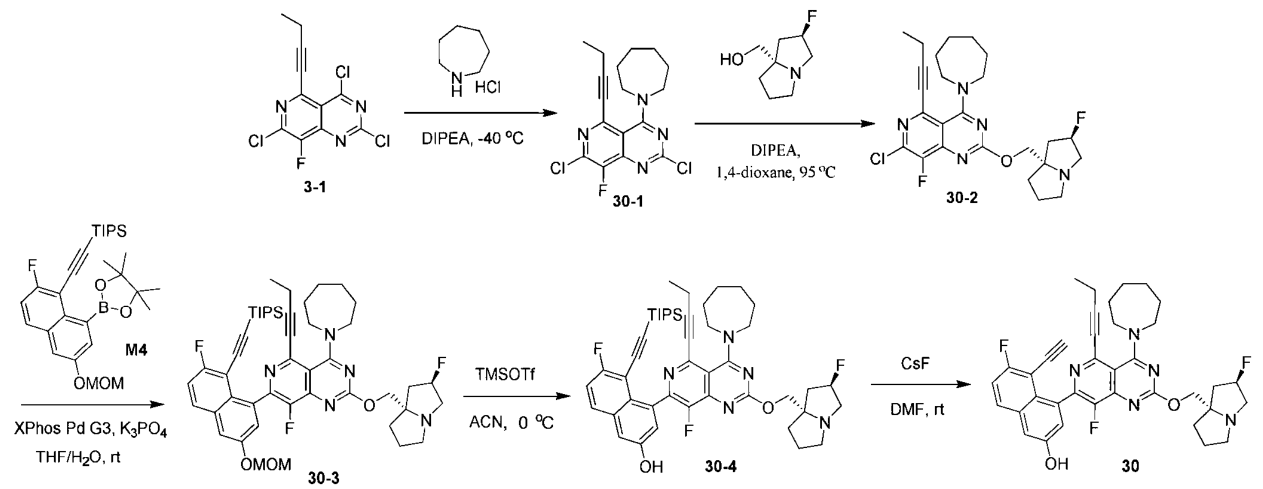


3-1, 30-1, 30-2, M4, 30-3, 30-4, 30

Step 1: Synthesis of Compound **30-1**

Into a 100 mL single-necked flask were added compound 3-1 (1.48 g, 4.86 mmol) and anhydrous dichloromethane (10 mL). The mixture was purged with nitrogen three times, cooled to -40°C and stirred until compound **3-1** was dissolved. Homopiperidine hydrochloride (0.66 g, 4.86 mmol) was added dropwise, followed by dropwise addition of DIPEA (4.02 mL, 24.3 mmol). The reaction mixture was stirred at -40 °C for 2 h. The reaction mixture was quenched by addition of saturated aqueous ammonium chloride solution (50 mL) and extracted with DCM (30 mL × 2). The combined organic layers were dried over anhydrous sodium sulfate and concentrated. The residue was purified by column chromatography using PE/EA (v/v = 92/8) as the eluent to obtain 484 mg of a yellow solid compound **30-1** with a yield of 27%. LC-MS (ESI, pos. ion) m/z:367.5 [M+H]$^+$.

Step 2: Synthesis of Compound **30-2**

Into a 100 mL single-necked flask were added compound **30-1** (480 mg, 1.31 mmol), ((2*R*,7a*S*)-2-fluorohexahydro-1*H*-pyrrolizin-7a-yl)methanol (310 mg, 1.97 mmol), DIPEA (0.43 mL, 2.62 mmol), and anhydrous 1,4-dioxane (10 mL). The mixture was purged with nitrogen three times. The mixture was heated to 95 °C and stirred for 33 h. The reaction

was stopped and cooled to room temperature. The reaction solution was concentrated, diluted with EA (30 mL), and washed with saturated ammonium chloride solution (50 mL). The phases were separated and extracted with EA (20 mL × 2). The combined organic phases were dried over anhydrous sodium sulfate and concentrated. The residue was purified by column chromatography using DCM/MeOH (v/v = 50/1) as eluent to obtain 341 mg of a yellow solid compound **30-2** with a yield of 53%. LC-MS (ESI, pos. ion) m/z: 490.2 $[M+H]^+$.

Step 3: Synthesis of Compound **30-3**

Into a 25 mL single-necked flask were added compound **30-2** (170 mg, 0.35 mmol, 1 equiv.), **M4** (360 mg, 0.70 mmol), $K_3PO_4 \cdot 7H_2O$ (360 mg, 0.10 mmol) and $THF/H_2O$ (v/v = 5 mL/1 mL). The mixture was purged with nitrogen three times, and Xphos Pd G3 (88 mg, 0.105 mmol) was added portionwise. The mixture was stirred at room temperature for 15 h. The reaction was stopped and the mixture was diluted with DCM (20 mL). The mixture was washed with saturated sodium chloride solution (20 mL) and extracted with DCM (20 mL × 2). The combined organic phases were dried over anhydrous sodium sulfate and concentrated. The residue was purified by column chromatography using DCM/MeOH (v/v = 98/2) as eluent to obtain 256 mg of a red oil compound **30-3** with a yield of 88%. LC-MS (ESI, pos. ion) m/z: 840.5 $[M+H]^+$.

Step 4: Synthesis of Compound **30-4**

Into a 25 mL single-necked flask were added compound **30-3** (256 mg, 0.30 mmol) and ACN (7 mL). The mixture was cooled to 0 °C and stirred for 5 min. TMSOTf (0.076 mL, 0.42 mmol) was added dropwise. After stirring at 0 °C for 0.5 h, the reaction was stopped and quenched with saturated aqueous sodium bicarbonate solution (20 mL). The mixture was extracted with DCM (20 mL × 2). The combined organic phases were dried over anhydrous sodium sulfate and concentrated. The residue was purified by column chromatography using DCM/MeOH (v/v = 30/1) as eluent to obtain 146 mg of a yellow solid compound **30-4** with a yield of 60%, LC-MS (ESI, pos. ion) m/z: 796.4 $[M+H]^+$.

Step 5: Synthesis of Compound **30**

Into a 25 mL single-necked flask were added compound **30-4** (146 mg, 0.18 mmol), cesium fluoride (410 mg, 2.70 mmol) and DMF (0.7 mL). The mixture was purged with nitrogen three times and stirred at room temperature for 15 h. The reaction was stopped, and water (20 mL) was added, precipitating a solid. Filtration afforded the crude solid. The crude product was purified by column chromatography using DCM/MeOH (v/v = 40/1) as eluent to obtain 103 mg of a yellow solid compound **30** with a yield of 87%, LC-MS (ESI, pos. ion) m/z: 640.3 $[M+H]^+$; HRMS (ESI): 640.2902 $[M+H]^+$; $^1H$ NMR (400 MHz, $CD_3OD$) δ 7.84 (dd, *J* = 8.9, 5.8 Hz, 1H), 7.37 - 7.27 (m, 2H), 7.26 - 7.16 (m, 1H), 5.30 (d, *J* = 53.9 Hz, 1H), 4.40 - 4.19 (m, 2H), 4.16 - 3.90 (m, 4H), 3.39 - 3.31 (m, 1H), 3.30 - 3.16 (m, 3H), 3.10 - 2.94 (m, 1H), 2.53 (q, *J* = 7.4 Hz, 2H), 2.42 - 2.07 (m, 3H), 2.06 - 1.79 (m, 7H), 1.68 - 1.51 (m, 4H), 0.93 - 0.79 (m, 3H). $^{19}F$ NMR (376 MHz, $CD_3OD$) δ -114.48 (1F), -141.02 (1F), -173.52 (1F).

**Example 31: Synthesis of Compound 31**

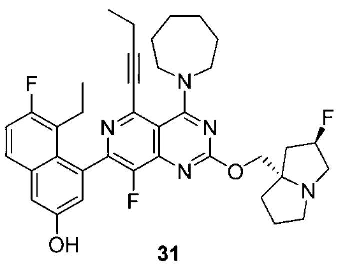

**31**

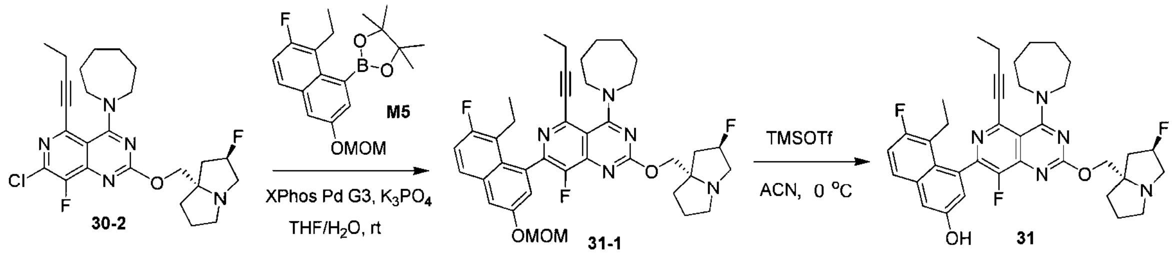


Step 1: Synthesis of Compound **31-1**

Into a 25 mL single-necked flask were added compound **30-2** (80 mg, 0.16 mmol, 1 equiv.), **M5** (116 mg, 0.32 mmol), $K_3PO_4 \cdot 7H_2O$ (165 mg, 0.48 mmol) and $THF/H_2O$ (v/v = 3 mL/0.6 mL). The mixture was purged with nitrogen three times, and Xphos Pd G3 (40 mg, 0.48 mmol) was added portionwise. The mixture was stirred at room temperature for 15 h. The reaction was quenched and diluted with DCM (20 mL). The mixture was washed with saturated sodium chloride solution (20 mL) and extracted with DCM (20 mL × 2). The combined organic phases were dried over anhydrous sodium sulfate and concentrated. The residue was purified by column chromatography using DCM/MeOH (v/v = 20/2) as eluent to obtain 123 mg of a red oil compound **31-1.** The yield was calculated as 100%. LC-MS (ESI, pos. ion) m/z: 689.0 $[M+H]^+$.

Step 4: Synthesis of Compound **31**

Into a 25 mL single-necked flask were added compound **31-1** (112 mg, 0.16 mmol) and ACN (4 mL). The mixture was cooled to 0 °C and stirred for 5 min. TMSOTf (0.043 mL, 0.24 mmol) was added dropwise. After stirring at 0 °C for 0.5 h, the reaction was stopped and quenched with saturated aqueous sodium bicarbonate solution (20 mL). The mixture was extracted with DCM (20 mL × 2). The combined organic phases were dried over anhydrous sodium sulfate and concentrated. The residue was purified by column chromatography using DCM/MeOH (v/v = 40/1) as eluent to obtain 58 mg of a yellow solid compound **31** with a yield of 55%, LC-MS (ESI, pos. ion) m/z: 644.4 $[M+H]^+$; HRMS (ESI): 644.3216 $[M+H]^+$; $^1H$ NMR (400 MHz, $CD_3OD$) δ 7.75 - 7.58 (m, 1H), 7.33 - 7.17 (m, 2H), 7.10 (s, 1H), 5.33 (d, *J* = 53.8 Hz, 1H), 4.43 - 4.22 (m, 2H), 4.21 - 3.91 (m, 4H), 3.44 - 3.33 (m, 1H), 3.29 - 3.21 (m, 2H), 3.16 - 2.97 (m, 1H), 2.54 (q, *J* = 7.3 Hz, 2H), 2.46 - 2.34 (m, 2H), 2.33 - 2.11 (m, 4H), 2.10 - 1.76 (m, 8H), 1.48 - 1.29 (m, 2H), 1.27 - 1.22 (m, 3H), 0.83 (t, *J* = 7.3 Hz, 3H). $^{19}F$ NMR (376 MHz, $CD_3OD$) δ -120.89 (1F), -140.68 (1F), -173.64 (1F).

**Example 32: Synthesis of Compound 32**

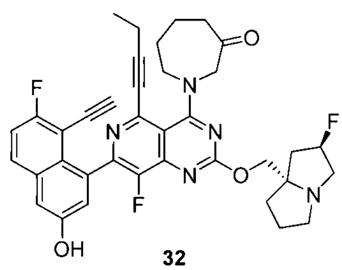

**32**

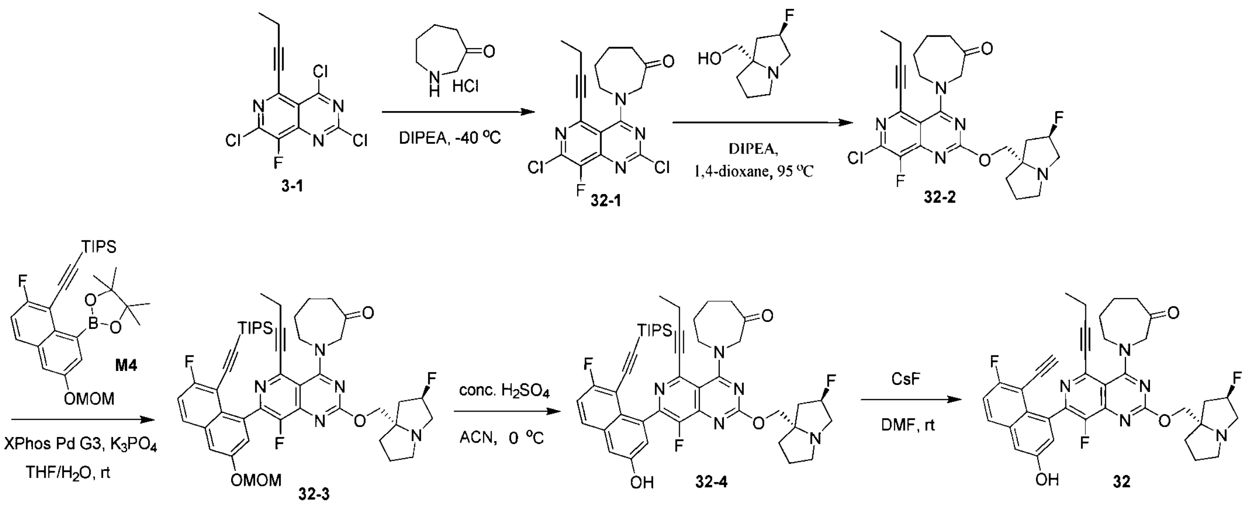

3-1 32-1 32-2 32-3 32-4 32

Step 1: Synthesis of Compound **32-1**

Into a 100 mL single-necked flask were added compound **3-1** (1.37 g, 4.50 mmol) and anhydrous dichloromethane (5 mL). The mixture was purged with nitrogen three times, cooled to -40 °C and stirred until compound **3-1** was dissolved. 3-Azacycloheptanone hydrochloride (0.34 g, 2.25 mmol) was added dropwise, followed by dropwise addition of DIPEA (2.97 mL, 18 mmol). The reaction mixture was stirred at -40 °C for 0.5 h. The reaction was quenched by addition of saturated aqueous ammonium chloride solution (30 mL) and the resulting mixture was extracted with DCM (20 mL × 2). The combined organic layers were dried over anhydrous sodium sulfate and concentrated. The residue was purified by column chromatography using PE/EA (v/v = 3/1) as the eluent to obtain 179 mg of a yellow solid compound **30-1** with a yield of 10%. LC-MS (ESI, pos. ion) m/z:381.1 $[M+H]^+$.

Step 2: Synthesis of Compound **32-2**

Into a 100 mL single-necked flask were added compound **32-1** (362 mg, 0.95 mmol), ((2*R*,7a*S*)-2-fluorohexahydro-1*H*-pyrrolizin-7a-yl)methanol (180 mg, 1.14 mmol), DIPEA (0.31 mL, 1.9 mmol) and anhydrous 1,4-dioxane (6 mL). The mixture was purged with nitrogen three times. The mixture was heated to 95 °C and stirred for 14.5 h. The reaction was stopped and cooled to room temperature. The reaction solution was concentrated, diluted with EA (20 mL), and washed with saturated ammonium chloride solution (20 mL). The phases were separated and the aqueous phase was extracted with EA (20 mL × 2). The combined organic phases were dried over anhydrous sodium sulfate and concentrated. The residue was purified by column chromatography using DCM/MeOH (v/v = 97/3) as eluent to obtain 288 mg of a yellow solid compound **32-2** with a yield of 60%. LC-MS (ESI, pos. ion) m/z: 504.3 $[M+H]^+$.

Step 3: Synthesis of Compound **32-3**

Into a 25 mL single-necked flask were added compound **32-2** (200 mg, 0.40 mmol, 1 equiv.), **M4** (420 mg, 0.80 mmol), $K_3PO_4 \cdot 7H_2O$ (405 mg, 1.2 mmol) and THF/$H_2O$ (v/v = 5 mL/1.6 mL). The mixture was purged with nitrogen three times, and Xphos Pd G3 (102 mg, 0.12 mmol) was added portionwise. The mixture was stirred at room temperature for 15 h. The reaction was quenched and diluted with DCM (20 mL). The mixture was washed with saturated sodium chloride solution (20 mL) and extracted with DCM (20 mL × 2). The combined organic phases were dried over anhydrous sodium sulfate and concentrated. The residue was purified by column chromatography using DCM/MeOH (v/v = 40/1) as eluent to obtain 339 mg of a red oil compound **32-3.** The yield was calculated as 100%. LC-MS (ESI, pos. ion) m/z: 854.4 $[M+H]^+$.

Step 4: Synthesis of Compound **32-4**

Into a 25 mL single-necked flask were added compound **32-3** (339 mg, 0.40 mmol) and ACN (8 mL). The mixture was cooled to 0 °C and stirred for 5 minutes, then concentrated sulfuric acid (0.033 mL, 0.60 mmol, 98% w/w) was added dropwise. After stirring at 0 °C for 15 min, the reaction was stopped and quenched with saturated aqueous sodium bicarbonate solution (20 mL). The mixture was extracted with DCM (20 mL × 2). The combined organic phases were dried over anhydrous sodium sulfate and concentrated. The residue was purified by column chromatography using DCM/MeOH (v/v = 40/1) as eluent to obtain 146 mg of a yellow solid compound **32-4** with a yield of 45%, LC-MS (ESI, pos. ion) m/z: 810.4 $[M+H]^+$.

Step 5: Synthesis of Compound **32**

Into a 25 mL single-necked flask were added compound **32-4** (146 mg, 0.18 mmol), cesium fluoride (410 mg, 2.70 mmol), and DMF (0.7 mL). The system was purged with nitrogen three times and stirred at room temperature for 12.5 h. The reaction was stopped, and water (20 mL) was added, precipitating a solid. Filtration afforded the crude solid. The crude product was purified by column chromatography using DCM/MeOH (v/v = 30/1) as eluent to obtain 42 mg of a yellow solid compound **32** with a yield of 36%, LC-MS (ESI, pos. ion) m/z: 654.3 $[M+H]^+$; HRMS (ESI): 654.2704 $[M+H]^+$; $^1H$ NMR (400 MHz, $CD_3OD$) δ 7.87 (dd, *J* = 9.1, 5.7 Hz, 1H), 7.40 - 7.28 (m, 2H), 7.24 (s, 1H), 5.44 (d, *J* = 51.7 Hz, 1H), 4.62 - 4.38 (m, 4H), 3.71 - 3.47 (m, 3H), 3.36 - 3.33 (m, 1H), 3.28 - 3.20 (m, 1H), 2.89 - 2.73 (m, 3H), 2.72 - 2.60 (m, 1H), 2.59 - 2.24 (m, 3H), 2.23 - 1.88 (m, 7H), 1.73 - 1.56 (m, 2H), 0.88 (t, *J*= 7.3 Hz, 3H). $^{19}F$ NMR (376 MHz, $CD_3OD$) δ -112.01 (1F), -145.55 (1F), -173.86 (1F).

**Example 33: Synthesis of Compound 33**

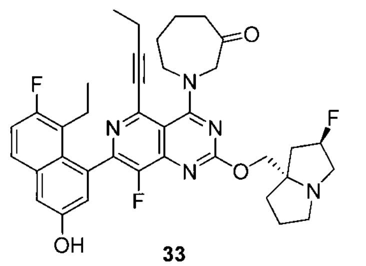

33

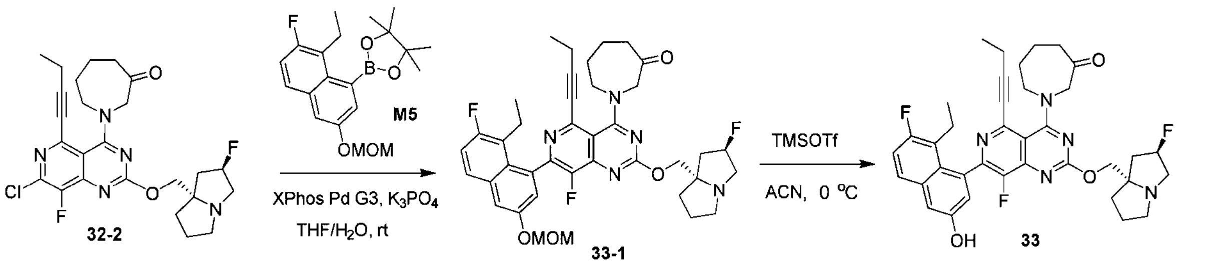


32-2 M5 33-1 33

Step 1: Synthesis of Compound **33-1**

Into a 25 mL single-necked flask were added compound **32-2** (80 mg, 0.16 mmol, 1 equiv.), **M5** (116 mg, 0.32 mmol), $K_3PO_4 \cdot 7H_2O$ (165 mg, 0.48 mmol) and THF/$H_2O$ (v/v = 3 mL/0.6 mL). The mixture was purged with nitrogen three times, and Xphos Pd G3 (40 mg, 0.048 mmol) was added portionwise. The mixture was stirred at room temperature for 15 h. The reaction was stopped and the mixture was diluted with DCM (20 mL). The resulting mixture was washed with saturated sodium chloride solution (20 mL) and extracted with DCM (20 mL × 2). The combined organic phases were dried over anhydrous sodium sulfate and concentrated. The residue was purified by column chromatography using DCM/MeOH (v/v = 97/3) as eluent to obtain 70 mg of a red oil compound **33-1** with a yield of 63%. LC-MS (ESI, pos. ion) m/z: 703.1 $[M+H]^+$.

Step 2: Synthesis of Compound **33**

Into a 25 mL single-necked flask were added compound **33-1** (66 mg, 0.094 mmol) and ACN (3 mL). The mixture was cooled to 0 °C and stirred for 5 min. TMSOTf (0.026 mL, 0.14 mmol) was added dropwise. After stirring at 0 °C for 30 min, the reaction was stopped and quenched with saturated aqueous sodium bicarbonate solution (20 mL). The mixture was extracted with DCM (20 mL × 2). The combined organic phases were dried over anhydrous sodium sulfate and concentrated. The residue was purified by column chromatography using DCM/MeOH (v/v = 15/1) as eluent to obtain 17 mg of a yellow solid compound **33** with a yield of 27%, LC-MS (ESI, pos. ion) m/z: 658.3 $[M+H]^+$; HRMS (ESI): 658.3020 $[M+H]^+$; $^1H$ NMR (400 MHz, $CD_3OD$) δ 7.68 (dd, *J* = 8.9, 5.9 Hz, 1H), 7.34 - 7.28 (m, 1H), 7.28 - 7.19 (m, 1H), 7.14 - 7.07 (m, 1H), 5.39 (d, *J* = 53.2 Hz, 1H), 4.57 - 4.37 (m, 3H), 4.35 - 4.25 (m, 2H), 4.23 - 4.09 (m, 1H), 3.76 - 3.35 (m, 4H), 3.25 - 3.08 (m, 1H), 2.91 - 2.64 (m, 1H), 2.56 (q, *J* = 7.5 Hz, 2H), 2.51 - 1.88 (m, 12H), 1.29 - 1.25 (m, 3H), 0.87 - 0.79 (m, 3H). $^{19}F$ NMR (376

MHz, $CD_3OD$) δ -112.79 (1F), -139.74 (1F), -173.65 (1F).

**Example 34: Synthesis of Compound 34**

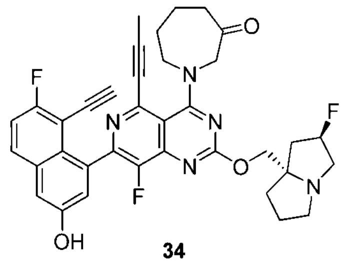

**34**

The synthesis of compound **34** was carried out by referring to the synthesis method of compound **32,** and compound **3-1** (structure and synthesis referred to step 1 of Example 1) was replaced with compound **3-1** to obtain yellow solid compound **34,** LC-MS (ESI, pos. ion) m/z: 640.3 $[M+H]^+$; $^1H$ NMR (400 MHz, $CD_3OD$) δ 7.85 (dd, *J* = 9.1, 5.7 Hz, 1H), 7.37 - 7.26 (m, 2H), 7.24 - 7.16 (m, 1H), 5.56 - 5.24 (m, 1H), 4.69 - 4.33 (m, 5H), 3.69 - 3.60 (m, 1H), 3.60 - 3.41 (m, 3H), 3.33 (s, 1H), 3.24 - 3.14 (m, 1H), 2.89 - 2.74 (m, 3H), 2.74 - 2.61 (m, 1H), 2.56 - 2.22 (m, 4H), 2.16 - 1.91 (m, 7H). $^{19}F$ NMR (376 MHz, $CD_3OD$) δ -111.98 (1F), -145.58 (1F), -173.83 (1F)。

**Example 35: Synthesis of Compound 35**

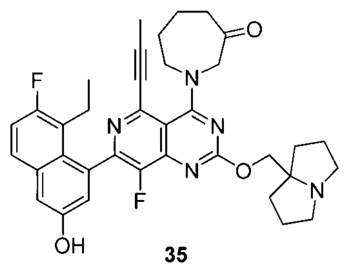

**35**

The synthesis of compound **35** was carried out by referring to the synthesis method of compound **32,** except that compound 1-1 (structure and synthesis see step 1 of Example 1) was used instead of **3-1,** (hexahydro-1*H*-pyrrolizin-7a-yl) methanol was used instead of ((2R, 7*aS*)-2-fluorohexahydro-1*H*-pyrrolizin-7*a*-yl)methanol, and **M5** was used instead of **M4** to obtain yellow solid compound **35,** LC-MS (ESI, pos. ion) m/z: 626.1 $[M+H]^+$; HRMS (ESI): 626.2943 $[M+H]^+$; $^1H$ NMR (400 MHz, $CD_3OD$) δ 7.69 (dd, *J= 9.0,* 5.7 Hz, 1H), 7.36 - 7.17 (m, 2H), 7.14 - 7.07 (m, 1H), 4.66 - 4.38 (m, 7H), 4.31 - 4.15 (m, 1H), 3.74 - 3.59 (m, 2H), 3.29 - 3.22 (m, 2H), 2.54 - 2.40 (m, 2H), 2.31 - 2.15 (m, 9H), 2.12 - 2.04 (m, 2H), 2.00 - 1.87 (m, 2H), 1.86 - 1.76 (m, 2H), 0.83 (t, *J* = 7.3 Hz, 3H). $^{19}F$ NMR (376 MHz, $CD_3OD$): δ -120.77 (1F), -139.84 (1F).

**Example 36: Synthesis of Compound 36**

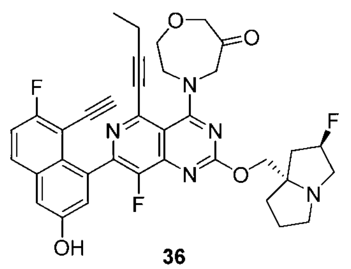

**36**

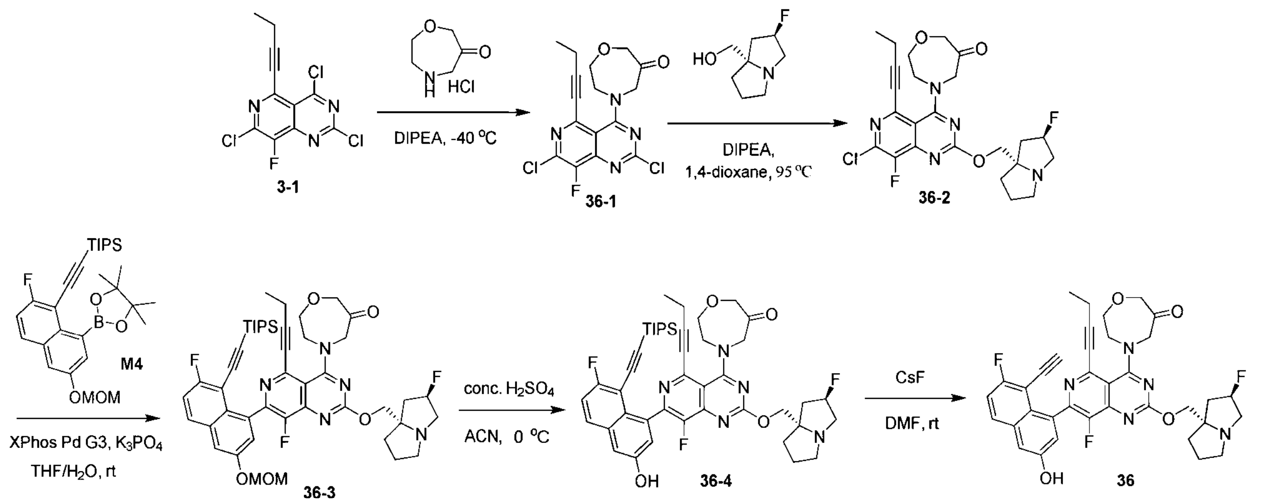


Step 1: Synthesis of Compound **36-1**

Into a 100 mL single-necked flask were added compound **3-1** (1.14 g, 3.74 mmol) and anhydrous dichloromethane (20 mL). The mixture was purged with nitrogen three times, cooled to -40 °C and stirred until the compound **3-1** was dissolved. 1,4-Oxazacycloheptane-6-one hydrochloride (0.55 g, 3.61 mmol) was added dropwise, followed by dropwise addition of DIPEA (2.99 mL, 18.05 mmol). The reaction mixture was stirred at -40 °C for 0.5 h. The reaction was quenched by addition of saturated aqueous ammonium chloride solution (50 mL), and the resulting mixture was extracted with DCM (30 mL × 2). The combined organic layers were dried over anhydrous sodium sulfate and concentrated. The residue was purified by column chromatography using PE/EA (v/v = 100/1-4/1) as the eluent to obtain 267 mg of a yellow solid compound **36-1** with a yield of 19%. LC-MS (ESI, pos. ion) m/z:383.3 $[M+H]^+$.

Step 2: Synthesis of Compound **36-2**

Into a 100 mL single-necked flask were added compound **36-1** (540 mg, 1.41 mmol), ((2*R*,7a*S*)-2-fluorohexahydro-1*H*-pyrrolizin-7a-yl)methanol (561 mg, 3.52 mmol), DIPEA (1.17 mL, 7.05 mmol) and anhydrous 1,4-dioxane (10 mL). The mixture was purged with nitrogen three times. The mixture was heated to 90 °C and stirred for 12 h. The reaction was stopped and cooled to room temperature. The reaction solution was concentrated, diluted with EA (20 mL), and washed with saturated ammonium chloride solution (20 mL). The phases were separated and the aqueous phase was extracted with EA (20 mL × 2). The combined organic phases were dried over anhydrous sodium sulfate and concentrated. The residue was purified by column chromatography using DCM/MeOH (v/v = 100/1-30/1) as eluent to obtain 450 mg of a yellow solid compound **36-2** with a yield of 63%. LC-MS (ESI, pos. ion) m/z: 506.1 $[M+H]^+$.

Step 3: Synthesis of Compound **36-3**

Into a 25 mL single-necked flask were added compound **36-2** (250 mg, 0.49 mmol, 1 equiv.), **M4** (502 mg, 0.98 mmol), $K_3PO_4 \cdot 7H_2O$ (830 mg, 2.45 mmol) and $THF/H_2O$ (v/v = 6 mL/0.5 mL). The mixture was purged with nitrogen three times, and Xphos Pd G3 (104 mg, 0.12 mmol) was added portionwise. The mixture was stirred at 30 °C for 6 h. The reaction was stopped and diluted with DCM (50 mL). The mixture was washed with saturated sodium chloride solution (20 mL × 2). The combined organic phases were dried over anhydrous sodium sulfate and concentrated. The residue was purified by column chromatography using DCM/MeOH (v/v = 100/1-30/1) as eluent to obtain 235 mg of a red oil compound **36-3** with a yield of 56%. LC-MS (ESI, pos. ion) m/z: 856.3 $[M+H]^+$.

Step 4: Synthesis of Compound **36-4**

Into a 25 mL single-necked flask were added compound **36-3** (300 mg, 0.35 mmol) and ACN (10 mL). The mixture was cooled to 0 °C and stirred for 5 minutes. Concentrated sulfuric acid (0.039 mL, 0.70 mmol, 98% purity) was added dropwise. After stirring at 0 °C for 30 minutes, the reaction was stopped and quenched with saturated aqueous sodium bicarbonate solution (20 mL). The mixture was extracted with DCM (30 mL × 2). The combined organic phases were dried over anhydrous sodium sulfate and concentrated to obtain 110 mg of a brown solid compound **36-4** with a yield of 40%, LC-MS (ESI, pos. ion) m/z: 812.3 $[M+H]^+$.

Step 5: Synthesis of Compound **36**

Into a 25 mL single-necked flask were added compound **36-4** (120 mg, 0.15 mmol), cesium fluoride (228 mg, 1.5 mmol) and DMF (2 mL). The mixture was purged with nitrogen three times and stirred at room temperature for 4 h. The reaction was stopped, and water (8 mL) was added, precipitating a solid. Filtration afforded the crude solid. The crude product was purified by column chromatography using DCM/MeOH (v/v = 12/1) as eluent to obtain 20 mg of a brown solid compound **36** with a yield of 21%, LC-MS (ESI, pos. ion) m/z: 656.2 [M+H]$^+$; HRMS (ESI): 656.2460 [M+H]$^+$; $^1$H NMR (400 MHz, $CD_3OD$) δ 7.86 (dd, *J* = 9.1, 5.8 Hz, 1H), 7.38 - 7.29 (m, 2H), 7.25 - 7.19 (m, 1H), 5.42 (d, *J* = 52.2 Hz, 1H), 4.83 - 4.68 (m, 2H), 4.57 - 4.40 (m, 3H), 4.12 - 4.02 (m, 2H), 3.70 - 3.61 (m, 1H), 3.59 - 3.42 (m, 3H), 3.27 - 3.17 (m, 1H), 2.90 - 2.72 (m, 2H), 2.60 - 2.21 (m, 4H), 2.20 - 2.10 (m, 2H), 2.08 - 1.97 (m, 1H), 1.74 - 1.60 (m, 2H), 0.88 (t, *J* = 7.4 Hz, 3H). $^{19}$F NMR (376 MHz, $CD_3OD$): δ -111.98 (1F), -144.65 (1F), -173.81 (1F).

**Example 37: Synthesis of Compound 37**

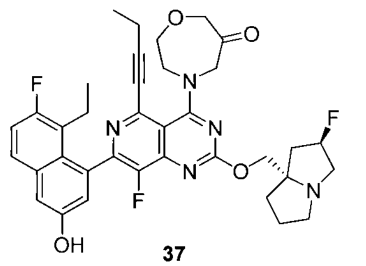

37

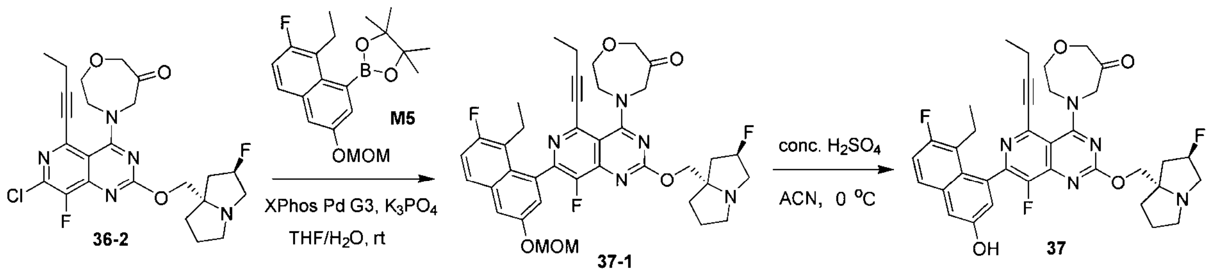


36-2 37-1 37

Step 1: Synthesis of Compound **37-1**

Into a 25 mL single-necked flask were added compound **36-2** (130 mg, 0.26 mmol, 1 equiv.), **M5** (190 mg, 0.52 mmol), $K_3PO_4 \cdot 7H_2O$ (440 mg, 1.3 mmol) and $THF/H_2O$ (v/v = 6 mL/0.5 mL). The mixture was purged with nitrogen three times, and Xphos Pd G3 (55 mg, 0.065 mmol) was added portionwise. The mixture was stirred at 30 °C for 6 h. The reaction was stopped and diluted with DCM (50 mL). The mixture was washed with saturated sodium chloride solution (20 mL × 2). The combined organic phases were dried over anhydrous sodium sulfate and concentrated. The residue was purified by column chromatography using DCM/MeOH (v/v = 100/1-30/1) as eluent to obtain 80 mg of a yellow soild compound **37-1** with a yield of 44%. LC-MS (ESI, pos. ion) m/z: 704.3 [M+H]$^+$.

Step 2: Synthesis of Compound **37**

Into a 25 mL single-necked flask were added compound **37-1** (80 mg, 0.11 mmol) and ACN (5 mL). The mixture was cooled to 0 °C and stirred for 5 minutes. Concentrated sulfuric acid (0.012 mL, 0.22 mmol, 98% w/w) was added dropwise. After stirring at 0 °C for 30 minutes, the reaction was stopped and quenched with saturated aqueous sodium bicarbonate solution (20 mL). The mixture was extracted with DCM (30 mL × 2). The combined organic phases were dried over anhydrous sodium sulfate and concentrated. The residue was purified by column chromatography using DCM/MeOH (v/v = 13/1) as eluent to obtain 13 mg of a yellow solid compound **37** with a yield of 20%. LC-MS (ESI, pos. ion) m/z: 660.2 [M+H]$^+$; HRMS (ESI): 660.2792 [M+H]$^+$; $^1$H NMR (400 MHz, $CD_3OD$) δ 7.68 (dd, *J* = 9.0, 5.9 Hz, 1H), 7.33 - 7.19 (m, 2H), 7.07 - 7.03 (m, 1H), 5.42 (d, *J* = 53.4 Hz, 1H), 5.02 - 4.92 (m, 1H), 4.83 - 4.67 (m, 2H), 4.55 - 4.41 (m, 2H), 4.11 - 4.04 (m, 2H), 3.68 - 3.60 (m, 1H), 3.58 - 3.42 (m, 3H), 3.25 - 3.18 (m, 1H), 2.87 - 2.76 (m, 2H), 2.58 - 2.35 (m, 3H), 2.34 - 2.21 (m, 2H), 2.20 - 2.10 (m, 2H), 2.09 - 1.97 (m, 1H), 1.69 - 1.59 (m, 2H), 0.87 (t, *J*= 7.4 Hz, 3H), 0.78 (t, *J*= 7.4 Hz, 3H). $^{19}$F NMR (376 MHz,

$CD_3OD$) δ -121.50 (1F), -143.77(1F), -173.81 (1F).

**Example 38: Synthesis of Compound 38**

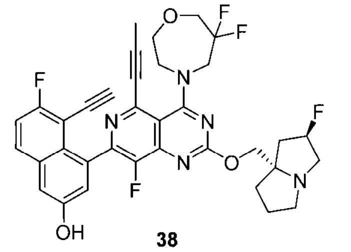

38

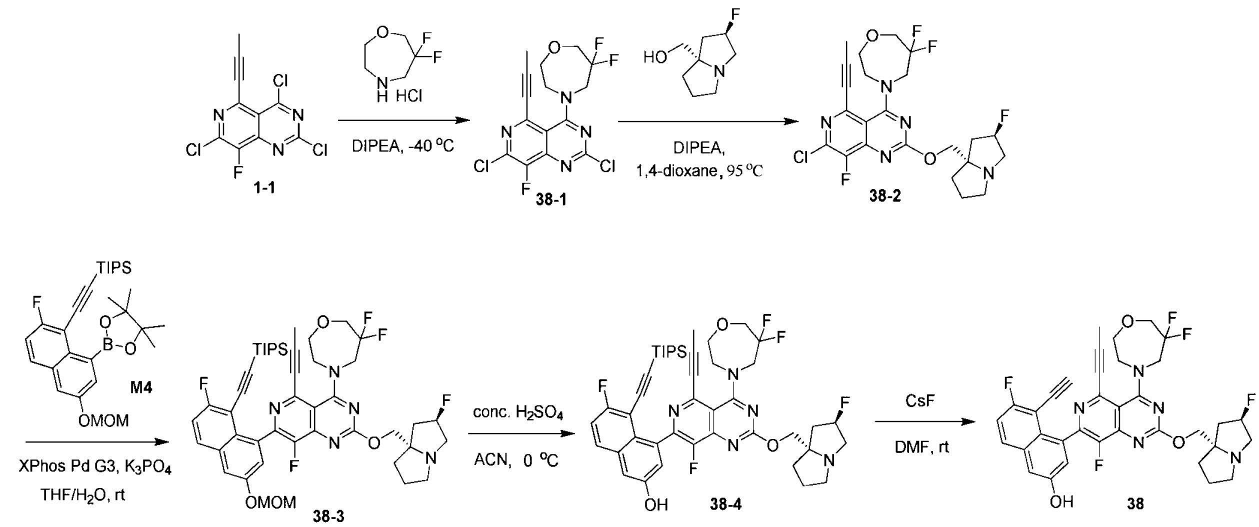


Step 1: Synthesis of Compound 38-1

Into a 100 mL single-necked flask were added compound **1-1** (1.15 g, 3.96 mmol) and anhydrous dichloromethane (10 mL). The mixture was purged with nitrogen three times and stirred until the compound **1-1** was dissolved. 6,6-difluoro-1,4-oxazacycloheptane hydrochloride (0.69 g, 3.96 mmol, Shanghai Hanhong Technology) was added dropwise, followed by dropwise addition of DIPEA (3.27 mL, 19.8 mmol). The reaction mixture was stirred at room temperature for 15 min. The reaction was stopped and quenched by addition of saturated aqueous ammonium chloride solution (30 mL) and extracted with DCM (30 mL × 2). The combined organic phases were dried over anhydrous sodium sulfate and concentrated. The residue was purified by column chromatography using PE/EA (v/v = 100/1-4/1) as the eluent to obtain 244 mg of a yellow solid compound **38-1** with a yield of 16%. LC-MS (ESI, pos. ion) m/z:353.1 $[M+H]^+$.

Step 2-Step 5: Synthesis of Compound **38**

The synthesis of compond 38 was carried out by referring to steps 2-5 in the synthesis procedure of compound **29** to obtain 30 mg of yellow solid compound **38,** LC-MS (ESI, pos. ion) m/z: 664.3 $[M+H]^+$; HRMS (ESI): 664.2353 $[M+H]^+$; $^1H$ NMR (400 MHz, $CD_3OD$) δ 7.87 (dd, $J$ = 9.0, 5.8 Hz, 1H), 7.39 - 7.29 (m, 2H), 7.27 - 7.23 (m, 1H), 5.31 (d, $J$ = 54.4 Hz, 1H), 4.75 - 4.64 (m, 1H), 4.61 - 4.44 (m, 3H), 4.39 - 4.17 (m, 4H), 4.15 - 3.81 (m, 4H), 3.23 - 3.12 (m, 2H), 3.09 - 2.96 (m, 1H), 2.41 - 2.24 (m, 2H), 2.21 (s, 3H), 2.16 - 2.08 (m, 1H), 2.06 - 1.83 (m, 3H). $^{19}F$ NMR (376 MHz, $CD_3OD$) δ -101.93 (2F), -111.47 (s, 1F), -139.64 (d, $J$ = 4.4 Hz, 1F), -173.66 (1F).

**Example 39: Synthesis of Compound 39**

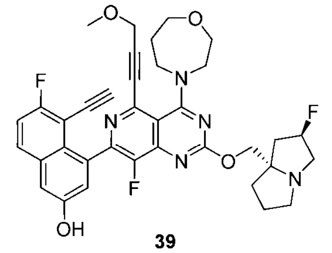

39

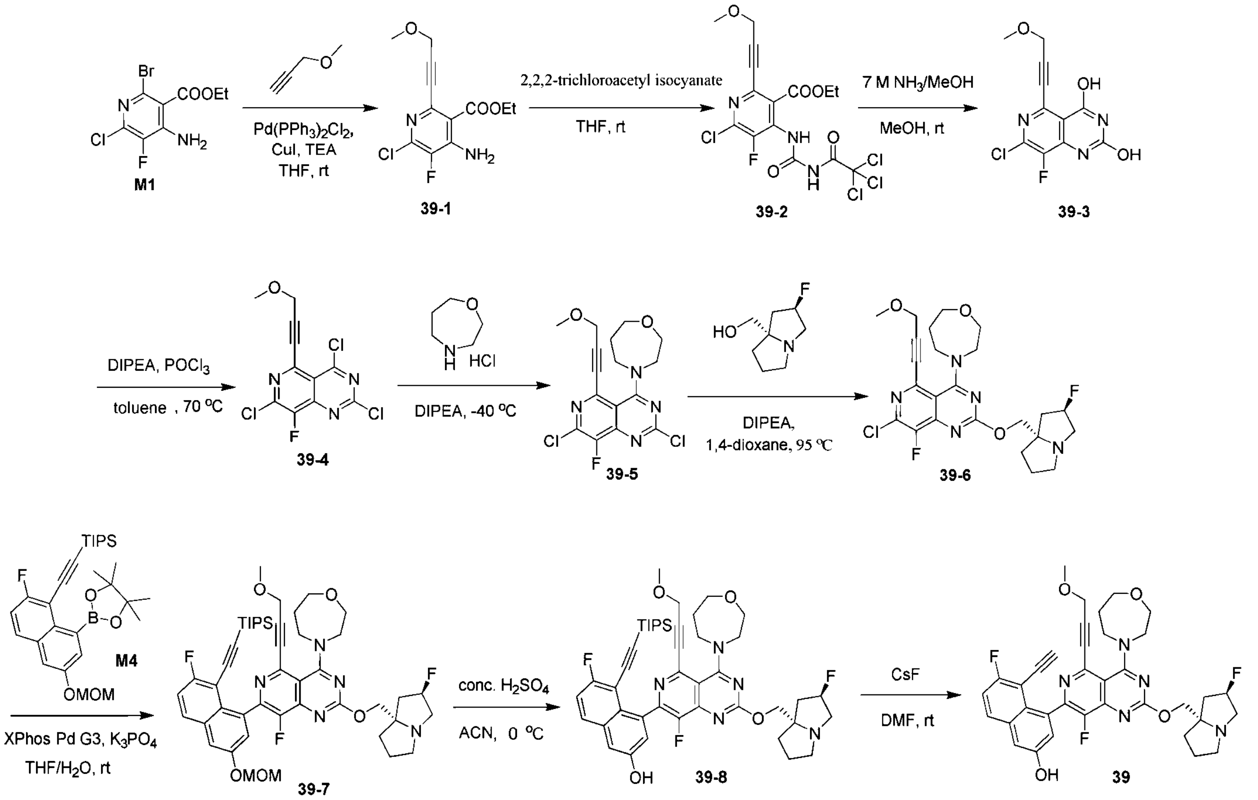


Step 1: Synthesis of Compound **39-1**

Into a 100 mL single-necked flask were added compound **M1** (5 g, 16.81 mmol), methyl propargyl ether (1.56 mL, 18.49 mmol), $Pd(PPh_3)_2Cl_2$ (1.77 g, 2.52 mmol), CuI(480 mg, 2.52 mmol), TEA (5.1 g, 50.43 mmol) and anhydrous THF (50 mL). The mixture was purged with nitrogen three times. The mixture was stirred at room temperature for 5 h. The reaction was stopped and filtered through a celite pad. The filtrate was diluted with dichloromethane (100 mL), washed sequentially with saturated ammonium chloride solution (20 mL × 3) and saturated brine (20 mL × 3), dried over anhydrous sodium sulfate, filtered, and concentrated. The residue was purified by column chromatography using PE/DCM (v/v = 4/1-1/1) as eluent to obtain 3.09 g of a yellow oil compound **39-1** with a yield 64%. LC-MS (ESI, pos. ion) m/z: 287.2 $[M+H]^+$

Step 2: Synthesis of Compound **39-2**

Into a 250 mL single-necked flask were added compound **39-1** (3.09 g, 10.78 mmol) and anhydrous THF (30 mL). The mixture was stirred to dissolve. Then, 2,2,2-trichloroacetyl isocyanate (1.54 mL, 12.94 mmol) was added. The resulting mixture was stirred at room temperature for 1 h. The reaction was stopped and concentrated to obtain a brown solid. The residue was used directly in the next step. The yield was calculated as 100%.

Step 3: Synthesis of Compound **39-3**

Into the compound **39-2** obtained in the previous step was added methanol (20 mL), and the mixture was stirred to dissolve compound **39-2**. Then, a solution of ammonia in methanol (7.69 mL, 53.85 mmol, 7 M) was added. The mixture

was stirred at room temperature for 0.5 h. The reaction was stopped and filtered to obtain a solid. The solid was slurried with 50 mL of MTBE for 10 minutes and filtered to obtain 3.03 g of a yellow solid compound **39-3** with a yield 99%. LC-MS (ESI, pos. ion) m/z: 284.2 $[M+H]^+$.

Step 4: Synthesis of Compound **39-4**

Into a 100 mL single-necked flask were added compound **39-3** (1.20 g, 4.23 mmol) and anhydrous toluene (20 mL). Then, phosphoryl chloride (1.16 mL, 12.69 mmol) and DIPEA (3.50 mL, 21.15 mmol) were added. The mixture was heated to 70 °C and stirred for 1 h under $N_2$ protection. The reaction was stopped and the mixture was cooled. The mixture was concentrated and used directly in the next step. The yield was calculated as 100%.

Step 5: Synthesis of Compound **39-5**

Into the compound **39-4** obtained in the previous step was added anhydrous dichloromethane (15 mL). The mixture was purged with nitrogen three times and stirred to dissolve. The mixture was cooled to -40 °C and stirred for 5 minutes. DIPEA (3.5 mL, 21.20 mmol) and homomorpholine (0.47 mL, 4.24 mmol)were added in turn. After stirring at -40 °C for 0.5 h, the reaction was stopped and the mixture was diluted with DCM (50 mL). The organic phase was washed with saturated ammonium chloride solution (20 mL × 2). The organic phase was separated, dried over anhydrous sodium sulfate, and concentrated. The residue was purified by column chromatography using PE/EA (v/v = 25/1-20/1) as eluent to obtain 430 mg of a yellow solid compound **39-5** with a yield of 26.3%. LC-MS (ESI, pos. ion) m/z: 385.1 $[M+H]^+$.

Step 6: Synthesis of Compound **39-6**

Into a 50 mL single-necked flask were added compound **39-5** (430 mg, 1.12 mmol), ((2*R*,7a*S*)-2-fluorohexahydro-1*H*-pyrrolizin-7*a*-yl)methanol (360 mg, 2.24 mmol), DIPEA (0.56 mL, 3.36 mmol) and anhydrous 1,4-dioxane (8 mL). The mixture was purged with nitrogen three times. The mixture was heated to 95 °C and stirred for 15 h. The reaction was stopped and cooled to room temperature. The reaction solution was concentrated, diluted with EA (30 mL), and washed with saturated ammonium chloride solution (20 mL × 2). The phases were separated. The organic phases were dried over anhydrous sodium sulfate and concentrated. The residue was purified by column chromatography using DCM/MeOH (v/v = 100/1-50/1) as eluent to obtain 146 mg of a yellow solid compound **39-6** with a yield of 26%. LC-MS (ESI, pos. ion) m/z: 508.2 $[M+H]^+$.

Step 7: Synthesis of Compound **39-7**

Into a 50 mL single-necked flask were added compound **39-6** (146 mg, 0.29 mmol), **M4** (300 mg, 0.58 mmol), Xphos Pd G3 (49 mg, 0.058 mmol), $K_3PO_4 \cdot 7H_2O$ (290 mg, 0.87 mmol) and THF/$H_2O$ (v/v = 4.5 mL/1.6 mL). The mixture was purged with nitrogen three times. The mixture was stirred at room temperature for 2 h. and Xphos Pd G3 was added several times until the reaction was complete. The reaction was stopped and saturated ammonium chloride solution (20 mL) was added into the mixture. The resulting mixture was extracted with DCM (20 mL × 2). The combined organic phases were dried over anhydrous sodium sulfate and concentrated. The residue was purified by column chromatography using DCM/MeOH (v/v = 25/1) as eluent to obtain 235 mg of a black oil compound **39-7** with a yield of 95%. LC-MS (ESI, pos. ion) m/z: 858.4 $[M+H]^+$.

Step 8: Synthesis of Compound **39-8**

Into a 50 mL single-necked flask were added compound **39-7** (235 mg, 0.27 mmol) and anhydrous acetonitrile (4 mL). The mixture was cooled to 0 °C and stirred for 5 minutes, then concentrated sulfuric acid (0.073 mL, 1.35 mmol, 98% purity) was added dropwise. After stirring for 30 minutes, the reaction was stopped and quenched with saturated aqueous sodium bicarbonate solution (20 mL). The resulting mixture was extracted with DCM (15 mL × 2). The combined organic phases were dried over anhydrous sodium sulfate and concentrated. The residue was purified by column chromatography using DCM/MeOH (v/v = 93/7) as eluent to obtain 77 mg of a yellow oil compound **39-8** with a yield of 35%, LC-MS (ESI, pos. ion) m/z: 814.4 $[M+H]^+$.

Step 9: Synthesis of Compound **39**

Into a 25 mL single-necked flask were added **39-8** (99 mg, 0.12 mmol), cesium fluoride (160 mg, 1.08 mmol) and DMF (0.5 mL). The mixture was purged with nitrogen three times and stirred at room temperature for 12.5 h. The reaction was stopped, and water (10 mL) was added, precipitating a solid. Filtration afforded the crude solid. The crude product was

purified by column chromatography using DCM/MeOH (v/v = 92/8) as eluent to obtain 37 mg of a yellow solid compound **39** with a yield of 44%. LC-MS (ESI, pos. ion) m/z: 658.3 $[M+H]^+$; HRMS (ESI): 658.2662 $[M+H]^+$; $^1H$ NMR (400 MHz, $CD_3OD$) δ 7.92 - 7.81 (m, 1H), 7.39 - 7.28 (m, 2H), 7.27 - 7.22 (m, 1H), 5.34 (d, *J* = 54.4 Hz, 1H), 4.43 (s, 2H), 4.40 - 4.19 (m, 4H), 4.19 - 4.05 (m, 2H), 3.98 - 3.88 (m, 2H), 3.86 - 3.76 (m, 2H), 3.44 (s, 3H), 3.42 - 3.37 (m, 1H), 3.36 - 3.32 (m, 3H), 3.14 - 3.03 (m, 1H), 2.45 - 2.22 (m, 2H), 2.20 - 1.88 (m, 6H). $^{19}F$ NMR (376 MHz, $CD_3OD$): δ -111.54 (1F), -139.56 (1F), -173.71 (1F).

**Example 40: Synthesis of Compound 40**

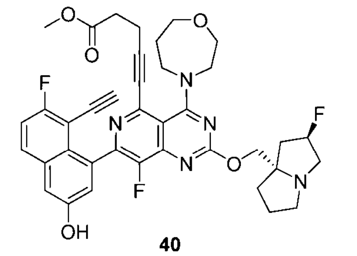

**40**

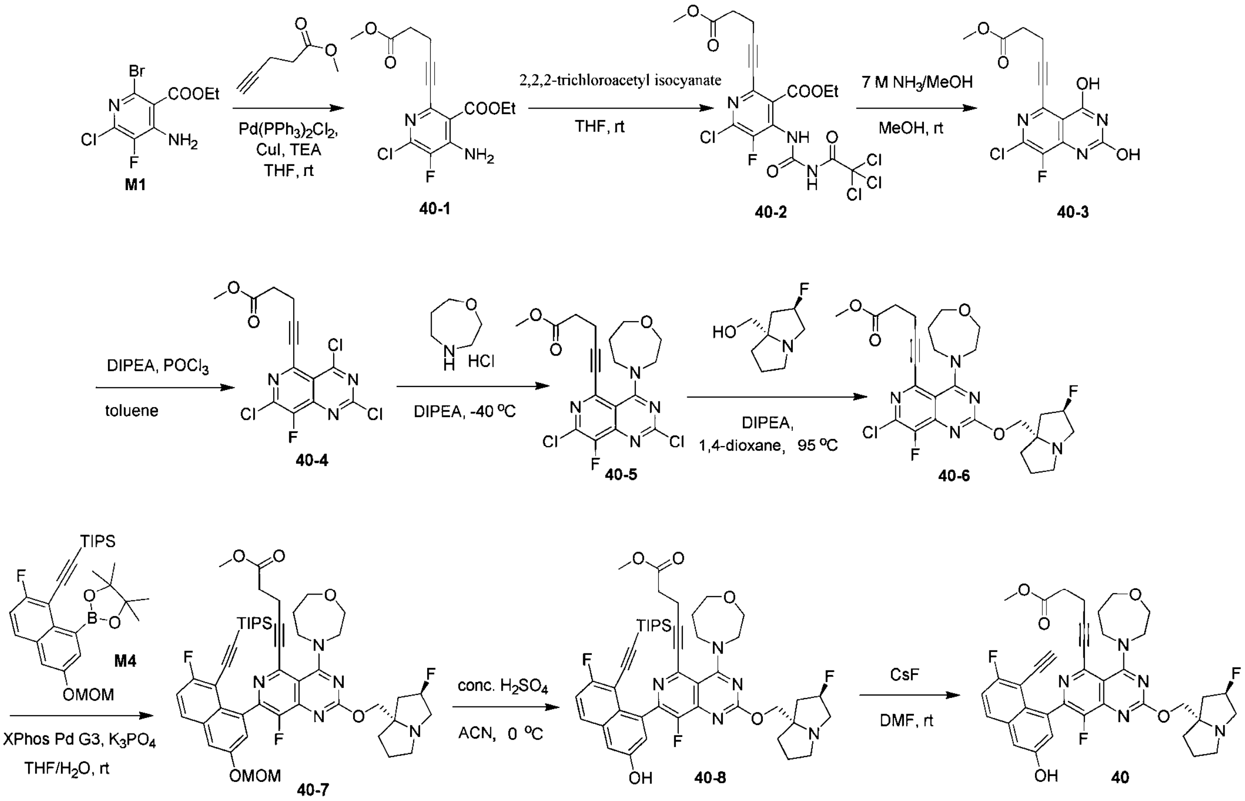


Step 1: Synthesis of Compound **40-1**

Into a 100 mL single-necked flask were added compound **M1** (1.8 g, 6.05 mmol), methyl 4-pentynoate (0.83 mL, 7.26 mmol), $Pd(PPh_3)_2Cl_2$ (0.85 g, 1.21 mmol), CuI(230 mg, 1.21 mmol), TEA (2.52 mL, 18.15 mmol) and anhydrous THF (25 mL). The mixture was purged with nitrogen three times. The mixture was stirred at room temperature for 2.5 h. The reaction was stopped and filtered through a celite pad. The filtrate was diluted with dichloromethane (100 mL), washed with saturated ammonium chloride solution (20 mL × 3) and saturated brine (20 mL × 3) in turn, dried over anhydrous sodium sulfate, filtered, and concentrated. The residue was purified by column chromatography using PE/DCM (v/v = 4/1) as eluent to obtain 1.43 g of a yellow solid compound **40-1** with a yield 72%. LC-MS (ESI, pos. ion) m/z: 329.1 $[M+H]^+$.

Step 2: Synthesis of Compound **40-2**

Into a 100 mL single-necked flask were added compound **40-1** (1.43 g, 4.37 mmol) and anhydrous THF (15 mL). The mixture was stirred to dissolve compound **40-1**. Then, 2,2,2-trichloroacetyl isocyanate (0.62 mL, 5.24 mmol) was added. The resulting mixture was stirred at room temperature for 0.5 h. The reaction was stopped and concentrated to obtain a dark solid. The residue was used directly in the next step.

Step 3: Synthesis of Compound **40-3**

Into the compound **40-2** obtained in the previous step was added methanol (20 mL), and the mixture was stirred to dissolve compound **40-2.** Then, a solution of ammonia in methanol (3.12 mL, 21.85 mmol, 7 M) was added. The mixture was stirred at room temperature for 0.5 h. The reaction was stopped and the reaction solution was concentrated to give the residue as a solid. The solid was slurried with 15 mL of MTBE for 10 minutes and filtered to obtain 1.13 g of a yellow solid compound **40-3.** The two-step yield was 79%. LC-MS (ESI, pos. ion) m/z: 324.1 $[M+H]^+$.

Step 4: Synthesis of Compound **40-4**

Into a 100 mL single-necked flask were added compound **40-3** (1.09 g, 3.35 mmol) and anhydrous toluene (15 mL). Then, phosphoryl chloride (1.83 mL, 20.1 mmol) and DIPEA (1.94 mL, 11.72 mmol) were added. The mixture was heated to 85 °C and stirred for 3 h under $N_2$. The reaction was stopped and cooled. The mixture was concentrated to obtain a black oil **40-4,** which was used directly in the next step. The yield was calculated as 100%.

Step 5: Synthesis of Compound **40-5**

Into the compound **40-4** obtained in the previous step was added anhydrous dichloromethane (10 mL). The mixture was purged with nitrogen three times and stirred to dissolve. The mixture was cooled to -40 °C and stirred for 5 minutes. DIPEA (2.76 mL, 16.7 mmol) and homomorpholine (0.34 g, 3.34 mmol) were added sequentially. After stirring at -40 °C for 0.5 h, the reaction was stopped and the mixture was diluted with DCM (50 mL). The organic phase was washed with saturated ammonium chloride solution (20 mL × 2). The organic phase was separated, dried over anhydrous sodium sulfate, and concentrated. The residue was purified by column chromatography using PE/EA (v/v = 10/1-4/1) as eluent to obtain 362 mg of a yellow solid compound **40-5** with a yield of 25%. LC-MS (ESI, pos. ion) m/z: 427.1 $[M+H]^+$.

Step 6: Synthesis of Compound **40-6**

Into a 100 mL single-necked flask were added compound **40-5** (362 mg, 0.85 mmol), ((2*R*,7a*S*)-2-fluorohexahydro-1*H*-pyrrolizin-7*a*-yl)methanol (410 mg, 2.55 mmol), DIPEA (0.42 mL, 2.55 mmol) and anhydrous 1,4-dioxane (8 mL). The mixture was purged with nitrogen three times. The mixture was heated to 95 °C and stirred for 15 h. The reaction was stopped and the mixture was cooled to room temperature. The reaction solution was concentrated, diluted with EA (30 mL), and washed with saturated ammonium chloride solution (20 mL × 2). The phases were separated. The organic phases were dried over anhydrous sodium sulfate and concentrated. The residue was purified by column chromatography using DCM/MeOH (v/v = 100/1-30/1) as eluent to obtain 303 mg of a yellow oil compound **40-6** with a yield of 65%. LC-MS (ESI, pos. ion) m/z: 550.2 $[M+H]^+$.

Step 7: Synthesis of Compound **40-7**

Into a 50 mL single-necked flask were added compound **40-6** (303 mg, 0.55 mmol), **M4** (420 mg, 0.83 mmol), Xphos Pd G3 (93 mg, 0.11 mmol), $K_3PO_4 \cdot 7H_2O$ (560 mg, 1.65 mmol) and THF/$H_2O$ (v/v = 6 mL/2 mL). The mixture was purged with nitrogen three times. The mixture was stirred at room temperature for 2 h. and Xphos Pd G3 was added several times until the reaction was complete. The reaction was stopped and added saturated ammonium chloride solution (20 mL) was added to the mixture. The resulting mixture was extracted with DCM (20 mL × 2). The combined organic phases were dried over anhydrous sodium sulfate and concentrated. The residue was purified by column chromatography using DCM/MeOH (v/v = 50/1) as eluent to obtain 280 mg of a yellow foamy solid compound **40-7** with a yield of 56%. LC-MS (ESI, pos. ion) m/z: 900.4 $[M+H]^+$.

Step 8: Synthesis of Compound **40-8**

Into a 50 mL single-necked flask were added compound **40-7** (280 mg, 0.31 mmol) and anhydrous acetonitrile (6 mL). The mixture was cooled to 0 °C and stirred for 8 minutes. Concentrated sulfuric acid (0.084 mL, 1.55 mmol, 98%

purity) was added dropwise. After stirring for 30 minutes, the reaction was stopped and quenched with saturated aqueous sodium bicarbonate solution (20 mL). The resulting mixture was extracted with DCM (20 mL × 2). The combined organic phases were dried over anhydrous sodium sulfate and concentrated to obtain a brown solid compound **40-8.** The yield was calculated as 100%, LC-MS (ESI, pos. ion) m/z: 856.4 $[M+H]^+$.

Step 9: Synthesis of Compound **40**

Into a 25 mL single-necked flask were added **40-8** (266 mg, 0.31 mmol), cesium fluoride (380 mg, 2.48 mmol) and DMF (0.8 mL). The mixture was purged with nitrogen three times and stirred at room temperature for 17.5 h. The reaction was stopped, and water (10 mL) was added, precipitating a solid. Filtration afforded the crude solid. The crude product was purified by column chromatography using DCM/MeOH (v/v = 92/8) as eluent to obtain 138 mg of a yellow solid compound **40** with a yield of 63%. LC-MS (ESI, pos. ion) m/z: 700.3 $[M+H]^+$; HRMS (ESI): 700.2756 $[M+H]^+$; $^1H$ NMR (400 MHz, $CD_3OD$) δ 7.87 (dd, *J* = 9.1, 5.7 Hz, 1H), 7.39 - 7.30 (m, 2H), 7.26 - 7.22 (m, 1H), 5.34 (d, *J* = 53.0 Hz, 1H), 4.66 - 4.59 (m, 4H), 4.39 - 4.21 (m, 4H), 4.19 - 4.03 (m, 2H), 3.98 - 3.90 (m, 2H), 3.85 - 3.78 (m, 2H), 3.72 (s, 3H), 3.42 - 3.38 (m, 1H), 2.85 (t, *J* = 6.9 Hz, 2H), 2.71 (t, *J* = 6.7 Hz, 2H), 2.42 - 2.23 (m, 2H), 2.19 - 1.92 (m, 6H). $^{19}F$ NMR (376 MHz, $CD_3OD$) δ -111.58 (1F), -140.50 (1F), -173.64 (1F).

**Examples 41-86 (i.e., compounds 41-86) were prepared by referring to the method of Example 1, and Examples 87-88 (i.e., compounds 87-88) were prepared by referring to the synthesis method of Example 13.** It should be noted that in the synthesis process of Examples 41-88, while referring to the synthesis method of the target compounds, it also includes the process of appropriate or necessary adjustment of the synthesis method by technicians based on conventional technical knowledge in the field. The structures and characterization data of Examples 41-88 are shown in Table A below.

Table A

| **Example Compound and Characterization Data** | **Example Compound and Characterization Data** |
|---|---|
| **Example 41**<br>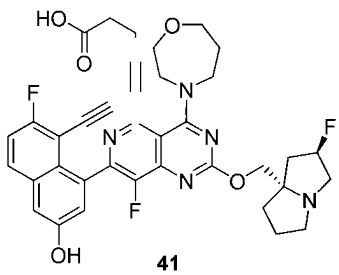<br>**41**<br>LC-MS (ESI, pos. ion) m/z:686.5 $[M+H]^+$. | **Example 42**<br>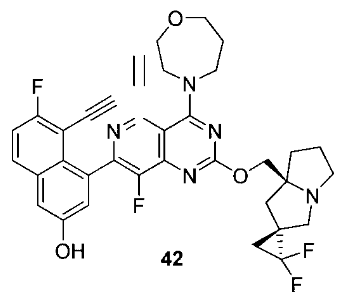<br>**42**<br>LC-MS (ESI, pos. ion) m/z:672.0 $[M+H]^+$. |
| **Example 43**<br>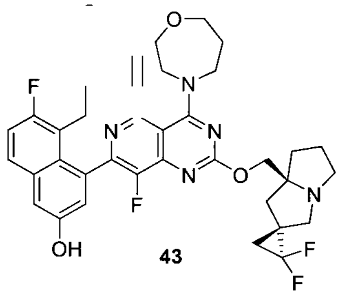<br>**43**<br>LC-MS (ESI, pos. ion) m/z:676.2 $[M+H]^+$. | **Example 44**<br>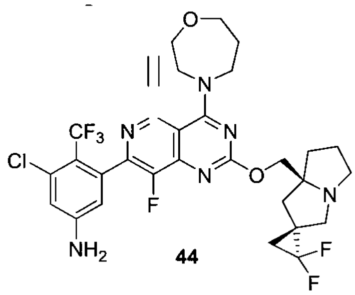<br>**44**<br>LC-MS (ESI, pos. ion) m/z:681.2 $[M+H]^+$. |
| **Example 45** | Example 46 |

(continued)

| Table A | |
|---|---|
| **Example Compound and Characterization Data** | **Example Compound and Characterization Data** |
| Example 45<br>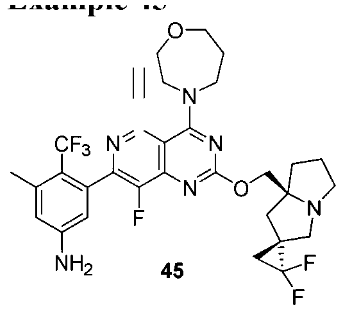<br>**45**<br>LC-MS (ESI, pos. ion) m/z:661.4 [M+H]+. | 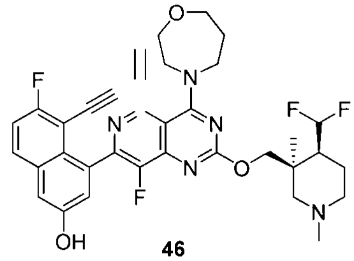<br>**46**<br>LC-MS (ESI, pos. ion) m/z:662.1 [M+H]+. |
| **Example 47**<br>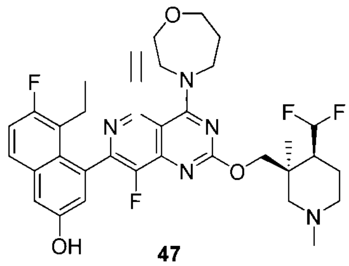<br>**47**<br>LC-MS (ESI, pos. ion) m/z:666.5 [M+H]+. | **Example 48**<br>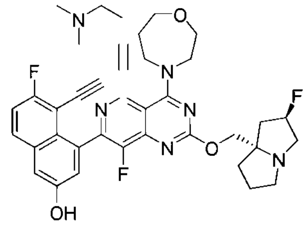<br>48<br>LC-MS (ESI, pos. ion) m/z: 685.5 [M+H]+. |
| **Example 49** | **Example** 50 |
| 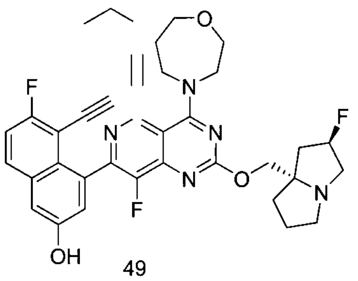<br>49<br>LC-MS (ESI, pos. ion) m/z: 656.2 [M+H]+. | 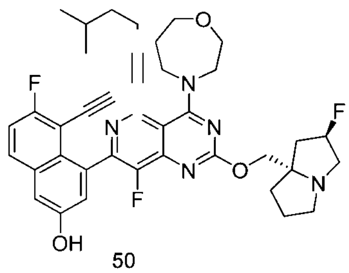<br>50<br>LC-MS (ESI, pos. ion) m/z: 684.5 [M+H]+. |
| **Example 51**<br>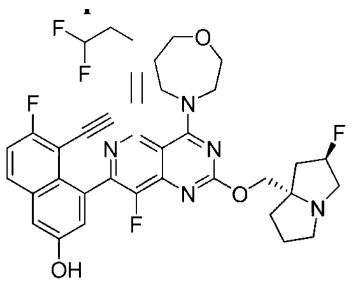<br>51<br>LC-MS (ESI, pos. ion) m/z: 692.2 [M+H]+. | **Example 52**<br>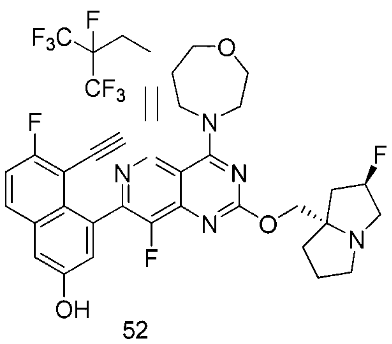<br>52<br>LC-MS (ESI, pos. ion) m/z: 810.0 [M+H]+. |
| **Example 53** | **Example 54** |

(continued)

| Table A | |
|---|---|
| **Example Compound and Characterization Data** | **Example Compound and Characterization Data** |
| 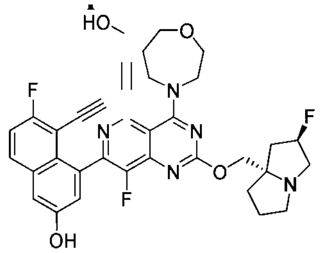<br>53<br>LC-MS (ESI, pos. ion) m/z: 644.3 [M+H]+. | 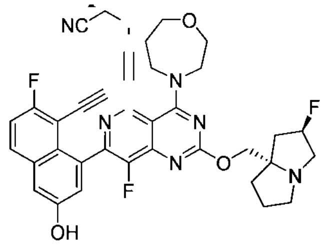<br>54<br>LC-MS (ESI, pos. ion) m/z: 667.1 [M+H]+. |
| **Example 55**<br>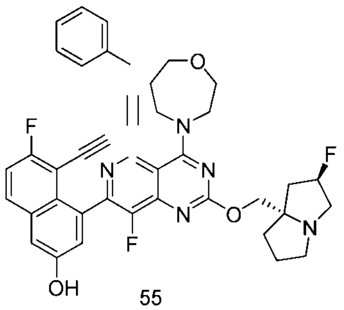<br>55<br>LC-MS (ESI, pos. ion) m/z: 704.3 [M+H]+. | **Example 56**<br>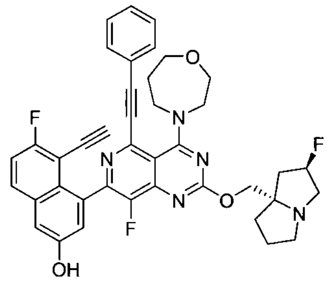<br>56<br>LC-MS (ESI, pos. ion) m/z: 690.2 [M+H]+. |
| **Example 57** | **Example 58** |
| 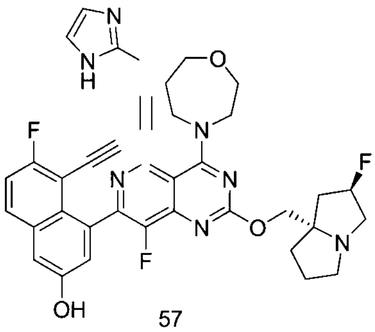<br>57<br>LC-MS (ESI, pos. ion) m/z: 694.5 [M+H]+. | 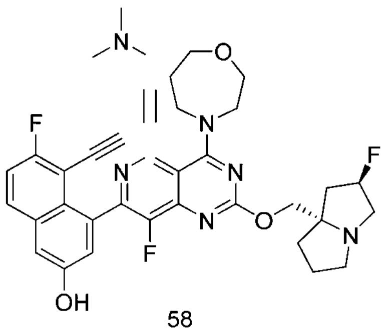<br>58<br>LC-MS (ESI, pos. ion) m/z: 671.6 [M+H]+. |
| **Example 59**<br>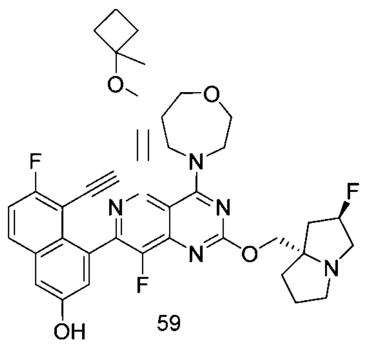<br>59<br>LC-MS (ESI, pos. ion) m/z: 712.5 [M+H]+. | **Examnle 60**<br>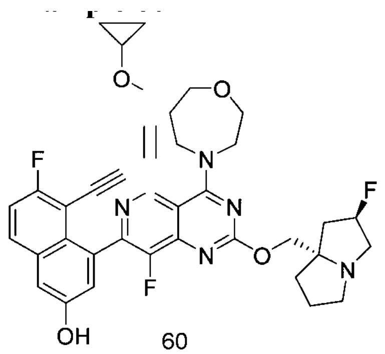<br>60<br>LC-MS (ESI, pos. ion) m/z: 684.0 [M+H]+. |
| **Example 61** | **Examnle 62** |

(continued)

| Table A | |
|---|---|
| **Example Compound and Characterization Data** | **Example Compound and Characterization Data** |
| 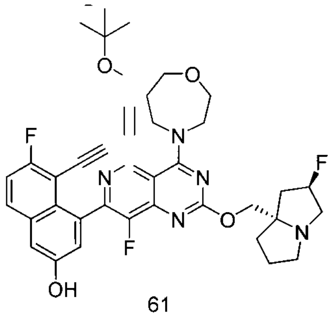<br>61<br>LC-MS (ESI, pos. ion) m/z: 700.5 $[M+H]^+$. | 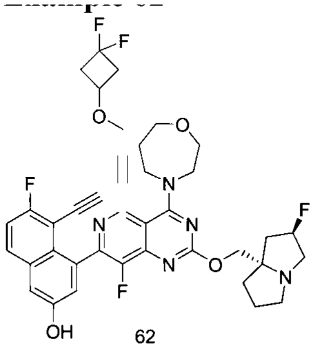<br>62<br>LC-MS (ESI, pos. ion) m/z: 734.0 $[M+H]^+$. |
| **Example 63**<br>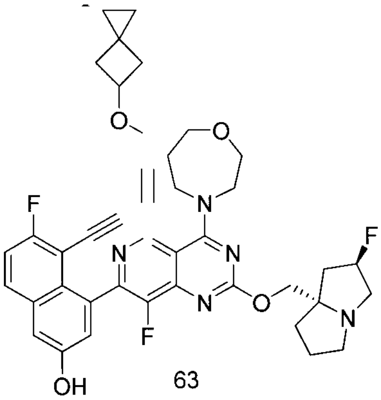<br>63<br>LC-MS (ESI, pos. ion) m/z: 724.5 $[M+H]^+$. | **Example 64**<br>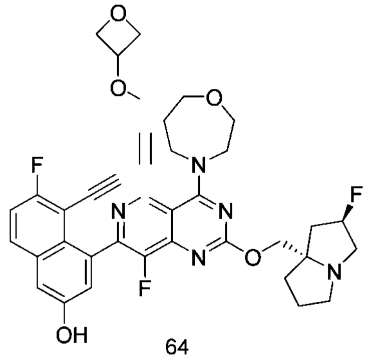<br>64<br>LC-MS (ESI, pos. ion) m/z: 700.2 $[M+H]^+$. |
| **Example 65** | **Example 66** |
| 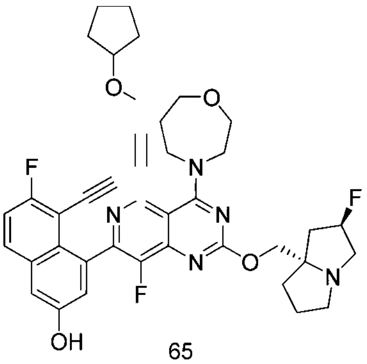<br>65<br>LC-MS (ESI, pos. ion) m/z: 712.5 $[M+H]^+$. | 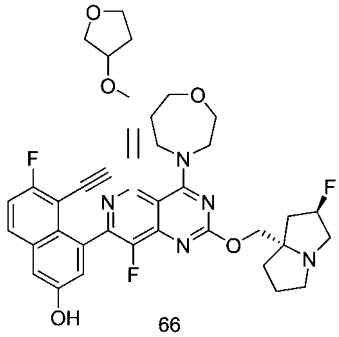<br>66<br>LC-MS (ESI, pos. ion) m/z: 714.5 $[M+H]^+$. |
| **Example 67** | **Example 68** |

(continued)

| Table A | |
|---|---|
| **Example Compound and Characterization Data** | **Example Compound and Characterization Data** |
| 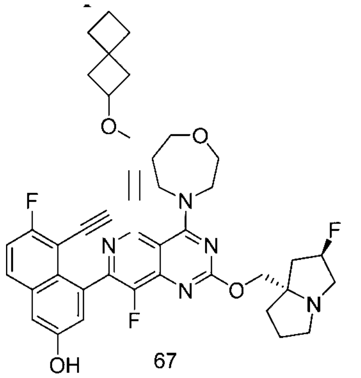 67 LC-MS (ESI, pos. ion) m/z: 738.3 $[M+H]^+$. | 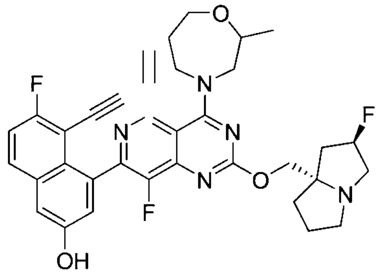 68 LC-MS (ESI, pos. ion) m/z: 642.2 $[M+H]^+$. |
| **Example 69** 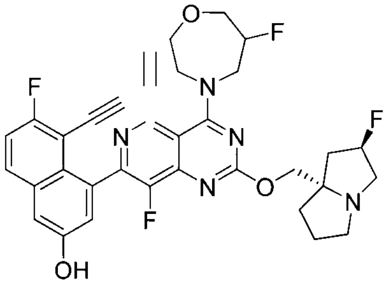 69 LC-MS (ESI, pos. ion) m/z: 646.2 $[M+H]^+$. | **Example 70** 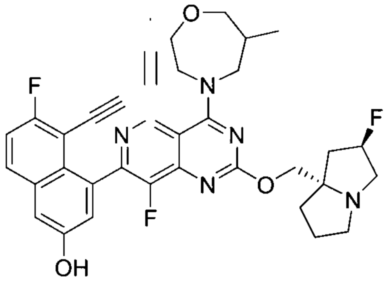 70 LC-MS (ESI, pos. ion) m/z: 641.5 $[M+H]^+$. |
| **Example 71** 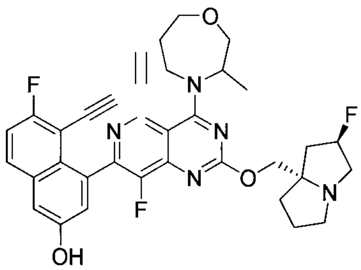 71 LC-MS (ESI, pos. ion) m/z: 641.5 $[M+H]^+$. | Example **72** 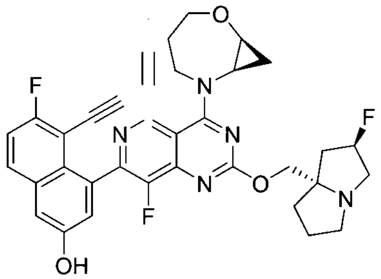 72 LC-MS (ESI, pos. ion) m/z: 640.2 $[M+H]^+$. |
| **Example 73** | **Example 74** |
| 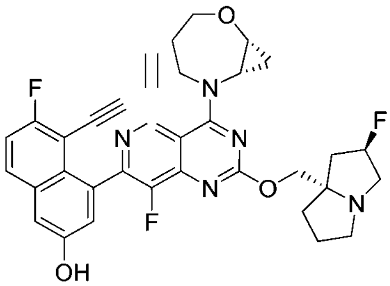 73 LC-MS (ESI, pos. ion) m/z: 640.2 $[M+H]^+$. | 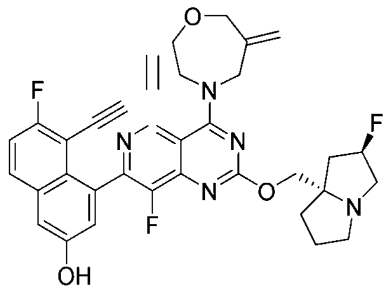 74 LC-MS (ESI, pos. ion) m/z: 640.2 $[M+H]^+$. |
| **Example** 75 | **Example** 76 |

(continued)

| Table A | |
|---|---|
| **Example Compound and Characterization Data** | **Example Compound and Characterization Data** |
| 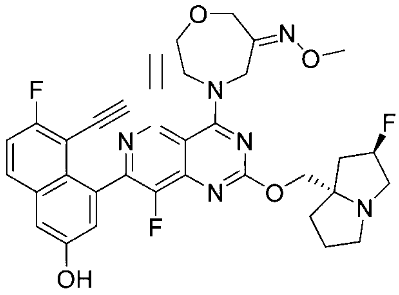<br>75<br>LC-MS (ESI, pos. ion) m/z: 671.0 [M+H]+. | 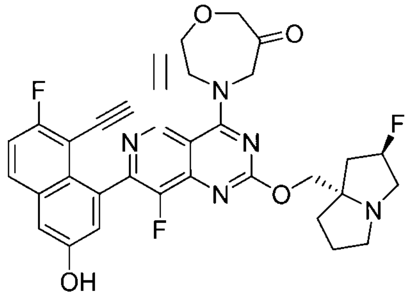<br>76<br>LC-MS (ESI, pos. ion) m/z: 642.2 [M+H]+. |
| **Example** 77<br>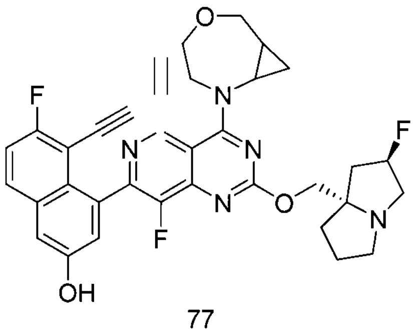<br>77<br>LC-MS (ESI, pos. ion) m/z: 640.2 [M+H]+. | **Example** 78<br>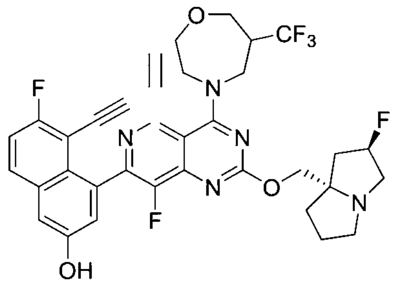<br>78<br>LC-MS (ESI, pos. ion) m/z: 696.5 [M+H]+. |
| **Example** 79<br>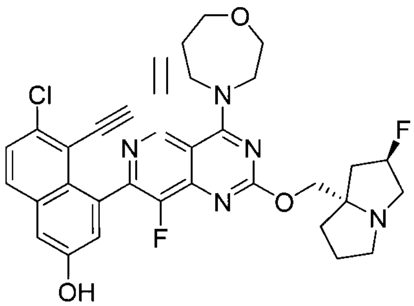<br>79<br>LC-MS (ESI, pos. ion) m/z: 644.0 [M+H]+. | **Example** 80<br>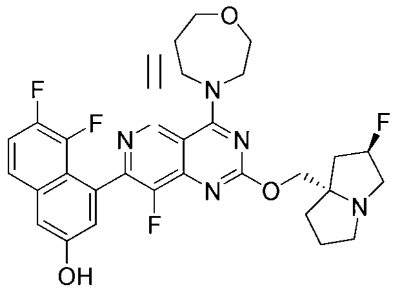<br>80<br>LC-MS (ESI, pos. ion) m/z: 622.2 [M+H]+. |
| **Example** 81 | **Example** 82 |
| 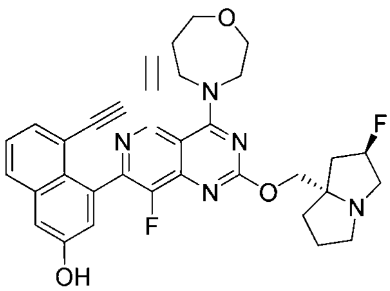<br>81<br>LC-MS (ESI, pos. ion) m/z: 610.2 [M+H]+. | 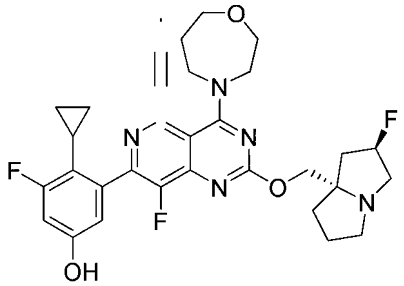<br>82<br>LC-MS (ESI, pos. ion) m/z: 694.4 [M+H]+. |

(continued)

| Table A | |
|---|---|
| **Example Compound and Characterization Data** | **Example Compound and Characterization Data** |
| **Example 83** 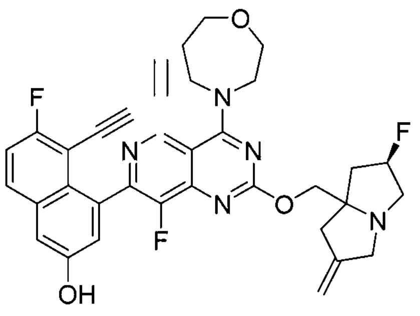 83 LC-MS (ESI, pos. ion) m/z: 640.2 [M+H]+. | **Example 84** 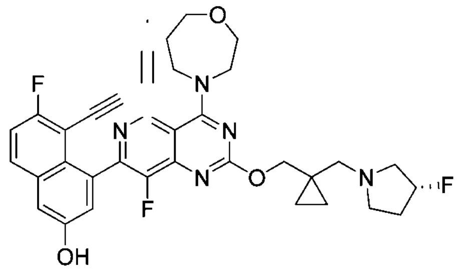 84 LC-MS (ESI, pos. ion) m/z: 642.6 [M+H]+. |
| **Example 85** 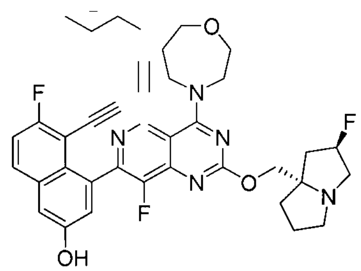 85 LC-MS (ESI, pos. ion) m/z: 670.4 [M+H]+. | **Example 86** 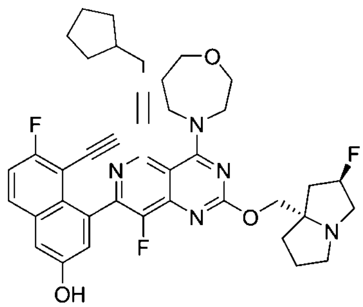 86 LC-MS (ESI, pos. ion) m/z: 696.5 [M+H]+. |
| **Example 87** 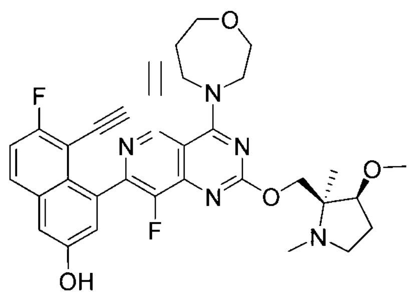 **87** LC-MS (ESI, pos. ion) m/z: 628.3 [M+H]+. | **Example 88** 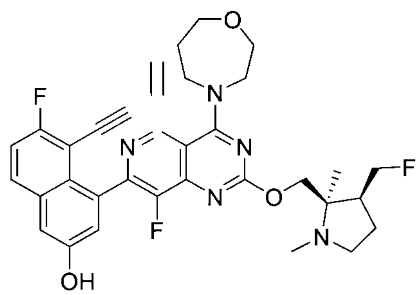 **88** LC-MS (ESI, pos. ion) m/z: 630.5 [M+H]+. |

**Biological Activity Examples**

**1. Determination of Inhibitory Effect on Intracellular pERK by In-Cell Western Assay**

Reagents Used

| Reagent | Source | Article No. |
|---|---|---|
| RPMI 1640 Medium | Gibco | A10491-01 |
| McCoy's 5A (modified) Medium | Invitrogen | 16600-108 |
| F-12K Medium | Gibco | 30-2004 |
| Fetal bovine serum (FBS) | Ausgenex | FBS500-S |
| DMEM Medium | Gibco | 11995065 |
| TrypLE™ Express Enzyme (1X), no phenol red | Gibco | 12604-021 |
| Penicillin-streptomycin | Gibco | 15140-122 |
| Blocking buffer: Odyssey Blocking Buffer (500mL) | LI-COR | 927-40000 |
| Secondary antibody: IRDye 800CW Goat anti-Rabbit IgG (H+L) (0.5 mg) | LI-COR | 926-32211 |
| Secondary antibody: IRDye 680RD Goat anti Mouse IgG (H+L) (0.5mg) | LI-COR | 926-68070 |
| Primary antibody: phospho-ERK Rabbit mAb | CST | 4370S |
| Primary antibody: GAPDH(D4C6R) Mouse mAb | CST | 97166S |
| Fixative solution: 8% fixative solution | Solarbio | P1112 |
| Dulbecco's Phosphate Buffered Saline (DPBS) | Gibco | 14190-144 |
| phosphate buffered saline(PBS) | Solarbio | P1010 |
| Tween-20 | Solarbio | T8220 |

Experimental steps:

(1) On the first day, the following cells were revived and cultured with the corresponding culture medium and incubated at 37 °C, 5% $CO_2$ until confluence.

| Cells | Culture Medium | Cell number in 384-well plate |
|---|---|---|
| H358 | RPMI 1640 Medium+10% Fetal bovine serum+1% Penicillin-streptomycin | 5000~8000 |
| MKN1 | RPMI 1640 Medium+10% Fetal bovine serum+1% Penicillin-streptomycin | |
| HPAC | DMEM Medium+10% Fetal bovine serum+1% Penicillin-streptomycin | |
| A549 | F12K Medium+10% Fetal bovine serum+1% | |
| | Penicillin-streptomycin | |
| PSN1 | RPMI 1640 Medium+10% Fetal bovine serum+1% Penicillin-streptomycin | |
| AGS | F-12K Medium+10% Fetal bovine serum+1% Penicillin-streptomycin | |
| HCT116 | McCoy's 5A (modified) Medium+10%+1% Penicillin-streptomycin | |
| NCI-H460 | RPMI 1640 Medium+10% Fetal bovine serum+1% Penicillin-streptomycin | |
| NCI-H727 | RPMI 1640 Medium+10% Fetal bovine serum+1% Penicillin-streptomycin | |

(2) On the third day, the culture medium was removed, the cells were rinsed once with DPBS, 2 mL of TrypLE™ trypsin was added for digestion, and the cells were placed at room temperature until the cells detached.
(3) 5 mL fresh culture medium was added to resuspend cells to terminate the digestion, and the cells were gently pipetted and centrifuged at 1000 rpm for 5 minutes at room temperature.
(4) The supernatant was removed, and 5 mL fresh culture medium was added to resuspend the cells and count cells.
(5) The cells were plated in a 384-well plate with appropriate cell concentration by adjusting with complete culture medium and incubated at 37 °C, 5% $CO_2$ for overnight.
(6) 200 nL of the prepared serial dilution compound (DMSO final concentration was 0.5%) was added and incubated at 37

°C, $CO_2$ for 3 h (HPAC cell lines for 1 h).
(7) The cells were fixed.
(8) The cells were washed twice with PBS.
(9) The blocking solution was added and the cells were incubated at room temperature for 1 h.
(10) The blocking solution was removed, and the primary antibody (phospho-ERK Rabbit mAb + GAPDH (D4C6R) Mouse mAb) was added and the cells were incubated at 4 °C for overnight.
(11) The cells were washed twice with PBST (PBS + 0.05% Tween 20) for 2 minutes each time.
(12) PBST was removed and secondary antibody (IRDye 800CW Goat anti-Rabbit IgG (H+L) + IRDye 680RD Goat anti Mouse IgG (H+L)) was added and incubated away from light at room temperature.
(14) Secondary antibody was discarded and the cell was washed twice with PBST, 2 minutes each time.
(15) 384-well plate was inverted and centrifuged at 1000 rpm at room temperature for 1 minute, and the 384-well plate was scanned with Odyssey CLx.
(16) Data analysis:

a) Assay stability check was peformed by using DMSO and reference control data:

Relative signal value = pERK (signal value of 800 channel) / GAPDH (signal value of 700 channel)

Inhibition rate % = 100-(Signal value of each sample-Relative signal value of quality control product)/(Signal value of DMSO well-Relative signal value of quality control product) × 100

b) nonlinear fitting of Compound $IC_{50}$:

$$Y=Bottom + (Top-Bottom) / (1+10^{\wedge} ((LogIC_{50}-X) \times HillSlope)).$$

The experimental test results are shown in Tables B and C, where "-" indicates that activity data were not measured.

Table B Inhibitory Effects of Compounds of the Present Invention on pERK in KRAS Wild-Type and Mutant Cell Lines

| Compound No. | H358, $IC_{50}$ (nM) | MKN1, $IC_{50}$ (nM) | HPAC, $IC_{50}$ (nM) | NCI-H727, $IC_{50}$ (nM) | A549, $IC_{50}$ (nM) | HCT 116,_ $IC_{50}$ (nM)_ |
|---|---|---|---|---|---|---|
| | KRAS *p.* G12C | KRAS WTdep | KRAS*p*.G12D | KRAS *p*.G12V | KRAS *p*.G12S | KRAS *p*.G13D |
| 1 | 0.4 | 1.9 | 0.3 | 1.0 | 0.6 | 2.1 |
| 2 | 2.0 | 6.4 | 1.6 | 4.8 | - | - |
| 3 | 0.7 | 3.7 | 0.6 | 2.2 | - | - |
| 7 | 1.3 | 3.3 | - | - | 5.7 | 11.6 |
| 10 | 0.7 | 1.6 | - | - | 1.4 | 6.2 |
| 11 | 2.0 | 7.4 | - | - | 20.2 | 28.6 |
| 25 | 0.8 | 3.0 | - | - | 2.3 | 7.9 |
| 26 | 3.9 | 21.5 | - | - | 15.1 | 47.3 |
| 27 | 2.0 | 4.5 | - | - | 5.1 | 19.5 |
| 29 | 2.2 | 3.6 | - | - | 6.2 | 15.2 |
| 38 | 0.4 | 1.41 | - | - | 1.2 | 5.4 |

As can be seen from Table B, the compounds of the present invention can effectively inhibit the phosphorylation of ERK protein in KRAS wild-type and KRAS mutant cell lines, including H358 (KRAS·G12C), MKN1.(KRAS WT), HPAC·(KRAS·G12D), NCI-H727:(KRAS.G12V), A549(KRAS G12S) and HCT-116(KRAS-G13D) cell lines.

Table C Inhibitory effect of the compounds of the present invention on pERK in AGS cells (KRAS G12D)

| Compound No. | AGS, $IC_{50}$ (nM) |
|---|---|
| | KRAS *p*.G12D |
| 1 | 0.2 |
| 2 | 2.2 |
| 3 | 0.5 |
| 4 | 7.5 |
| 5 | 8.6 |
| 7 | 0.6 |
| 8 | 7.1 |
| 9 | 17.2 |
| 10 | 0.8 |
| 11 | 2.6 |
| 12 | 5.8 |
| 14 | 1.2 |
| 15 | 15 |
| 16 | 8.1 |
| 17 | 0.8 |
| 19 | 6.4 |
| 20 | 0.4 |
| 22 | 2.8 |
| 23 | 6.1 |
| 24 | 5.4 |
| 25 | 0.8 |
| 26 | 3.2 |
| 27 | 5.2 |
| 29 | 1.4 |
| 30 | 6.8 |
| 33 | 4.1 |
| 35 | 4.4 |
| 38 | 1.6 |

As shown in Table C, the compounds of the present invention can effectively inhibit the phosphorylation of ERK protein in KRAS G12D mutant cell lines.

2. KRAS G12D/cRAF Binding Assay

Experimental steps:

(1) Compounds were prepared in DMSO and serially diluted 4-fold in DMSO.
(2) 0.1 μL of serially diluted compounds was added to a 384-well plate.
(3) 5 μL of a specific concentration of Tag2-KRAS G12D&GTP was added to the 384-well plate and the mixture was centrifuged at 1000 rpm for 1 minute.

(4) 5 μL of a specific concentration of Tag1-cRAF was added to the 384-well plate and the mixture was centrifuged at 1000 rpm for 1 minute.
(5) Incubated at 25 °C for 15 minutes.
(6) 10 μL of a mixture of anti-Tag1-$Tb^{3+}$ and anti-Tag2-XL665 was added to the 384-well plate.
(7) Centrifuged at 1000 rpm for 1 minute and incubated at 4 °C for 3 hours.
(8) The 665/615 nm ratio was read on a microplate reader.
(9) Data Analysis: GraphPad Prism 8.0 software was used to analyze the data and calculate the $IC_{50}$ values, with the logarithmic value of the compound concentration as the horizontal axis and the 665/615 nm ratio as the vertical axis.

The analysis results showed that the compounds of the present invention can effectively bind to KRAS G12D-GTP and inhibit the binding of KRAS to cRAF protein. Specific experimental results of some compounds are shown in Table D.

**3. KRAS G12D/SOS1 Binding Assay**

Experimental steps:

(1) Compounds were prepared in DMSO and serially diluted 4-fold in DMSO.
(2) 0.1 μL of serially diluted compounds was added to a 384-well plate.
(3) 5 μL of a specific concentration of Tag2-KRASG12D&GTP was added to the 384-well plate and the mixture was centrifuged at 1000 rpm for 1 minute.
(4) 5 μL of a specific concentration of Tag1-SOS1 was added to the 384-well plate and the mixture was centrifuged at 1000 rpm for 1 minute.
(5) Incubated at 25 °C for 15 minutes.
(6) 10 μL of a mixture of anti-Tag1-$Tb^{3+}$ and anti-Tag2-XL665 was added to the 384-well plate.
(7) Centrifuged at 1000 rpm for 1 minute and incubated at 4 °C for 3 hours.
(8) The 665/615 nm ratio was read on a microplate reader.
(9) Data Analysis: GraphPad Prism 8.0 software was used to analyze the data and calculate the $IC_{50}$ values, with the logarithmic value of the compound concentration as the horizontal axis and the 665/615 nm ratio as the vertical axis.

The analysis results showed that the compounds of the present invention can effectively bind to KRAS G12D-GTP and inhibit the binding of KRAS to SOS1 protein. Specific experimental results of some compounds are shown in Table D.

Table D Competitive binding experiments of compounds to KRAS G12D-GTP/cRAF and KRAS G12D-GTP/SOS1

| Compound No. | KRAS G12D-GTP & cRAF, HTRF, $IC_{50}$ (nM) | KRAS G12D-GTP & SOS1, HTRF, $IC_{50}$ (nM) |
|---|---|---|
| 1 | 7.9 | 16.3 |
| 2 | 13.7 | 20.8 |
| 3 | 7.4 | 14.8 |

**4. 3D-CTG method was used to detect the effects of compounds on the inhibition of GP2D, HPAC, AGS, MKN1, NCI-H727 and NCI-H358 cell proliferation:**

Experimental steps:

(1) AGS cells were revived with F12K medium containing 10% fetal bovine serum, GP2D and HPAC cells were revived with DMEM medium containing 10% fetal bovine serum, and MKN1, NCI-H727, and NCI-H358 cells were revived with RPMI 1640 medium containing 10% fetal bovine serum and passaged twice. After the cells recovered, they were ready for use in experiments.
(2) On the first day, the cells were rinsed once with PBS, preheated trypsin was added, and the cells were placed in a 37 °C, 5% $CO_2$ incubator for digestion for 3-5 minutes. The digestion was terminated by adding an appropriate amount of complete culture medium (containing 10% fetal bovine serum), and the cells were transferred to a centrifuge tube and

centrifuged at 1000 rpm for 5 minutes.

(3) The cells were resuspended with complete culture medium, counted, and adjusted to the appropriate cell concentration with complete culture medium.

(4) Using the ECHO 655 ultrasonic liquid handling system, 0.2 μl of the prepared 200× serial dilution compound (final DMSO concentration of 0.5%) was added to a 384-well plate. 40 μl of the cell suspension counted in step (3) was then added to each well and the plate was incubated in a 5% $CO_2$ incubator at 37 °C.

(6) After the fourth day of incubation of AGS and the eighth day of incubationof GP2D, HPAC, MKN1, NCI-H727, and NCI-H358, 3D CTG detection reagent was added to each well.

(7) Signal values were read using Envision.

(8) Data analysis:

a) DMSO and culture medium were used to test the stability of the experimental method:

H = DMSO well
L = Culture medium well

H, CV% (DMSO well) = 100 × (SD value of DMSO well / average value of DMSO well) L, CV% (corresponding culture medium well) = 100 × (SD value of culture medium well / average value of corresponding culture medium well)

Z' = 1-3 × (SD value of DMSO well + SD value of culture medium well) / (average value of DMSO well - average value of corresponding culture medium well)

Inhibition rate, % = (average value of DMSO well - average value of sample well) / (average value of DMSO well - average value of corresponding culture medium well) x 100

b) IC50 values were fitted using the nonlinear regression equation in XLfit 5.5.0 software.

$$Y=Bottom + (Top-Bottom)/(1+10^{\wedge}((LogIC50-X)*HillSlope))$$

X: Log value of compound concentration
Y: Inhibition rate (% inh)

Conclusion: The $IC_{50}$ value of the compounds of the present invention on the inhibition of GP2D, HPAC, AGS, MKN1, NCI-H727, and NCI-H358 cell proliferation is less than 1 μM, and most of the compounds have an $IC_{50}$ of less than 100 nM; among them, a large part of the compounds have an $IC_{50}$ of less than 10 nM. Therefore, the compounds of the present invention have good inhibitory effects on the proliferation of GP2D, HPAC, AGS, MKN1, NCI-H727, and NCI-H358 cells.

**5. Pharmacokinetic evaluation of the compounds of the present invention by intravenous injection or oral gavage in mice**

The inventors conducted a pharmacokinetic evaluation of the compounds of this invention in mice. Wherein, detailed animal information is provided in Table E.

Table E: Animal Information Table

| Strain | Grade | Sex | Weight | Age | Source |
|---|---|---|---|---|---|
| BALB/c mice | SPF | male | 18- 30 g | 6-8 weeks | Hunan SJA Laboratory Animal Co., Ltd |

Test method

The compounds of the present invention were administered to test animals in the form of a solution of 5% DMSO + 30% PEG400 + 65% saline, 10% DMSO + 10% Kolliphor HS15 + 80% Saline, 10% DMSO + 89% (25% SBE-B-CD) + (2% HCl), 20% PEG400 + 80% sterile water for injection, or 10% DMA + 10% HS15 + 30% PEG400 + 50% sterile water for

injection. The animals were fasted for 12 hours before administration and had free access to water. For the intravenous administration group, the dose was 2 mg/kg/day, qd. Venous blood samples (approximately 0.15 mL) were collected at the following time points after administration: 0.083, 0.25, 0.5, 1.0, 2.0, 5.0, 7.0 and 24 hours. EDTA-K2 was pre-added to the blood collection tube as an anticoagulant. Blood samples were centrifuged at 12,000 rpm for 2 minutes, and plasma was collected and stored at -20°C or -70°C. For the intravenous administration group, the dose was 5 mg/kg/day, qd. Venous blood samples (approximately 0.15 mL) were collected at the following time points after administration: 0.25, 0.5, 1.0, 2.0, 5.0, 7.0 and 24 hours. EDTA-K2 was pre-added to the blood collection tube as an anticoagulant. Blood samples were centrifuged at 12,000 rpm for 2 minutes, and plasma was collected and stored at -20°C or -70°C.

The collected plasma samples were processed (frozen plasma was thawed at room temperature and vortexed for 15 seconds. 10-20 μL of plasma was added to 120-150 μL of acetonitrile solution containing the internal standard and vortexed for 5 minutes. The sample was centrifuged at 4,000 rpm for 5 minutes. 100 μL of the supernatant was added to 120-150 μL of methanol/water (v/v = 1/1) and mixed). The concentrations of the compounds in the plasma were analyzed by LC-MS/MS.

The analysis results show that the compounds of the present invention have good pharmacokinetic properties in mice, such as compound 1 and compound 25 of the present invention, indicating that the compounds of the present invention have good druggability and better clinical application prospects. The pharmacokinetic parameters of some compounds of the present invention in mice are shown in Table F.

Table F Pharmacokinetic parameters of the compounds of the present invention in mice

| compound | Dosage (mg/kg/day) | $T_{max}$ | $C_{max}$ | $AUC_{last}$ | $AUC_{INF}$ | $T_{1/2}$ | $MRT_{last}$ | Cl | Vss | $F_AUC_{T NF}$ |
|---|---|---|---|---|---|---|---|---|---|---|
| | | (h) | (ng/mL ) | (h*ng/mL) | (h*ng/mL) | (h) | (h) | (mL/min/kg) | (L/kg) | (%) |
| 25 | 2 (iv administration) | 0.083 | 9950 | 10600 | 10800 | 1.2 | 1.3 | 3.2 | 0.2 7 | - |
| | 5 (ig administration) | 0.42 | 2660 | 5960 | 6140 | 1.3 | 1.7 | - | - | 23 |
| 26 | 2 (iv administration) | 0.083 | 8280 | 8050 | 8110 | 1 | 1.2 | 4.1 | 0.3 | - |
| | 5 (ig administration) | 0.5 | 1630 | 4120 | 4240 | 1.3 | 1.8 | - | - | 21 |

Reference throughout this specification to "one embodiment," "some embodiments," "certain implementations," "example," "specific example," or "some examples" means that a particular feature, structure, material, or characteristic described in connection with the embodiment or example is included in at least one embodiment or example of the present disclosure. In this specification, the schematic expressions of the above terms do not necessarily refer to the same embodiment or example. Furthermore, the particular features, structures, materials, or characteristics may be combined in any suitable manner in one or more embodiments or examples. In addition, those skilled in the art can integrate and combine different embodiments, implementations, examples or the features of them as long as they are not contradictory to one another.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure, the scope of the invention is defined by the claims and their equivalents.

## Claims

**1.** A compound having Formula (I) or a stereoisomer, a tautomer, an N-oxide, a solvate, a metabolite, a pharmaceutically acceptable salt or a prodrug thereof,

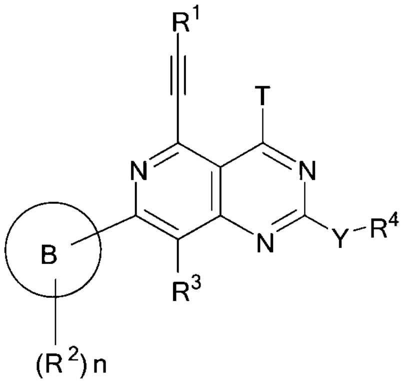

(I),

wherein,

$R^1$ is -H, -D, -CN, $-NH_2$, -C(=O)H, -C(=O)OH, $-C(=O)OR^{6a}$, $-C(=O)NR^6R^7$, $-NR^6C(=O)R^7$, $-NR^{6a}C(=O)NR^6R^7$, $-C(=O)R^{6a}$, $-C(=O)OR^{6a}$, $-NR^6S(=O)_2R^7$, $-S(=O)_2NR^6R^7$, $-NR^{6a}S(=O)_2NR^6R^7$, $-NR^6R^7$, $-C_{1-6}$ alkyl $NR^6C(=O)R^7$, $-C_{1-6}$ alkyl $NR^6R^7$, $-C_{1-6}$ alkyl-$C(=O)OR^6$, $-C_{1-6}$ alkyl-$C(=O)NR^6R^7$, $C_{1-6}$ alkyl, $C_{1-6}$ cyanoalkyl, $C_{1-6}$ hydroxyalkyl, $C_{1-6}$ haloalkyl, $C_{1-6}$ alkoxy $C_{1-6}$ alkyl, ($C_{3-12}$ cycloalkyl)-$C_{1-6}$ alkyl, (3-12 membered heterocyclyl)-$C_{1-6}$ alkyl, ($C_{6-10}$ aryl)-$C_{1-6}$ alkyl, (5-12 membered heteroaryl)-$C_{1-6}$ alkyl, ($C_{3-12}$ cycloalkyl)-O-$C_{1-6}$ alkyl, (3-12 membered heterocyclyl)-O-$C_{1-6}$ alkyl, $C_{1-6}$ mercaptoalkyl, $C_{6-12}$ aryl, 5-12 membered heteroaryl, $C_{3-12}$ cycloalkyl or 3-12 membered heterocyclyl; wherein the $C_{1-6}$ alkoxy $C_{1-6}$ alkyl, ($C_{3-12}$ cycloalkyl)-$C_{1-6}$ alkyl, (3-12 membered heterocyclyl)-$C_{1-6}$ alkyl, ($C_{6-10}$ aryl)-$C_{1-6}$ alkyl, (5-12 membered heteroaryl)-$C_{1-6}$ alkyl, ($C_{3-12}$ cycloalkyl)-O-$C_{1-6}$ alkyl, (3-12 membered heterocyclyl)-O-$C_{1-6}$ alkyl, $C_{6-12}$ aryl, 5-12 membered heteroaryl, $C_{3-12}$ cycloalkyl and 3-12 membered heterocyclyl are each independently optionally substituted with 1, 2, 3 or 4 substituents selected from D, -OH, -F, -Cl, -Br, -I, CN, $-C(=O)OR^6$, $-NR^6R^7$, $-C(=O)NR^6R^7$, $-NR^6C(=O)R^7$ and $C_{1-6}$ alkyl;
T is

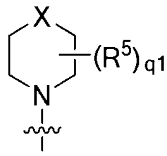

;

X is a bond, -O-, -S-, -NH-, $-CH_2-$, $-CH_2-NH-$, $-CH_2-NH-CH_2-$, $-(CH_2)_3-$, $-(CH_2)_2-$, $-NH-CH_2-$, $-O-CH_2-$, $-CH_2-O-$, $-CH_2-S-$ or $-S-CH_2-$;
each $R^5$ is independently H, -D, -OH, -F, -Cl, -Br, -I, -CN, -SH, -COOH, oxo, -NHC(=O)H, $-C(=O)R^{6c}$, $-C(=O)OR^{6c}$, $-C(=O)NR^{6b}R^{7b}$, $-NR^{6b}C(=O)R^{7b}$, $-NR^{6b}S(=O)_2R^{7b}$, $-S(=O)_2N(R^{7b})_2$, $-NR^{6b}C(=O)N(R^{7b})_2$, $-NR^{6b}S(=O)_2N(R^{7b})_2$, $-OS(=O)_2N(R^{7b})_2$, $-S(=O)_2R^{6c}$, $-C_{1-4}$ alkylene $-S(=O)_2N(R^{7b})_2$, $C_{1-6}$ alkyl, $C_{1-6}$ alkylthio, $C_{1-6}$ alkoxy, $C_{2-6}$ alkenyl, $C_{2-6}$ alkynyl, $C_{1-6}$ cyanoalkyl, $C_{1-6}$ hydroxyalkyl, $C_{1-6}$ haloalkyl, -NH($C_{1-6}$ alkyl), -N($C_{1-6}$ alkyl)$_2$, $C_{2-6}$ haloalkenyl, $C_{2-6}$ haloalkynyl, $C_{1-6}$ haloalkoxy, $C_{1-6}$ haloalkylthio, $C_{6-10}$ aryl, 5-10 membered heteroaryl, $C_{3-6}$ cycloalkyl or 3-6 membered heterocyclyl, wherein the $C_{1-6}$ alkyl, $C_{1-6}$ alkylthio, $C_{1-6}$ alkoxy, $C_{2-6}$ alkenyl, $C_{2-6}$ alkynyl, -NH($C_{1-6}$ alkyl), -N($C_{1-6}$ alkyl)$_2$, $C_{6-10}$ aryl, 5-10 membered heteroaryl, $C_{3-6}$ cycloalkyl and 3-6 membered heterocyclyl are each independently optionally substituted with 1, 2, 3 or 4 $R^8$;
or, two $R^5$ attached to the same carbon atom together form

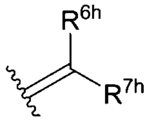 or 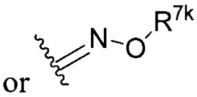 ;

or, two $R^5$ attached to the same carbon atom together with the carbon atom to which they are attached form a $C_{3-6}$ cycloalkyl or 3-6 membered heterocyclyl, wherein the $C_{3-6}$ cycloalkyl and 3-6 membered heterocyclyl are each independently optionally substituted with 1, 2, 3 or 4 $R^8$;
or, two $R^5$ attached to two adjacent atoms together with the two adjacent atoms to which they are attached form a $C_{3-6}$ cycloalkyl or 3-6 membered heterocyclyl, wherein the $C_{3-6}$ cycloalkyl and 3-6 membered heterocyclyl are each independently optionally substituted with 1, 2, 3 or 4 $R^8$;

each $R^8$ is independently -D, -OH, -F, -Cl, -Br, -I, -CN, -$NH_2$, -C(=O)OH, -C(=O)$NH_2$, oxo, $C_{1-6}$ alkyl, $C_{1-6}$ alkoxy, $C_{1-6}$ hydroxyalkyl, $C_{6-10}$ aryl, 6-10 membered heteroaryl, -C(=O)O$C_{1-6}$ alkyl, -C(=O)NH$C_{1-6}$ alkyl or -C(=O)N($C_{1-6}$ alkyl)$_2$;

with the proviso that T is not

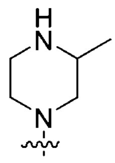 , 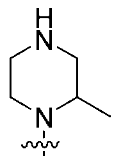 , 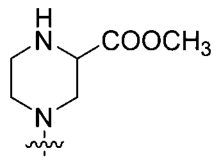 , 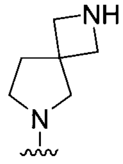 ,

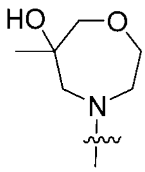 or 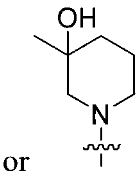 ;

Ring B is $C_{6-12}$ aryl or 5-12 membered heteroaryl;

each $R^2$ is independently -D, -OH, -F, -Cl, -Br, -I, -CN, -SH, -$CH_2$C(=O)$NR^{6b}R^{7b}$, -C(=O)$R^{6c}$, -C(=O)$OR^{6c}$, -C(=O)$NR^{6b}R^{7b}$, -$NR^{6b}$C(=O)$R^{7b}$, -$NR^{6b}R^{7b}$, $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, $C_{6-12}$ aryl, 5-12 membered heteroaryl, $C_{3-6}$ cycloalkyl or 3-6 membered heterocyclyl, wherein the $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, $C_{6-12}$ aryl, 5-12 membered heteroaryl, $C_{3-6}$ cycloalkyl and 3-6 membered heterocyclyl are each independently optionally substituted with 1, 2, 3 or 4 substituents selected from -D, -OH, -F, -Cl, -Br, -I, -CN, -$NH_2$, -C(=O)H, -C(=O)OH, $C_{1-6}$ alkyl, $C_{1-6}$ alkoxy, $C_{3-6}$ cycloalkyl and 3-6 membered heterocyclyl;

$R^3$ is -H, -D, -OH, -SH, -F, -Cl, -Br, -I, -CN, methyl, ethyl, n-propyl, i-propyl, n-butyl or $C_{1-4}$ haloalkyl;

Y is bond, O or S;

$R^4$ is 3-10 membered heterocyclyl, -L-(3-12 membered heterocyclyl), -L-($C_{3-12}$ cycloalkyl), -L-(5-12 membered heteroaryl) or -L-($C_{6-10}$ aryl); wherein the 3-10 membered heterocyclyl, -L-($C_{3-12}$ cycloalkyl), -L-(5-12 membered heteroaryl) and -L-($C_{6-10}$ aryl) are each independently optionally substituted with 1, 2, 3 or 4 $R^{9a}$; and the -L-(3-12 membered heterocyclyl) is optionally substituted with 1, 2, 3 or 4 $R^{9b}$.

$R^{9a}$ and $R^{9b}$ are each independently -D, -OH, -F, -Cl, -Br, -I, -CN, -$NR^{6d}R^{7d}$, -C(=O)$NR^{6d}R^{7d}$, -$CH_2NR^{6d}R^{7d}$, -$CH_2$OC(=O)$NR^{6d}R^{7d}$, $C_{1-6}$ alkyl, $C_{1-6}$ alkoxy, $C_{1-6}$ haloalkyl, $C_{1-6}$ haloalkoxy, ($C_{6-10}$ aryl)-$C_{1-6}$ alkyl, (5-12 membered heteroaryl)-$C_{1-6}$ alkyl, (3-6 membered heterocyclyl)-$C_{1-6}$ alkyl, ($C_{3-6}$ cycloalkyl)-$C_{1-6}$ alkyl, $C_{3-6}$ cycloalkyl or 3-6 membered heterocyclyl; wherein the $C_{1-6}$ alkyl, $C_{1-6}$ alkoxy, $C_{1-6}$ haloalkyl, $C_{1-6}$ haloalkoxy, ($C_{6-10}$ aryl)-$C_{1-6}$ alkyl, (5-12 membered heteroaryl)-$C_{1-6}$ alkyl, (3-6 membered heterocyclyl)-$C_{1-6}$ alkyl, ($C_{3-6}$ cycloalkyl)-$C_{1-6}$ alkyl, $C_{3-6}$ cycloalkyl and 3-6 membered heterocyclyl are each independently optionally substituted with 1, 2, 3 or 4 substituents selected from -D, -F, -Cl, -Br, -I, -OH, -CN, -$NR^{6e}R^{7e}$, -C(=O)$C_{1-6}$ alkyl and $C_{1-6}$ alkyl;

or, two $R^{9b}$ attached to the same ring carbon atom together form

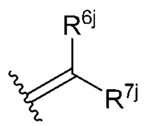 ;

L is $C_{1-6}$ alkylene;

$R^6$, $R^7$, $R^{6b}$, $R^{7b}$, $R^{6d}$, $R^{7d}$, $R^{6e}$ and $R^{7e}$ are each independently -H, -D or $C_{1-6}$ alkyl, wherein the $C_{1-6}$ alkyl is independently optionally substituted with 1, 2, 3 or 4 substituents selected from -D, -OH, -F, -Cl, -Br, -I, -CN, -C(=O)H, -C(=O)OH, -$NR^{6g}R^{7g}$, $C_{1-6}$ alkoxy, $C_{6-12}$ aryl, $C_{3-6}$ cycloalkyl and 3-6 membered heterocyclyl;

or $R^6$ and $R^7$, or $R^{6b}$ and $R^{7b}$, or $R^{6d}$ and $R^{7d}$, or $R^{6e}$ and $R^{7e}$, independently together with the N atom to which they are attached form a 4-6 membered heterocyclic ring; wherein the 4-6 membered heterocyclic ring is optionally substituted with 1, 2, 3 or 4 substituents selected from -D, -OH, -F, -Cl, -Br, -I, -CN, -$NH_2$, $C_{1-6}$ alkyl, $C_{1-6}$ alkylamino, $C_{3-6}$ cycloalkyl, 3-6 membered heterocyclyl, $C_{1-6}$ alkoxy, $C_{1-6}$ cyanoalkyl, $C_{1-6}$ hydroxyalkyl, $C_{1-6}$

haloalkoxy and $C_{1-6}$ haloalkyl;
$R^{6a}$, $R^{6c}$, $R^{6g}$, $R^{7g}$, $R^{6h}$, $R^{7h}$, $R^{7k}$, $R^{6j}$ and $R^{7j}$ are each independently -H, -D or $C_{1-6}$ alkyl;
n is 0, 1, 2, 3, 4, 5, 6 or 7;
q1 is 0, 1, 2, 3, 4, 5, 6, 7 or 8.

**2.** The compound of claim 1, wherein, $R^1$ is -H, -D, -CN, $-NH_2$, -C(=O)H, -C(=O)OH, $-C(=O)OR^{6a}$, $-C(=O)NR^6R^7$, $-NR^6C(=O)R^7$, $-NR^{6a}C(=O)NR^6R^7$, $-C(=O)R^{6a}$, $-C(=O)OR^{6a}$, $-NR^6S(=O)_2R^7$, $-S(=O)_2NR^6R^7$, $-NR^{6a}S(=O)_2NR^6R^7$, $-NR^6R^7$, $-C_{1-4}$ alkyl $NR^6C(=O)R^7$, $-C_{1-4}$ alkyl $NR^6R^7$, $-C_{1-4}$ alkyl-$C(=O)OR^6$, $-C_{1-4}$ alkyl-$C(=O)NR^6R^7$, $C_{1-6}$ alkyl, $C_{1-4}$ cyanoalkyl, $C_{1-4}$ hydroxyalkyl, $C_{1-4}$ haloalkyl, $C_{1-4}$ alkoxy $C_{1-4}$ alkyl, ($C_{3-6}$ cycloalkyl)-$C_{1-4}$ alkyl, ($C_{7-12}$ cycloalkyl)-$C_{1-4}$ alkyl, (3-6 membered heterocyclyl)-$C_{1-4}$ alkyl, (7-12 membered heterocyclyl)-$C_{1-4}$ alkyl, phenyl-$C_{1-4}$ alkyl, (5-6 membered heteroaryl)-$C_{1-4}$ alkyl, ($C_{3-6}$ cycloalkyl)-O-$C_{1-4}$ alkyl, ($C_{7-12}$ cycloalkyl)-O-$C_{1-4}$ alkyl, (3-6 membered heterocyclyl)-O-$C_{1-6}$ alkyl, (3-7 membered heterocyclyl)-O-$C_{1-6}$ alkyl, (7-12 membered heterocyclyl)-O-$C_{1-4}$ alkyl, $C_{1-4}$ mercaptoalkyl, $C_{6-10}$ aryl, 5-12 membered heteroaryl, $C_{3-6}$ cycloalkyl or 3-6 membered heterocyclyl; wherein the $C_{1-4}$ alkoxy $C_{1-4}$ alkyl, ($C_{3-6}$ cycloalkyl)-$C_{1-4}$ alkyl, ($C_{7-12}$ cycloalkyl)-$C_{1-4}$ alkyl, (3-6 membered heterocyclyl)-$C_{1-4}$ alkyl, (7-12 membered heterocyclyl)-$C_{1-4}$ alkyl, phenyl-$C_{1-4}$ alkyl, (5-6 membered heteroaryl)-$C_{1-4}$ alkyl, ($C_{3-6}$ cycloalkyl)-O-$C_{1-4}$ alkyl, ($C_{7-12}$ cycloalkyl)-O-$C_{1-4}$ alkyl, (3-6 membered heterocyclyl)-O-$C_{1-6}$ alkyl, (3-7 membered heterocyclyl)-O-$C_{1-6}$ alkyl, (7-12 membered heterocyclyl)-O-$C_{1-4}$ alkyl, $C_{6-10}$ aryl, 5-12 membered heteroaryl, $C_{3-6}$ cycloalkyl and 3-6 membered heterocyclyl are each independently optionally substituted with 1, 2, 3 or 4 substituents selected from D, -OH, -F, -Cl, -Br, -I, CN, $-C(=O)OR^6$, $-NR^6R^7$, $-C(=O)NR^6R^7$, $-NR^6C(=O)R^7$ and $C_{1-4}$ alkyl;

**3.** The compound of any one of claims 1-2, wherein, $R^1$ is -H, -D, -CN, $-NH_2$, -C(=O)H, -C(=O)OH, $-C(=O)OR^{6a}$, $-C(=O)NR^6R^7$, $-NR^6C(=O)R^7$, $-NR^{6a}C(=O)NR^6R^7$, $-C(=O)R^{6a}$, $-C(=O)OR^{6a}$, $-NR^6S(=O)_2R^7$, $-S(=O)_2NR^6R^7$, $-NR^{6a}$-$S(=O)_2NR^6R^7$, $-NR^6R^7$, $-CH_2NR^6C(=O)R^7$, $-CH_2NR^6R^7$, $-(CH_2)_2NR^6R^7$, $-CH_2C(=O)OR^6$, $-(CH_2)_2C(=O)OR^6$, $-(CH_2)_3C(=O)OR^6$, $-CH_2C(=O)NR^6R^7$, $-(CH_2)_2C(=O)NR^6R^7$, $-(CH_2)_3C(=O)NR^6R^7$, $-CH_3$, $-CH_2CH_3$, $-(CH_2)_2CH_3$, $-(CH_2)_2CH(CH_3)_2$, $-(CH_2)_3CH_3$, $-C(CH_3)_3$, $-CH(CH_3)_2$, $-CH_2CH(CH_3)_2$, $-CH_2CN$, $-(CH_2)_2CN$, $-(CH_2)_3CN$, $-CH(CH_3)CN$, $-C(CH_3)_2CN$, $-CH_2OH$, $-(CH_2)_2OH$, $-(CH_2)_3OH$, $-CH(OH)CH_3$, $-(CH_2)_2CHF_2$, $-(CH_2)_2CF(CF_3)_2$, $-CF_3$, $-CHF_2$, $-CH_2F$, $-(CH_2)_2F$, $-(CH_2)_2Cl$, $-CH_2CF_3$, $-CH_2OCH_3$, $-(CH_2)_2OCH_3$, $-(CH_2)_2OCH_2CH_3$, $-CH_2OCH_2CH_3$, $-CH_2OC(CH_3)_3$, $-CH_2$-cyclopropyl, $-CH_2$-cyclobutyl, $-CH_2$-cyclopentyl, $-CH_2$-cyclohexyl, $-(CH_2)_2$-cyclopentyl, $-(CH_2)_3$-cyclopentyl, $-(CH_2)_2$-cyclohexyl, $-CH_2$-phenyl, $-CH_2$-imidazolyl, $-CH_2$-pyrazolyl, $-CH_2O$-cyclopropyl, $-CH_2O$-cyclobutyl, $-(CH_2)_2O$-cyclobutyl, $-CH_2O$-cyclopentyl, $-(CH_2)_2O$-cyclopentyl, $-CH_2O$-azetidinyl, $-CH_2O$-oxetanyl, $-CH_2O$-tetrahydrofuranyl, $-CH_2O$-pyrrolidinyl, $-CH_2O$-spiro[2.3]hexyl, $-CH_2O$-spiro[3.3]heptanyl, $-CH_2SH$, $-(CH_2)_2SH$, phenyl, naphthyl, pyridinyl, pyrimidinyl, cyclopropyl, cyclobutyl, cyclopentyl, cyclohexyl, oxiranyl, oxetanyl, tetrahydropyranyl, azetidinyl or pyrrolidinyl; wherein the $-CH_2OCH_3$, $-(CH_2)_2OCH_3$, $-(CH_2)_2OCH_2CH_3$, $-CH_2OCH_2CH_3$, $-CH_2OC(CH_3)_3$, $-CH_2$-cyclopropyl, $-CH_2$-cyclobutyl, $-CH_2$-cyclopentyl, $-CH_2$-cyclohexyl, $-(CH_2)_2$-cyclopentyl, $-(CH_2)_3$-cyclopentyl, $-(CH_2)_2$-cyclohexyl, $-CH_2$-phenyl, $-CH_2$-imidazolyl, $-CH_2$-pyrazolyl, $-CH_2O$-cyclopropyl, $-CH_2O$-cyclobutyl, $-(CH_2)_2O$-cyclobutyl, $-CH_2O$-cyclopentyl, $-(CH_2)_2O$-cyclopentyl, $-CH_2O$-azetidinyl, $-CH_2O$-oxetanyl, $-CH_2O$-tetrahydrofuranyl, $-CH_2O$-pyrrolidinyl, $-CH_2O$-spiro[2.3]hexanyl, $-CH_2O$-spiro[3.3]heptanyl, phenyl, naphthyl, pyridinyl, pyrimidinyl, cyclopropyl, cyclobutyl, cyclopentyl, cyclohexyl, oxiranyl, oxetanyl, tetrahydropyranyl, azetidinyl and pyrrolidinyl are each independently optionally substituted with 1, 2, 3 or 4 substituents selected from D, -OH, -F, -Cl, -Br, -I, CN, $-C(=O)OR^6$, $-NR^6R^7$, $-C(=O)NR^6R^7$, $-NR^6C(=O)R^7$, methyl, ethyl, n-propyl, isopropyl, n-butyl, isobutyl and *tert*-butyl.

**4.** The compound of any one of claims 1-3, wherein, T is

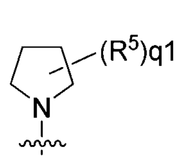 , 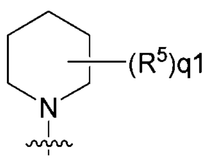 ,

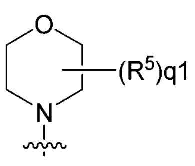 , 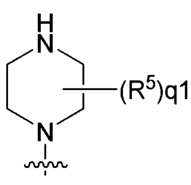 , 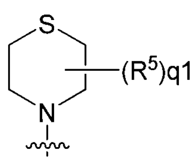 , 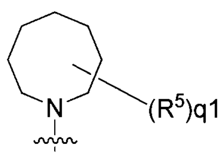 , 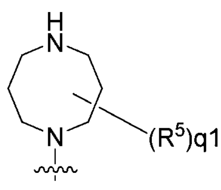 ,

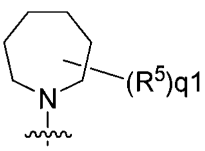, 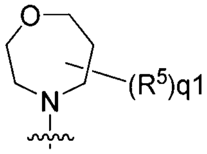, 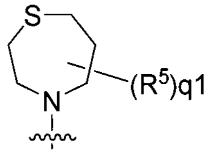 or 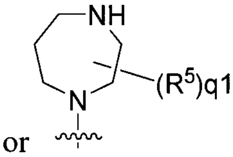,

each $R^5$ is independently-H, -D, -OH, -F, -Cl, -Br, -I, -CN, -SH, -COOH, oxo, -NHC(=O)H, $-C(=O)R^{6c}$, $-C(=O)OR^{6c}$, $-C(=O)NR^{6b}R^{7b}$, $-NR^{6b}C(=O)R^{7b}$, $-NR^{6b}S(=O)_2R^{7b}$, $-S(=O)_2N(R^{7b})_2$, $-NR^{6b}C(=O)N(R^{7b})_2$, $-NR^{6b}S(=O)_2N(R^{7b})_2$, $-OS(=O)_2N(R^{7b})_2$, $-S(=O)_2R^{6c}$, $-C_{1-4}$ alkylene-$S(=O)_2N(R^{7b})_2$, $C_{1-4}$ alkyl, $C_{1-4}$ alkylthio, $C_{1-4}$ alkoxy, $C_{2-4}$ alkenyl, $C_{2-4}$ alkynyl, $C_{1-4}$ cyanoalkyl, $C_{1-4}$ hydroxyalkyl, $C_{1-4}$ haloalkyl, -NH($C_{1-4}$ alkyl), -N($C_{1-4}$ alkyl)$_2$, $C_{2-4}$ haloalkenyl, $C_{2-4}$ haloalkynyl, $C_{1-4}$ haloalkoxy, $C_{1-4}$ haloalkylthio, 6-10 membered aryl, 5-10 membered heteroaryl, $C_{3-6}$ cycloalkyl or 3-6 membered heterocyclyl; wherein the $C_{1-4}$ alkyl, $C_{1-4}$ alkylthio, $C_{1-4}$ alkoxy, $C_{2-4}$ alkenyl, $C_{2-4}$ alkynyl, -NH($C_{1-4}$ alkyl), -N($C_{1-4}$ alkyl)$_2$, 6-10 membered aryl, 5-10 membered heteroaryl, $C_{3-6}$ cycloalkyl and 3-6 membered heterocyclyl are each independently optionally substituted with 1, 2, 3 or 4 $R^8$;

or, two $R^5$ attached to the same carbon atom together form

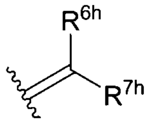 or 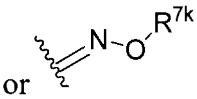 ;

or, two $R^5$ attached to the same carbon atom together with the carbon atom to which they are attached form a $C_{3-6}$ cycloalkyl or 3-6 membered heterocyclyl, wherein the $C_{3-6}$ cycloalkyl and 3-6 membered heterocyclyl are each independently optionally substituted with 1, 2, 3 or 4 $R^8$;

or, two $R^5$ attached to two adjacent atoms together with the two adjacent atoms to which they are attached form a $C_{3-6}$ cycloalkyl or 3-6 membered heterocyclyl, wherein the $C_{3-6}$ cycloalkyl and 3-6 membered heterocyclyl are each independently optionally substituted with 1, 2, 3 or 4 $R^8$;

each $R^8$ is independently -D, -OH, -F, -Cl, -Br, -I, -CN, $-NH_2$, -C(=O)OH, $-C(=O)NH_2$, oxo, $C_{1-4}$ alkyl, $C_{1-4}$ alkoxy, $C_{1-4}$ hydroxyalkyl, $C_{6-10}$ aryl, 6-10 membered heteroaryl, -C(=O)O$C_{1-4}$ alkyl, -C(=O)NH$C_{1-4}$ alkyl, -C(=O)N($C_{1-4}$ alkyl)$_2$;

$R^{6h}$, $R^{7h}$ and $R^{7k}$ each are independently -H, -D or $C_{1-4}$ alkyl.

**5.** The compound of any one of claims 1-4, wherein, T is

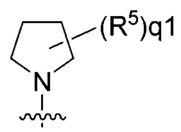, 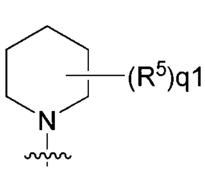, 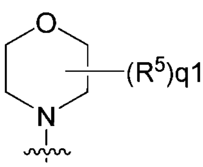, 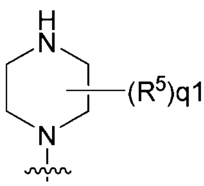, 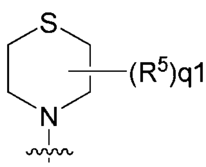,

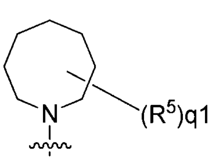, 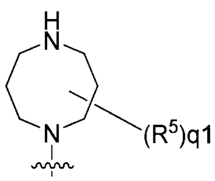, 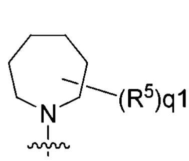, 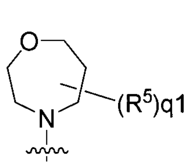,

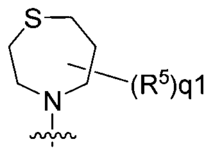 or 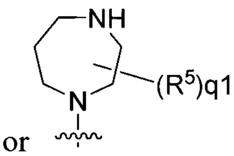 ,

each $R^5$ is independently -H, -D, -OH, -F, -Cl, -Br, -I, -CN, -SH, -COOH, oxo, -NH(C=O)H, $-C(=O)R^{6c}$, $-C(=O)OR^{6c}$, $-C(=O)NR^{6b}R^{7b}$, $-NR^{6b}C(=O)R^{7b}$, $-NR^{6b}S(=O)_2R^{7b}$, $-S(=O)_2N(R^{7b})_2$, $-NR^{6b}C(=O)N(R^{7b})_2$, $-NR^{6b}S(=O)_2N(R^{7b})_2$, $-OS(=O)_2N(R^{7b})_2$, $-S(=O)_2R^{6c}$, $-CH_2S(=O)_2N(R^{7b})_2$, $-(CH_2)_2S(=O)_2N(R^{7b})_2$, $-CH_3$, $-CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-S(CH_2)_2CH_3$, $-SCH_2CH(CH_3)_2$, $-SCH(CH_3)_2$, $-OCH_3$, $-OCH_2CH_3$, $-O(CH_2)_2CH_3$, $-OCH_2CH(CH_3)_2$, $-OCH(CH_3)_2$, $-CH=CH_2$, $-CH=CHCH_3$, $-CH_2CH=CH_2$, $-C\equiv CH$, $-C\equiv CCH_3$, $-CH_2C\equiv CH$, $-CH_2CN$, $-(CH_2)_2CN$, $-(CH_2)_3CN$, $-CH_2OH$, $-(CH_2)_2OH$, $-(CH_2)_3OH$, $-CH(OH)CH_3$, $-CF_3$, $-CHF_2$, $-CH_2F$, $-(CH_2)_2F$, $-(CH_2)_2Cl$, $-CH_2CF_3$, $-NHCH_3$, $-NH(CH_2CH_3)$, $-NH((CH_2)_2CH_3)$, $-NH((CH_2)_3CH_3)$, $-NH(CH(CH_3)_2)$, $-N(CH_3)_2$, $-N(CH_2CH_3)_2$, $-N((CH_2)_2CH_3)_2$, $-CHFCH=CH_2$, -CH=CHF, -CH=CHCl, $-CH=CHCH_2F$, $-C=CCH_2F$, $-OCF_3$, $-OCH_2F$, $-OCHF_2$, $-OCH_2CF_3$, $-OCH_2CHF_2$, $-SCF_3$, $-SCH_2F$, $-SCHF_2$, $-SCH_2CF_3$, $-SCH_2CHF_2$, phenyl, furyl, imidazolyl, isoxazolyl, oxazolyl, pyrrolyl, pyrazolyl, pyridinyl, pyrimidinyl, pyridazinyl, pyrazinyl, thiophenyl, thiazolyl, triazolyl, tetrazolyl, benzopyridinyl, benzimidazolyl, benzopyrrolyl, benzopyrazolyl, cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein the $-CH_3$, $-CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-S(CH_2)_2CH_3$, $-SCH_2CH(CH_3)_2$, $-SCH(CH_3)_2$, $-OCH_3$, $-OCH_2CH_3$, $-O(CH_2)_2CH_3$, $-OCH_2CH(CH_3)_2$, $-OCH(CH_3)_2$, $-CH=CH_2$, $-CH=CHCH_3$, $-CH_2CH=CH_2$, $-C\equiv CH$, $-C\equiv CCH_3$, $-CH_2C\equiv CH$, $-CH_2CN$, $-(CH_2)_2CN$, $-(CH_2)_3CN$, $-CH_2OH$, $-(CH_2)_2OH$, $-(CH_2)_3OH$, $-CH(OH)CH_3$, $-CHF_2$, $-CH_2F$, $-(CH_2)_2F$, $-(CH_2)_2Cl$, $-CH_2CF_3$, $-NHCH_3$, $-NH(CH_2CH_3)$, $-NH((CH_2)_2CH_3)$, $-NH((CH_2)_3CH_3)$, $-NH(CH(CH_3)_2)$, $-N(CH_3)_2$, $-N(CH_2CH_3)_2$, $-N((CH_2)_2CH_3)_2$, $-CHFCH=CH_2$, -CH=CHF, -CH=CHCl, $-CH=CHCH_2F$, $-C=CCH_2F$, $-OCH_2F$, $-OCHF_2$, $-OCH_2CF_3$, $-OCH_2CHF_2$, $-SCH_2F$, $-SCHF_2$, $-SCH_2CF_3$, $-SCH_2CHF_2$, phenyl, furyl, imidazolyl, isoxazolyl, oxazolyl, pyrrolyl, pyrazolyl, pyridinyl, pyrimidinyl, pyridazinyl, pyrazinyl, thiophenyl, thiazolyl, triazolyl, tetrazolyl, benzopyridinyl, benzimidazolyl, benzopyrrolyl, benzopyrazolyl, cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl are each independently optionally substituted with 1, 2, 3 or 4 $R^8$;

or, two $R^5$ attached to the same carbon atom together form

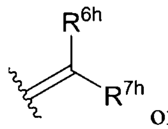 or 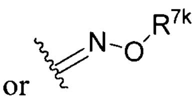 ;

or, two $R^5$ attached to the same carbon atom, together with the carbon atom to which they are attached, form a cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein the cyclopropyl, cyclobutyl, cyclopentyl, cyclohexyl, oxiranyl, aziridinyl, azetidinyl, oxetanyl, thietanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl are each independently optionally substituted with 1, 2, 3 or 4 $R^8$;

or, two $R^5$ attached to two adjacent atoms together with the two adjacent atoms to which they are attached, form a cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl or morpholinyl; wherein the cyclopropyl, cyclobutyl, cyclopentyl, cyclohexyl, oxiranyl, aziridinyl, azetidinyl, oxetanyl, pyrrolidinyl, tetrahydrofuranyl, tetrahydrothiophenyl, thiazolidinyl, pyrazolidinyl, pyrazolinyl, oxazolidinyl, imidazolidinyl, piperidyl, piperazinyl and morpholinyl are each independently optionally substituted with 1, 2, 3 or 4 $R^8$;

each $R^8$ is independently -D, -OH, -F, -Cl, -Br, -I, -CN, $-NH_2$, -C(=O)OH, $-C(=O)NH_2$, oxo, $-CH_3$, $-CH_2CH_3$, $-(CH_2)_2CH_3$, $-(CH_2)_3CH_3$, $-CH(CH_3)_2$, $-CH_2CH(CH_3)_2$, $-C(CH_3)_3$, $-OCH_3$, $-OCH_2CH_3$, $-O(CH_2)_2CH_3$, $-CH_2OH$, $-(CH_2)_2OH$, $-(CH_2)_3OH$, $-CH(OH)CH_3$, phenyl, furyl, imidazolyl, isoxazolyl, oxazolyl, pyrrolyl, pyrazolyl, pyridinyl, pyrimidinyl, pyridazinyl, pyrazinyl, thiophenyl, thiazolyl, triazolyl, tetrazolyl, benzopyridinyl, benzimidazolyl,

benzopyrrolyl, benzopyrazolyl, $-C(=O)OCH_3$, $-C(=O)OCH_2CH_3$, $-C(=O)O(CH_2)_2CH_3$, $-C(=O)OCH(CH_3)_2$, $-C(=O)NHCH_3$, $-C(=O)NHCH_2CH_3$, $-C(=O)N(CH_3)_2$ or $-C(=O)N(CH_3)CH_2CH_3$;
$R^{6h}$, $R^{7h}$ and $R^{7k}$ are each independently -H, -D, methyl, ethyl, *n*-propyl, isopropyl, *n*-butyl, isobutyl or *tert*-butyl.

**6.** The compound of any one of claims 1-5, wherein, ring B is one of the following sub-structures,

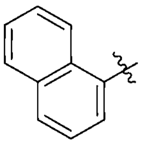, 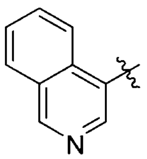, 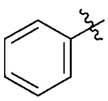, 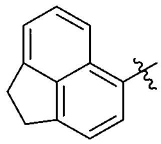, 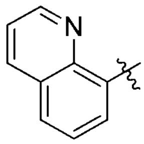, 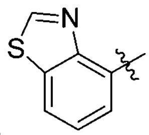, 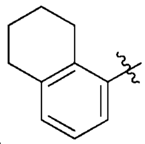, 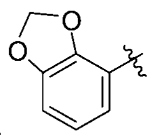,

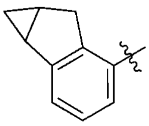, 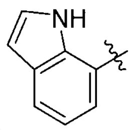, 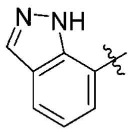, 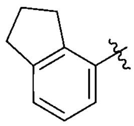, 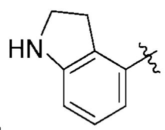, 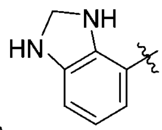, 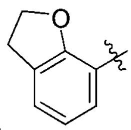, 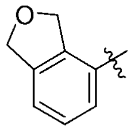,

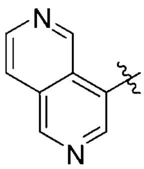, 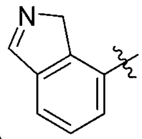, 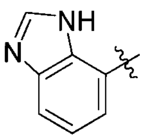, 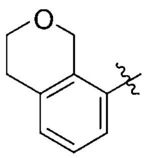, 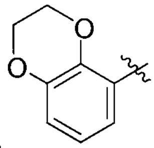 or 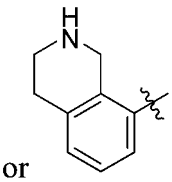;

each $R^2$ is independently -D, -OH, -F, -Cl, -Br, -I, -CN, -SH, $-CH_2C(=O)NR^{6b}R^{7b}$, $-C(=O)R^{6c}$, $-C(=O)OR^{6c}$, $-C(=O)NR^{6b}R^{7b}$, $-NR^{6b}C(=O)R^{7b}$, $-NR^{6b}R^{7b}$, $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, $C_{6-10}$ aryl, 5-12 membered heteroaryl, $C_{3-6}$ cycloalkyl or 3-6 membered heterocyclyl; wherein the $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, $C_{6-10}$ aryl, 5-12 membered heteroaryl, $C_{3-6}$ cycloalkyl and 3-6 membered heterocyclyl are each independently optionally substituted with 1, 2, 3 or 4 substituents selected from -D, -OH, -F, -Cl, -Br, -I, -CN, $-NH_2$, -C(=O)H, -C(=O)OH, $C_{1-4}$ alkyl, $C_{1-4}$ alkoxy, $C_{3-6}$ cycloalkyl and 3-6 membered heterocyclyl.

**7.** The compound of any one of claims 1-6, wherein,
each $R^2$ is independently-D, -OH, -F, -Cl, -Br, -I, -CN, -SH, $-CH_2C(=O)NR^{6b}R^{7b}$, $-C(=O)R^{6c}$, $-C(=O)OR^{6c}$, $-C(=O)NR^{6b}R^{7b}$, $-NR^{6b}C(=O)R^{7b}$, $-NR^{6b}R^{7b}$, $-CH_3$, $-CH_2CH_3$, -(CH2)2CH3, $-(CH_2)_3CH_3$, $-C(CH_3)_3$, $-CH(CH_3)_2$, $-SCH_3$, $-SCH_2CH_3$, $-CH=CH_2$, $-CH=CHCH_3$, $-CH_2CH=CH_2$, -C≡CH, $-C=CCH_3$, $-CH_2C≡CH$, $-C≡CCH_2OH$, $-C≡C(CH_2)_2OH$, $-OCH_3$, $-OCH_2CH_3$, $-O(CH_2)_2CH_3$, $-CH_2CN$, $-(CH_2)_2CN$, $-(CH_2)_3CN$, $-CH_2OH$, $-(CH_2)_2OH$, $-(CH_2)_3OH$, $-CH(OH)CH_3$, $-(CH_2)_2F$, $-CH_2CHF_2$, $-CF_3$, $-CH_2CF_3$, $-CHF_2$, $-CH_2F$, $-(CH_2)_2Cl$, -CH=CHF, -CH=CHCl, $-CH=CHCH_2F$, $-C≡CCH_2F$, $-C≡C(CH_2)_2F$, -C=CF, $-OCF_3$, $-OCHF_2$, $-OCH_2CHF_2$, $-OCH_2CF_3$, $-OCHClCHCl_2$, $-OCH_2CH_2F$, $-SCF_3$, $-SCH_2CF_3$, $-SCH_2CHF_2$, phenyl, naphthyl, pyridinyl, pyrimidinyl, cyclopropyl, cyclobutyl, cyclopentyl, cyclohexyl, pyrrolidinyl, oxazolidinyl, tetrahydrofuranyl, piperidyl or piperazinyl, wherein the-$CH_3$, $-CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-CH=CH_2$, $-CH=CHCH_3$, $-CH_2CH=CH_2$, -C≡CH, $-C≡CCH_3$, $-CH_2C≡CH$, $-C≡CCH_2OH$, $-C≡C(CH_2)_2OH$, $-OCH_3$, $-OCH_2CH_3$, $-O(CH_2)_2CH_3$, $-CH_2CN$, $-(CH_2)_2CN$, $-(CH_2)_3CN$, $-CH_2OH$, $-(CH_2)_2OH$, $-(CH_2)_3OH$, $-CH(OH)CH_3$, $-(CH_2)_2F$, $-CH_2CHF_2$, $-CH_2CF_3$, $-CHF_2$, $-CH_2F$, $-(CH_2)_2Cl$, -CH=CHF, -CH=CHCl, $-CH=CHCH_2F$, $-C≡CCH_2F$, $-C≡C(CH_2)_2F$, $-OCHF_2$, $-OCH_2CHF_2$, $-OCH_2CF_3$, $-OCHClCHCl_2$, $-OCH_2CH_2F$, $-SCH_2CF_3$, $-SCH_2CHF_2$, phenyl, naphthyl, pyridinyl, pyrimidinyl, cyclopropyl, cyclobutyl, cyclopentyl, cyclohexyl, pyrrolidinyl, oxazolidinyl, tetrahydrofuranyl, piperidyl and piperazinyl are each independently optionally substituted with 1, 2, 3 or 4 substituents selected from -D, -OH, -F, -Cl, -Br, -I, -CN, $-NH_2$, -C(=O)H, -C(=O)OH, methyl, ethyl, n-propyl, isopropyl, methoxy, ethoxy, n-propoxy, i-propoxy, cyclopropyl, cyclobutyl, cyclopentyl, cyclohexyl, pyrrolidinyl, oxazolidinyl, tetrahydrofuranyl, piperidyl or piperazinyl.

**8.** The compound of any one of claims 1-7, wherein,

$R^4$ is a 3-6 membered heterocyclyl,

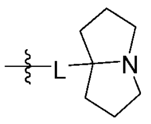, 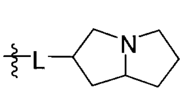, 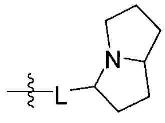, 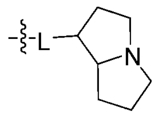, 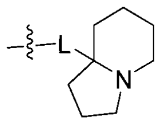, 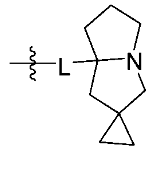,

-L-pyrrolidinyl, -L-piperidyl, -L-morpholinyl, -L-oxetanyl, -L-oxiranyl, -L-tetrahydrofuranyl, -L-octahydroindolizinyl, -L-cyclopropyl, -L-cyclopentyl, -L-octahydropentalenyl, -L-octahydro-1*H*-indenyl, -L-decalinyl, -L-pyridinyl, -L-pyrazolyl or -L-phenyl; wherein the 3-6 membered heterocyclyl, -L-cyclopropyl, -L-cyclopentyl, -L-octahydropentalenyl, -L-octahydro-1*H*-indenyl, -L-decalinyl, -L-pyridinyl, -L-pyrazolyl and -L-phenyl are each independently optionally substituted with 1, 2, 3 or 4 $R^{9a}$; and

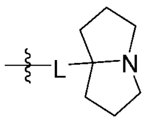, 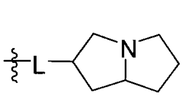, 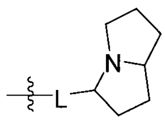, 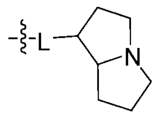, 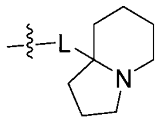, 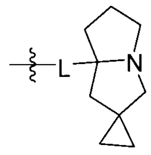,

-L-pyrrolidinyl, -L-piperidinyl, -L-morpholinyl, -L-oxetanyl, -L-oxiranyl, -L-tetrahydrofuranyl and -L-octahydroindolizinyl are each optionally substituted with 1, 2, 3 or 4 $R^{9b}$;

$R^{9a}$ and $R^{9b}$ are each independently -D, -OH, -F, -Cl, -Br, -I, -CN, $-NR^{6d}R^{7d}$, $-C(=O)NR^{6d}R^{7d}$, $-CH_2NR^{6d}R^{7d}$, $-CH_2OC(=O)NR^{6d}R^{7d}$, $C_{1-4}$ alkyl, $C_{1-4}$ alkoxy, $C_{1-4}$ haloalkyl, $C_{1-4}$ haloalkoxy, phenyl-$C_{1-4}$ alkyl, (5-6 membered heteroaryl)-$C_{1-4}$ alkyl, (3-6 membered heterocyclyl)-$C_{1-4}$ alkyl, ($C_{3-6}$ cycloalkyl)-$C_{1-4}$ alkyl, $C_{3-6}$ cycloalkyl or 3-6 membered heterocyclyl; wherein the $C_{1-4}$ alkyl, $C_{1-4}$ alkoxy, $C_{1-4}$ haloalkyl, $C_{1-4}$ haloalkoxy, phenyl-$C_{1-4}$ alkyl, (5-6 membered heteroaryl)-$C_{1-4}$ alkyl, (3-6 membered heterocyclyl)-$C_{1-4}$ alkyl, ($C_{3-6}$ cycloalkyl)-$C_{1-4}$ alkyl, $C_{3-6}$ cycloalkyl and 3-6 membered heterocyclyl are each independently optionally substituted with 1, 2, 3 or 4 substituents selected from -D, -F, -Cl, -Br, -I, -OH, -CN, $-NR^{6e}R^{7e}$, $-C(=O)C_{1-4}$ alkyl and $C_{1-4}$ alkyl; or,

two $R^{9b}$ attached to the same carbon atom together form

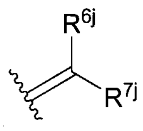;

L is $C_{1-4}$ alkylene;

$R^{6j}$ and $R^{7j}$ are each independently -H, -D or $C_{1-4}$ alkyl.

**9.** The compound of any one of claims 1-8, wherein,

$R^4$ is piperidyl, piperazinyl, pyrrolidinyl, imidazolidinyl,

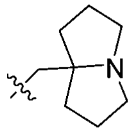, 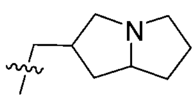, 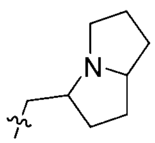, 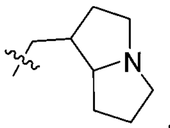, 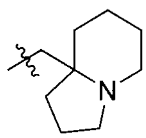, 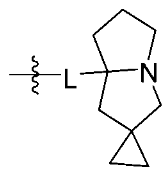,

$-CH_2$-pyrrolidinyl, $-CH_2$-morpholinyl, $-(CH_2)_2$-morpholinyl, $-CH_2$-oxetanyl, $-CH_2$-oxiranyl, $-CH_2$-tetrahydrofuranyl, $-CH_2$-octahydroindolizinyl, $-CH_2$-cyclopropyl, $-CH_2$-cyclopentyl, $-CH_2$-octahydropentalenyl, $-CH_2$-octahydro-1H-indenyl, $-CH_2$-decahydronaphthalenyl, $-CH_2$-pyridinyl, $-(CH_2)_2$-pyridinyl, $-CH_2$-pyrazolyl, $-(CH_2)_2$-pyrazolyl or $-CH_2$-phenyl, wherein the piperidyl, piperazinyl, pyrrolidinyl, imidazolidinyl, $-CH_2$-cyclopropyl, $-CH_2$-cyclopentyl, $-CH_2$-octahydropentalenyl, $-CH_2$-octahydro-1H-indenyl, $-CH_2$-decahydronaphthalenyl, $-CH_2$-pyridinyl, $-(CH_2)_2$-pyridinyl, $-CH_2$-pyrazolyl, $-(CH_2)_2$-pyrazolyl and $-CH_2$-phenyl are each independently optionally substituted with 1, 2, 3 or 4 $R^{9a}$; and the

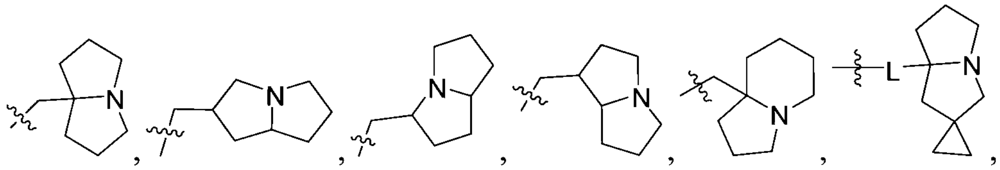
, , , , , ,

$-CH_2$-pyrrolidinyl, $-CH_2$-morpholinyl, $-(CH_2)_2$-morpholinyl, $-CH_2$-oxetanyl, $-CH_2$-oxiranyl, $-CH_2$-tetrahydrofuranyl and $-CH_2$-octahydroindolizinyl are each optionally substituted with 1, 2, 3 or 4 $R^{9b}$;
$R^{9a}$ and $R^{9b}$ are each independently -D, -OH, -F, -Cl, -Br, -I, -CN, $-NR^{6d}R^{7d}$ $-C(-O)NR^{6d}R^{7d}$, $-CH_2NR^{6d}R^{7d}$, $-CH_2OC(=O)NR^{6d}R^{7d}$, methyl, ethyl, n-propyl, isopropyl, n-butyl, isobutyl, ***tert-butyl,*** methoxy, ethoxy, isopropoxy, $-CHF_2$, $-CF_3$, $-OCF_3$, benzyl, pyridinylmethyl, pyrazolylmethyl, morpholinylmethyl, pyrrolidinylmethyl, piperazinylmethyl, azetidinylmethyl, piperidinylmethyl, tetrahydropyranylmethyl, cyclopropylmethyl, cyclopentylmethyl, cyclohexylmethyl, cyclonentyl, cyclohexyl, morpholinyl, piperidinyl, pyrrolidinyl, piperazinyl or azetidinyl, wherein the methyl, ethyl, *n*-propyl, isopropyl, *n*-butyl, isobutyl, *tert*-butyl*,* methoxy, ethoxy, isopropoxy, $-CHF_2$, benzyl, pyridinylmethyl, pyrazolylmethyl, morpholinylmethyl, pyrrolidinylmethyl, piperazinylmethyl, azetidinylmethyl, piperidinylmethyl, tetrahydropyranylmethyl, cyclopropylmethyl, cyclopentylmethyl, cyclohexylmethyl, cyclopentyl, cyclohexyl, morpholinyl, piperidinyl, pyrrolidinyl, piperazinyl and azetidinyl are each independently optionally substituted with 1, 2, 3 or 4 substituents selected from -D, -F, -Cl, -Br, -I, -OH, -CN, $-NH_2$, $-NHCH_3$, $-N(CH_3)_2$, $-NHCH_2CH_3$, $-C(=O)CH_3$, $-C(=O)CH_2CH_3$, methyl, ethyl, *n*-propyl and isopropyl;
or,
two $R^{9b}$ attached to the same carbon atom together form

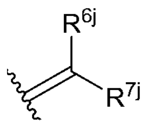
;

$R^{6j}$ and $R^{7j}$ are each independently -H, -D, methyl, ethyl, *n*-propyl, isopropyl, *n*-butyl, isobutyl or *tert*-butyl.

**10.** The compound of any one of claims 1-9, wherein, $R^6$, $R^7$, $R^{6b}$, $R^{7b}$, $R^{6d}$, $R^{7d}$, $R^{6e}$ and $R^{7e}$ are each independently -H, -D or $C_{1-4}$ alkyl, wherein the $C_{1-4}$ alkyl is optionally substituted with 1, 2, 3 or 4 substituents selected from -D, -OH, -F, -Cl, -Br, -I, -CN, -C(=O)H, -C(=O)OH, $-NR^{6g}R^{7g}$, $C_{1-4}$ alkoxy, $C_{6-10}$ aryl, $C_{3-6}$ cycloalkyl and 3-6 membered heterocyclyl;

or $R^6$ and $R^7$, or $R^{6b}$ and $R^{7b}$, or $R^{6d}$ and $R^{7d}$, or $R^{6e}$ and $R^{7e}$, independently together with the N atom to which they are attached form a 4-6 membered heterocyclic ring, wherein the 4-6 membered heterocyclic ring is optionally substituted with 1, 2, 3 or 4 substituents selected from -D, -OH, -F, -Cl, -Br, -I, -CN, $-NH_2$, $C_{1-4}$ alkyl, $C_{1-4}$ alkylamino, $C_{3-6}$ cycloalkyl, 3-6 membered heterocyclyl, $C_{1-4}$ alkoxy, $C_{1-4}$ cyanoalkyl, $C_{1-4}$ hydroxyalkyl, $C_{1-4}$ haloalkoxy and $C_{1-4}$ haloalkyl;
$R^{6a}$, $R^{6c}$, $R^{6g}$ and $R^{7g}$ are each independently -H, -D or $C_{1-4}$ alkyl.

**11.** The compound of any one of claims 1-10, wherein,

$R^6$, $R^7$, $R^{6b}$, $R^{7b}$ , $R^{6d}$, $R^{7d}$, $R^{6e}$ and $R^{7e}$ are each independently -H, -D, methyl, ethyl, *n*-propyl, isopropyl, *n*-butyl, isobutyl or *tert*-butyl, wherein the methyl, ethyl, *n*-propyl, isopropyl, *n*-butyl, isobutyl and *tert*-butyl are each independently optionally substituted with 1, 2, 3 or 4 substituents selected from -D, -OH, -F, -Cl, -Br, -I, -CN, C(=O)H, -C(=O)OH, $-NR^{6g}R^{7g}$, methoxy, ethoxy, *n*-propoxy, isopropoxy, isobutoxy, cyclopropyl, cyclobutyl, cyclopentyl, cyclohexyl, phenyl, oxiranyl, oxetanyl, azetidinyl and pyrrolidinyl;
or $R^6$ and $R^7$, or $R^{6b}$ and $R^{7b}$, or $R^{6d}$ and $R^{7d}$, or $R^{6e}$ and $R^{7e}$, together with the nitrogen atom to which they are attached form pyrrolidine, piperazine, piperidine, morpholinyl, oxazolidine or imidazolidine, wherein the pyrrolidine, piperazine, piperidine, morpholinyl, oxazolidine and imidazolidine are each independently optionally substituted with 1, 2, 3 or 4 substituents selected from -D, -OH, -F, -Cl, -Br, -I, -CN, $-NH_2$, methyl, ethyl, *n*-propyl, isopropyl, *n*-butyl, isobutyl, *tert*-butyl, methylamino, dimethylamino, ethylamino, cyclopropyl, cyclopentyl, pyrrolidinyl, methoxy, ethoxy, isopropoxy, cyanomethyl, hydroxymethyl, hydroxyethyl, trifluoromethoxy, fluoromethyl, difluoromethyl, trifluoromethyl or 1,2-dichloroethyl;
$R^{6a}$, $R^{6c}$, $R^{6g}$ and $R^{7g}$ are each independently -H, -D, methyl, ethyl, *n*-propyl, isopropyl, *n*-butyl, isobutyl or *tert*-butyl.

**12.** The compound of any one of claims 1-11 having Formula (I-1) or a stereoisomer, a tautomer, an *N*-oxide, a solvate, a metabolite, a pharmaceutically acceptable salt or a prodrug thereof,

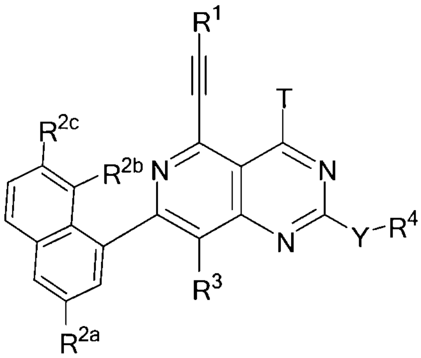

(I-1),

wherein $R^1$, $R^3$, $R^4$, Y and T are each as defined in any one of claims 1-11; $R^{2a}$, $R^{2b}$ and $R^{2c}$ are each as defined for $R^2$ in any one of claims 1-11.

**13.** The compound of any one of claims 1-12 having the following structure or a stereoisomer, a tautomer, an *N*-oxide, a solvate, a metabolite, a pharmaceutically acceptable salt or a prodrug thereof,

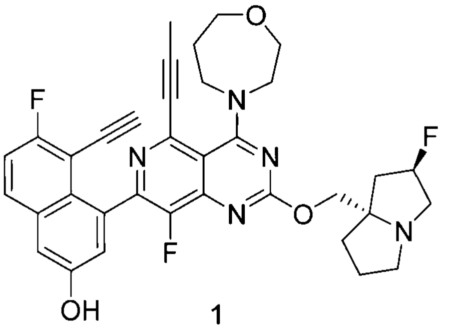

1,

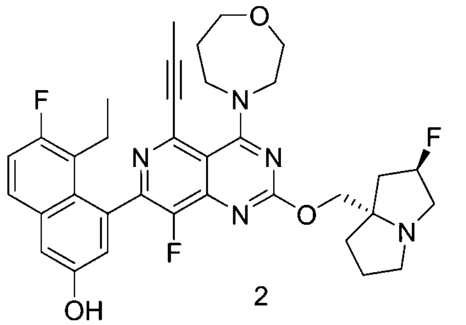

2,

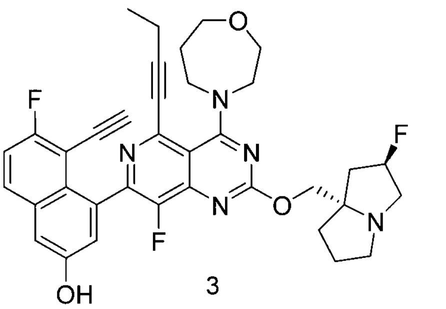

3,

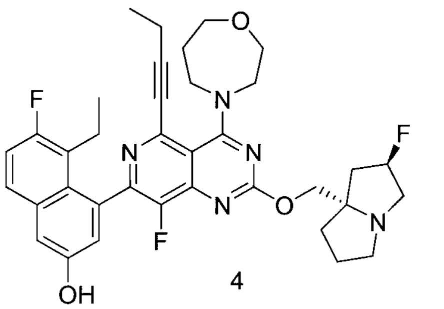

4,

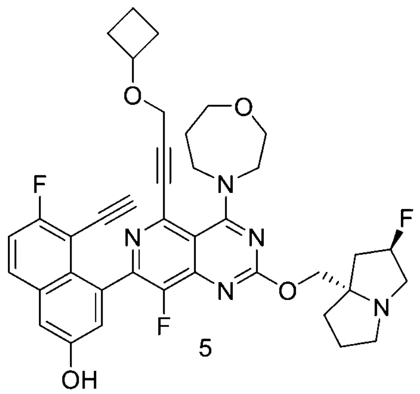
5
,
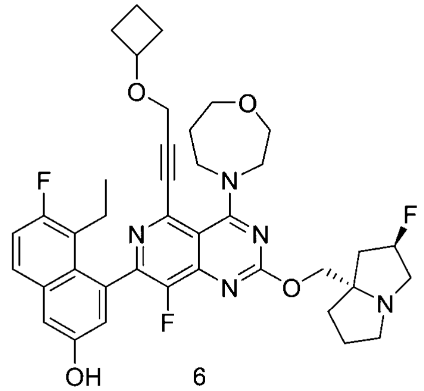
6
,
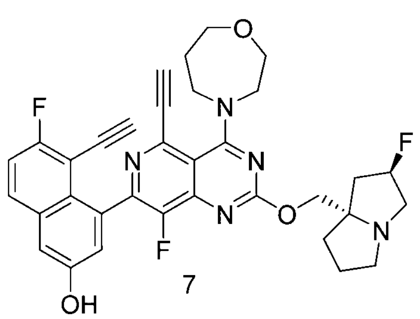
7
,
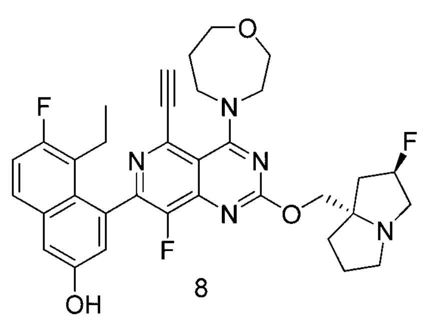
8
,
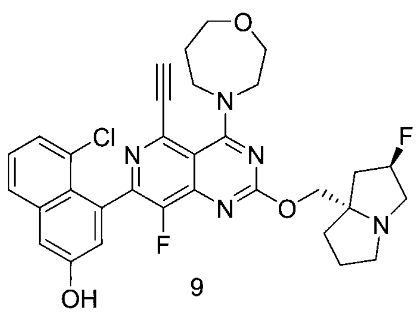
9
,
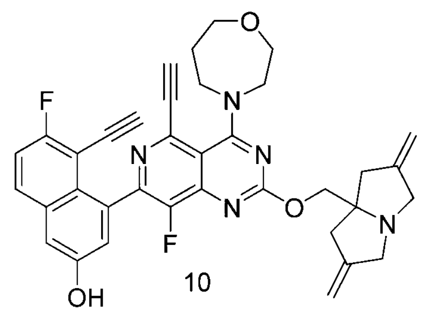
10
,
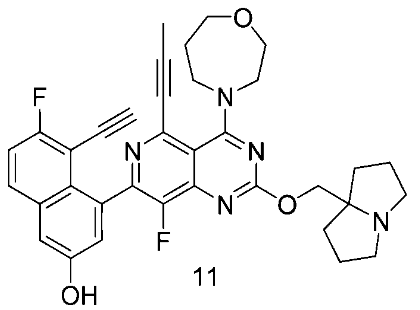
11
,
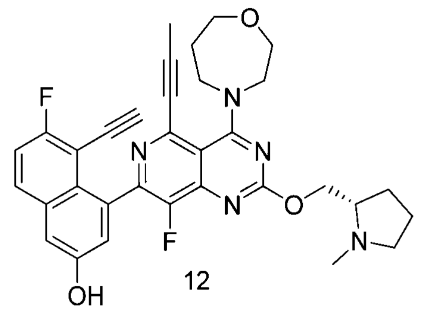
12
,

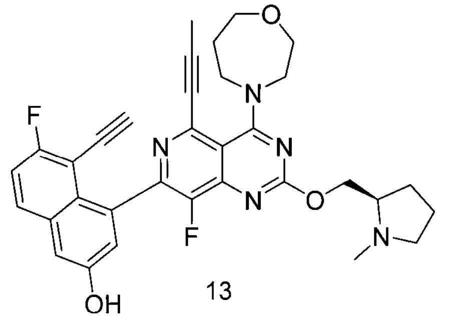
13 ,

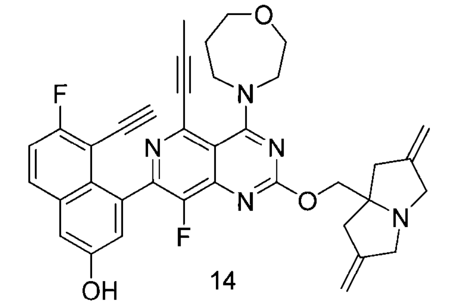
14 ,

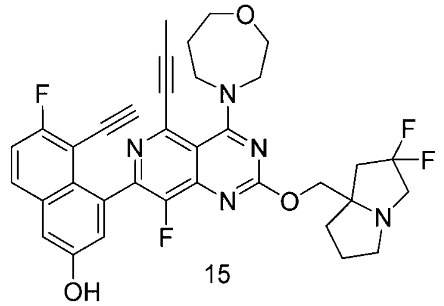
15 ,

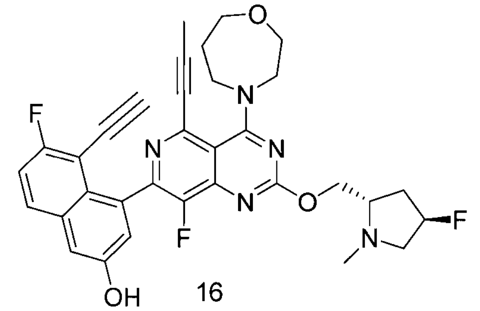
16 ,

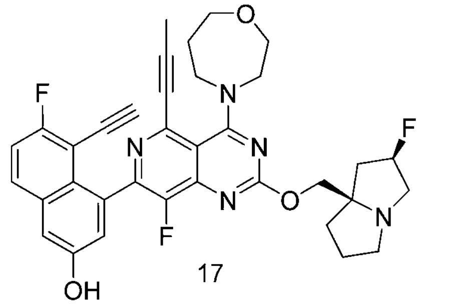
17 ,

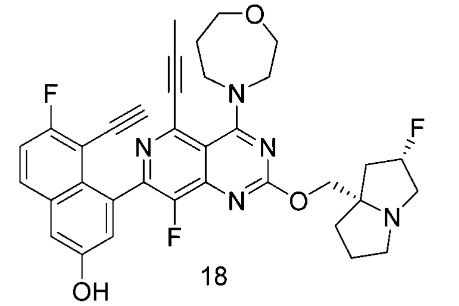
18 ,

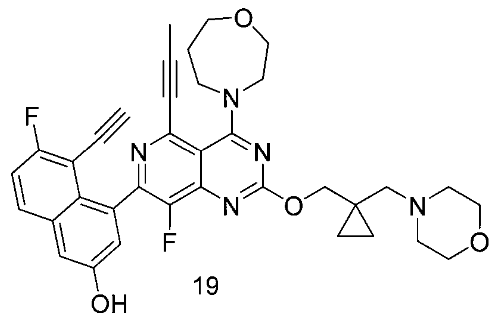
19 ,

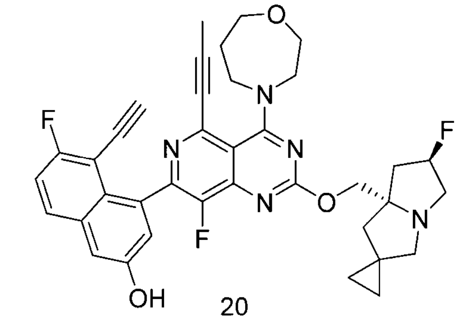
20 ,

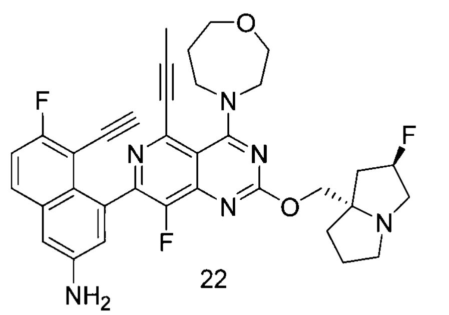
22 ,

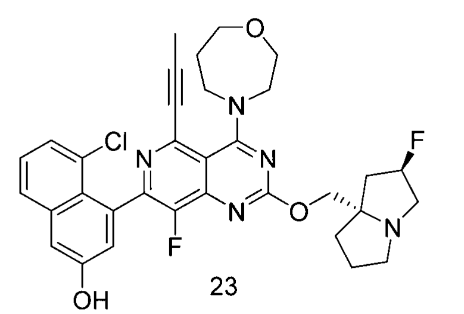
23 ,

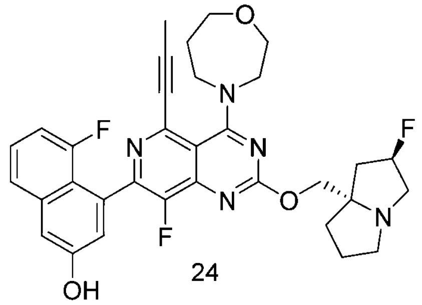
24
,

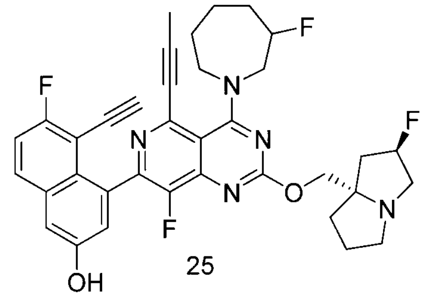
25
,

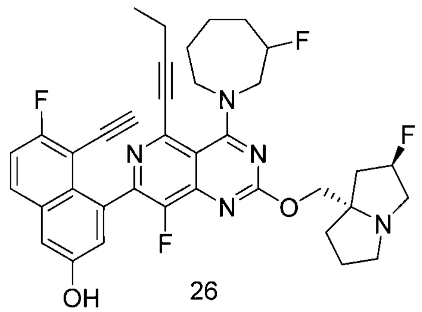
26
,

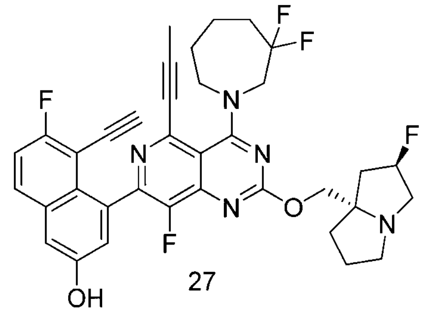
27
,

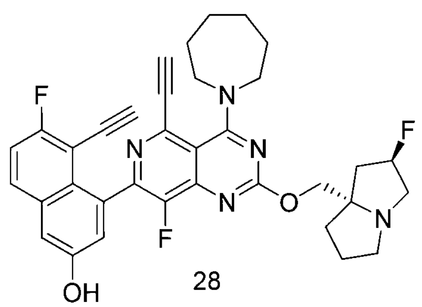
28
,

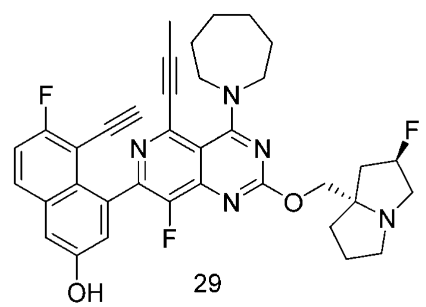
29
,

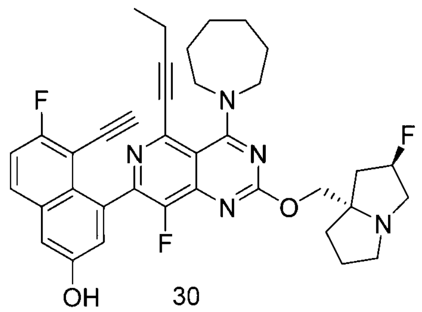
30
,

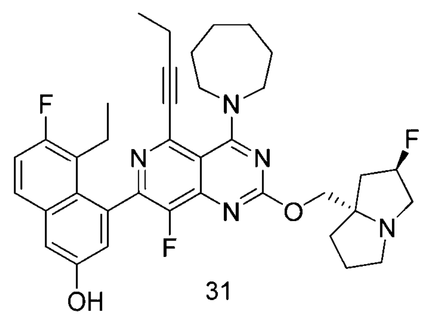
31
,

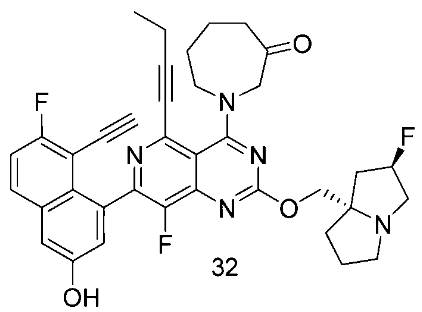
32
,

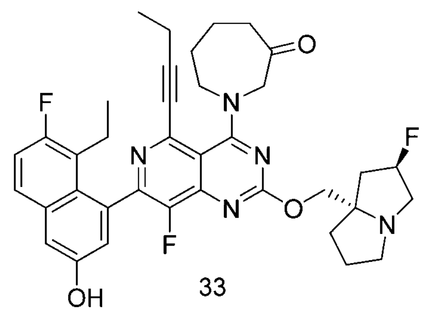
33
,

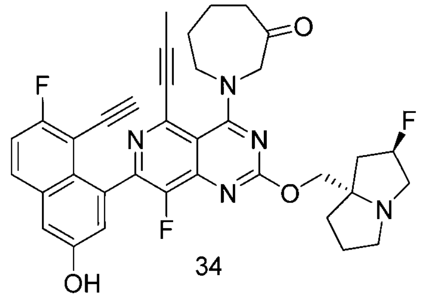
34
,

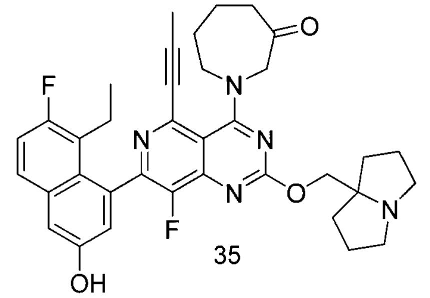
35
,

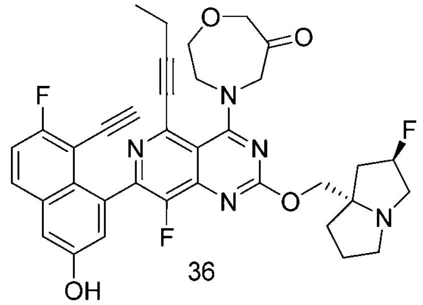
36
,

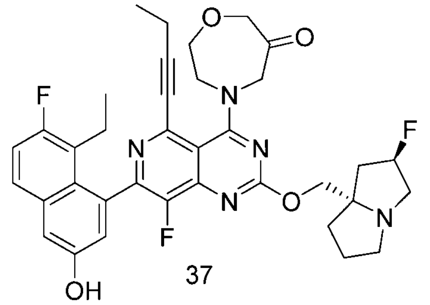
37
,

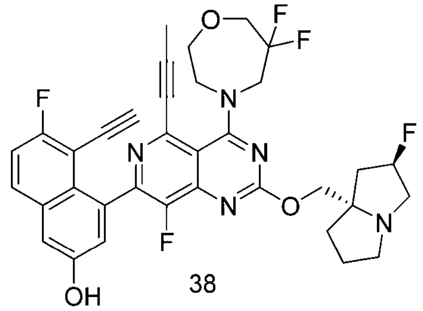
38
,

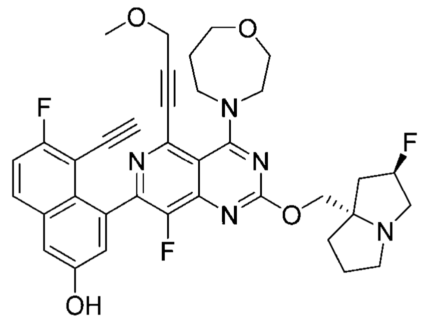
39
,

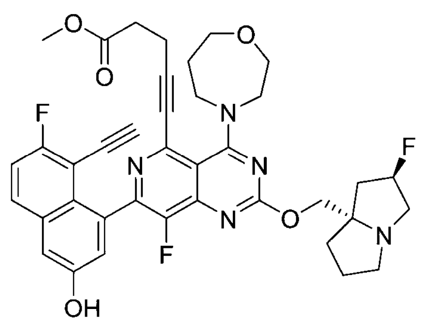
40
,

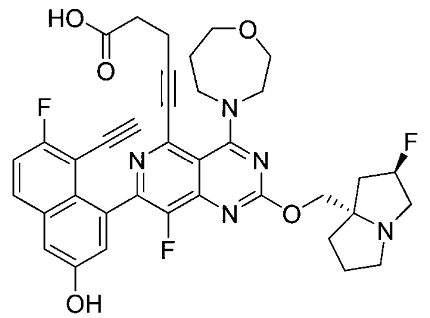
41
,

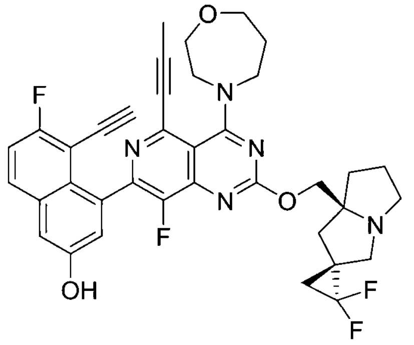

42 ,

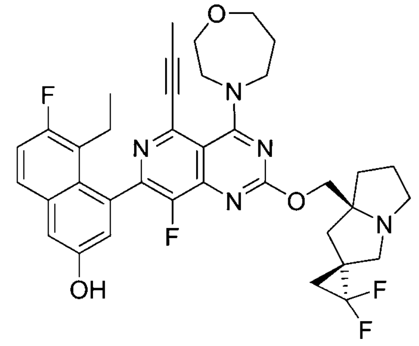

43 ,

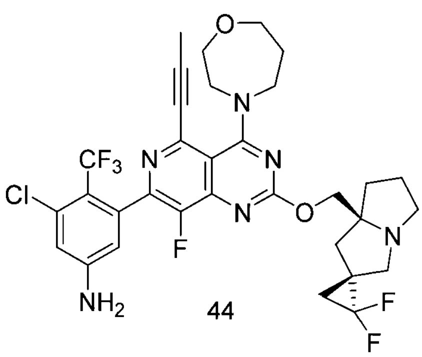

44 ,

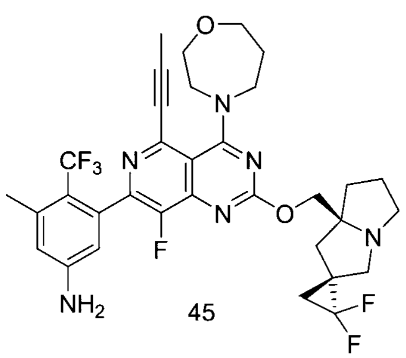

45 ,

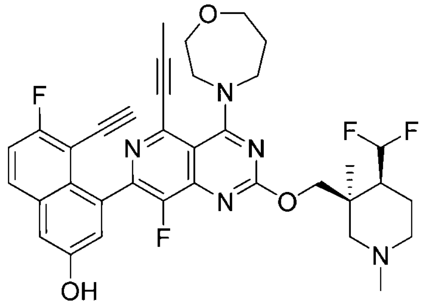

46 ,

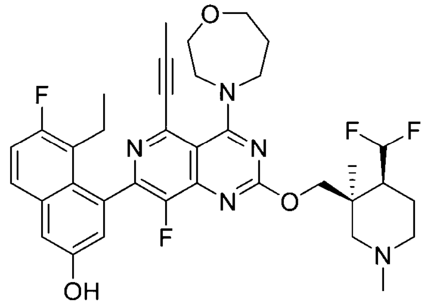

47 ,

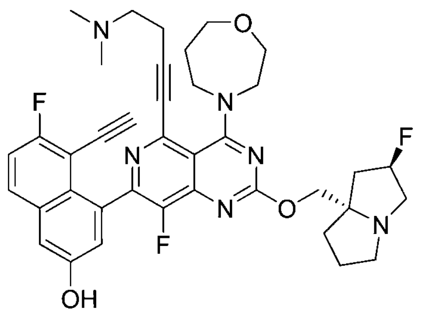

48 ,

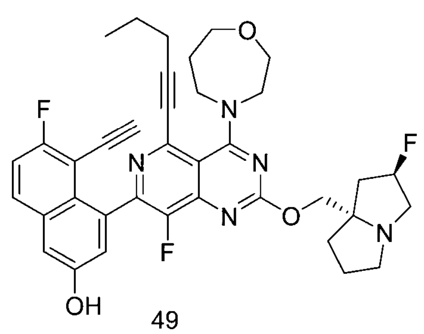

49 ,

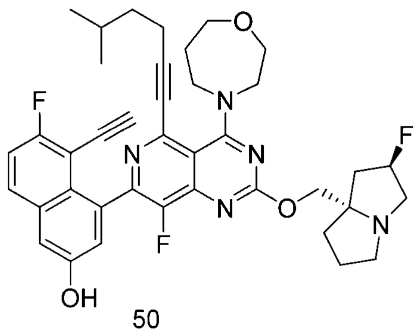

50

,

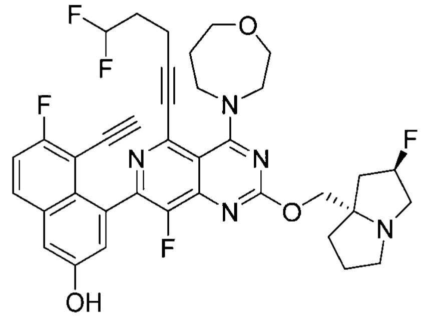

51

,

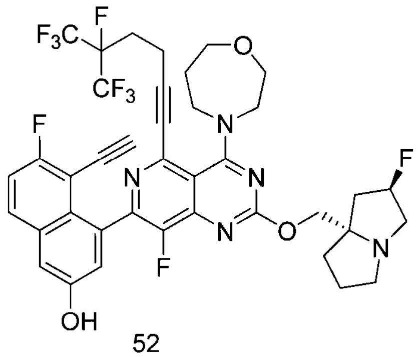

52

,

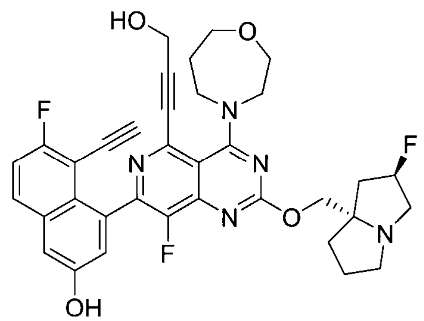

53

,

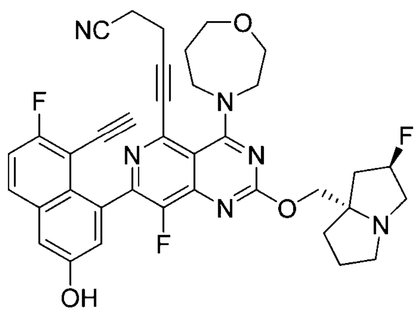

54

,

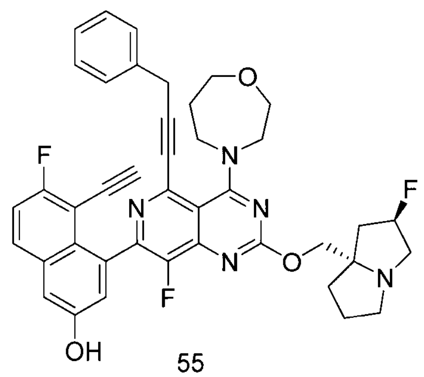

55

,

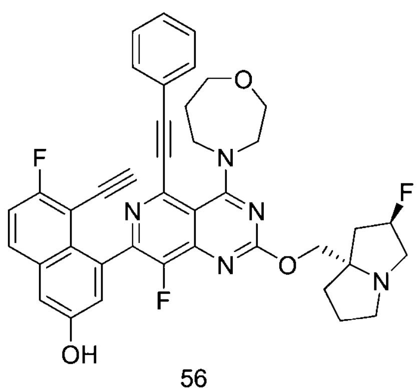

56

,

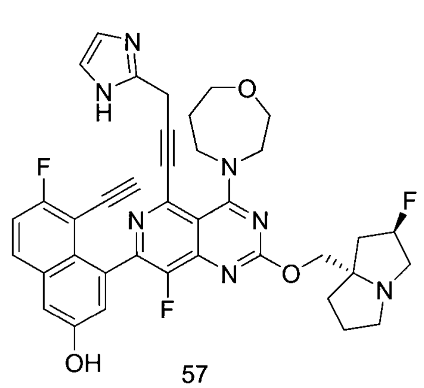

57

,

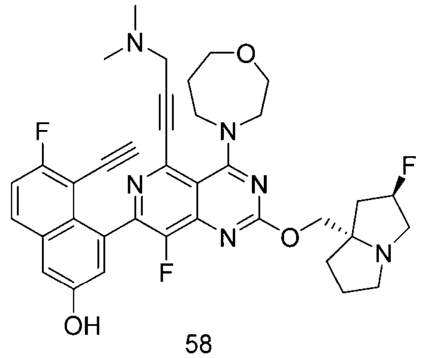

58

,

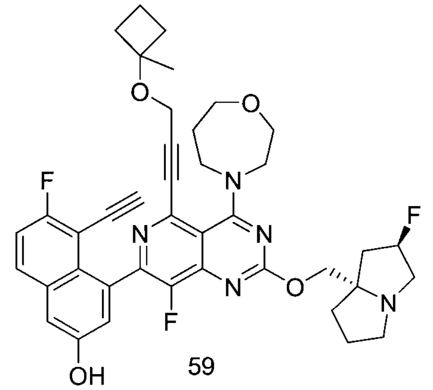

59

,

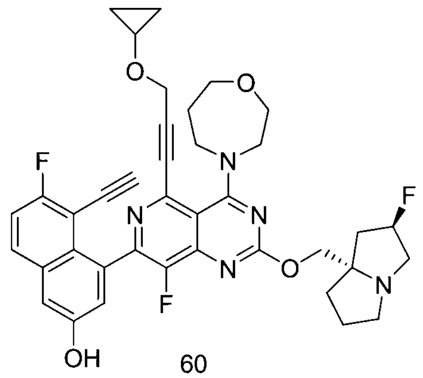

60

,

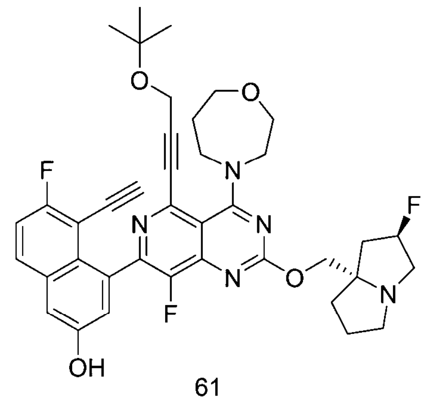

61

,

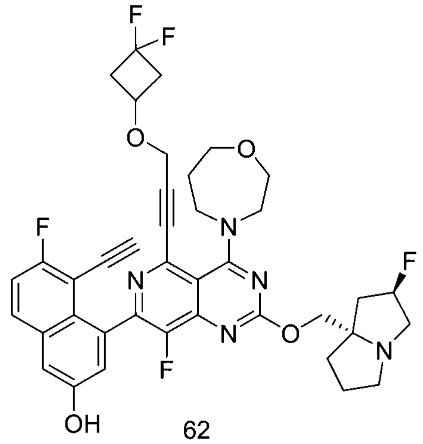

62

,

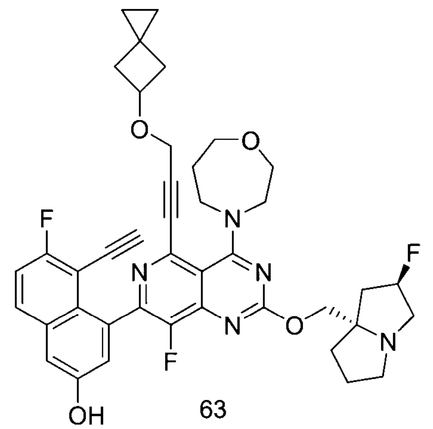

63

,

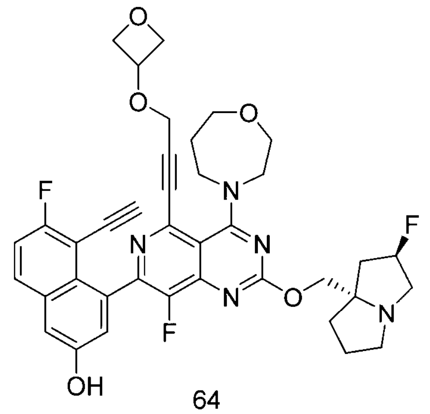

64 ,

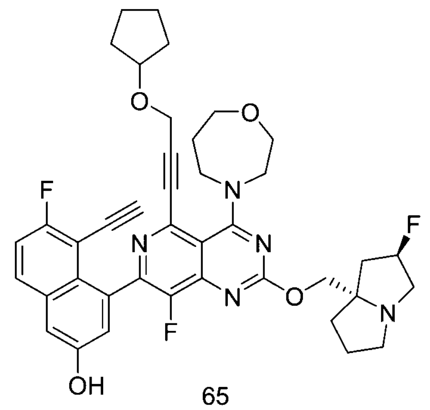

65 ,

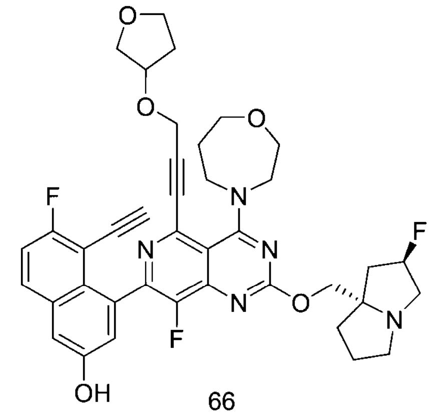

66 ,

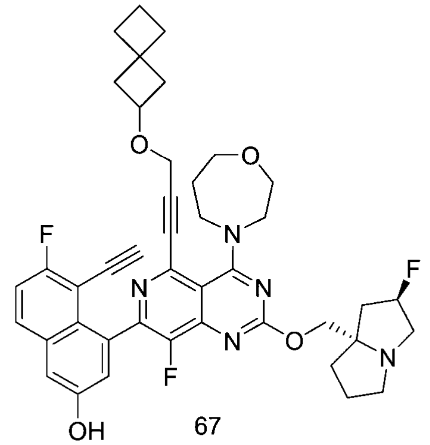

67 ,

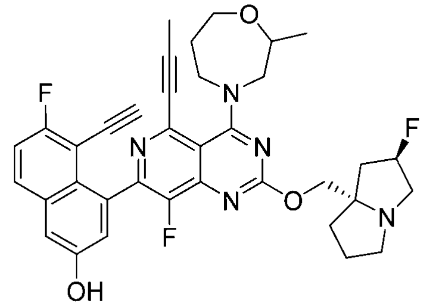

68 ,

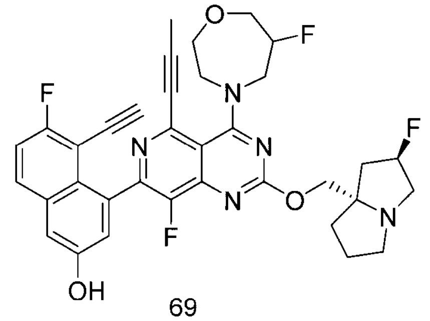

69 ,

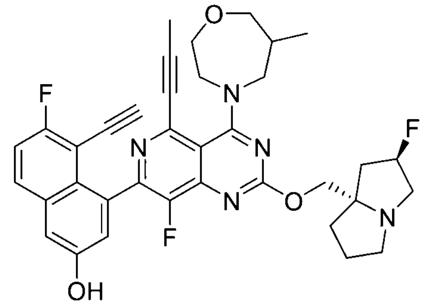

70 ,

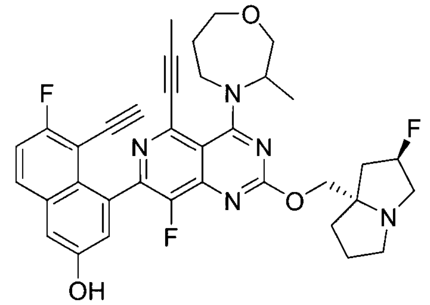

71 ,

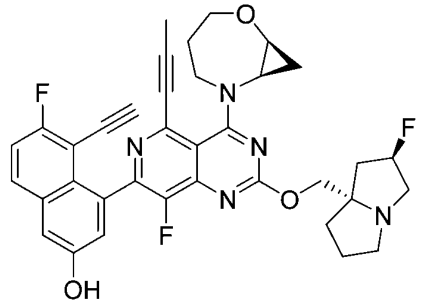

72

,

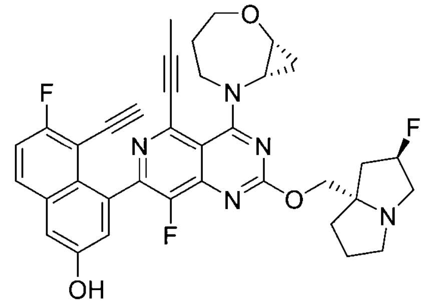

73

,

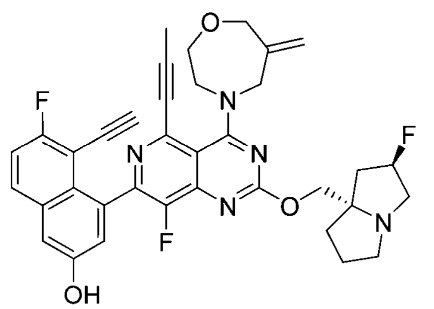

74

,

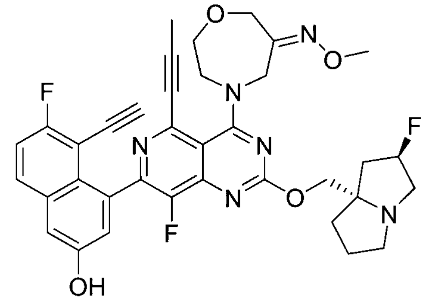

75

,

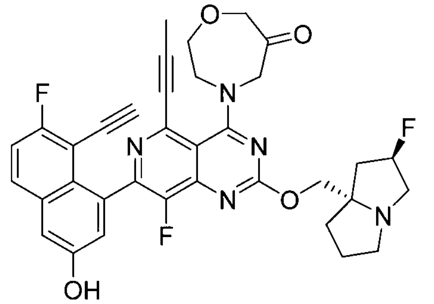

76

,

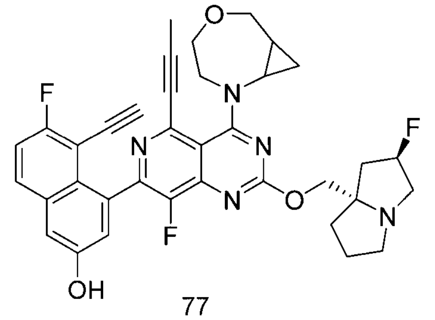

77

,

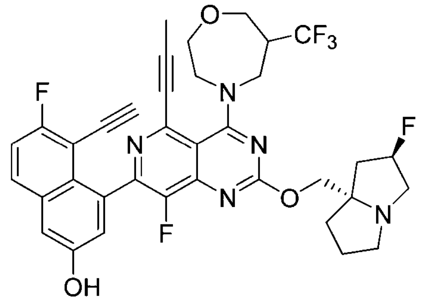

78

,

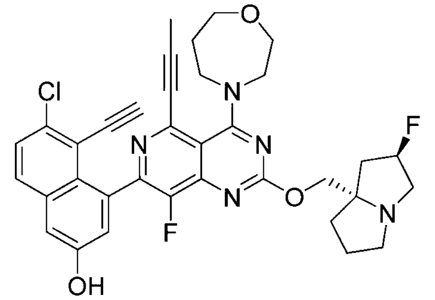

79

,

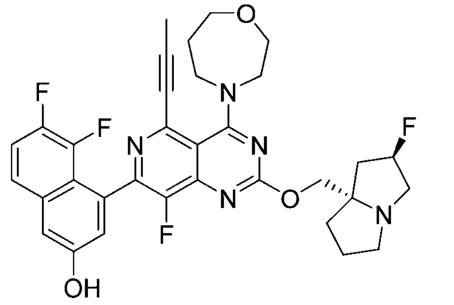

80 ,

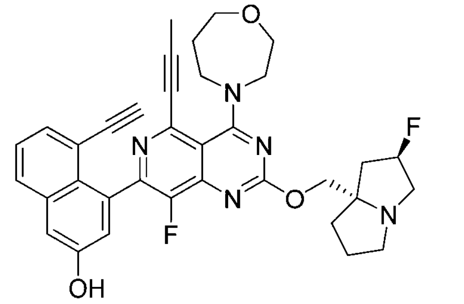

81 ,

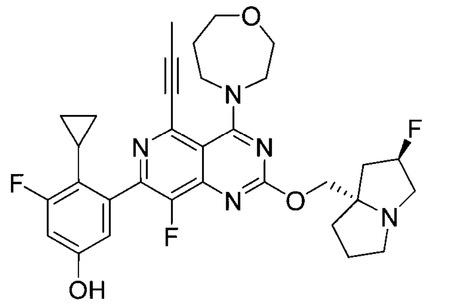

82 ,

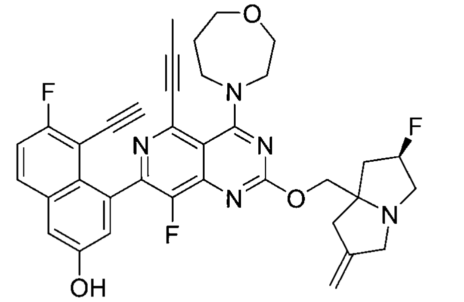

83 ,

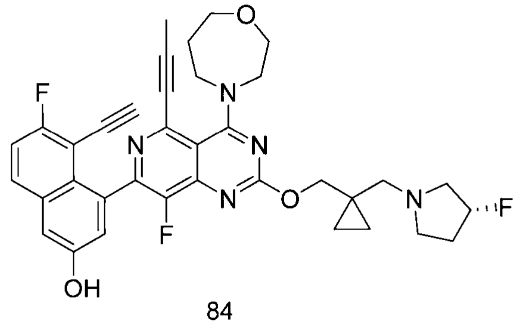

84 ,

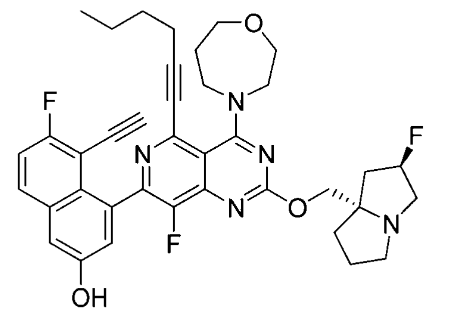

85 ,

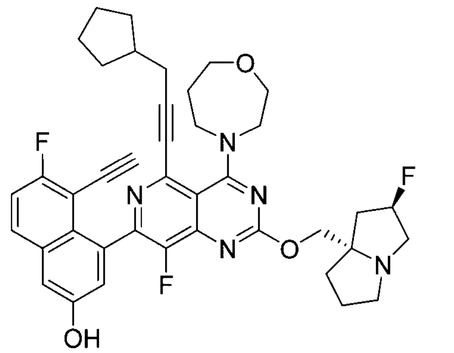

86 ,

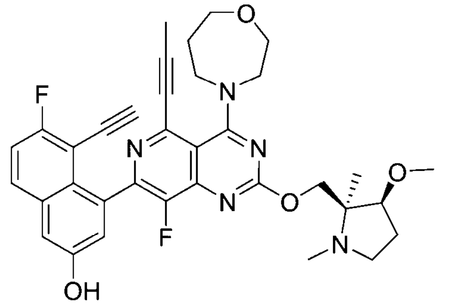

87 or

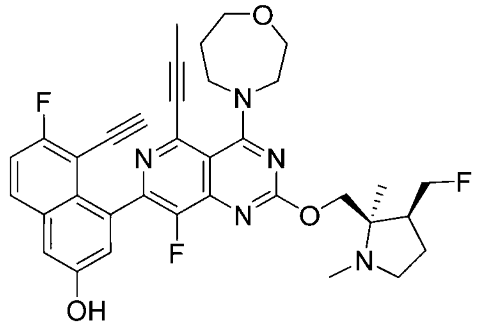

88

.

**14.** A pharmaceutical composition comprising the compound of any one of claims 1-13; optionally, the pharmaceutical composition further comprises a pharmaceutically acceptable adjuvant.

**15.** Use of the compound of any one of claims 1-13 or the pharmaceutical composition of claim 14 in the manufacture of a medicament for preventing, treating, or alleviating a disease associated with KRAS wild-type, or KRAS G12A, KRAS G12C, KRAS G12D, KRAS G12R, KRAS G12S, KRAS G12V, KRAS G13D, or KRAS Q61H mutation.

**16.** The use of claim 15, wherein, the disease associated with KRAS wild-type, or KRAS G12A, KRAS G12C, KRAS G12D, KRAS G12R, KRAS G12S, KRAS G12V, KRAS G13D or KRAS Q61H mutation according to the present invention is cancer.

**17.** The use of claim 16, wherein, the cancer is cardiac cancer: sarcoma, myxoma, rhabdomyoma, fibroma, lipoma or teratoma; lung cancer: bronchial carcinoma, non-small cell lung cancer, small cell lung cancer, alveolar carcinoma, bronchial adenoma, sarcoma, lymphoma, chondromatous hamartoma or mesothelioma; gastrointestinal cancer: esophageal cancer, gastric cancer, pancreatic cancer, small intestinal cancer or large intestinal cancer; urogenital tract cancer: kidney cancer, bladder and urethral cancer, prostate cancer or testicular cancer; liver cancer: hepatocellular carcinoma, hepatoblastoma, angiosarcoma, hepatocellular adenoma or hemangioma; biliary tract cancer: gallbladder cancer, ampullary cancer or cholangiocarcinoma; bone cancer: osteosarcoma, fibrosarcoma, malignant fibrous histiocytoma, chondrosarcoma, Ewing sarcoma, malignant lymphoma, multiple myeloma, malignant giant cell tumor, chordoma, enchondroma, chondroblastoma, chondromyxoid fibroma, osteoid osteoma or giant cell tumor; nervous system cancer: skull tumor or brain cancer; gynecological cancer: uterine cancer, vulvar cancer, vaginal cancer, fallopian tube carcinoma, ovarian cancer or breast cancer; hematologic cancer: acute or chronic myeloid leukemia, acute lymphoblastic leukemia, chronic lymphocytic leukemia, Hodgkin's disease or non-Hodgkin lymphoma; skin cancer: melanoma, basal cell carcinoma, squamous cell carcinoma, Kaposi sarcoma, dysplastic nevus, lipoma, hemangioma, dermatofibroma, keloid or psoriasis; adrenal cancer: neuroblastoma.

**INTERNATIONAL SEARCH REPORT**

International application No. **PCT/CN2024/087520**

**A. CLASSIFICATION OF SUBJECT MATTER**

C07D471/04(2006.01)i； C07D519/00(2006.01)i； A61P35/00(2006.01)i； A61P35/02(2006.01)i； A61K31/519(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

万方, WANFANG, CNKI, CNTXT, VEN, STNext-REGISTRY, CAPLUS, MARPAT: 广东东阳光, KRAS抑制剂, 癌, 肿瘤, 嘧啶并吡啶, MRTX1133, pyrimidopyridine, cancer, tumor, 大鼠肉瘤病毒癌基因, rat sarcoma viral oncogene homolog, STN-structure search

**C. DOCUMENTS CONSIDERED TO BE RELEVANT**

| Category* | Citation of document, with indication, where appropriate, of the relevant passages | Relevant to claim No. |
|---|---|---|
| X | WO 2023020523 A1 (JACOBIO PHARMACEUTICALS CO., LTD.) 23 February 2023 (2023-02-23)<br>see claims 1, 2, 21, and 30-38, and description, pages 79 and 81 | 1-17 |
| X | WO 2023020519 A1 (JACOBIO PHARMACEUTICALS CO., LTD.) 23 February 2023 (2023-02-23)<br>see claims 1, 22, and 71-79 | 1-17 |
| E | CN 118047801 A (GUANGDONG HEC PHARMACEUTICAL CO., LTD.) 17 May 2024 (2024-05-17)<br>see claims 13 and 14 | 1-17 |
| PX | CN 115974896 A (GUANGDONG HEC PHARMACEUTICAL CO., LTD.) 18 April 2023 (2023-04-18)<br>see claims 1 and 9 | 1-17 |
| PX | WO 2024061333 A1 (GAN & LEE PHARM CO., LTD.) 28 March 2024 (2024-03-28)<br>see claims 1, 7, and 14 | 1-17 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **22 July 2024** | **23 July 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/087520**

**C. DOCUMENTS CONSIDERED TO BE RELEVANT**

| Category* | Citation of document, with indication, where appropriate, of the relevant passages | Relevant to claim No. |
|---|---|---|
| PX | WO 2024008068 A1 (JACOBIO PHARMACEUTICALS CO., LTD.) 11 January 2024 (2024-01-11) see claims 1 and 66 | 1-17 |
| PX | CN 117624170 A (TYLIGAND BIOSCIENCE (SHANGHAI) LIMITED) 01 March 2024 (2024-03-01) see claims 1, 9, and 14 | 1-17 |
| A | WO 2022042630 A1 (INVENTISBIO CO., LTD. et al.) 03 March 2022 (2022-03-03) see entire document | 1-17 |
| A | WO 2022199586 A1 (SUZHOU ZELGEN BIOPHARMACEUTICALS CO., LTD. et al.) 29 September 2022 (2022-09-29) see entire document | 1-17 |
| A | WO 2022194191 A1 (GUANGDONG NEWOPP BIOPHARMACEUTICALS CO., LTD.) 22 September 2022 (2022-09-22) see entire document | 1-17 |

Form PCT/ISA/210 (second sheet) (July 2022)

**INTERNATIONAL SEARCH REPORT**
**Information on patent family members**

International application No.
**PCT/CN2024/087520**

| Patent document cited in search report | Publication date (day/month/year) | Patent family member(s) | Publication date (day/month/year) |
|---|---|---|---|
| WO 2023020523 A1 | 23 February 2023 | None | |
| WO 2023020519 A1 | 23 February 2023 | EP 4387967 A1 | 26 June 2024 |
| | | TW 202328124 A | 16 July 2023 |
| | | WO 2023020519 A9 | 23 March 2023 |
| CN 118047801 A | 17 May 2024 | WO 2024104425 A1 | 23 May 2024 |
| CN 115974896 A | 18 April 2023 | WO 2023061463 A1 | 20 April 2023 |
| WO 2024061333 A1 | 28 March 2024 | None | |
| WO 2024008068 A1 | 11 January 2024 | None | |
| CN 117624170 A | 01 March 2024 | WO 2024041606 A1 | 29 February 2024 |
| WO 2022042630 A1 | 03 March 2022 | EP 4204412 A1 | 05 July 2023 |
| | | JP 2023540228 A | 22 September 2023 |
| | | US 2023357233 A1 | 09 November 2023 |
| WO 2022199586 A1 | 29 September 2022 | None | |
| WO 2022194191 A1 | 22 September 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021041671 A
- WO 2022132200 A


### Non-patent literature cited in the description


- *Nature*, 2013, vol. 503, 548-551
- McGraw-Hill Dictionary of Chemical Terms. McGraw-Hill Book Company, 1984
- **ELIEL, E.** ; **WILEN, S.** Stereochemistry of Organic Compounds. John Wiley & Sons, Inc., 1994
- **S. M. BERGE et al.** *J. Pharmaceutical Sciences*, 1977, vol. 66, 1-19
- Remington: The Science and Practice of Pharmacy. Lippincott Williams& Wilkins, 2005
- Encyclopedia of Pharmaceutical Technology. Marcel Dekker, 1988